# EUROPEAN PATENT SPECIFICATION

(11) **EP 2 066 648 B1**
(45) Date of publication and mention of the grant of the patent: **12.04.2017**
(21) Application number: 07788185.2
(22) Date of filing: 03.08.2007
(51) Int. Cl.: C07D 295/08, C07D 295/12, C07D 295/14, C07D 413/08, A61K 31/5377, A61K 31/4453, A61K 31/445, A61K 31/4523, A61K 31/495, A61P 25/00, C07C 69/74

(54) **SUBSTITUTED DIMETHYLCYCLOBUTYL COMPOUNDS, THEIR PREPARATION AND USE IN MEDICAMENTS**
SUBSTITUIERTE DIMETHYLCYCLOBUTYLVERBINDUNGEN, IHRE HERSTELLUNG UND VERWENDUNG IN MEDIKAMENTEN
COMPOSÉS DIMÉTHYLCYCLOBUTYLE SUBSTITUÉS, LEUR PRÉPARATION ET UTILISATION DANS DES MÉDICAMENTS

(30) Priority: 04.08.2006 EP 06384013
(43) Date of publication of application: 10.06.2009
(73) Proprietor: Laboratorios del Dr. Esteve, S.A., 08041 Barcelona (ES)
(72) Inventor: CUBERES ALTISEN, Maria, Rosa, 08190 San Cugat Del Valles (ES); CORBERA ARJONA, Jordi, 08225 Terrasa (ES); HOLENZ, Jorg, 15023 Enhörna (SE); ORTUÑO MINGARRO, Rosa, María, 08031 Barcelona (ES); IZQUIERDO SALADO, Sandra, 08206 Sabadell (ES)
(74) Representative: Carpintero Lopez, Francisco
(86) International application number: PCT/EP2007/058047
(87) International publication number: WO 2008/015266

(56) References cited:
- US-A- 3 387 021
- US-A- 3 453 327
- AVOTINS ET AL.: LATVIJAS PSR ZINATNU AKADEMIJAS VESTIS, KIMIJAS SERIJA, vol. 4, 1983, pages 465-469, XP001248169 RUSSIA
- SCHENONE, P. ET AL.: "Aminoalcoli derivati dal ciclobutano" IL FARMACO, vol. 25, no. 7, 1970, pages 533-541, XP009075142 Italy
- SCHENONE ET AL: BOLL. CHIM. FARM., vol. 110, 1971, pages 380-384, XP009075215 Italy
- HERGUETA ET AL.: CHEM. PHARM. BULL., vol. 49, no. 9, 2001, pages 1174-1177, XP001247962
- FERANDEZ ET AL: NUCLEOSIDES, NUCLEOTIDES AND NUCLEIC ACIDS, vol. 20, no. 4-7, 2001, pages 1129-1131, XP009075127
- BLANCO ET AL.: CHEM. PHARM. BULL., vol. 47, no. 9, 1999, pages 1314-1317, XP001248179
- KULA ET AL.: POLISH JOURNAL OF CHEMISTRY, vol. 68, 1994, pages 1699-1706, XP001247964 Poland
- COLLET ET AL: TETRAHEDRON, vol. 31, 1975, pages 2243-2246, XP002408409
- INOKUCHI ET AL.: J. ORG. CHEM., vol. 56, no. 7, 1991, pages 2416-2421, XP002408410
- LIU ET AL.: TETRAHEDRON LETTERS, vol. 45, 2004, pages 6097-6100, XP002408411
- MOGLIONI ET AL.: J. ORG. CHEM., vol. 65, 2000, pages 3934-3940, XP002408413
- LEWIS ET AL: JOURNAL OF CHEMICAL AND ENGINEERING DATA, vol. 14, no. 3, 1969, pages 401-402, XP002409743
- LIU ET AL.: OPPI BRIEFS, vol. 29, no. 4, 1997, pages 473-476, XP001248167
- HARISPE M ET AL: COMPTES RENDUS DES SEANCES DE L'ACADEMIE DES SCIENCES. SERIE I: MATHEMATIQUES, EDITIONS SCIENTIFIQUES & MEDICALES ELSEVIER, FR, 1957, pages 1550-1552, XP009075140 ISSN: 0764-4442
- DATABASE CAPLUS [Online] CHEMICAL ABSTRACTS SERVICE, COLUMBUS, OHIO, US; GUHA, P. C. ET AL: "Synthetic experiments in the pinane group. III. Synthesis and configuration of pinic acid" XP002409787 retrieved from STN Database accession no. 1937:47839 & BERICHTE DER DEUTSCHEN CHEMISCHEN GESELLSCHAFT [ABTEILUNG] B: ABHANDLUNGEN , 70B, 1505-12 CODEN: BDCBAD; ISSN: 0365-9488, 1937,
- DATABASE CAPLUS [Online] CHEMICAL ABSTRACTS SERVICE, COLUMBUS, OHIO, US; MINACHEV, KH. ET AL.: "Effect of thiophene on the properties of alumina-platinum catalyst in the conditions of methylcyclopentane dehydroisomerization" XP002409788 retrieved from STN Database accession no. 1965:462522
- DATABASE CAPLUS [Online] CHEMICAL ABSTRACTS SERVICE, COLUMBUS, OHIO, US; PARK, JOSEPH D. ET AL: "Hydroxyalkyl and olefinic substituted gem-dimethylcyclobutanes" XP002409789 retrieved from STN Database accession no. 1965:462521 & INDUSTRIAL & ENGINEERING CHEMISTRY PRODUCT RESEARCH AND DEVELOPMENT , 4(3), 149-53 CODEN: IEPRA6; ISSN: 0196-4321, 1965,
- DATABASE CAPLUS [Online] CHEMICAL ABSTRACTS SERVICE, COLUMBUS, OHIO, US; MOGLIONI, ALBERTINA G. ET AL: "Stereoselective synthesis of novel cyclobutane dehydro amino acids from (+)-.alpha.-pinene" XP002409790 retrieved from STN Database accession no. 1998:330842 & TETRAHEDRON LETTERS , 39(21), 3593-3596 CODEN: TELEAY; ISSN: 0040-4039, 1998,
- DATABASE CAPLUS [Online] CHEMICAL ABSTRACTS SERVICE, COLUMBUS, OHIO, US; OSTLING, G. J.: "Preparation of glycols corresponding with pinic, norpinic, and d-camphoric acids and their derivatives" XP002409791 retrieved from STN Database accession no. 1922:13382 & OVERSIKI FINSKA VETENSKAPS SOC. , 57 [A](NO. 7), 19 PP., 1914,
- DATABASE CAPLUS [Online] CHEMICAL ABSTRACTS SERVICE, COLUMBUS, OHIO, US; THOMAS, ALAN F. ET AL: "The Baeyer-Villiger reaction of pinanones (bicyclo[3.1.1]heptanones)" XP002409792 retrieved from STN Database accession no. 1992:235889 & TETRAHEDRON , 48(10), 1927-42 CODEN: TETRAB; ISSN: 0040-4020, 1992,
- DATABASE CAPLUS [Online] CHEMICAL ABSTRACTS SERVICE, COLUMBUS, OHIO, US; JAOUI, MOHAMMED ET AL: "Mass balance of gaseous and particulate products analysis from .alpha.-pinene/NOx/air in the presence of natural sunlight" XP002409793 retrieved from STN Database accession no. 2001:533378 & JOURNAL OF GEOPHYSICAL RESEARCH, [ATMOSPHERES] , 106(D12), 12541-12558 CODEN: JGRDE3; ISSN: 0148-0227, 2001,
- FIGUEIRA ET AL.: SYNTHESIS, no. 10, 2000, pages 1459-1463, XP002408414
- DATABASE HCAPLUS [Online] CHEMICAL ABSTRACTS SERVICE, COLUMBUS, OHIO, US; HERGUETA, ANTONIO R. ET AL: "Synthesis of two enantiomerically pure precursors of cyclobutane carbocyclic nucleosides" XP002409794 retrieved from STN Database accession no. 2003:928830 & TETRAHEDRON: ASYMMETRY , 14(23), 3773-3778 CODEN: TASYE3; ISSN: 0957-4166, 2003,
- DATABASE CAPLUS [Online] CHEMICAL ABSTRACTS SERVICE, COLUMBUS, OHIO, US; FRANCOIS, HUGUETTE ET AL: "(.+-.)-cis-Pinic acid derivatives. I. Pinic amino acids" XP002409795 retrieved from STN Database accession no. 1967:115820 & BULLETIN DE LA SOCIETE CHIMIQUE DE FRANCE , (2), 535-9 CODEN: BSCFAS; ISSN: 0037-8968, 1967,
- DATABASE CAPLUS [Online] CHEMICAL ABSTRACTS SERVICE, COLUMBUS, OHIO, US; FRANCOIS, HUGUETTE ET AL: "(.+-.)-cis-Pinic acid derivatives. III. Acidolysis of urea by (.+-.)-cis-pinic acid. Pinic diamine" XP002409796 retrieved from STN Database accession no. 1967:115822 & BULLETIN DE LA SOCIETE CHIMIQUE DE FRANCE , (2), 542-4 CODEN: BSCFAS; ISSN: 0037-8968, 1967,

## Description

The present invention relates to substituted dimethylcyclobutyl compounds of general formula I, 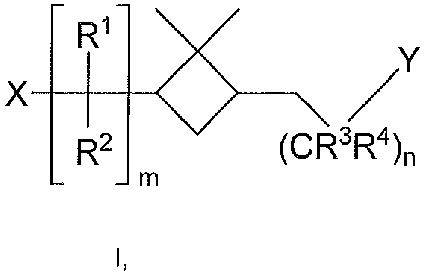 a process for their preparation, medicaments comprising said substituted dimethylcyclobutyl compounds as well as the use of said substituted dimethylcyclobutyl compounds for the preparation of medicaments, which are particularly suitable for the prophylaxis and/or treatment of disorders or diseases that are at least partially mediated via sigma receptors.

The search for new therapeutic agents has been greatly aided in recent years by better understanding of the structure of proteins and other biomolecules associated with target diseases. One important class of these proteins is the sigma receptor, a cell surface receptor of the central nervous system (CNS) which may be related to the dysphoric, hallucinogenic and cardiac stimulant effects of opioids.

From studies of the biology and function of sigma receptors, evidence has been presented that sigma receptor ligands may be useful in the treatment of psychosis and movement disorders such as dystonia and tardive dyskinesia, and motor disturbances associated with Huntington's chorea or Tourette's syndrome and in Parkinson's disease (Walker, J.M. et al, Pharmacological Reviews, 1990, 42, 355). It has been reported that the known sigma receptor ligand rimcazole clinically shows effects in the treatment of psychosis (Snyder, S.H., Largent, B.L. J. Neuropsychiatry 1989, 1, 7). The sigma binding sites have preferential affinity for the dextrorotatory isomers of certain opiate benzomorphans, such as (+)SKF 10047, (+)cyclazocine, and (+)pentazocine and also for some narcoleptics such as haloperidol.

The sigma receptor has at least two subtypes, which may be discriminated by stereoselective isomers of these pharmacoactive drugs. Possible sigma-site-mediated drug effects include modulation of glutamate receptor function, neurotransmitter response, neuroprotection, behaviour, and cognition (Quirion, R. et al. Trends Pharmacol. Sci., 1992, 13:85-86). Most studies have implied that sigma binding sites (receptors) are plasmalemmal elements of the signal transduction cascade. Drugs reported to be selective sigma ligands have been evaluated as antipsychotics (Hanner, M. et al. Proc. Natl. Acad. Sci., 1996, 93:8072-8077). The existence of sigma receptors in the CNS, immune and endocrine systems have suggested a likelihood that it may serve as link between the three systems.

Thus, there is a need to find compounds that have pharmacological activity towards the sigma receptor, being both effective and selective, and having good "drugability" properties, i.e. good pharmaceutical properties related to administration, distribution, metabolism and excretion.

Therefore an object of the present invention was to provide compounds that are particularly suitable as active ingredients in medicaments, especially in medicaments for the prophylaxis and/or treatment of disorders or diseases related to sigma receptors, preferably sigma-1 receptors, such as food intake related disorders or pain.

Surprisingly, it has been found that the substituted dimethylcyclobutyl compounds of general formula I given below show good to excellent affinity for sigma receptors, in particular they show good to excellent affinity to sigma-1 receptors. These compounds are therefore particularly suitable as pharmacologically active agents in a medicament for the prophylaxis and/or treatment of disorders or diseases related to sigma receptors, preferably related to sigma-1 receptors.

Thus, in one of its aspects the present invention relates to a dimethylcyclobutyl compound of general formula I, 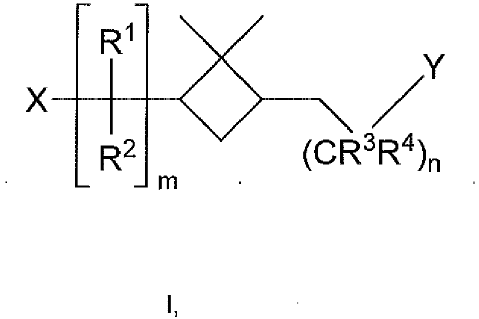 wherein
m is 1 or 2;
n is 0 or 1;
X and Y are different
X represents a -OR⁵ moiety or a -NR⁶R⁷ moiety; or a (hetero)cycloaliphatic radical selected from the group consisting of pyrrolidinyl, piperidinyl, morpholinyl, thiomorpholinyl, piperazinyl, pyrazolidinyl, indolinyl, isoindolinyl, (1,2,3,4)-tetrahydroquinolinyl and (1,2,3,4)-tetrahydroisoquinolinyl, which may be unsubstituted or optionally substituted with 1, 2, 3, 4 or 5 substituent(s) independently selected from the group consisting of oxo (=0), thioxo (=S), methyl, ethyl, n-propyl, isopropyl, n-butyl, tert-butyl, sec-butyl, isobutyl, n-pentyl, -O-CH₃, -O-C₂H₅, F, Cl, Br, I, -CN, -CF₃, -OH, -SH and -NH₂;
Y represents a -OR⁸ moiety; a -NR⁹R¹⁰ moiety; a -C(=O)-OR¹¹ moiety; or a (hetero)cycloaliphatic radical selected from the group consisting of pyrrolidinyl, piperidinyl, morpholinyl, thiomorpholinyl, piperazinyl, pyrazolidinyl, indolinyl, isoindolinyl, (1,2,3,4)-tetrahydroquinolinyl, and (1,2,3,4)-tetrahydroisoquinolinyl, which may be unsubstituted or optionally substituted with 1, 2, 3, 4 or 5 substituent(s) independently selected from the group consisting of methyl, ethyl, n-propyl, isopropyl, n-butyl, tert-butyl, sec-butyl, isobutyl, n-pentyl, -O-CH₃, -O-C₂H₅, F, Cl, Br, I, -CN, -CF₃, -OH, -SH and -NH₂;
R¹ represents a hydrogen atom or a radical selected from the group consisting of methyl, ethyl, n-propyl, isopropyl, n-butyl, sec-butyl, isobutyl, tert-butyl and n-pentyl;
R² represents a hydrogen atom; a radical selected from the group consisting of methyl, ethyl, n-propyl, isopropyl, n-butyl, sec-butyl, isobutyl, tert-butyl and n-pentyl; or an aryl radical selected from the group consisting of phenyl and naphthyl, which may be unsubstituted or optionally substituted with 1, 2, 3, 4 or 5 substituent(s) independently selected from the group consisting of methyl, ethyl, n-propyl, isopropyl, n-butyl, tert-butyl, sec-butyl, isobutyl, n-pentyl, -O-CH₃, -O-C₂H₅ -S-CH₃, -S-C₂H₅, -C(=O)-OH, -C(=O)-O-CH₃, -C(=O)-O-C₂H₅, -C(=O)-CH₃, -C(=O)-C₂H₅, F, Cl, Br, I, -CN, -CF₃,-OCF₃, -SCF₃, -OH, -SH, -NH₂, -NO₂, -CHO, -CF₂H and -CFH₂;
R³ and R⁴, both represent a hydrogen atom
R⁵ and R⁸, independently of one another, each represent a hydrogen atom; a radical selected from the group consisting of methyl, ethyl, n-propyl, isopropyl, n-butyl, sec-butyl, isobutyl, tert-butyl and n-pentyl; an aryl radical selected from the group consisting of phenyl and naphthyl, which may be bonded via a -(CH₂)_{1,2 or 3}-group and which may be unsubstituted or optionally substituted with 1, 2, 3, 4 or 5 substituent(s) independently selected from the group consisting of methyl, ethyl, n-propyl, isopropyl, n-butyl, tert-butyl, sec-butyl, isobutyl, n-pentyl, -O-CH₃, -O-C₂H₅, -S-CH₃, -S-C₂H₅, -C(=O)-OH, -C(=O)-O-CH₃, -C(=O)-O-C₂H₅, -C(=O)-CH₃, -C(=O)-C₂H₅, F, Cl, Br, I, -CN, -CF₃, -OCF₃, -SCF₃, -OH, -SH, -NH₂, -NO₂, -CHO, -CF₂H,-CFH₂ and -S(=O)₂-CH₃; or a -C(=O)-R¹² moiety;with the proviso that
if R⁵ represents hydrogen and m is 1,
R¹ represents a radical selected from the group consisting of methyl, ethyl, n-propyl, isopropyl, n-butyl, sec-butyl, isobutyl, tert-butyl and n-pentyl; and
R² represents a radical selected from the group consisting of methyl, ethyl, n-propyl, isopropyl, n-butyl, sec-butyl, isobutyl, tert-butyl and n-pentyl; or an aryl radical selected from the group consisting of phenyl and naphthyl, which may be unsubstituted or optionally substituted with 1, 2, 3, 4 or 5 substituent(s) independently selected from the group consisting of methyl, ethyl, n-propyl, isopropyl, n-butyl, tert-butyl, sec-butyl, isobutyl, n-pentyl, -O-CH₃, -O-C₂H₅-S-CH₃, -S-C₂H₅, -C(=O)-OH, -C(=O)-O-CH₃, -C(=O)-O-C₂H₅,-C(=O)-CH₃, -C(=O)-C₂H₅, F, Cl, Br, I, -CN, -CF₃, -OCF₃, -SCF₃, -OH, -SH,-NH₂, -NO₂, -CHO, -CF₂H and -CFH₂
R⁶, R⁷, R⁹ and R¹⁰, independently of one another, each represent a a radical selected from the group consisting of methyl, ethyl, n-propyl, isopropyl, n-butyl, sec-butyl, isobutyl, tert-butyl and n-pentyl; or an aryl radical selected from the group consisting of phenyl and naphthyl, which may be bonded via a -(CH₂)_{1, 2 or 3}-group and which may be unsubstituted or optionally substituted with 1, 2, 3, 4 or 5 substituent(s) independently selected from the group consisting of methyl, ethyl, n-propyl, isopropyl, n-butyl, tert-butyl, sec-butyl, isobutyl, -O-CH₃, -O-C₂H₅, -S-CH₃, -S-C₂H₅,-C(=O)-OH, -C(=O)-O-CH₃, -C(=O)-O-C₂H₅, -C(=O)-CH₃, -C(=O)-C₂H₅, F, Cl, Br, I, -CN, -CF₃, -OCF₃, -SCF₃, -OH, -SH, -NH₂-NO₂, -CHO, -CF₂H, -CFH₂,-C(=O)-NH₂ and -S(=O)₂-CH₃;
or R⁶ and R⁷, together with the bridging nitrogen atom form a moiety selected from the group consisting of 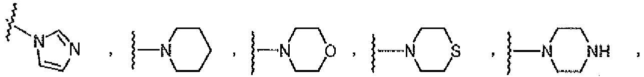 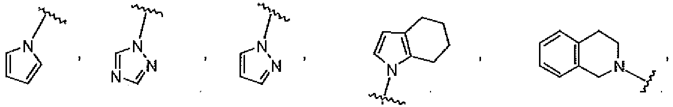 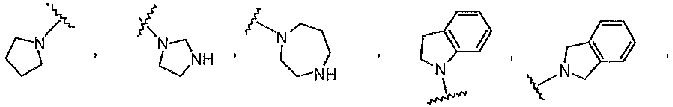 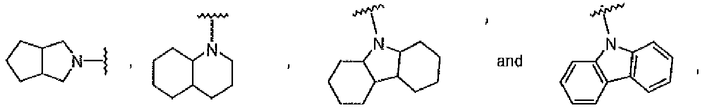 which may be unsubstituted or optionally substituted in any position including the -NH-groups with 1, 2, 3, 4 or 5 substituent(s) independently selected from the group consisting of oxo (=O), methyl, ethyl, n-propyl, isopropyl, n-butyl, tert-butyl, sec-butyl, -O-CH₃, -O-C₂H₅, -S-CH₃, -S-C₂H₅,-C(=O)-OH, -C(=O)-O-CH₃, -C(=O)-O-C₂H₅, -C(=O)-O-CH₂-CH₂-CH₃, -C(=O)-O-CH(CH₃)₂, -C(=O)-O-C(CH₃)₃, -C(=O)-CH₃, -C(=O)-C₂H₅, -C(=O)-CH₂-CH₂-CH₃, -C(=O)-CH(CH₃)₂, -C(=O)-C(CH₃)₃, F, Cl, Br, I, -CN, -CF₃, -OCF₃,-SCF₃, -OH, -SH, -NH₂, -NO₂, -CHO, -CF₂H, -CFH₂, -C(=O)-NH₂, -C(=O)-NH-CH₃, -C(=O)-NH-C₂H₅, -C(=O)-N(CH₃)₂, -C(=O)-N(C₂H₅)₂, -S(=O)₂-CH₃, phenyl, phenethyl and benzyl, whereby said cyclic substituents may be unsubstituted or substituted by 1, 2 or 3 substituent(s) independently selected from the group consisting of methyl, ethyl, n-propyl, isopropyl, methoxy, ethoxy, F, Cl, Br, -CN, -CF₃, -OCF₃, -SCF₃, -OH, -SH, -NH₂ and-NO₂;
or R⁹ and R¹⁰, together with the bridging nitrogen atom form a moiety selected from the group consisting of; 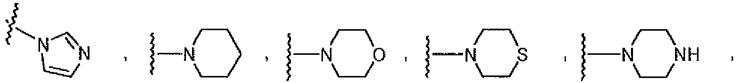 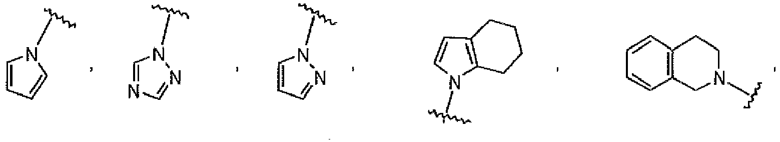 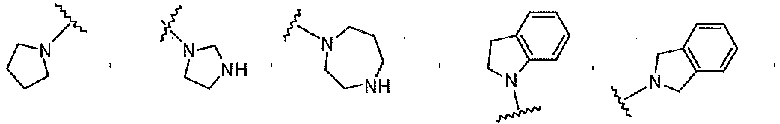 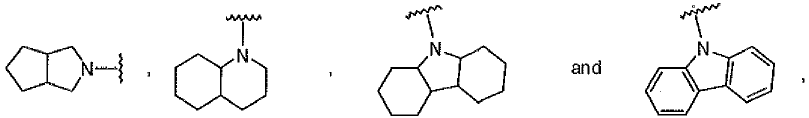 which may be unsubstituted or optionally substituted in any position including the -NH-groups with 1, 2, 3, 4 or 5 substituent(s) independently selected from the group consisting of oxo (=O), methyl, ethyl, n-propyl, isopropyl, n-butyl, tert-butyl, sec-butyl, -O-CH₃, -O-C₂H₅, -S-CH₃, -S-C₂H₅, -C(=O)-OH, -C(=O)-O-CH₃, -C(=O)-O-C₂H₅, -C(=O)-O-CH₂-CH₂-CH₃, -C(=O)-O-CH(CH₃)₂,-C(=O)-O-C(CH₃)₃, -C(=O)-CH₃, -C(=O)-C₂H₅, -C(=O)-CH₂-CH₂-CH₃, -C(=O)-CH(CH₃)₂, -C(=O)-C(CH₃)₃, F, Cl, Br, I, -CN, -CF₃, -OCF₃, -SCF₃, -OH, -SH,-NH₂, -NO₂, -CHO, -CF₂H, -CFH₂, -C(=O)-NH₂, -C(=O)-NH-CH₃, -C(=O)-NH-C₂H₅, -C(=O)-N(CH₃)₂, -C(=O)-N(C₂H₅)₂, -S(=O)₂-CH₃, phenyl, phenethyl and benzyl, whereby said cyclic substituents may be unsubstituted or substituted by 1, 2 or 3 substituent(s) independently selected from the group consisting of methyl, ethyl, n-propyl, isopropyl, methoxy, ethoxy, F, Cl, Br, -CN, -CF₃,-OCF₃, -SCF₃, -OH, -SH, -NH₂ and -NO₂;
R¹¹ represents a radical selected from the group consisting of methyl, ethyl, n-propyl, isopropyl, n-butyl, sec-butyl, isobutyl, tert-butyl and n-pentyl; and
R¹² represents a hydrogen atom; a radical selected from the group consisting of methyl, ethyl, n-propyl, isopropyl, n-butyl, sec-butyl, isobutyl, tert-butyl and n-pentyl;
optionally in form of one of its stereoisomers, preferably enantiomers or diasteromers, a racemate or in form of a mixture of at least two of its stereoisomers, preferably enantiomers and/or diastereomers, in any mixing ratio, or a salt thereof, or a corresponding solvate thereof.

Preferred is a compound of general formula I, wherein
X and Y are different; wherein
if R⁵ represents hydrogen and m is 1,
R¹ represents a radical selected from the group consisting of methyl, ethyl, n-propyl, isopropyl, n-butyl, sec-butyl, isobutyl, tert-butyl and n-pentyl;
R² represents a radical selected from the group consisting of methyl, ethyl, n-propyl, isopropyl, n-butyl, sec-butyl, isobutyl, tert-butyl and n-pentyl ; or an aryl radical, selected from the group consisting of phenyl and naphthyl, which may be unsubstituted or optionally substituted with 1, 2, 3, 4 or 5 substituent(s) independently selected from the group consisting of methyl, ethyl, n-propyl, isopropyl, n-butyl, tert-butyl, sec-butyl, isobutyl, n-pentyl, -O-CH₃, -O-C₂H₅-S-CH₃, -S-C₂H₅, -C(=O)-OH, -C(=O)-O-CH₃,-C(=O)-O-C₂H₅, -C(=O)-CH₃, -C(=O)-C₂H₅, F, Cl, Br, I, -CN, -CF₃, -OCF₃,-SCF₃, -OH, -SH, -NH₂, -NO₂, -CHO, -CF₂H and -CFH₂;
Wherein
R⁶, R⁷, R⁹ and R¹⁰, independently of one another, each represent a radical selected from the group consisting of methyl, ethyl, n-propyl, isopropyl, n-butyl, sec-butyl, isobutyl, tert-butyl and n-pentyl;
or an aryl radical selected from the group consisting of phenyl and naphthyl, which may be bonded via a -(CH₂)_{1, 2 or 3}-group and which may be unsubstituted or optionally substituted with 1, 2, 3, 4 or 5 substituent(s) independently selected from the group consisting of methyl, ethyl, n-propyl, isopropyl, n-butyl, tert-butyl, sec-butyl, isobutyl, -O-CH₃, -O-C₂H₅, -S-CH₃, -S-C₂H₅, -C(=O)-OH, -C(=O)-O-CH₃, -C(=O)-O-C₂H₅, -C(=O)-CH₃, -C(=O)-C₂H₅, F, Cl, Br, I, -CN, -CF₃, -OCF₃, -SCF₃, -OH, -SH, -NH₂-NO₂, -CHO, -CF₂H,-CFH₂, -C(=O)-NH₂ and -S(=O)₂-CH₃
or R⁶ and R⁷, together with the bridging nitrogen atom form a moiety selected from the group consisting of 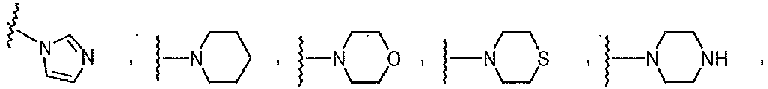 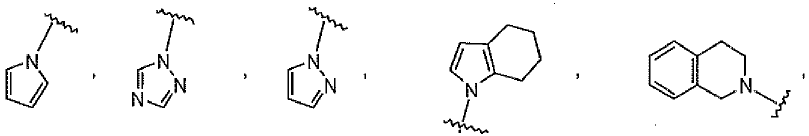 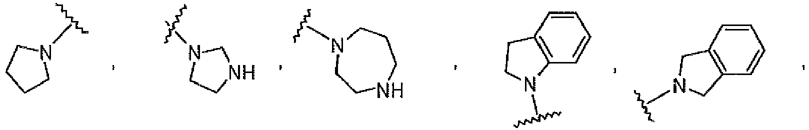 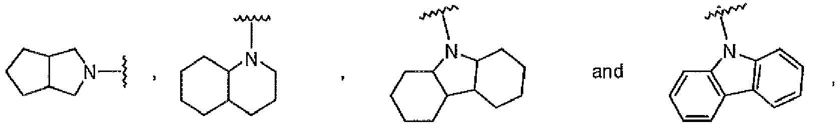 which may be unsubstituted or optionally substituted in any position including the -NH-groups with 1, 2, 3, 4 or 5 substituent(s) independently selected from the group consisting of oxo (=O), methyl, ethyl, n-propyl, isopropyl, n-butyl, tert-butyl, sec-butyl, -O-CH₃, -O-C₂H₅, -S-CH₃, -S-C₂H₅, -C(=O)-OH, -C(=O)-O-CH₃, -C(=O)-O-C₂H₅, -C(=O)-O-CH₂-CH₂-CH₃, -C(=O)-O-CH(CH₃)₂,-C(=O)-O-C(CH₃)₃, -C(=O)-CH₃, -C(=O)-C₂H₅, -C(=O)-CH₂-CH₂-CH₃, -C(=O)-CH(CH₃)₂, -C(=O)-C(CH₃)₃, F, Cl, Br, I, -CN, -CF₃, -OCF₃, -SCF₃, -OH, -SH,-NH₂, -NO₂, -CHO, -CF₂H, -CFH₂, -C(=O)-NH₂, -C(=O)-NH-CH₃, -C(=O)-NH-C₂H₅, -C(=O)-N(CH₃)₂, -C(=O)-N(C₂H₅)₂, -S(=O)₂-CH₃, phenyl, phenethyl and benzyl, whereby said cyclic substituents may be unsubstituted or substituted by 1, 2 or 3 substituent(s) independently selected from the group consisting of methyl, ethyl, n-propyl, isopropyl, methoxy, ethoxy, F, Cl, Br, -CN, -CF₃,-OCF₃, -SCF₃, -OH, -SH, -NH₂ and -NO₂;
or R⁹ and R¹⁰, together with the bridging nitrogen atom form a moiety selected from the group consisting of; 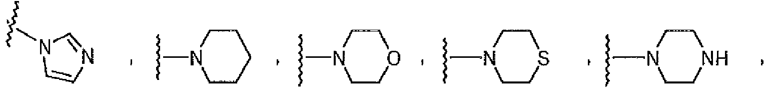 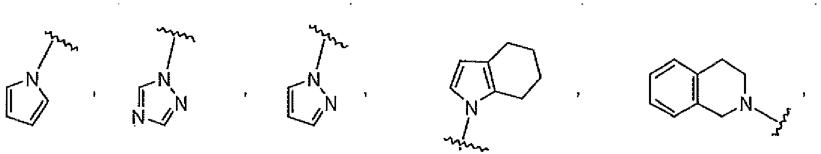 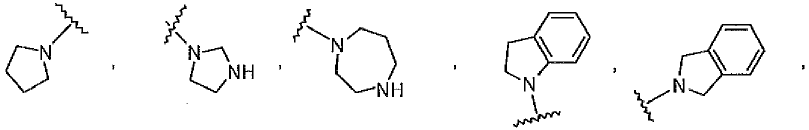 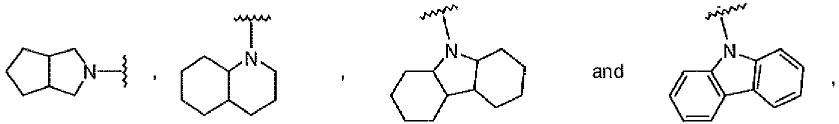 which may be unsubstituted or optionally substituted in any position including the -NH-groups with 1, 2, 3, 4 or 5 substituent(s) independently selected from the group consisting of oxo (=O), methyl, ethyl, n-propyl, isopropyl, n-butyl, tert-butyl, sec-butyl, -O-CH₃, -O-C₂H₅, -S-CH₃, -S-C₂H₅, -C(=O)-OH, -C(=O)-O-CH₃, -C(=O)-O-C₂H₅, -C(=O)-O-CH₂-CH₂-CH₃, -C(=O)-O-CH(CH₃)₂,-C(=O)-O-C(CH₃)₃, -C(=O)-CH₃, -C(=O)-C₂H₅, -C(=O)-CH₂-CH₂-CH₃, -C(=O)-CH(CH₃)₂, -C(=O)-C(CH₃)₃, F, Cl, Br, I, -CN, -CF₃, -OCF₃, -SCF₃, -OH, -SH,-NH₂, -NO₂, -CHO, -CF₂H, -CFH₂, -C(=O)-NH₂, -C(=O)-NH-CH₃, -C(=O)-NH-C₂H₅, -C(=O)-N(CH₃)₂, -C(=O)-N(C₂H₅)₂, -S(=O)₂-CH₃, phenyl, phenethyl and benzyl, whereby said cyclic substituents may be unsubstituted or substituted by 1, 2 or 3 substituent(s) independently selected from the group consisting of methyl, ethyl, n-propyl, isopropyl, methoxy, ethoxy, F, Cl, Br, -CN, -CF₃,-OCF₃, -SCF₃, -OH, -SH, -NH₂ and -NO₂;
R¹¹ represents a radical selected from the group consisting of methyl, ethyl, n-propyl, isopropyl, n-butyl, sec-butyl, isobutyl, tert-butyl and n-pentyl; and
R¹² represents a hydrogen atom or a radical selected from the group consisting of methyl, ethyl, n-propyl, isopropyl, n-butyl, sec-butyl, isobutyl, tert-butyl and n-pentyl;
Suitable alkylene groups include -(CH₂)-, -CH(CH₃)-, -CH(phenyl), -(CH₂)₂-,-(CH₂)₃-,-(CH₂)₄-, -(CH₂)₅ and -(CH₂)₆-, suitable alkenylene groups include-CH=CH-, -CH₂-CH=CH- and -CH=CH-CH₂- and suitable alkinylene groups include -C≡C-, -CH₂-C≡C- and -C≡C-CH₂-.

The term "condensed" according to the present invention means that a ring or ring system is attached to another ring or ring system, whereby the terms "annulated" or "annelated" are also used by those skilled in the art to designate this kind of attachment.

An alkenyl radical comprises at least one carbon-carbon double bond, an alkinyl radical comprises at least one carbon-carbon triple bond.

Preferred is a substituted dimethylcyclobutyl compound of general formula I, wherein the stereoisomers have the general formulae la or Ib or Ic or Id, 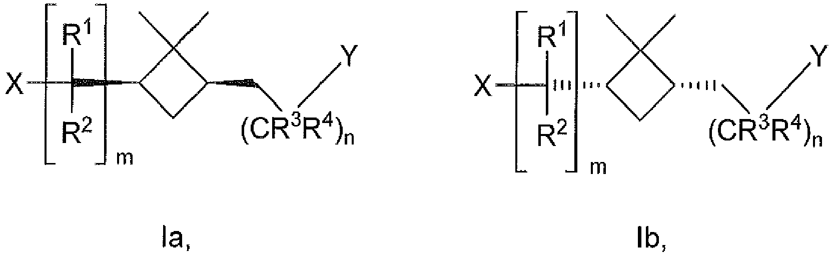 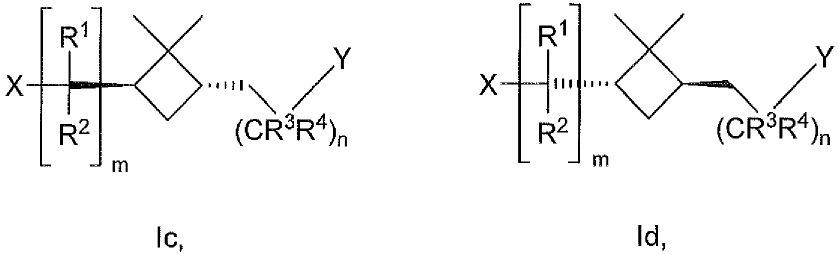 wherein
m, X, Y, R¹, R², R³, R⁴ and n have the above defined meanings.

Also preferred is a dimethylcyclobutyl compound of general formula I, wherein n is 0 or 1;
and the other substituents have any of the above defined meanings, optionally in form of one of its stereoisomers, preferably enantiomers or diasteromers, a racemate or in form of a mixture of at least two of its stereoisomers, preferably enantiomers and/or diastereomers, in any mixing ratio, or a corresponding solvate thereof.

Also preferred is a dimethylcyclobutyl compound of general formula I, wherein m is 1;
and the other substituents have any of the above defined meanings,
optionally in form of one of its stereoisomers, preferably enantiomers or diasteromers, a racemate or in form of a mixture of at least two of its stereoisomers, preferably enantiomers and/or diastereomers, in any mixing ratio, or a corresponding solvate thereof.

Also preferred is a dimethylcyclobutyl compound of general formula I, wherein
X represents a -OR⁵ moiety or a -NR⁶R⁷ moiety;
and the other substituents have any of the above defined meanings, optionally in form of one of its stereoisomers, preferably enantiomers or diasteromers, a racemate or in form of a mixture of at least two of its stereoisomers, preferably enantiomers and/or diastereomers, in any mixing ratio, or a corresponding solvate thereof.

Also preferred is a dimethylcyclobutyl compound of general formula I, wherein
Y represents a -OR⁸ moiety; a -NR⁹R¹⁰ moietyor a -C(=O)-OR¹¹ moiety;
and the other substituents have any of the above defined meanings, optionally in form of one of its stereoisomers, preferably enantiomers or diasteromers, a racemate or in form of a mixture of at least two of its stereoisomers, preferably enantiomers and/or diastereomers, in any mixing ratio, or a corresponding solvate thereof.

Also preferred is a dimethylcyclobutyl compound of general formula I, wherein
R¹ represents a hydrogen atom or a radical selected from the group consisting of methyl, ethyl, n-propyl, isopropyl, n-butyl, sec-butyl, isobutyl, tert-butyl and n-pentyl,
and the other substituents have any of the above defined meanings,
optionally in form of one of its stereoisomers, preferably enantiomers or diasteromers, a racemate or in form of a mixture of at least two of its stereoisomers, preferably enantiomers and/or diastereomers, in any mixing ratio, or a corresponding solvate thereof.

Also preferred is a dimethylcyclobutyl compound of general formula I, wherein
R² represents a hydrogen atom; a radical selected from the group consisting of methyl, ethyl, n-propyl, isopropyl, n-butyl, sec-butyl, isobutyl, tert-butyl, n-pentyl,; or phenylwhich may be unsubstituted or optionally substituted with 1, 2, 3, 4 or 5 substituent(s) independently selected from the group consisting of methyl, ethyl, n-propyl, isopropyl, n-butyl, tert-butyl, sec-butyl, isobutyl, n-pentyl, -O-CH₃, -O-C₂H₅, -F, Cl, Br, I, -CN, -CF₃, -OCF₃, -SCF₃;
and the other substituents have any of the above defined meanings, optionally in form of one of its stereoisomers, preferably enantiomers or diasteromers, a racemate or in form of a mixture of at least two of its stereoisomers, preferably enantiomers and/or diastereomers, in any mixing ratio, or a corresponding solvate thereof.

Also preferred is a dimethylcyclobutyl compound of general formula I, wherein R³ and R⁴ both represent a hydrogen atom;
and the other substituents have any of the above defined meanings, optionally in form of one of its stereoisomers, preferably enantiomers or diasteromers, a racemate or in form of a mixture of at least two of its stereoisomers, preferably enantiomers and/or diastereomers, in any mixing ratio, or a corresponding solvate thereof.

Also preferred is a dimethylcyclobutyl compound of general formula I, wherein
R⁵ and R⁸, independently of one another, each represent a hydrogen atom; a radical selected from the group consisting of methyl, ethyl, n-propyl, isopropyl, n-butyl, sec-butyl, isobutyl, tert-butyl, n-pentyl, an aryl radical selected from the group consisting of phenyl and naphthylwhich may be bonded via a -(CH₂)_{1, 2 or 3}-group and which may be unsubstituted or optionally substituted with 1, 2, 3, 4 or 5 substituent(s) independently selected from the group consisting of methyl, ethyl, n-propyl, isopropyl, n-butyl, tert-butyl, -O-CH₃, -O-C₂H₅, F, Cl, Br, I, -CN, -CF₃, -OCF₃, -SCF₃, -OH, -SH, -NH₂, -NO₂, -CHO, -CF₂H, -CFH₂, and -S(=O)₂-CH₃; or a -C(=O)-R¹² moiety;
and the other substituents have any of the above defined meanings, optionally in form of one of its stereoisomers, preferably enantiomers or diasteromers, a racemate or in form of a mixture of at least two of its stereoisomers, preferably enantiomers and/or diastereomers, in any mixing ratio, or a corresponding solvate thereof.

Also preferred is a dimethylcyclobutyl compound of general formula I, wherein
R⁶, R⁷, R⁹ and R¹⁰, independently of one another, each represent a radical selected from the group consisting of methyl, ethyl, n-propyl, isopropyl, n-butyl, sec-butyl, isobutyl, tert-butyl, n-pentylor a phenyl radicalwhich may be bonded via a -(CH₂)_{1, 2 or 3}-group and which may be unsubstituted or
optionally substituted with 1, 2, 3, 4 or 5 substituent(s) independently selected from the group consisting of methyl, ethyl, n-propyl, isopropyl, n-butyl, tert-butyl, sec-butyl, isobutyl, -O-CH₃, -O-C₂H₅, F, Cl, Br, I, -CN, -CF₃,-OCF₃ and -SCF₃ ; and the other substituents have any of the above defined meanings, optionally in form of one of its stereoisomers, preferably enantiomers or diasteromers, a racemate or in form of a mixture of at least two of its stereoisomers, preferably enantiomers and/or diastereomers, in any mixing ratio, or a corresponding solvate thereof.

Also preferred is a dimethylcyclobutyl compound of general formula I, wherein
R¹¹ and R¹², independently of one another, represent a hydrogen atom; a radical selected from the group consisting of methyl, ethyl, n-propyl, isopropyl, n-butyl, sec-butyl, isobutyl, tert-butyl, n-pentyl and the other substituents have any of the above defined meanings, optionally in form of one of its stereoisomers, preferably enantiomers or diasteromers, a racemate or in form of a mixture of at least two of its stereoisomers, preferably enantiomers and/or diastereomers, in any mixing ratio, or a corresponding solvate thereof.

More particularly preferred is a dimethylcyclobutyl compound of general formula I, wherein
m is 1;
n is 0 or 1;
X represents a -OR⁵ moiety or a -NR⁶R⁷ moiety;
Y represents a -OR⁸ moiety; a -NR⁹R¹⁰ moiety or a -C(=O)-OR¹¹ moiety;
R¹ represents a hydrogen atom or a radical selected from the group consisting of methyl, ethyl, n-propyl, isopropyl, n-butyl, sec-butyl, isobutyl, tert-butyl and n-pentyl;
R² represents a hydrogen atom; a radical selected from the group consisting of methyl, ethyl, n-propyl, isopropyl, n-butyl, sec-butyl, isobutyl, tert-butyl and n-pentyl; or an aryl radical selected from the group consisting of phenyl and naphthyl, which may be unsubstituted or substituted with 1, 2, 3, 4 or 5 substituent(s) independently selected from the group consisting of methyl, ethyl, n-propyl, isopropyl, n-butyl, tert-butyl, sec-butyl, isobutyl, n-pentyl, -O-CH₃, -O-C₂H₅ -S-CH₃, -S-C₂H₅, -C(=O)-OH, -C(=O)-O-CH₃, -C(=O)-O-C₂H₅,-C(=O)-CH₃, -C(=O)-C₂H₅, F, Cl, Br, I, -CN, -CF₃, -OCF₃, -SCF₃, -OH, -SH,-NH₂, -NO₂, -CHO, -CF₂H and -CFH₂;
R³ and R⁴ both represent a hydrogen atom;
R⁵ and R⁸, independently of one another, each represent a hydrogen atom; a radical selected from the group consisting of methyl, ethyl, n-propyl, isopropyl, n-butyl, sec-butyl, isobutyl, tert-butyl and n-pentyl; an aryl radical selected from the group consisting of phenyl and naphthyl, which may be bonded via a -(CH₂)_{1, 2 or 3}-group and which may be unsubstituted or optionally substituted with 1, 2, 3, 4 or 5 substituent(s) independently selected from the group consisting of methyl, ethyl, n-propyl, isopropyl, n-butyl, tert-butyl, -O-CH₃, -O-C₂H₅-F, Cl, Br, I, -CN, -CF₃, -OCF₃, -SCF₃, -OH,-SH, -NH₂, -NO₂, -CHO, -CF₂H, -CFH₂ and -S(=O)2-CH3; or a -C(=O)-R¹² moiety; R⁶, R⁷, R⁹ and R¹⁰, independently of one another, each represent a radical selected from the group consisting of methyl, ethyl, n-propyl, isopropyl, n-butyl, sec-butyl, isobutyl, tert-butyl and n-pentyl; or an aryl radical selected from the group consisting of phenyl and naphthyl, which may be bonded via a -(CH₂)_{1, 2 or 3}-group and which may be unsubstituted or optionally substituted with 1, 2, 3, 4 or 5 substituent(s) independently selected from the group consisting of methyl, ethyl, n-propyl, isopropyl, n-butyl, tert-butyl, sec-butyl, isobutyl, -O-CH₃, -O-C₂H₅, -S-CH₃, -S-C₂H₅, F, Cl, Br, I, -CN, -CF₃, -OCF₃,-SCF₃, -OH, -SH, -NH₂-NO₂, -CHO, -CF₂H, -CFH₂, -C(=O)-NH₂ and -S(=O)₂-CH₃;
or R⁶ and R⁷ together with the bridging nitrogen atom form a moiety selected from the group consisting of 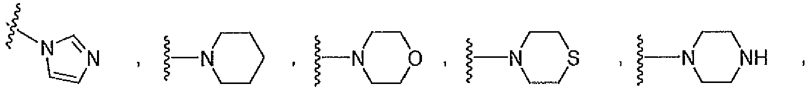 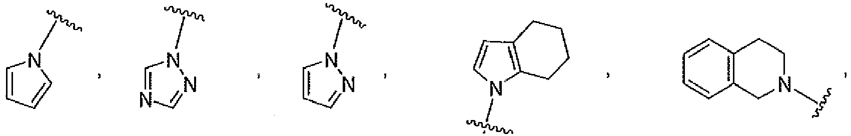 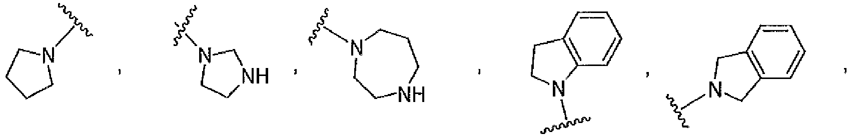 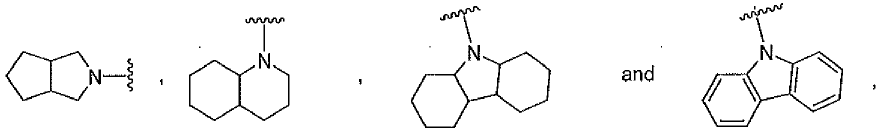 which may be unsubstituted or optionally substituted in any position including the -NH-groups with 1, 2, 3, 4 or 5 substituent(s) independently selected from the group consisting of oxo (=0), methyl, ethyl, n-propyl, isopropyl, n-butyl, tert-butyl, sec-butyl, -O-CH₃, -O-C₂H₅, -S-CH₃, -S-C₂H₅, -C(=O)-OH, -C(=O)-O-CH₃, -C(=O)-O-C₂H₅, -C(=O)-O-CH₂-CH₂-CH₃, -C(=O)-O-CH(CH₃)₂,-C(=O)-O-C(CH₃)₃, -C(=O)-CH₃, -C(=O)-C₂H₅, -C(=O)-CH₂-CH₂-CH₃, -C(=O)-CH(CH₃)₂, -C(=O)-C(CH₃)₃, F, Cl, Br, I, -CN, -CF₃, -OCF₃, -SCF₃, -OH, -SH,-NH₂, -NO₂, -CHO, -CF₂H, -CFH₂, -C(=O)-NH₂, -C(=O)-NH-CH₃, -C(=O)-NH-C₂H₅, -C(=O)-N(CH₃)₂, -C(=O)-N(C₂H₅)₂, -S(=O)₂-CH₃, phenyl, phenethyl and benzyl, whereby said cyclic substituents may be unsubstituted or substituted by 1, 2 or 3 substituent(s) independently selected from the group consisting of methyl, ethyl, n-propyl, isopropyl, methoxy, ethoxy, F, Cl, Br, -CN, -CF₃,-OCF₃, -SCF₃, -OH, -SH, -NH₂ and -NO₂;
or R⁹ and R¹⁰ together with the bridging nitrogen atom form a moiety selected from the group consisting of 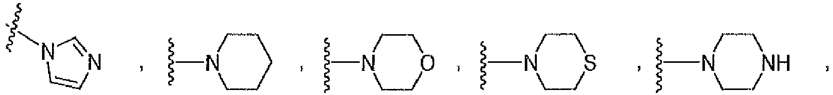 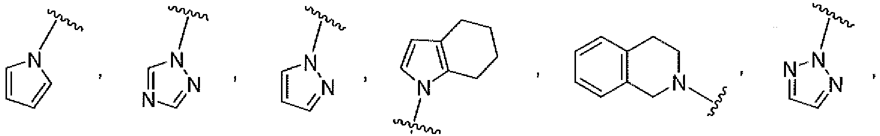 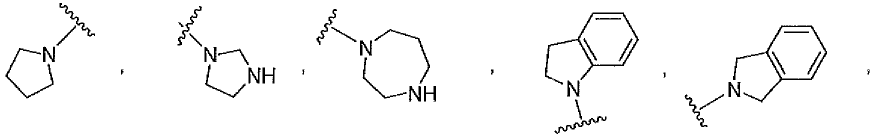 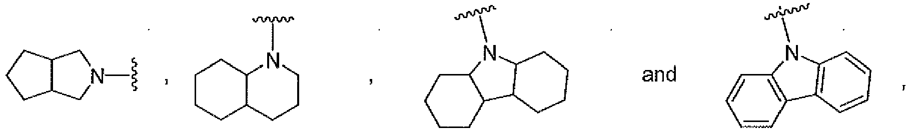 which may be unsubstituted or optionally substituted in any position including the -NH-groups with 1, 2, 3, 4 or 5 substituent(s) independently selected from the group consisting of oxo (=0), methyl, ethyl, n-propyl, isopropyl, n-butyl, tert-butyl, sec-butyl, -O-CH₃, -O-C₂H₅, -S-CH₃, -S-C₂H₅, -C(=O)-OH, -C(=O)-O-CH₃, -C(=O)-O-C₂H₅, -C(=O)-O-CH₂-CH₂-CH₃, -C(=O)-O-CH(CH₃)₂,-C(=O)-O-C(CH₃)₃, -C(=O)-CH₃, -C(=O)-C₂H₅, -C(=O)-CH₂-CH₂-CH₃, -C(=O)-CH(CH₃)₂, -C(=O)-C(CH₃)₃, F, Cl, Br, I, -CN, -CF₃, -OCF₃, -SCF₃, -OH, -SH,-NH₂, -NO₂, -CHO, -CF₂H, -CFH₂, -C(=O)-NH₂, -C(=O)-NH-CH₃, -C(=O)-NH-C₂H₅, -C(=O)-N(CH₃)₂, -C(=O)-N(C₂H₅)₂, -S(=O)₂-CH₃, phenyl, phenethyl and benzyl, whereby said cyclic substituents may be unsubstituted or substituted by 1, 2 or 3 substituent(s) independently selected from the group consisting of methyl, ethyl, n-propyl, isopropyl, methoxy, ethoxy, F, Cl, Br, -CN, -CF₃,-OCF₃, -SCF₃, -OH, -SH, -NH₂ and -NO₂;
R¹¹ represent a a radical selected from the group consisting of methyl, ethyl, n-propyl, isopropyl, n-butyl, sec-butyl, isobutyl, tert-butyl and n-pentyl; and R¹² represents a hydrogen atom; a radical selected from the group consisting of methyl, ethyl, n-propyl, isopropyl, n-butyl, sec-butyl, isobutyl, tert-butyl and n-pentyl;
optionally in form of one of its stereoisomers, preferably enantiomers or diasteromers, a racemate or in form of a mixture of at least two of its stereoisomers, preferably enantiomers and/or diastereomers, in any mixing ratio, or a salt thereof, or a corresponding solvate thereof.

Even more particularly preferred is a dimethylcyclobutyl compound of general formula I, wherein
m is 1;
n is 0 or 1;
X represents a -OR⁵ moiety or a -NR⁶R⁷ moiety;
Y represents a -OR⁸ moiety; a -NR⁹R¹⁰ moiety or a -C(=O)-OR¹¹ moiety;
R¹ represents a hydrogen atom or a radical selected from the group consisting of methyl, ethyl, n-propyl, isopropyl, n-butyl, sec-butyl, isobutyl, tert-butyl and n-pentyl;
R² represents a hydrogen atom; a radical selected from the group consisting of methyl, ethyl, n-propyl, isopropyl, n-butyl, sec-butyl, isobutyl, tert-butyl and n-pentyl; or a phenyl radical which may be unsubstituted or substituted with 1, 2, 3, 4 or 5 substituent(s) independently selected from the group consisting of methyl, ethyl, n-propyl, isopropyl, n-butyl, tert-butyl, sec-butyl, isobutyl, n-pentyl, -O-CH₃, -O-C₂H₅, F, Cl, Br, I, -CN, -CF₃, -OCF₃ and -SCF₃;
R³ and R⁴ both represent a hydrogen atom;
R⁵ and R⁸, independently of one another, each represent a hydrogen atom; a phenyl radical, which may be bonded via a -(CH₂)_{1, 2 or 3}-group and which may be unsubstituted or optionally substituted with 1, 2, 3, 4 or 5 substituent(s) independently selected from the group consisting of methyl, ethyl, n-propyl, isopropyl, n-butyl, tert-butyl, -O-CH₃, -O-C₂H₅, F, Cl, Br, I, -CN, -CF₃ and-OCF₃; or a -C(=O)-R¹² moiety; R⁶, R⁷, R⁹ and R¹⁰, independently of one another, each represent a radical selected from the group consisting of methyl, ethyl, n-propyl, isopropyl, n-butyl, sec-butyl, isobutyl, tert-butyl and n-pentyl; or a phenyl radical, which may be bonded via a -(CH₂)_{1, 2} or ₃-group and which may be unsubstituted or optionally substituted with 1, 2, 3, 4 or 5 substituent(s) independently selected from the group consisting of methyl, ethyl, n-propyl, isopropyl, n-butyl, tert-butyl, sec-butyl, isobutyl, -O-CH₃, -O-C₂H₅, F, Cl, Br, I, -CN, -CF₃,-OCF₃ and -SCF₃;
or R⁶ and R⁷ together with the bridging nitrogen atom form a moiety selected from the group consisting of 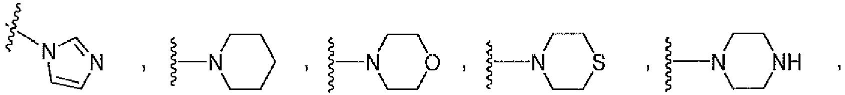 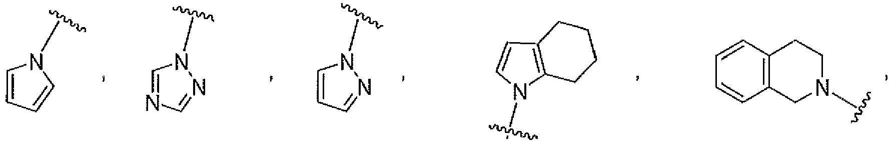 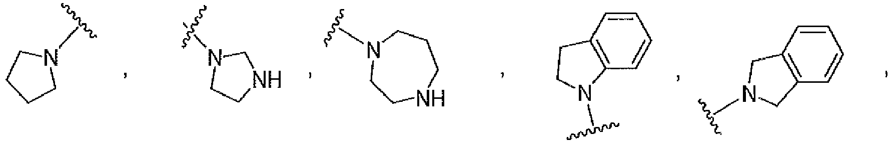 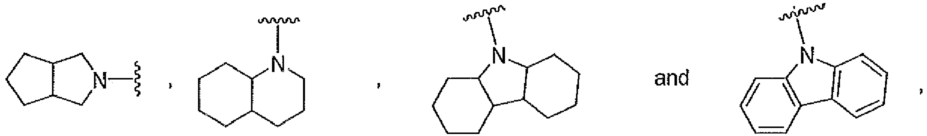 which may be unsubstituted or optionally substituted in any position including the -NH-groups with 1, 2, 3, 4 or 5 substituent(s) independently selected from the group consisting of oxo (=O), methyl, ethyl, n-propyl, isopropyl, n-butyl, tert-butyl, sec-butyl, phenyl, phenethyl and benzyl, whereby said cyclic substituents may be unsubstituted or substituted by 1, 2 or 3 substituent(s) independently selected from the group consisting of methyl, ethyl, n-propyl, isopropyl, methoxy, ethoxy, F, Cl, Br, -CN, -CF₃, -OCF₃, -SCF₃, -OH, -SH,-NH₂ and -NO₂;
or R⁹ and R¹⁰ together with the bridging nitrogen atom form a moiety selected from the group consisting of 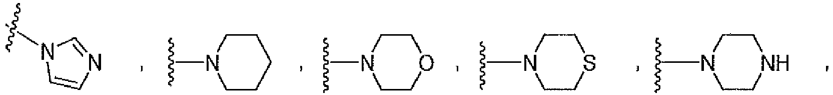 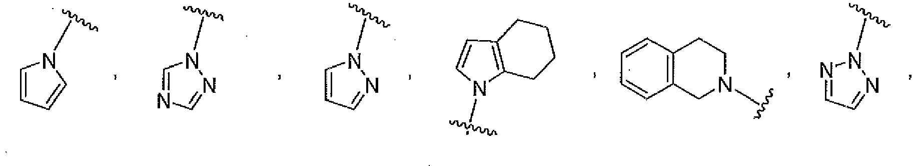 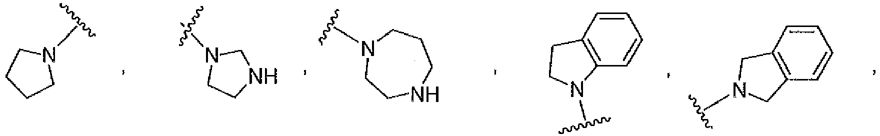 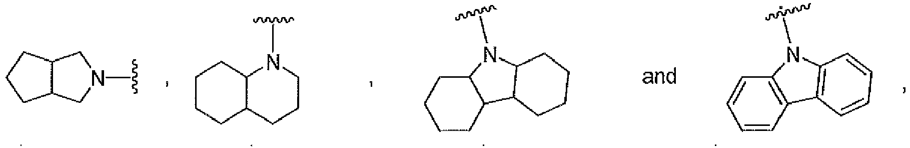 which may be unsubstituted or optionally substituted in any position including the -NH-groups with 1, 2, 3, 4 or 5 substituent(s) independently selected from the group consisting of oxo (=0), methyl, ethyl, n-propyl, isopropyl, n-butyl, tert-butyl, sec-butyl, phenyl, phenethyl and benzyl, whereby said cyclic substituents may be unsubstituted or substituted by 1, 2 or 3 substituent(s) independently selected from the group consisting of methyl, ethyl, n-propyl, isopropyl, methoxy, ethoxy, F, Cl, Br, -CN, -CF₃, -OCF₃, -SCF₃, -OH, -SH,-NH₂ and -NO₂;
and R¹¹ and R¹², independently of one another, represent a radical selected from the group consisting of methyl, ethyl, n-propyl, isopropyl, n-butyl, sec-butyl, isobutyl, tert-butyl and n-pentyl;
optionally in form of one of its stereoisomers, preferably enantiomers or diasteromers, a racemate or in form of a mixture of at least two of its
stereoisomers, preferably enantiomers and/or diastereomers, in any mixing ratio, or a salt thereof, or a corresponding solvate thereof.

Also even more particularly preferred is a dimethylcyclobutyl compound of general formula I, wherein
m is 1;
n is 0 or 1;
X represents a -OR⁵ moiety or a -NR⁶R⁷ moiety;
Y represents a -OR⁸ moiety; a -NR⁹R¹⁰ moiety or a -C(=O)-OR¹¹ moiety;
R¹ represents a hydrogen atom or a radical selected from the group consisting of methyl, ethyl, n-propyl, isopropyl, n-butyl, sec-butyl, isobutyl, tert-butyl and n-pentyl;
R² represents a hydrogen atom; a radical selected from the group consisting of methyl, ethyl, n-propyl, isopropyl, n-butyl, sec-butyl, isobutyl, tert-butyl and n-pentyl; or a phenyl radical which may be unsubstituted or substituted with 1, 2, 3, 4 or 5 substituent(s) independently selected from the group consisting of methyl, ethyl, n-propyl, isopropyl, n-butyl, tert-butyl, sec-butyl, isobutyl, n-pentyl, -O-CH₃, -O-C₂H₅, F, Cl, Br, I, -CN, -CF₃, -OCF₃ and -SCF₃;
R³ and R⁴ both represent a hydrogen atom;
R⁵ and R⁸, independently of one another, each represent a hydrogen atom; a phenyl radical, which may be bonded via a -(CH₂)_{1, 2 or 3}-group and which may be unsubstituted or optionally substituted with 1, 2, 3, 4 or 5 substituent(s) independently selected from the group consisting of methyl, ethyl, n-propyl, isopropyl, n-butyl, tert-butyl, -O-CH₃, -O-C₂H₅, F, Cl, Br, I, -CN, -CF₃ and-OCF₃ or a -C(=O)-R¹² moiety;
R⁶, R⁷, R⁹ and R¹⁰, independently of one another, each represent a radical selected from the group consisting of methyl, ethyl, n-propyl, isopropyl, n-butyl, sec-butyl, isobutyl, tert-butyl and n-pentyl; or a phenyl radical, which is bonded via a -(CH₂)_{1, 2 or 3}-group and which may be unsubstituted or optionally substituted with 1, 2, 3, 4 or 5 substituent(s) independently selected from the group consisting of methyl, ethyl, n-propyl, isopropyl, n-butyl, tert-butyl, sec-butyl, isobutyl, -O-CH₃, -O-C₂H₅, F, Cl, Br, I, -CN, -CF₃,-OCF₃ and -SCF₃;
or R⁶ and R⁷ together with the bridging nitrogen atom form a moiety selected from the group consisting of 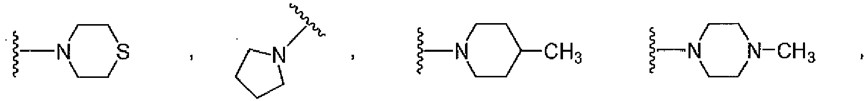 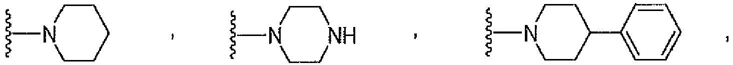 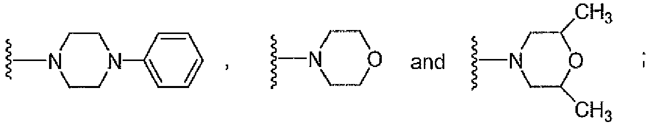 or R⁹ and R¹⁰ together with the bridging nitrogen atom form a moiety selected from the group consisting of 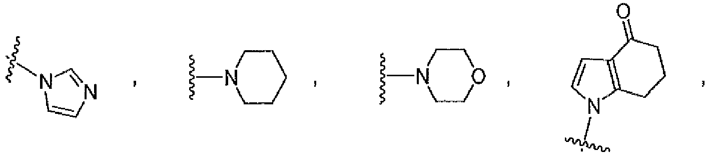 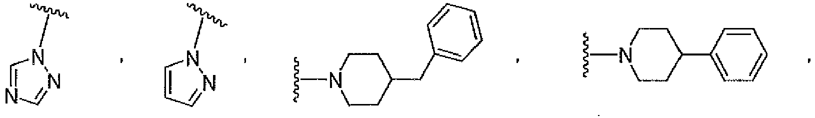 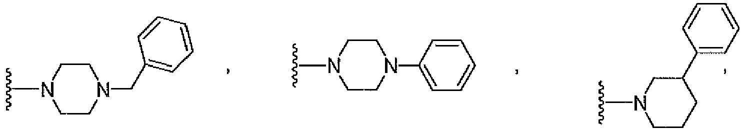 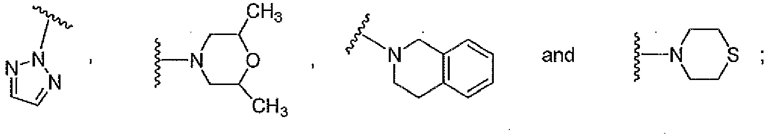 and R¹¹ and R¹², independently of one another, represent a radical selected from the group consisting of methyl, ethyl, n-propyl, isopropyl, n-butyl, sec-butyl, isobutyl, tert-butyl and n-pentyl;
optionally in form of one of its stereoisomers, preferably enantiomers or diasteromers, a racemate or in form of a mixture of at least two of its stereoisomers, preferably enantiomers and/or diastereomers, in any mixing ratio, or a salt thereof, or a corresponding solvate thereof.

Also preferred is a dimethylcyclobutyl compound of general formula Ie, 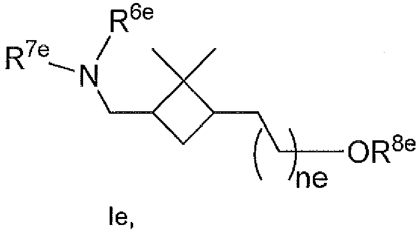 wherein
ne is 0 or 1;
R^{6e} and R^{7e}, independently of one another, each represent a radical selected from the group consisting of methyl, ethyl, n-propyl, isopropyl, n-butyl, sec-butyl, isobutyl, tert-butyl and n-pentyl; or an aryl or heteroaryl radical selected from the group consisting of phenyl and naphthyl, which may be bonded via a -(CH₂)_{1, 2 or 3}-group and which may be unsubstituted or optionally substituted with 1, 2, 3, 4 or 5 substituent(s) independently selected from the group consisting of methyl, ethyl, n-propyl, isopropyl, n-butyl, tert-butyl, sec-butyl, isobutyl, -O-CH₃, -O-C₂H₅, -S-CH₃, -S-C₂H₅, F, Cl, Br, I, -CN, -CF₃, -OCF₃,-SCF₃, -OH, -SH, -NH₂-NO₂, -CHO, -CF₂H, -CFH₂, -C(=O)-NH₂ and -S(=O)₂-CH₃;
or R^{6e} and R^{7e} together with the bridging nitrogen atom form a moiety selected from the group consisting of 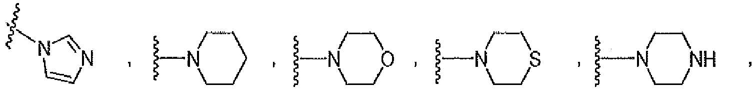 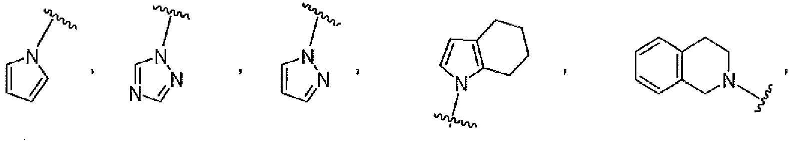 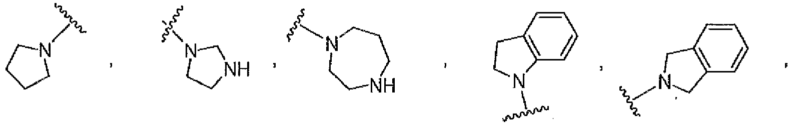 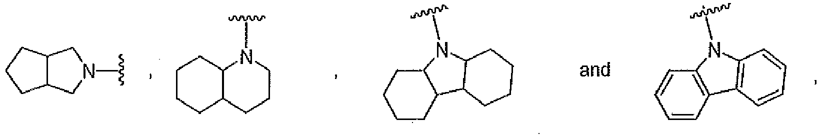 which may be unsubstituted or optionally substituted in any position including the -NH-groups with 1, 2, 3, 4 or 5 substituent(s) independently selected from the group consisting of oxo (=O), methyl, ethyl, n-propyl, isopropyl, n-butyl, tert-butyl, sec-butyl, -O-CH₃, -O-C₂H₅, -S-CH₃, -S-C₂H₅, -C(=O)-OH, -C(=O)-O-CH₃, -C(=O)-O-C₂H₅, -C(=O)-O-CH₂-CH₂-CH₃, -C(=O)-O-CH(CH₃)₂,-C(=O)-O-C(CH₃)₃, -C(=O)-CH₃, -C(=O)-C₂H₅, -C(=O)-CH₂-CH₂-CH₃, -C(=O)-CH(CH₃)₂, -C(=O)-C(CH₃)₃, F, Cl, Br, I, -CN, -CF₃, -OCF₃, -SCF₃, -OH, -SH,-NH₂, -NO₂, -CHO, -CF₂H, -CFH₂, -C(=O)-NH₂, -C(=O)-NH-CH₃, -C(=O)-NH-C₂H₅, -C(=O)-N(CH₃)₂, -C(=O)-N(C₂H₅)₂, -S(=O)₂-CH₃, phenyl, phenethyl and benzyl, whereby said cyclic substituents may be unsubstituted or substituted by 1, 2 or 3 substituent(s) independently selected from the group consisting of methyl, ethyl, n-propyl, isopropyl, methoxy, ethoxy, F, Cl, Br, -CN, -CF₃,-OCF₃, -SCF₃, -OH, -SH, -NH₂ and -NO₂;
R^{8e} represents a hydrogen atom; a radical selected from the group consisting of methyl, ethyl, n-propyl, isopropyl, n-butyl, sec-butyl, isobutyl, tert-butyl and n-pentyl; an aryl radical selected from the group consisting of phenyl and naphthyl, which may be bonded via a -(CH₂)_{1, 2 or 3}-group and which may be unsubstituted or optionally substituted with 1, 2, 3, 4 or 5 substituent(s) independently selected from the group consisting of methyl, ethyl, n-propyl, isopropyl, n-butyl, tert-butyl, -O-CH₃, -O-C₂H₅-F, Cl, Br, I, -CN, -CF₃, -OCF₃,-SCF₃, -OH, -SH, -NH₂, -NO₂, -CHO, -CF₂H, -CFH₂ and -S(=O)₂-CH₃; or a-C(=O)-R^{12e} moiety;
   and R^{12e} represents a radical selected from the group consisting of methyl, ethyl, n-propyl, isopropyl, n-butyl, sec-butyl, isobutyl, tert-butyl and n-pentyl; optionally in form of one of its stereoisomers, preferably enantiomers or diasteromers, a racemate or in form of a mixture of at least two of its stereoisomers, preferably enantiomers and/or diastereomers, in any mixing ratio, or a salt thereof, or a corresponding solvate thereof.

Preferred is a substituted dimethylcyclobutyl compound of general formula Ie, wherein the stereoisomers have the general formulae If or Ig or Ih or Ij, 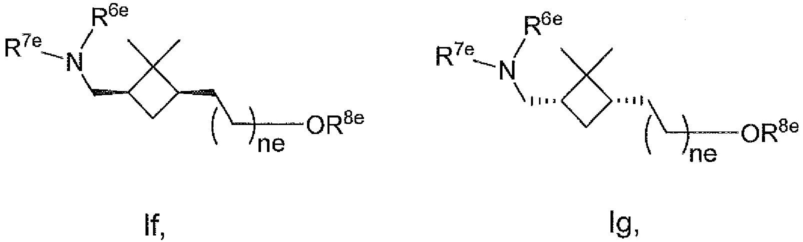 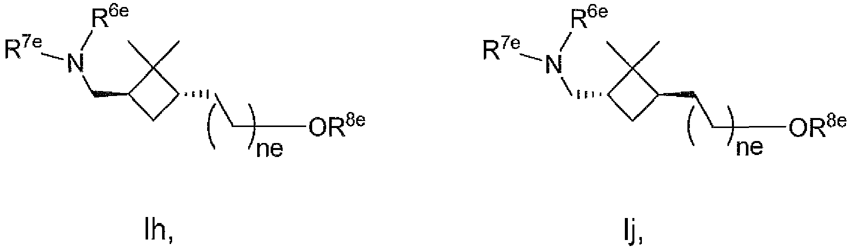 wherein ne, R^{6e}, R^{7e} and R^{8e} have the above defined meanings.

Also preferred is a dimethylcyclobutyl compound of general formula Ik, 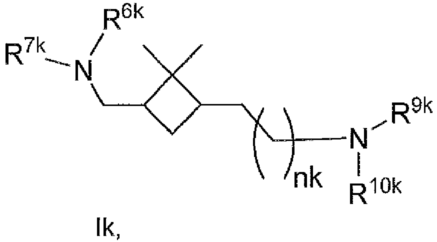 wherein
R^{6k} R^{7k}N- and R^{9k}R^{10k}N- are different
nk is 0 or 1;
R^{6k}, R^{7k}, R^{9k} and R^{10k}, independently of one another, each represent a radical selected from the group consisting of methyl, ethyl, n-propyl, isopropyl, n-butyl, sec-butyl, isobutyl, tert-butyl and n-pentyl; or an aryl radical selected from the group consisting of phenyl and naphthyl, which may be bonded via a -(CH₂)_{1,2 or 3}-group and which may be unsubstituted or optionally substituted with 1, 2, 3, 4 or 5 substituent(s) independently selected from the group consisting of methyl, ethyl, n-propyl, isopropyl, n-butyl, tert-butyl, sec-butyl, isobutyl, -O-CH₃, -O-C₂H₅, -S-CH₃, -S-C₂H₅, F, Cl, Br, I, -CN, -CF₃, -OCF₃,-SCF₃, -OH, -SH, -NH₂-NO₂, -CHO, -CF₂H, -CFH₂, -C(=O)-NH₂ and -S(=O)₂-CH₃;
or R^{6k} and R^{7k} together with the bridging nitrogen atom form a moiety selected from the group consisting of 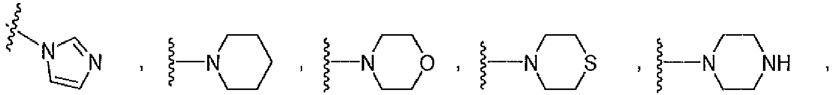 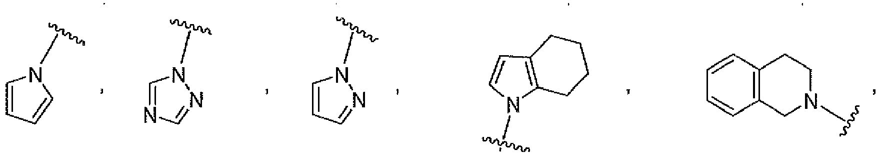 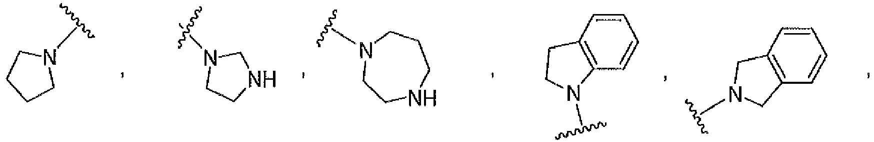 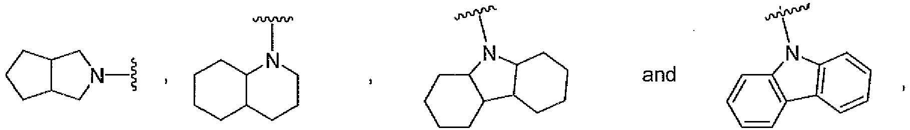 which may be unsubstituted or optionally substituted in any position including the -NH-groups with 1, 2, 3, 4 or 5 substituent(s) independently selected from the group consisting of oxo (=O), methyl, ethyl, n-propyl, isopropyl, n-butyl, tert-butyl, sec-butyl, -O-CH₃, -O-C₂H₅, -S-CH₃, -S-C₂H₅, -C(=O)-OH, -C(=O)-O-CH₃, -C(=O)-O-C₂H₅, -C(=O)-O-CH₂-CH₂-CH₃, -C(=O)-O-CH(CH₃)₂,-C(=O)-O-C(CH₃)₃, -C(=O)-CH₃, -C(=O)-C₂H₅, -C(=O)-CH₂-CH₂-CH₃, -C(=O)-CH(CH₃)₂, -C(=O)-C(CH₃)₃, F, Cl, Br, I, -CN, -CF₃, -OCF₃, -SCF₃, -OH, -SH,-NH₂, -NO₂, -CHO, -CF₂H, -CFH₂, -C(=O)-NH₂, -C(=O)-NH-CH₃, -C(=O)-NH-C₂H₅, -C(=O)-N(CH₃)₂, -C(=O)-N(C₂H₅)₂, -S(=O)₂-CH₃, phenyl, phenethyl and benzyl, whereby said cyclic substituents may be unsubstituted or substituted by 1, 2 or 3 substituent(s) independently selected from the group consisting of methyl, ethyl, n-propyl, isopropyl, methoxy, ethoxy, F, Cl, Br, -CN, -CF₃,-OCF₃, -SCF₃, -OH, -SH, -NH₂ and -NO₂;
or R^{9k} and R^{10k} together with the bridging nitrogen atom form a moiety selected from the group consisting of 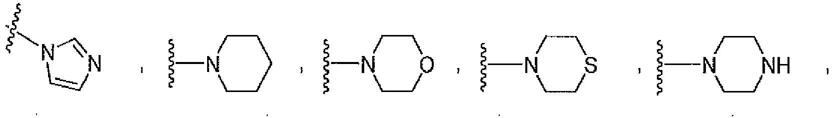 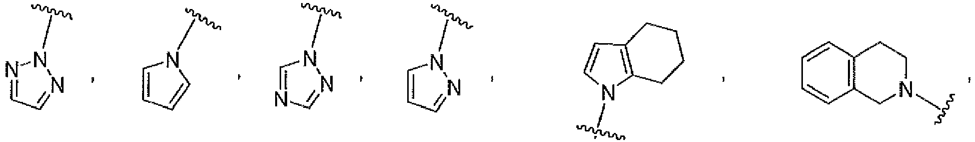 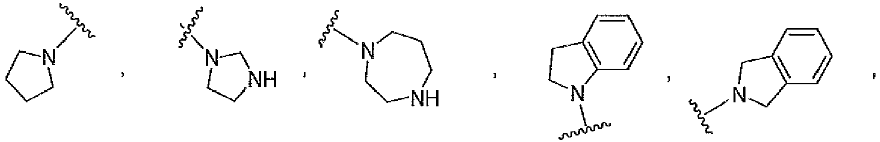 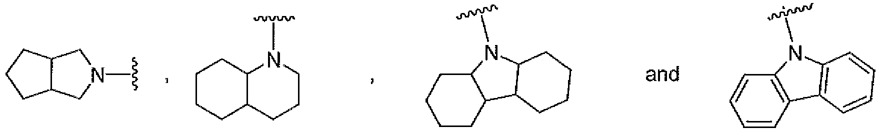 which may be unsubstituted or optionally substituted in any position including the -NH-groups with 1, 2, 3, 4 or 5 substituent(s) independently selected from the group consisting of oxo (=0), methyl, ethyl, n-propyl, isopropyl, n-butyl, tert-butyl, sec-butyl, -O-CH₃, -O-C₂H₅, -S-CH₃, -S-C₂H₅, -C(=O)-OH, -C(=O)-O-CH₃, -C(=O)-O-C₂H₅, -C(=O)-O-CH₂-CH₂-CH₃, -C(=O)-O-CH(CH₃)₂,-C(=O)-O-C(CH₃)₃, -C(=O)-CH₃, -C(=O)-C₂H₅, -C(=O)-CH₂-CH₂-CH₃, -C(=O)-CH(CH₃)₂, -C(=O)-C(CH₃)₃, F, Cl, Br, I, -CN, -CF₃, -OCF₃, -SCF₃, -OH, -SH,-NH₂, -NO₂, -CHO, -CF₂H, -CFH₂, -C(=O)-NH₂, -C(=O)-NH-CH₃, -C(=O)-NH-C₂H₅, -C(=O)-N(CH₃)₂, -C(=O)-N(C₂H₆)₂, -S(=O)₂-CH₃, phenyl, phenethyl and benzyl, whereby said cyclic substituents may be unsubstituted or substituted by 1, 2 or 3 substituent(s) independently selected from the group consisting of methyl, ethyl, n-propyl, isopropyl, methoxy, ethoxy, F, Cl, Br, -CN, -CF₃,-OCF₃, -SCF₃, -OH, -SH, -NH₂ and -NO₂;
optionally in form of one of its stereoisomers, preferably enantiomers or diasteromers, a racemate or in form of a mixture of at least two of its stereoisomers, preferably enantiomers and/or diastereomers, in any mixing ratio, or a salt thereof, or a corresponding solvate thereof.

Preferred is a substituted dimethylcyclobutyl compound of general formula Ik, wherein the stereoisomers have the general formulae Im or In or Io or Ip, 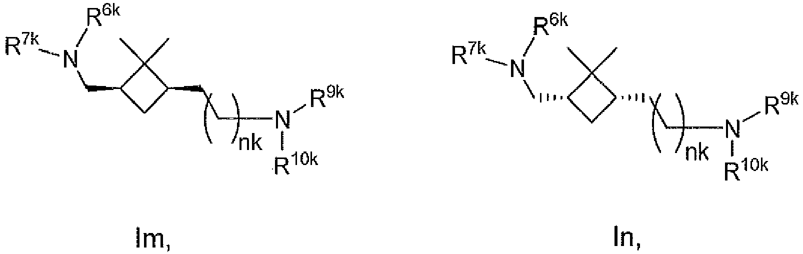 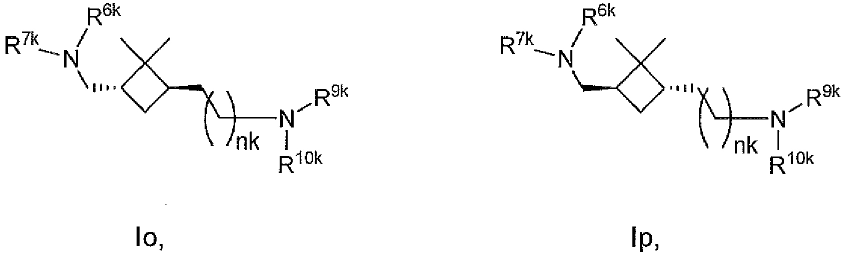 wherein nk, R^{6k}, R^{7k}, R^{9k} and R^{10k} have the above defined meanings.

Most particularly preferred is a dimethylcyclobutyl compound selected from the group consisting of
[1] 4-(((1S,3S)-3-(2-(benzyloxy)ethyl)-2,2-dimethylcyclobutyl)methyl)-morpholine
[2] 1-(((1S,3S)-3-(2-(benzyloxy)ethyl)-2,2-dimethylcyclobutyl)methyl)-piperidine
[3] 1-(((1S,3S)-3-(2-(benzyloxy)ethyl)-2,2-dimethylcyclobutyl)methyl)-4-phenylpiperidine
[4] 1-(((1S,3S)-3-(2-(benzyloxy)ethyl)-2,2-dimethylcyclobutyl)methyl)-4-phenylpiperazine
[5] 1-(((1S,3S)-3-(2-(benzyloxy)ethyl)-2,2-dimethylcyclobutyl)methyl)-4-methylpiperazine
[6] 4-(((1R,3S)-3-(benzyloxymethyl)-2,2-dimethylcyclobutyl)methyl)-morpholine
[7] 1-(((1R,3S)-3-(benzyloxymethyl)-2,2-dimethylcyclobutyl)methyl)-piperidine
[8] 1-(((1R,3S)-3-(benzyloxymethyl)-2,2-dimethylcyclobutyl)methyl)-4-phenylpiperidine
[9] 1-(((1R,3S)-3-(benzyloxymethyl)-2,2-dimethylcyclobutyl)methyl)-4-phenylpiperazine
[10] 1-(((1R,3S)-3-(benzyloxymethyl)-2,2-dimethylcyclobutyl)methyl)-4-methylpiperazine
[13] 1-((1R,3R)-2,2-dimethyl-3-(2-morpholinoethyl)cyclobutyl)-1-phenylethanol
[14] 1-((1R,3R)-2,2-dimethyl-3-(2-(piperidin-1-yl)ethyl)cyclobutyl)-1-phenylethanol
[15] 4-(((1R,3R)-2,2-dimethyl-3-(2-phenoxyethyl)cyclobutyl)methyl)-morpholine
[16] 1-(((1R,3R)-2,2-dimethyl-3-(2-phenoxyethyl)cyclobutyl)methyl)piperidine
[17] 2-((1R,3R)-2,2-dimethyl-3-(piperidin-1-ylmethyl)cyclobutyl)ethanol
[18] 2-((1S,3S)-2,2-dimethyl-3-(piperidin-1-ylmethyl)cyclobutyl)ethanol
[19] 2-((1R,3R)-2,2-dimethyl-3-(morpholinomethyl)cyclobutyl)ethanol
[20] 2-((1S,3S)-2,2-dimethyl-3-(morpholinomethyl)cyclobutyl)ethanol
[21] 2-((1R,3R)-2,2-dimethyl-3-((4-methylpiperidin-1-yl)methyl)cyclobutyl)-ethanol
[22] 2-((1R,3R)-2,2-dimethyl-3-((4-phenylpiperidin-1-yl)methyl)cyclobutyl)-ethanol
[23] 2-((1R,3R)-3-(((2S,6R)-2,6-dimethylmorpholino)methyl)-2,2-dimethylcyclobutyl)ethanol
[24] 2-((1R,3R)-3-(N-benzyl-N-methylamino)methyl)-2,2-dimethylcyclobutyl)-ethanol
[26] methyl 2-((1R,3R)-2,2-dimethyl-3-(piperidin-1-ylmethyl)cyclobutyl)-acetate
[27] 1-(((1R,3R)-3-(2-(benzyloxy)ethyl)-2,2-dimethylcyclobutyl)methyl)-piperidine
[28] 1-(((1R,3R)-3-(2-(benzyloxy)ethyl)-2,2-dimethylcyclobutyl)methyl)-4-methylpiperidine
[29] 1-(((1R,3R)-3-(2-(benzyloxy)ethyl)-2,2-dimethylcyclobutyl)methyl)-4-phenylpiperidine
[30] 4-(((1R,3R)-3-(2-(benzyloxy)ethyl)-2,2-dimethylcyclobutyl)methyl)-thiomorpholine
[31] 4-(((1R,3R)-3-(2-(benzyloxy)ethyl)-2,2-dimethylcyclobutyl)methyl)-morpholine
[32] (2S,6R)-4-(((1R,3R)-3-(2-(benzyloxy)ethyl)-2,2-dimethylcyclobutyl)-methyl)-2,6-dimethylmorpholine
[33] 1-(((1R,3R)-3-(2-(benzyloxy)ethyl)-2,2-dimethylcyclobutyl)methyl)-pyrrolidine
[34] 1-(((1R,3R)-3-(2-(benzyloxy)ethyl)-2,2-dimethylcyclobutyl)methyl)-4-methylpiperazine
[35] 1-(((1R,3R)-3-(2-(benzyloxy)ethyl)-2,2-dimethylcyclobutyl)methyl)-4-methylpiperazine oxalate
[36] 1-(((1R,3R)-3-(2-(benzyloxy)ethyl)-2,2-dimethylcyclobutyl)methyl)-4-phenylpiperazine
[37] 1-(((1R,3R)-3-(2-(benzyloxy)ethyl)-2,2-dimethylcyclobutyl)methyl)-4-phenylpiperazine oxalate
[38] 1-(2-((1R,3R)-3-(benzyloxymethyl)-2,2-dimethylcyclobutyl)ethyl)-piperidine
[39] 4-(2-((1R,3R)-3-(benzyloxymethyl)-2,2-dimethylcyclobutyl)ethyl)-morpholine
[40] 4-(((1R,3R)-2,2-dimethyl-3-(2-(3-phenylpiperidin-1-yl)ethyl)cyclobutyl)-methyl)-morpholine
[41] 4-(((1R,3R)-2,2-dimethyl-3-(2-(4-phenylpiperldin-1-yl)ethyl)cyclobutyl)-methyl)morpholine
[42] 4-(((1R,3R)-3-(2-(4-benzylpiperidin-1-yl)ethyl)-2,2-dimethylcyclobutyl)-methyl)morpholine
[43] 4-(((1R,3R)-3-(2-(1H-imidazol-1-yl)ethyl)-2,2-dimethylcyclobutyl)-methyl)morpholine
[44] 4-(((1R,3R)-3-(2-(1H-pyrazol-1-yl)ethyl)-2,2-dimethylcyclobutyl)-methyl)morpholine
[45] 4-(((1R,3R)-3-(2-(1H-1,2,4-triazol-1-yl)ethyl)-2,2-dimethylcyclobutyl)-methyl)morpholine
[46] 1-(2-((1R,3R)-2,2-dimethyl-3-(morpholinomethyl)cyclobutyl)ethyl)-6,7-dihydro-1H-indol-4(5H)-one
[47] 2-(2-((1R,3R)-2,2-dimethyl-3-(morpholinomethyl)cyclobutyl)ethyl)-1,2,3,4-tetrahydroisoquinoline
[48] 1-(((1R,3R)-3-(2-(1H-imidazol-1-yl)ethyl)-2,2-dimethylcyclobutyl)-methyl)piperidine
[49] 1-(((1R,3R)-3-(2-(1H-pyrazol-1-yl)ethyl)-2,2-dimethylcyclobutyl)-methyl)piperidine
[50] 1-(((1R,3R)-3-(2-(1H-1,2,4-triazol-1-yl)ethyl)-2,2-dimethylcyclobutyl)-methyl)piperidine
[51] 1-(2-((1R,3R)-2,2-dimethyl-3-(piperidin-1-ylmethyl)cyclobutyl)ethyl)-4-phenylpiperidine
[52] 4-benzyl-1-(2-((1R,3R)-2,2-dimethyl-3-(piperidin-1-ylmethyl)cyclobutyl)-ethyl)piperidine
[53] 1-(2-((1R,3R)-2,2-dimethyl-3-(piperidin-1-ylmethyl)cyclobutyl)ethyl)-6,7-dihydro-1H-indol-4(5H)-one and
[54] 2-(2-((1R,3R)-2,2-dimethyl-3-(piperidin-1-ylmethyl)cyclobutyl)ethyl)-1,2,3,4-tetrahydroisoquinoline;
[55] 2-((1*R*,3*R*)-2,2-Dimethyl-3-((piperidin-1-yl)methyl)cyclobutyl)ethyl pivalate
[56] 2-((1*R*,3*R*)-2,2-Dimethyl-3-((piperidin-1-yl)methyl)cyclobutyl)ethyl acetate
optionally in form of one of its stereoisomers, preferably enantiomers or diasteromers, a racemate or in form of a mixture of at least two of its stereoisomers, preferably enantiomers and/or diastereomers, in any mixing ratio, or a salt thereof, or a corresponding solvate thereof.

In still another aspect the present invention relates to a process for the preparation of a substituted dimethylcyclobutyl compound of general formula I, wherein at least one compound of general formula II, 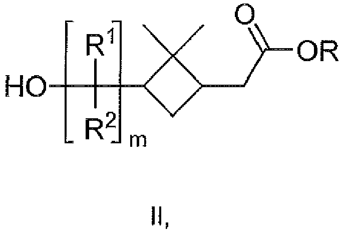 wherein m, R¹ and R² have the meaning given above and R represents a linear or branched C₁₋₅-alkyl radical, is reacted with methane sulfonylchloride, *p*-toluene sulfonylchloride, trifluormethane sulfonylchloride, thionyl chloride or tetrabromethane, preferably in a reaction medium selected from the group consisting of diethylether, tetrahydrofuran, tert-butylmethylether and dichloromethane or a mixture thereof, more preferably in dichloromethane, preferably in the presence of at least one base selected from the group consisting of pyridine, triethylamine, diisopropylamine, diisopropylethylamine, 4-methylmorpholine and morpholine, to yield at least one compound of general formula III, 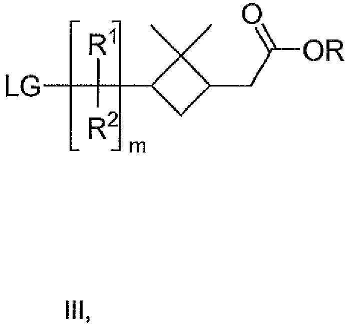 wherein m, R¹ and R² have the meaning given above, R represents a linear or branched C₁₋₅-alkyl radical and LG represents -O-S(=O)₂-CH₃, -O-S(=O)₂-p-toluyl, -O-S(=O)₂-CF₃, Cl or Br, which is optionally purified and/or isolated, and at least one compound of general formula III is reacted with at least one compound of general formula HNR⁶R⁷, wherein R⁶ and R⁷ have the meaning given above, preferably in a reaction medium selected from the group consisting of dimethylformamide, acetonitrile, dichloromethane and tetrahydrofuran or a mixture thereof, more preferably in tetrahydrofuran, preferably in the presence of at least one base selected from the group consisting of pyridine, triethylamine, diisopropylamine, diisopropylethylamine, 4-methylmorpholine and morpholine, to yield at least one compound of general formula IV, 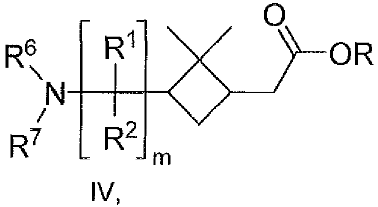 wherein m, R¹, R², R⁶ and R⁷ have the meaning given above and R represents a linear or branched C₁₋₅-alkyl radical, which is optionally purified and/or isolated,
and at least one compound of general formula IV is reacted with at least one reducing agent selected from the group consisting of lithium borohydride, sodium borohydride, lithium aluminium hydride and diborane, preferably with lithium borohydride, preferably in a reaction medium selected from the group consisting of methanol, ethanol, hexane and tetrahydrofuran or a mixture thereof, more preferably in methanol and/or tetrahydrofuran, to yield at least one compound of general formula V, 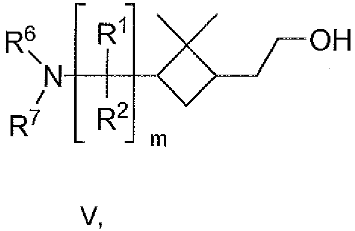 wherein m, R¹, R², R⁶ and R⁷ have the meaning given above, which is optionally purified and/or isolated,
and at least one compound of general formula V is reacted with methane sulfonylchloride, *p*-toluene sulfonylchloride, trifluormethane sulfonylchloride, thionyl chloride or tetrabromethane, preferably in a reaction medium preferably selected from the group consisting of tetrahydrofurane, diethylether, tert-butylmethylether and dichloromethane or a mixture thereof, more preferably in dichloromethane, preferably in the presence of at least one base selected from the group consisting of pyridine, triethylamine, diisopropylamine, diisopropylethylamine, 4-methylmorpholine and morpholine, to yield at least one compound of general formula VI, 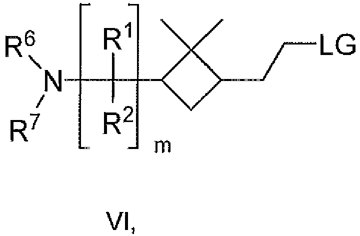 wherein m, R¹, R², R⁶ and R⁷ have the meaning given above and LG represents -O-S(=O)₂-CH₃, -O-S(=O)₂-p-toluyl, -O-S(=O)₂-CF₃, Cl or Br, which is optionally purified and/or isolated, and at least one compound of general formula VI is reacted with at least one compound of general formula HNR⁹R¹⁰, wherein R⁹ and R¹⁰ have the meaning given above, preferably in a reaction medium selected from the group consisting of dimethylformamide, acetonitrile, dichloromethane and tetrahydrofuran or a mixture thereof, preferably in the presence of at least one base selected from the group consisting of pyridine, triethylamine, diisopropylamine, diisopropylethylamine, 4-methylmorpholine and morpholine, to yield at least one compound of general formula VII, 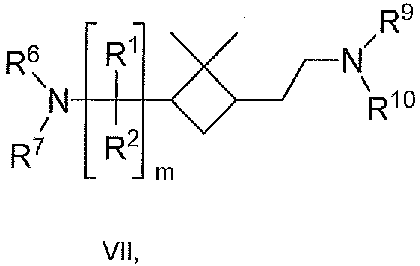 wherein m, R¹, R², R⁶, R⁷, R⁹ and R¹⁰ have the meaning given above, which is optionally purified and/or isolated,
or at least one compound of general formula VI is reacted with at least one compound of general formula M-OR⁸, wherein R⁸ has the meaning given above and M represents a monovalent kation selected from the group consisting of sodium, magnesium, potassium and lithium, preferably M represents a lithium kation, preferably in a reaction medium selected from the group consisting of dimethylformamide, dichloromethane and tetrahydrofuran or a mixture thereof, more preferably in tetrahydrofuran, preferably in the presence of at least one base selected from the group consisting of pyridine, triethylamine, diisopropylamine, diisopropylethylamine, 4-methylmorpholine and morpholine, to yield at least one compound of general formula VIII, 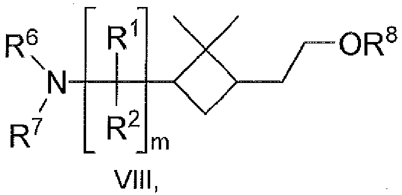 wherein m, R¹, R², R⁶, R⁷ and R⁸ have the meaning given above, which is optionally purified and/or isolated;
and optionally at least one compound of general formula VIII, wherein R⁸ represents hydrogen, is reacted with at least one compound of general formula LG-C(=O)-R¹², wherein R¹² has the above defined meaning and LG represents a leaving group, preferably a leaving group selected from the group consisting of chlorine and bromine, preferably in a reaction medium, preferably in the presence of at least one base selected from the group consisting of pyridine, triethylamine, diisopropylamine, diisopropylethylamine, 4-methylmorpholine and morpholine,
or with at least one compound of general formula HO-C(=O)-R¹², wherein R¹² has the above defined meaning, preferably in a reaction medium, preferably in the presence of at least one base selected from the group consisting of pyridine, triethylamine, diisopropylamine, diisopropylethylamine, 4-methylmorpholine and morpholine, preferably in the presence of at least one coupling agent,
to yield at least one compound of general formula VIIIa, 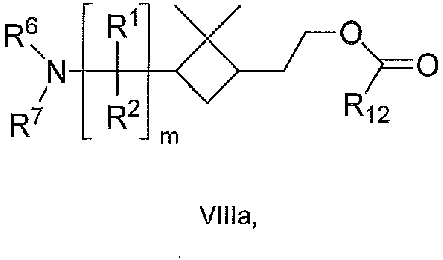 wherein m, R¹, R², R⁶, R⁷ and R¹² have the meaning given above, which is optionally purified and/or isolated.

In still another aspect the present invention relates to a process for the preparation of a substituted dimethylcyclobutyl compound of general formula I, wherein at least one compound of general formula II, 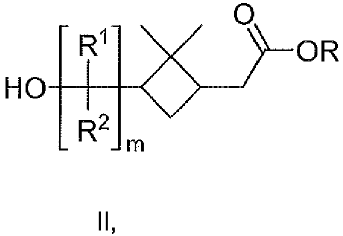 wherein m, R¹ and R² have the meaning given above and R represents a linear or branched C₁₋₅-alkyl radical, is reacted with at least one compound of general formula R⁵-LG, wherein R⁵ has the meaning given above and LG represents a leaving group, preferably LG represents a leaving group selected from the group consisting of chlorine, bromine, -O-S(=O)₂-CH₃, -O-S(=O)₂-CF₃ and -O-S(=O)₂-*p*-toluyl, more preferably LG represents bromine, preferably in a reaction medium selected from the group consisting of tetrahydrofuran, diethylether, tert-butylmethylether and dichloromethane or a mixture thereof, more preferably in dichloromethane, preferably in the presence of at least one base, more preferably in the presence of at least one base selected from the group consisting of sodium hydride, butyllithium, sodium ethoxide, potassium tert-butoxide and potassium hydride, to yield at least one compound of general formula IX, 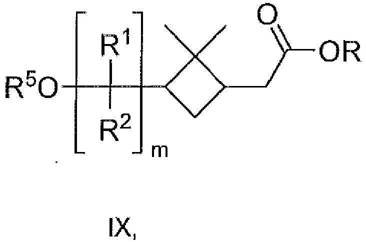 wherein m, R¹, R² and R⁵ have the meaning given above and R represents a linear or branched C₁₋₅-alkyl radical, which is optionally purified and/or isolated,
and at least one compound of general formula IX is reacted with at least one reducing agent selected from the group consisting of lithium borohydride, lithium aluminium hydride and diborane, sodium borohydride, preferably with lithium borohydride, preferably in a reaction medium selected from the group consisting of methanol, ethanol, hexane and tetrahydrofuran or a mixture thereof, more preferably in methanol and/or tetrahydrofuran,, to yield at least one compound of general formula X, 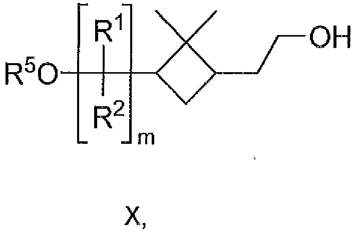 wherein m, R¹, R² and R⁵ have the meaning given above, which is optionally purified and/or isolated,
and optionally at least one compound of general formula X, is reacted with at least one compound of general formula LG-C(=O)-R¹², wherein R¹² has the meaning given above and LG represents a leaving group, preferably a leaving group selected from the group consisting of chlorine and bromine, preferably in a reaction medium, preferably in the presence of at least one base selected from the group consisting of pyridine, triethylamine, diisopropylamine, diisopropylethylamine, 4-methylmorpholine and morpholine,
or with at least one compound of general formula HO-C(=O)-R¹², wherein R¹² has the meaning given above, preferably in a reaction medium, preferably in the presence of at least one base, preferably in the presence of at least one coupling agent,
to yield at least one compound of general formula Xa, 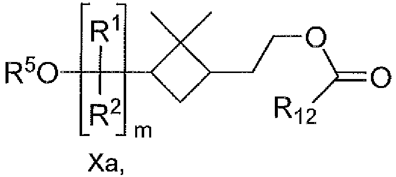 wherein m, R¹, R² and R⁵ have the meaning given above, which is optionally purified and/or isolated,
and at least one compound of general formula X is reacted with methane sulfonylchloride, *p*-toluene sulfonylchloride, trifluormethane sulfonylchloride, thionyl chloride or tetrabromethane, preferably in a reaction medium selected from the group consisting of diethylether, tetrahydrofuran, tert-butylmethylether and dichloromethane or a mixture thereof, more preferably in dichloromethane, preferably in the presence of at least one base selected from the group consisting of pyridine, triethylamine, diisopropylamine, diisopropylethylamine, 4-methylmorpholine and morpholine, to yield at least one compound of general formula XI, 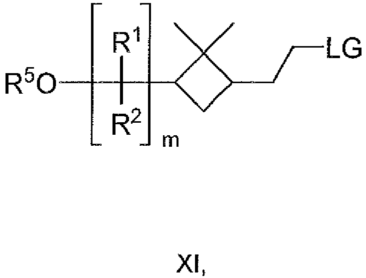 wherein m, R¹, R² and R⁵ have the meaning given above and LG represents -O-S(=O)₂-CH₃, -O-S(=O)₂-p-toluyl, -O-S(=O)₂-CF₃, Cl or Br, which is optionally purified and/or isolated,
and at least one compound of general formula XI is reacted with at least one compound of general formula HNR⁹R¹⁰, wherein R⁹ and R¹⁰ have the meaning given above, preferably in a reaction medium selected from the group consisting of dimethylformamide, acetonitrile, dichloromethane and tetrahydrofuran or a mixture thereof, preferably in the presence of at least one base selected from the group consisting of pyridine, triethylamine, diisopropylamine, diisopropylethylamine, 4-methylmorpholine and morpholine, to yield at least one compound of general formula XII, 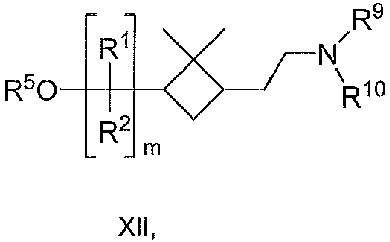 wherein m, R¹, R², R⁵, R⁹ and R¹⁰ have the meaning given above, which is optionally purified and/or isolated.

In still another aspect the present invention relates to a process for the preparation of a substituted dimethylcyclobutyl compound of general formula I, wherein at least one compound of general formula XIII, 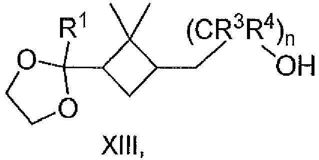 wherein R¹, R³ and R⁴ have the meaning given above, is reacted with methane sulfonylchloride, *p*-toluene sulfonylchloride, trifluormethane sulfonylchloride, thionyl chloride or tetrabromethane, preferably in a reaction medium selected from the group consisting of diethylether, tetrahydrofuran, tert-butylmethylether and dichloromethane or a mixture thereof, more preferably in dichloromethane, preferably in the presence of at least one base selected from the group consisting of pyridine, triethylamine, diisopropylamine, diisopropylethylamine, 4-methylmorpholine and morpholine, to yield at least one compound of general formula XIV, 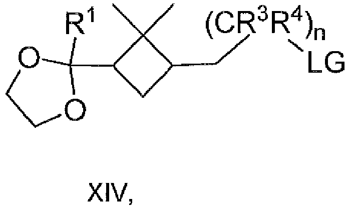 wherein R¹, R³ and R⁴ have the meaning given above and LG represents - O-S(=O)₂-CH₃, -O-S(=O)₂-p-toluyl, -O-S(=O)₂-CF₃, Cl or Br, which is optionally purified and/or isolated,
and at least one compound of general formula XIV is reacted with at least one compound of general formula HNR⁹R¹⁰, wherein R⁹ and R¹⁰ have the meaning given above, preferably in a reaction medium selected from the group consisting of dimethylformamide, acetonitrile, dichloromethane and tetrahydrofuran or a mixture thereof, preferably in the presence of at least one base selected from the group consisting of pyridine, triethylamine, diisopropylamine, diisopropylethylamine, 4-methylmorpholine and morpholine, to yield at least one compound of general formula XV, 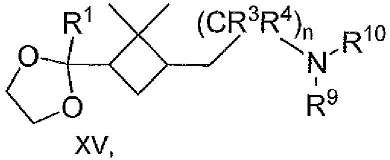 wherein R¹, R³, R⁴, R⁹ and R¹⁰ have the meaning given above, which is optionally purified and/or isolated,
and at least one compound of general formula XV is reacted with at least one reagent selected from the group consisting of hydrochloric acid, pyridinium p-toluenesulfonate, sulfonic acid and trifluoroacetic acid, preferably with pyridinium *p*-toluenesulfonate, preferably in a reaction medium selected from the group consisting of acetone and water or a mixture thereof, to yield at least one compound of general formula XVI, 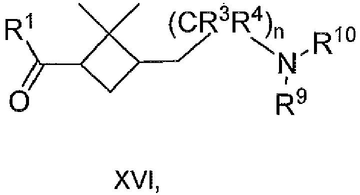 wherein R¹, R³, R⁴, R⁹ and R¹⁰ have the meaning given above, which is optionally purified and/or isolated,
and at least one compound of general formula XVI is reacted with at least one compound of general formula R²-Li, wherein R² has the meaning given above, or R²-Mg-Z, wherein R² has the meaning given above, and Z represents an anion selected from the group consisting of bromide and chloride, preferably in a reaction medium, selected from the group consisting of ether and tetrahydrofurane, to yield at least one compound of general formula XVII, 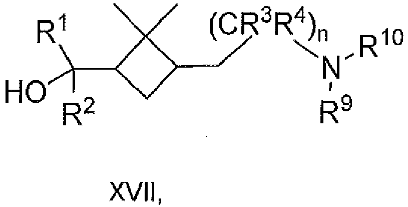 wherein R¹, R², R³, R⁴, R⁹ and R¹⁰ have the meaning given above, which is optionally purified and/or isolated,
and optionally at least one compound of general formula XVII is reacted with at least one compound of general formula R⁵-LG, wherein R⁵ has the meaning given above and LG represents a leaving group, preferably LG represents a leaving group selected from the group consisting of chlorine, bromine, -O-S(=O)₂-CH₃, -O-S(=O)₂-CF₃ and -O-S(=O)₂-*p*-toluyl, more preferably LG represents bromine, preferably in a reaction medium selected from the group consisting of tetrahydrofuran, diethylether, tert-butylmethylether and dichloromethane or a mixture thereof, more preferably in dichloromethane, preferably in the presence of at least one base, more preferably in the presence of at least one base selected from the group consisting of sodium hydride, butyllithium, sodium ethoxide, potassium tert-butoxide and potassium hydride, to yield at least one compound of general formula XVIII, 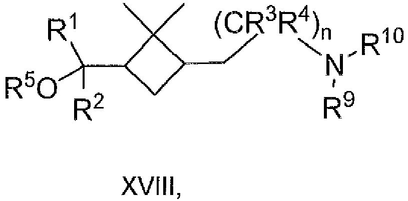 wherein R¹, R², R³, R⁴, R⁵, R⁹ and R¹⁰ have the meaning given above, which is optionally purified and/or isolated.

In still another aspect the present invention relates to a process for the preparation of a compound of genera formula I, characterized in that at least one compound of general formula IXX, 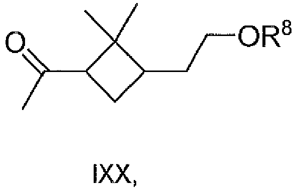 wherein R⁸ has the meaninggiven above, is reacted with NaOBr, preferably in a reaction medium, more preferably in a reaction medium selected from the group consisting of dioxane, THF and water or a mixture thereof, to yield at least one compound of general formula XX, 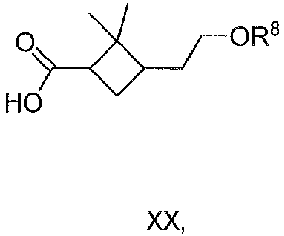 wherein R⁸ has the meaning gieven above, which is optionally purified and/or isolated,
and at least one compound of general formula XX is reacted with methyl iodide, preferably in a reaction medium, preferably in the presence of at least one base, more preferably in the presence of caesium carbonate, to yield at least one compound of general formula XXI, 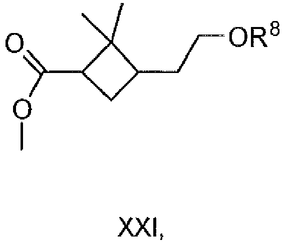 wherein R⁸ has the meaning given above, which is optionally purified and/or isolated,
and at least one compound of general formula XXI is reacted with at least one reducing agent selected from the group consisting of lithium borohydride, sodium borohydride, lithium aluminium hydride and diborane, preferably with lithium borohydride, preferably in a reaction medium, to yield at least one compound of general formula XXII, 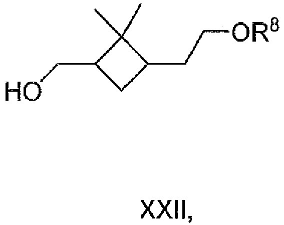 wherein R⁸ has the meaning given above, which is optionally purified and/or isolated.

The compounds of general formulae I, la, Ib, Ic, Id, Ie, If, Ig, Ih, Ij, Ik, Im, In, Io and Ip given above may be purified and/or isolated according to methods well known to those skilled in the art. Preferably, the compounds of general formulae I, la, Ib, Ic, Id, Ie, If, Ig, Ih, Ij, Ik, Im, In, Io and Ip may be isolated by evaporating the reaction medium, addition of water and adjusting the pH value to obtain the compound in form of a solid that can be isolated by filtration, or by extraction with a solvent that is not miscible with water such as chloroform and purification by chromatography or recrystallisation from a suitable solvent.

The compounds of general formulae HNR⁶R⁷, HNR⁹R¹⁰, MOR⁸, R⁵-LG, R²-Li and R²-Mg-Z are commercially available or may also be prepared according to standard methods known in the prior art.

During some synthetic reactions described above or while preparing the compounds of general formulae I, la, Ib, Ic, Id, Ie, If, Ig, Ih, Ij, Ik, Im, In, Io and Ip the protection of sensitive or reactive groups may be necessary and/or desirable. This can be performed by using conventional protective groups like those described in Protective groups in Organic Chemistry, ed. J. F. W. McOmie, Plenum Press, 1973; T.W. Greene & P.G.M. Wuts and Protective Groups in Organic Chemistrv. John Wilev & sons. 1993. 3rd edition. The protective groups may be eliminated when convenient by means well-known to those skilled in the art.

If the substituted dimethylcyclobutyl compounds of general formulae I, la, Ib, Ic, Id, Ie, If, Ig, Ih, Ij, Ik, Im, In, Io and Ip are obtained in form of a mixture of stereoisomers, particularly enantiomers or diastereomers, said mixtures may be separated by standard procedures known to those skilled in the art, e.g. chromatographic methods or crystallization with chiral reagents.

The substituted dimethylcyclobutyl compounds of general formulae I, la, Ib, Ic, Id, Ie, If, Ig, Ih, Ij, Ik, Im, In, Io and Ip and in each case stereoisomers thereof may be obtained in form of a corresponding salt according to methods well known to those skilled in the art, e.g. by reacting said compound with at least one inorganic and/or organic acid, preferably in a suitable reaction medium. Suitable reaction media include, for example, any of the ones given above. Suitable inorganic acids include but are not limited to hydrochloric acid, hydrobromic acid, phosphoric acid, oxalic acid, sulfuric acid, nitric acid, suitable organic acids include but are not limited to citric acid, maleic acid, fumaric acid, tartaric acid, or derivatives thereof, p-toluenesulfonic acid, methanesulfonic acid or camphersulfonic acid.

The term "salt" is to be understood as meaning any form of the substituted dimethylcyclobutyl compounds in which they assume an ionic form or are charged and are coupled with a counter-ion (a cation or anion) or are in solution. By this are also to be understood complexes of the active compound with other molecules and ions, in particular complexes which are complexed via ionic interactions.

The term "physiologically acceptable salt" is understood in particular, in the context of this invention, as salt (as defined above) formed either with a physiologically tolerated acid, that is to say salts of the particular active compound with inorganic or organic acids which are physiologically tolerated - especially if used on humans and/or mammals - or with at least one, preferably inorganic, cation which are physiologically tolerated - especially if used on humans and/or mammals. Examples of physiologically tolerated salts of particular acids are salts of: hydrochloric acid, hydrobromic acid, sulfuric acid, hydrobromide, monohydrobromide, monohydrochloride or hydrochloride, methiodide, methanesulfonic acid, formic acid, acetic acid, oxalic acid, succinic acid, malic acid, tartaric acid, mandelic acid, fumaric acid, lactic acid, citric acid, glutamic acid, hippuric acid picric acid and/or aspartic acid. Examples of physiologically tolerated salts of particular bases are salts of alkali metals and alkaline earth metals and with NH₄.

Solvates, preferably hydrates, of the substituted dimethylcyclobutyl compounds of general formulae I, la, Ib, Ic, Id, Ie, If, Ig, Ih, Ij, Ik, Im, In, Io and Ip and in each case of corresponding stereoisomers may also be obtained by standard procedures known to those skilled in the art.

The term "solvate" according to this invention is to be understood as meaning any form of the substituted dimethylcyclobutyl compounds in which they have attached to it via non-covalent binding another molecule (most likely a polar solvent) especially including hydrates and alcoholates, e.g. ethanolate.

A further aspect of the present invention relates to a medicament comprising at least one substituted dimethylcyclobutyl compound of general formulae I, la, Ib, Ic, Id, Ie, If, Ig, Ih, Ij, Ik, Im, In, Io and Ip given above, optionally in form of one of its stereoisomers, preferably enantiomers or diasteromers, a racemate or in form of a mixture of at least two stereoisomers, preferably enantiomers and/or diastereomers, in any mixing ratio, or a physiologically acceptable salt thereof, or a corresponding solvate thereof, and optionally at least one physiologically acceptable auxiliary agent.
Said medicament is particularly suitable for sigma receptor regulation, preferably for sigma-1 receptor regulation, and can therefore be used for the prophylaxis and/or treatment of a disorder or a disease that is least partially mediated via sigma receptors, preferably sigma-1 receptors.

Preferably said medicament is suitable for the prophylaxis and/or treatment of a disorder or disease related to food intake, preferably for the regulation of appetite, for the maintenance, increase or reduction of body weight, for the prophylaxis and/or treatment of obesity, bulimia, anorexia, cachexia, type II diabetes (non insulin dependent diabetes mellitus), preferably type II diabetes that is caused by obesity; for the prophylaxis and/or treatment of diarrhoea; lipoprotein disorders, preferably selected from the group consisting of hypercholesterolemia (type II hyperlipoproteinemia); hypertriglyceridemia; hypoalphalipoproteinemia and high lipoprotein(a) levels; arthritis; hypertension; arrhythmia; ulcer; tardive dyskinesia; stress; cancer; stroke; ischemic stroke; migraine; epilepsy; anxiety; panic attacks; depression; cognitive disorders; cognitive dysfunction associated with psychiatric diseases; memory disorders; psychosis; schizophrenia; for the prophylaxis and/or treatment of drug addiction and/or withdrawal or for the prophylaxis and/or treatment of cocaine addiction and/or withdrawal.

Also preferably said medicament is suitable for the prophylaxis and/or treatment of pain, preferably neuropathic pain, allodynia, analgesia, causalgia, central pain, dysesthesia, hyperesthesia, hyperalgesia, hypoalgesia, hypoesthesia, or neuralgia, more preferably neuropathic pain, hyperalgesia or allodynia.

In another aspect the present invention relates to the use of at least one substituted dimethylcyclobutyl compound of general formulae I, Ia, Ib, Ic, Id, Ie, If, Ig, Ih, Ij, Ik, Im, In, Io and Ip given above, optionally in form of one of their stereoisomers, preferably enantiomers or diasteromers, a racemate or in form of a mixture of at least two stereoisomers, preferably enantiomers and/or diastereomers, in any mixing ratio, or a physiologically acceptable salt thereof, or a corresponding solvate thereof, for the preparation of a medicament suitable for sigma receptor regulation, preferably for sigma-1 receptor regulation and/or preferably for the prophylaxis and/or treatment of a disorder or a disease that is least partially mediated via sigma receptors, preferably sigma-1 receptors.

The use of at least one substituted dimethylcyclobutyl compound of general formulae I, Ia, Ib, Ic, Id, Ie, If, Ig, Ih, Ij, Ik, Im, In, Io and Ip given above, optionally in form of one of its stereoisomers, preferably enantiomers or diasteromers, a racemate or in form of a mixture of at least two stereoisomers, preferably enantiomers and/or diastereomers, in any mixing ratio, or a physiologically acceptable salt thereof, or a corresponding solvate thereof, for the manufacture of a medicament for the prophylaxis and/or treatment of a disorder or disease related to food intake, preferably for the regulation of appetite, for the maintenance, increase or reduction of body weight, for the prophylaxis and/or treatment of obesity, bulimia, anorexia, cachexia, type II diabetes (non insulin dependent diabetes mellitus), preferably type II diabetes that is caused by obesity; for the prophylaxis and/or treatment of diarrhoea; lipoprotein disorders, preferably selected from the group consisting of hypercholesterolemia (type II hyperlipoproteinemia); hypertriglyceridemia; hypoalphalipoproteinemia and high lipoprotein(a) levels; arthritis; hypertension; arrhythmia; ulcer; tardive dyskinesia; stress; cancer; stroke; ischemic stroke; migraine; epilepsy; anxiety; panic attacks; depression; cognitive disorders; cognitive dysfunction associated with psychiatric diseases; memory disorders; psychosis; schizophrenia; for the prophylaxis and/or treatment of drug addiction and/or withdrawal or for the prophylaxis and/or treatment of cocaine addiction and/or withdrawal is preferred.

The use of at least one substituted dimethylcyclobutyl compound of general formulae I, la, Ib, Ic, Id, Ie, If, Ig, Ih, Ij, Ik, Im, In, Io and Ip given above, optionally in form of one of its stereoisomers, preferably enantiomers or diasteromers, a racemate or in form of a mixture of at least two stereoisomers, preferably enantiomers and/or diastereomers, in any mixing ratio, or a physiologically acceptable salt thereof, or a corresponding solvate thereof, for the manufacture of a medicament for the prophylaxis and/or treatment of pain, preferably neuropathic pain, allodynia, analgesia, causalgia, central pain, dysesthesia, hyperesthesia, hyperalgesia, hypoalgesia, hypoesthesia, or neuralgia, more preferably neuropathic pain, hyperalgesia or allodynia, is preferred.

The use of at least one substituted dimethylcyclobutyl compound of general formulae I, la, Ib, Ic, Id, Ie, If, Ig, Ih, Ij, Ik, Im, In, Io and Ip given above, optionally in form of one of its stereoisomers, preferably enantiomers or diasteromers, a racemate or in form of a mixture of at least two stereoisomers, preferably enantiomers and/or diastereomers, in any mixing ratio, or a physiologically acceptable salt thereof, or a corresponding solvate thereof, as pharmacological tool or as anxiolytic or as immunosuppressant is preferred.

Any medicament according to the present invention may be in any form suitable for the application to humans and/or animals, preferably humans including infants, children and adults. The medicament can be produced by standard procedures known to those skilled in the art, e.g. from the table of contents of "Pharmaceutics: The Science of Dosage Forms", Second Edition, Aulton, M.E. (ED. Churchill Livingstone, Edinburgh (2002); "Encyclopedia of Pharmaceutical Technology", Second Edition, Swarbrick, J. and Boylan J.C. (Eds.), Marcel Dekker, Inc. New York (2002); "Modern Pharmaceutics", Fourth Edition, Banker G.S. and Rhodes C.T. (Eds.) Marcel Dekker, Inc. New York 2002 y "The Theory and Practice of Industrial Pharmacy", Lachman L., Lieberman H. And Kanig J. (Eds.), Lea & Febiger, Philadelphia (1986). The composition of the medicament may vary depending on the route of administration.

The medicament of the present invention may, for example, be administered parentally in combination with conventional injectable liquid carriers, such as water or suitable alcohols. Conventional pharmaceutical excipients for injection, such as stabilizing agents, solubilizing agents, and buffers, may be included in such injectable compositions. These medicaments may for example be injected intramuscularly, intraperitoneally, or intravenously. Medicaments according to the present invention may also be formulated into orally administrable compositions containing one or more physiologically compatible carriers or excipients, in solid or liquid form. These compositions may contain conventional ingredients such as binding agents, fillers, lubricants, and acceptable wetting agents. The compositions may take any convenient form, such as tablets, pellets, granules, capsules, lozenges, aqueous or oily solutions, suspensions, emulsions, or dry powdered forms suitable for reconstitution with water or other suitable liquid medium before use, for immediate or retarded release. The multiparticulate forms, such as pellets or granules, may e.g. be filled into a capsule, compressed into tablets or suspended in a suitable liquid.

Suitable controlled release formulations, materials and methods for their preparation are known from the prior art, e.g. from the table of contents of "Modified-Release Drug Delivery Technology", Rathbone, M.J. Hadgraft, J. and Roberts, M.S. (Eds.), Marcel Dekker, Inc., New York (2002); "Handbook of Pharmaceutical Controlled Release Technology", Wise, D.L. (Ed.), Marcel Dekker, Inc. New York, (2000); "Controlled Drug Delivery", Vol, I, Basic Concepts, Bruck, S.D. (Ed.), CRD Press Inc., Boca Raton (1983) y de Takada, K. and Yoshikawa, H., "Oral Drug Delivery", Encyclopedia of Controlled Drug Delivery, Mathiowitz, E. (Ed.), John Wiley & Sons, Inc., New York (1999), Vol. 2, 728-742; Fix, J., "Oral drug delivery, small intestine and colon", Encyclopedia of Controlled Drug Delivery, Mathiowitz, E. (Ed.), John Wiley & Sons, Inc., New York (1999), Vol. 2, 698-728.

Medicaments according to the present invention may also comprise an enteric coating, so that their dissolution is dependent on pH-value. Due to said coating the medicament can pass the stomach undissolved and the respective dimethylcyclobutyl compound is liberated in the intestinal tract. Preferably the enteric coating is soluble at a pH value of 5 to 7.5. Suitable materials and methods for the preparation are known from the prior art.

Typically, the medicaments according to the present invention may contain 1-60 % by weight of one or more substituted dimethylcyclobutyl compounds as defined herein and 40-99 % by weight of one or more auxiliary substances (additives).

The liquid oral forms for administration may also contain certain additives such as sweeteners, flavoring, preservatives, and emulsifying agents. Nonaqueous liquid compositions for oral administration may also be formulated, containing edible oils. Such liquid compositions may be conveniently encapsulated in e.g., gelatin capsules in a unit dosage amount.

The compositions of the present invention may also be administered topically or via a suppository.

The daily dosage for humans and animals may vary depending on factors that have their basis in the respective species or other factors, such as age, sex, weight or degree of illness and so forth. The daily dosage for humans may preferably be in the range from 1 to 2000 mg, preferably 1 to 1500 mg, more preferably 1 to 1000, even more preferably 1 to 500 mg, most preferably 1 to 100 mg of active substance to be administered during one or several intakes per day.

In the following methods for determining the pharmacological activity of the substituted dimethylcyclobutyl compounds are described.

### PHARMACOLOGICAL METHODS:

Some representative compounds of the invention were tested for their activity as sigma (sigma-1 and sigma-2) inhibitors. The following protocols were followed:

### Sigma-1 receptor binding

Brain membrane preparation and binding assays for the sigma-1 receptor were performed as described (DeHaven-Hudkins et al., Characterization of the binding of [3H]-(+)-pentazocine to recognition sites in guinea pig brain, Eur. J. Pharmacol. 227, 371-378) with some modifications.

In brief, guinea pig brains were homogenized in 10 vols. (w/v) of Tris-HCl 50 mM, 0.32 M sucrose, pH 7.4, with a Kinematica Polytron PT 3000 at 15000 r.p.m. for 30 s. The homogenate was centrifuged at 1000 g for 10 min at 4 °C and the supernatants collected and centrifuged again at 48000 g for 15 min at 4 °C. The pellet was resuspended in 10 volumes of Tris-HCl buffer (50 mM, pH 7.4), incubated at 37 °C for 30 min, and centrifuged at 48000 g for 20 min at 4 °C. Following this, the pellet was resuspended in fresh Tris-HCl buffer (50 mM, pH 7.4) and stored on ice until use.

Each assay tube contained 10 µL of [³H]-(+)-pentazocine (final concentration of 0.5 nM), 900 µL of the tissue suspension to a final assay volume of 1 mL and a final tissue concentration of approximately 30 mg tissue net weight/mL. Non-specific binding was defined by addition of a final concentration of 1 M haloperidol. All tubes were incubated at 37 °C for 150 min before termination of the reaction by rapid filtration over Schleicher & Schuell GF 3362 glass fibre filters [previously soaked in a solution of 0,5% polyethylenimine for at least 1 h]. Filters were then washed with four times with 4 mL of cold Tris-HCl buffer (50 mM, pH 7.4). Following addition of scintillation cocktail, the samples were allowed to equilibrate overnight. The amount of bound radioactivity was determined by liquid scintillation spectrometry using a Wallac Winspectral 1414 liquid scintillation counter. Protein concentrations were determined by the method of Lowry et al. (Protein measurement with the Folin phenol reagent, J. Biol. Chem, 193, 265).

### Sigma-2 receptor binding

Binding studies for sigma-2 receptor were performed as described (Radesca et al., Synthesis and Receptor Binding of Enantiomeric N-Substituted cis-N-[2-(3,4-Dichlorophenyl)ethyl]-2-(1-pyrrolidinyl)ciclohexylamines as High-Affinity Receptor Ligands, J. Med. Chem. 34, 3065-3074) with some modifications. In brief, brains from sigma receptor type I (sigma-1) knockout mice (Langa, F., Codony X., Tovar V., Lavado A., Giménez E., Cozar P., Cantero M., Dordal A., Hernández E., Pérez R., Monroy X., Zamanillo D., Guitart X., Montoliu L1., 2003, Generation and phenotypic analysis of sigma receptor type I (Sigma1) knockout mice, European Journal of Neuroscience, Vol. 18, 2188-2196) were homogenized in a volume of 10 mUg tissue net weight of ice-cold 10 mM Tris-HCl, pH 7.4, containing 320 mM sucrose (Tris-sucrose buffer) with a Potter-Elvehjem homogenizer (10 strokes at 500 r.p.m.) The homogenates were then centrifuged at 1000g for 10 min at 4 °C, and the supernatants were saved. The pellets were resuspended by vortexing in 2 mUg ice-cold Tris-sucrose buffer and centrifuged again at 1000 g for 10 min. The combined 1000g supernatants were centrifuged at 31000 g for 15 min at 4 °C. The pellets were resuspended by vortexing in 3 mL/g 10 mM Tris-HCl, pH 7.4, and the suspension was kept at 25 °C for 15 min. Following centrifugation at 31000 g for 15 min, the pellets were resuspended by gentle Potter Elvehjem homogenisation to a volume of 1.53 mL/g in 10 mM Tris-HCl pH 7.4.

The assay tubes contained 10 µL of [³H]-DTG (final concentration of 3 nM), 400 µL of the tissue suspension (5.3 mL/g in 50 mM Tris-HCl, pH 8.0) to a final assay volume of 0.5 mL. Non-specific binding was defined by addition of a final concentration of 1 M haloperidol. All tubes were incubated at 25 °C for 120 min before termination of the reaction by rapid filtration over Schleicher & Schuell GF 3362 glass fibre filters [previously soaked in a solution of 0,5% polyethylenimine for at least 1 h]. Filters were washed with three times with 5 mL volumes of cold Tris-HCl buffer (10 mM, pH 8.0). Following addition of scintillation cocktail samples were allowed to equilibrate overnight. The amount of bound radioactivity was determined by liquid scintillation spectrometry using a Wallac Winspectral 1414 liquid scintillation counter. Protein concentrations were determined by the method of Lowry et al. (Protein measurement with the Folin phenol reagent, J. Biol. Chem, 193, 265).

The present invention is illustrated below with the aid of examples.

### Examples

A compound of general formula II, wherein both R¹ and R² represent hydrogen and R represents tert-butyl, can be prepared starting from (-)-α-pinene as depicted in scheme 1. below.

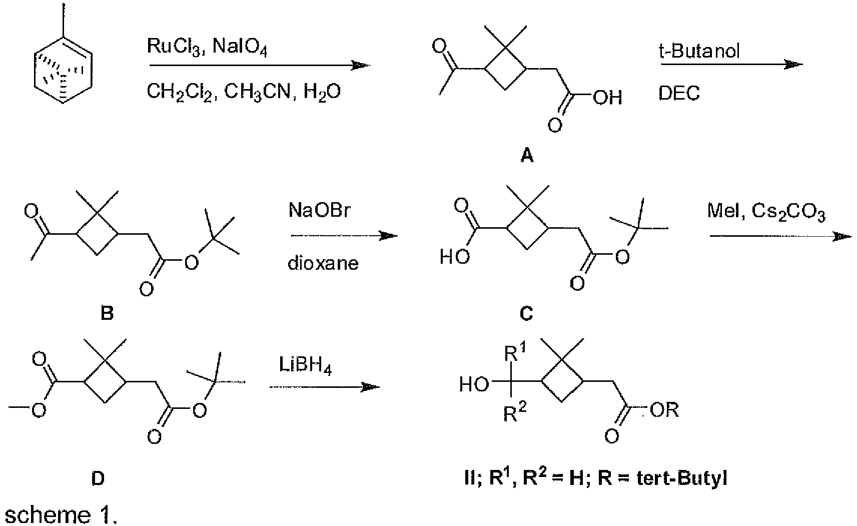

Exact reaction conditions for the each reaction step in scheme 1. are given in A. G. Moglioni et al. J. Org. Chem. 2000, 65, 3934-3940.

### Preparation of Compound C1:

### (1R,3R)-3-((tert-Butoxycarbonyl)methyl)-2,2-dimethylcyclobutanecarboxylic acid

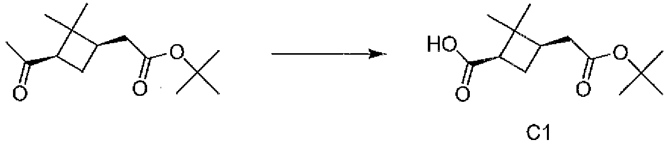

Aqueous NaOBr prepared from bromine (9.4 mL, 183.2 mmol), NaOH (28.4 g, 710.9 mmol) and water (237 mL) was added to a solution of tert-butyl 2-((1R,3R)-3-acetyl-2,2-dimethylcyclobutyl)acetate (5.5 g, 22.9 mmol) in 1,4-dioxane (138 mL) and water (38 mL). The reaction mixture was cooled to -5 °C and stirred at temperature between -5 and 0 °C for 5 h. The reaction mixture was washed with dichloromethane (4 x 200 mL). 40% aqueous NaHS₂O₃ was added to the basic aqueous layer at 0 °C until pH 5-6 was reached and the solution was acidified to pH 2 with conc. aqueous HCl. The acidic solution was extracted with dichloromethane (6x200 mL) and the organic extracts were dried over MgSO₄. The solvent was evaporated under reduced pressure to afford 4.9 g of (1*R*,3*R*)-3-((*tert*-butoxycarbonyl)methyl)-2,2-dimethylcyclobutanecarboxylic acid (90% yield), which was used in the next step without further purification.

### • ¹H-NMR (250 MHz, CDCl₃)

1.0 (s, 3H), 1.3 (s, 3H), 1.5 (s, 9H), 2.0 (m, 1 H), 2.1 (m, 1 H), 2.3 (m, 2H), 2.5 (m, 1H), 2.8 (dd, J=10 Hz, *J*=10.5 Hz, 1H).

Compound C2 (1*S*,3*S*)-3-((*tert*-Butoxycarbonyl)methyl)-2,2-dimethylcyclobutanecarboxylic acid was prepared analogous to compound C1.

### Preparation of Compound D1:

### Methyl (1R,3R)-3-((tert-butoxycarbonyl)methyl)-2,2-dimethylcyclobutane-carboxylate

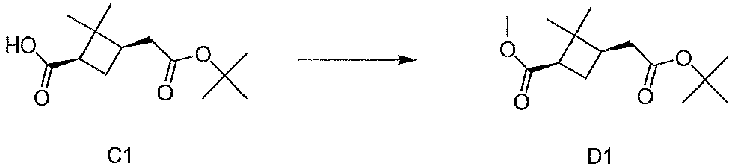

Cs₂CO₃ (7.9 g, 24.2 mmol) and Mel (1.5 mL, 24.2 mmol) were added to an ice-cooled solution of (1*R*,3*R*)-3-((*tert*-butoxycarbonyl)methyl)-2,2-dimethylcyclobutanecarboxylic acid (4.9 g, 20.2 mmol) in DMF (150 mL), and the reaction mixture was stirred at room temperature for 8 h. The reaction mixture was poured into EtOAc (200 mL) and the solution was washed with saturated aqueous NaHCO₃ (6 x 100 mL). The organic layer was dried over MgSO₄ and the solvents were removed under vacuo to afford 4.1 g of methyl (1R,3R)-3-((*tert*-butoxycarbonyl)methyl)-2,2-dimethylcyclobutanecarboxylate (80% yield), which was used in the next step without further purification.

### • ¹H-NMR (250 MHz, CDCl₃)

0.9 (s, 3H), 1.2 (s, 3H), 1.5 (s, 9H), 2.0 (m, 1H), 2.1 (m, 1H), 2.3 (m, 2H), 2.4 (m, 1H), 2.8 (dd, *J*=10.5 Hz, *J*=11 Hz, 1H), 3.7 (s, 3H).

Compound D2 Methyl (1*S*,3*S*)-3-((*tert*-butoxycarbonyl)methyl)-2,2-dimethylcyclobutanecarboxylate was prepared analogous to compound D1.

### Preparation of compounds of general formula II:

### tert-Butyl 2-((1R,3R)-3-(hydroxymethyl)-2,2-dimethylcyclobutyl)acetate

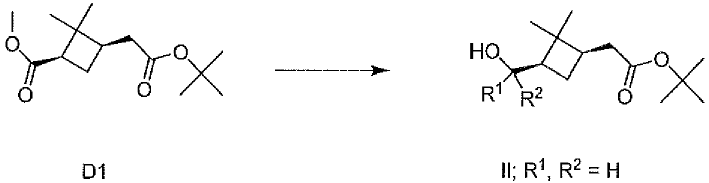

2 M LiBH₄ in THF (40 mL, 80.0 mmol) and dry MeOH (4 mL) was successively added to a solution of methyl (1R,3R)-3-((*tert-*butoxycarbonyl)methyl)-2,2-dimethylcyclobutanecarboxylic acid (4.1 g, 16.0 mmol) in anhydrous THF under nitrogen atmosphere. The mixture was allowed to stand for 15 min then heated to reflux for 12 h. Excess hydride was destroyed with MeOH and water, and the solution was extracted with CH₂Cl₂ (4x150 mL). The organic extracts were dried over MgSO₄ and the solvents were evaporated at reduced pressure to afford tert-butyl 2-((1R,3R)-3-(hydroxymethyl)-2,2-dimethylcyclobutyl)acetate (2.9 g, 79% yield), which was used in the next step without further purification.

### • ¹H-NMR (250 MHz, CDCl₃)

0.9 (s, 3H), 1.2 (s, 3H), 1.5 (s, 9H), 2.1 (m, 3H), 2.2 (m, 3H), 3.6 (m, 2H).

The compound *tert*-Butyl 2-((1*S*,3*S*)-3-(hydroxymethyl)-2,2-dimethylcyclobutyl)-acetate was prepared as described above.

### Preparation of compounds of general formula III:

### ((1R,3R)-3-((tert-Butoxycarbonyl)methyl)-2,2-dimethylcyclobutyl)methyl methanesulfonate

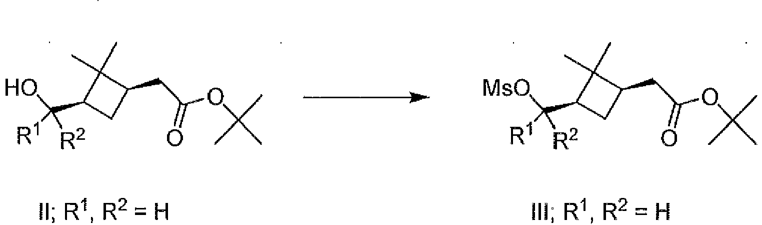

Triethylamine (1.5 mL, 10.4 mmol) and mesyl chloride (0.8 mL, 9.6 mmol) were added to an ice-cooled solution of tert-butyl 2-((1R,3R)-3-(hydroxymethyl)-2,2-dimethylcyclobutyl)acetate (1.7 g, 7.4 mmol) in anhydrous dichloromethane (70 mL) under nitrogen atmosphere. After stirring at 0 °C for 1 h, the reaction mixture was washed with saturated aqueous NaHCO₃ (4 x 50 mL) and dried over MgSO₄. The solvent was removed to afford ((1*R*,3*R*)-3-((*tert*-butoxycarbonyl)methyl)-2,2-dimethylcyclobutyl)methyl methanesulfonate (1.9 g, 83% yield) that was used in the next step without further purification.

### • ¹H-NMR (250 MHz, CDCl₃)

1.0 (s, 3H), 1.1 (s, 3H), 1.5 (s, 9H), 2.2 (m, 6H), 3.0 (s, 3H), 4.2 (m, 2H).

The compounds ((1*S*,3*S*)-3-((*tert*-Butoxycarbonyl)methyl)-2,2-dimethylcyclobutyl)methyl methanesulfonate and 2-((1*R*,3*R*)-2,2-Dimethyl-3-(2-methyl-1,3-dioxolan-2-yl)cyclobutyl)ethyl methanesulfonate (compound of general formula (XIV) were prepared as described above.

### Preparation of compounds of general formula IV:

### tert-Butyt 2-((1R,3R)-2,2-dimethyl-3-(morpholinomethyl)cyclobutyl)acetate

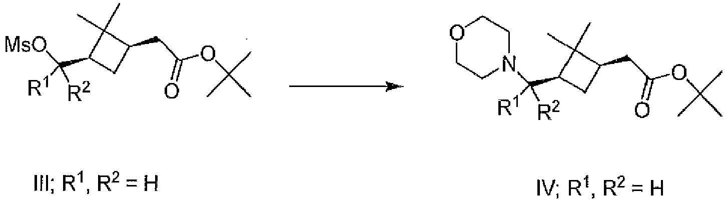

A mixture of ((1*R*,3*R*)-3-((*tert*-butoxycarbonyl)methyl)-2,2-dimethylcyclobutyl)methyl methanesulfonate (1 g, 1.5 mmol) and morpholine (25 mL, 287 mmol) were heated at 80 °C for 7 days under nitrogen atmosphere. Then the mixture was cooled, EtOAc (25 mL) was added and the resultant solution was subsequently washed with saturated sodium NaHCO₃ and dried over MgSO₄. Solvent was removed to provide tert-butyl 2-((1R,3R)-2,2-dimethyl-3-(morpholinomethyl)cyclobutyl)acetate (920 mg, 95% yield).
- ¹H-NMR (250 MHz, CDCl₃)
   0.9 (s, 3H), 1.1 (s, 3H), 1.5 (s, 9H), 2.2 (m, 5H), 2.4 (m, 7H), 3.7 (m, 4H).
   The following compounds of general formula IV were prepared as described above:
   *tert*-Butyl 2-((1*S*,3*S*)-2,2-dimethyl-3-(morpholinomethyl)cyclobutyl)acetate
   *tert*-Butyl 2-((1*R*,3*R*)-2,2-Dimethyl-3-((piperidin-1-yl)methyl)cyclobutyl)acetate
- ¹H-NMR (250 MHz, CDCl₃)
   0.9 (s, 3H), 1.1 (s, 3H), 1.4 (s, 9H), 1.6 (m, 6H), 2.1 (m, 8H), 2.3 (m, 4H).

### tert-Butyl 2-((1S,3S)-2,2-Dimethyl-3-((piperidin-1-yl)methyl)cyclobutyl)acetate

### Preparation of compounds of general formula V:

### 2-((1R,3R)-2,2-Dimethyl-3-(morpholinomethyl)cyclobutyl)ethanol

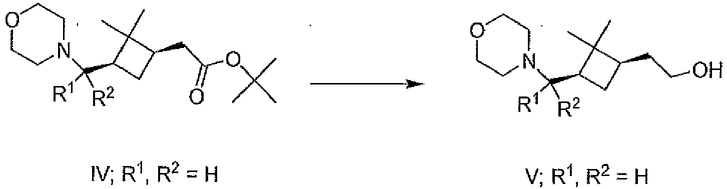

2 M LiBH₄ in THF (10 mL, 20.0 mmol) and dry methanol (1 mL) was successively added to a solution of tert-butyl 2-((1R,3R)-2,2-dimethyl-3-(morpholinomethyl)cyclobutyl)acetate (720 mg, 2.4 mmol) in anhydrous THF (20 mL) under nitrogen atmosphere. After 15 minutes standing, the mixture was heated to reflux for 24 h. Excess hydride was destroyed with methanol and the reaction mixture was poured into water. The resultant solution was extracted with CH₂Cl₂ (5x60 mL) and the organic extracts were dried over MgSO₄. Solvents were removed under reduced pressure to afford 700 g of a residue that was chromatographed on Baker^{®} silica gel using 1% TEA - 1:9 MeOH-CH₂Cl₂ as eluent to give pure 2-((1R,3R)-2,2-dimethyl-3-(morpholinomethyl)cyclobutyl)ethanol (410 mg, 75% yield).
- ¹H-NMR (250 MHz, CDCl₃)
   0.9 (s, 3H), 1.1 (s, 3H), 1.6 (m, 3H), 1.9 (m, 1 H), 2.2 (m, 3H), 2.4 (m, 5H), 3.6 (m, 2H), 3.7 (m, 4H).
- ¹³C-NMR (67.5 MHz, CDCl₃)
   16.4 (1C), 30.1 (1C), 30.5 (1C), 33.4 (1C), 39.8 (1C), 39.9 (1C), 40.2 (1C), 53.9 (2C), 60.0 (1C), 61.6 (1C), 67.0 (2C).
- MS (IFE-IT), m/z (%): 228.1 (M+1⁺,100)

The following products of general formulae and V and XVII were prepared as described above:

### 2-((1S,3S)-2,2-Dimethyl-3-(morpholinomethyl)cyclobutyl)ethanol [α]= - 23 (c=0.8, CH₂Cl₂)

### 2-((1R,3R)-2,2-Dimethyl-3-((piperidin-1-yl)methyl)cyclobutyl)ethanol

- ¹H-NMR (250 MHz, CDCl₃)
   0.9 (s, 3H), 1.1 (s, 3H), 1.5 (m, 10H), 1.9 (m, 1H), 2.1 (m, 2H), 2.4 (m, 5H), 3.6 (m, 2H).
- ¹³C-NMR (67.5 MHz, CDCl₃)
   16.4 (1C), 24.2 (1C), 25.8 (2C), 30.1 (1C), 31.3 (1C), 33.4 (1C), 40.0 (1C), 40.2 (1 C), 40.4 (1 C), 54.8 (2C), 60.2 (1C), 61.6 (1C).
- MS (IFE-IT), m/z (%): 226.1 (M+1⁺,100)
- [α]= + 16 (c=0.3, CH₂Cl₂)

### 2-((1S,3S)-2,2-Dimethyl-3-((piperidin-1-yl)methyl)cyclobutyl)ethanol • [α]= - 22 (c=1.2, CH₂Cl₂)

### 2-((1R,3R)-2,2-Dimethyl-3-((4-phenylpiperidin-1-yl)methyl)cyclobutyl)ethanol

- ¹H-NMR (250 MHz, CDCl₃)
   1.0 (s, 3H), 1.1 (s, 3H), 1.6 (m, 3H), 1.8 (m, 5H), 2.1 (m, 7H), 2.5 (dd, J=12 Hz, 1H), 3.0 (m, 1H), 3.6 (m, 2H), 7.3 (m, 5H).
- ¹³C-NMR (67.5 MHz, CDCl₃)
   16.4 (1C), 30.1 (1C), 31.2 (1C), 33.0 (1C), 33.2 (1C). 33.3 (1C), 39.9 (1C), 40.3 (1C). 40.7 (1C), 42.8 (1C), 54.3 (1C), 55.3 (1C), 60.0 (1C), 61.6 (1C), 126.2 (1C). 127.0 (2C), 128.3 (2C), 146.5 (1C).
- MS (IFE-IT), m/z (%): 302.2 (M+1⁺,100)
- [α]= +8 (*c*=1.3, CH₂Cl₂)

### 2-((1R,3R)-2,2-Dimethyl-3-(((2S,6R)-2,B-dimethyimorpholino)-methyl)cyclobutyl)ethanol

- ¹H-NMR (250 MHz, CDCl₃)
   1.1 (s, 3H), 1.2 (s, 3H), 1.3 (d, J= 4.5 Hz, 6H), 1.5 (m, 2H), 1.7 (m, 3H), 1.9 (m, 1H), 2.1 (m, 3H), 2.4 (dd, *J*=11.5 Hz, 1H), 2.7 (m, 2H), 3.6 (m, 2H), 3.7 (m, 2H).
- MS (IFE-IT), m/z (%): 256.2 (M+1⁺,100)

### 2-((1R,3R)-2,2-Dimethyl-3-((4-mothylpiperidin-1-yl)methyl)cyclobutyl)ethanol

- ¹H-NMR (250 MHz, CDCl₃)
   0.9 (s, 3H), 0.9 (d, *J*=5 Hz, 3H), 1.1 (s, 3H), 1.5 (m, 2H), 1.7 (m, 4H), 1.9 (m, 4H), 2.1 (m, 4H), 2.5 (dd, *J*=15 Hz, *J*=12.5 Hz, 1H), 2.9 (m, 2H), 3.6 (m, 2H).
- MS (IFE-IT), m/z (%): 240.1 (M+1⁺,100)

### 2-((1R,3R)-2,2-Dimethyl-3-(N-benzyl-N-methylamino)methyl)cyclobutyl)ethanol

- ¹H-NMR (250 MHz, CDCl₃)
   0.9 (s, 3H), 1.1 (s, 3H), 1.6 (m, 4H), 1.9 (m, 1H), 2.1 (m, 2H), 2.2 (s, 3H), 2.5 (m, 1 H), 3.4 (d, J= 11.2 Hz, 1 H), 3.5 (d, *J*= 11.2 Hz, 1H), 3.6 (m, 2H), 7.3 (m, 5H).
- MS (IFE-IT), m/z (%): 262.1 (M+1⁺,100)

### ((1R,3R)-(2-(4-Benzylpiperidin-1-yl)ethyl)-2,2-dimethylcyclobutyl)methanol

- ¹H-NMR (250 MHz, CDCl₃)
   0.94 (s, 3H), 1.1 (s, 3H), 1.6 (m, 9H), 2.0 (m, 4H), 2.3 (m, 2H), 2.5 (d, *J*=6.7 Hz, 2H), 3.0 (m, 2H), 3.5 (m, 2H), 7.2 (m, 5H).

### Preparation of compounds of general formula VI:

### 2-((1R,3R)-2,2-Dimethyl-3-(morpholinomethyl)cyclobutyl)ethyl methanesulfonate

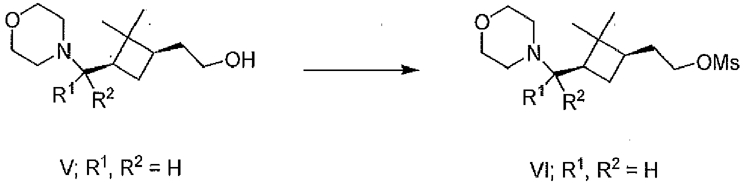

Triethylamine (1.2 mL, 8.7 mmol) and mesyl chloride 0.6 mL, 8.1 mmol) were added to an ice-cooled solution of alcohol 2-((1R,3R)-2,2-dimethyl-3-(morpholinomethyl)cyclobutyl}ethanol (1.4 g, 6.2 mmol) under nitrogen atmosphere. After 1 h stirring the reaction mixture was washed with saturated aqueous NaHCO₃ (4x50 mL), and the organic phase was dried over MgSO₄. The solvent was evaporated in vacuo to afford mesylate 2-((1*R*,3*R*)-2,2-Dimethyl-3-(morpholinomethyl)cyclobutyl)ethyl methanesulfonate (1.6 g, 85% yield) that was used in the next step without further purification.
- ¹H-NMR (250 MHz, CDCl₃)
   1.0 (s, 3H), 1.1 (s, 3H), 1.6 (m, 2H), 1.9 (m, 2H), 2.2 (m, 3H), 2.4 (m, 5H), 3.0 (s, 3H), 3.7 (m, 4H), 4.2 (m, 2H).

The following product of general formula VI was prepared as described above:

### 2-((1R,3R)-2,2-Dimethyl-3-((piperldin-1-yl)methyl)cyclobutyl)ethyl methanesulfonate

- ¹H-NMR (250 MHz, CDCl₃)
   0.9 (s, 3H), 1.1 (s, 3H), 1.4 (m, 2H), 1.6 (m, 6H), 1.8 (m, 2H), 2.1 (m, 3H), 2.4 (m, 5H), 3.0 (s, 3H), 4.2 (m, 2H).

### Preparation of example compound 46:

### 142-((1R,3R)-2,2-dimethyl-3-(morpholinomethyl)cyclobutyl)ethyl)-6,7-dihydro-1H-indol,4(5H)-one

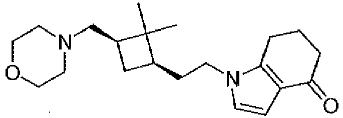

1.6 M BuLi in hexane (1.1 mL, 1.8 mmol) was added dropwise to an ice-cooled solution of 2-((1R,3R)-2,2-dimethyl-3-(morpholinomethyl)cyclobutyl)ethyl methanesulfonate (500 mg, 1.6 mmol) and 6,7-dihydro-1*H-*indol-4(5*H*)-one (245 mg, 1.8 mmol) in anhydrous DMF (45 mL) under nitrogen atmosphere. The light-protected mixture was heated to reflux for 9 days, then cooled to room temperature and diluted with EtOAc (60 mL). The resultant solution was washed with saturated aqueous NaHCO₃ and dried over MgSO₄. The solvents were removed at reduced pressure affording 750 mg of crude 1-(2-((1R,3R)-2,2-dimethyl-3-(morpholinomethyl)cyclobutyl)ethyl)-6,7-dihydro-1H-indol-4(5H)-one, which was chromatographed on Baker^{®} silica gel using 1% Triethylamine in 1:9 MeOH-CH₂Cl₂ as eluent to provide pure 1-(2-((1R,3R)-2,2-dimethyl-3-(morpholinomethyl)cyclobutyl)ethyl)-6,7-dihydro-1H-indol-4(5H)-one (325 mg, 58% yield).
- **¹H-NMR (250 MHz, CDCl₃)**
   1.0 (s, 3H), 1.1 (s, 3H), 1.6 (m, 2H), 1.8 (m, 2H), 2.1 (m, 2H), 2.2 (m, 3H), 2.5 (m, 7H), 2.7 (m, 2H), 3.7 (m, 6H), 6.6 (s, 2H).
- **¹³C-NMR (67.5 MHz, CDCl₃**)
   16.2 (1C), 21.6 (1C), 23.5 (1C), 29.8 (1C), 30.0 (1C), 31.4 (1C), 37.5 (1C), 39.4 (1C), 40.0 (1C), 40.2 (1C), 45.1 (1C), 53.7 (2C), 59.6 (1C), 66.7 (2C), 105.3 (1C), 120.5 (1C), 121.9 (1C), 142.9 (1C), 194.2 (1C).
- **MS (IFE-IT), m/z (%):** 345.2 (M+1⁺,100)

The following example compounds were prepared as described above:

### Example compound 53: 6,7-Dihydro-1-(2-((1R,3R)-2,2-dimethyl-3-((piperidin-1-yl)methyl)cyclobutyl)ethyl)-1H-indol-(5H)-one

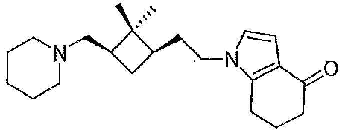

- **¹H-NMR (250 MHz, CDCl₃)**
   0.9 (s, 3H), 1.1 (s, 3H), 1.6 (m, 6H), 1.8 (m, 3H), 2.1 (m, 6H), 2.5 (m, 7H), 2.7 (m, 2H), 3.7 (m, 2H), 7.3 (s, 2H).
- **¹³C-NMR (67.5 MHz, CDCl₃)**
   16.5 (1C), 21.6 (1C), 22.7 (1C), 23.6 (2C), 23.7 (1C), 29.5 (1C), 30.9 (1C), 31.2 (1C), 37.6 (1C), 38.0 (1C), 40.6 (2C), 45.1 (1C), 53.8 (2C), 58.8 (1C), 105.4 (1C), 120.7 (1C), 121.9 (1C), 142.9 (1C), 194.0 (1C).
- **MS (IFE-IT), m/z (%):** 343.2 (M+1⁺,100)

### Example compound 42:4-(((1R,3R)-3-(2-(4-Benzylpiperidin-1-yl)ethyl)-2,2-dimethylcyclobutyl)methyl)morpholine

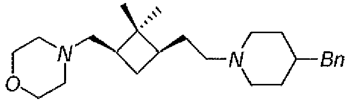

- ¹**H-NMR (250 MHz, CDCl₃)**
   0.9 (s, 3H), 1.1 (s, 3H), 1.3 (m, 4H), 1.6 (m, 3H), 1.8 (m, 3H), 2.1 (m, 6H), 2.4 (m, 5H), 2.5 (d, *J*= 7.8 Hz, 2H), 2.9 (m, 2H), 3.7 m, 4H), 7.2 (m, 3H), 7.3 (m, 2H).
- **MS (IFE-IT), m/z (%)**: 385.3 (M+1⁺,100)

### Example compound 52: 4-Benzyl-1-(2..((1R,3R)..2,2-dimethyl-3-((piperidin-1-yl)methyl)cyclobutyl)ethyl)piperidine

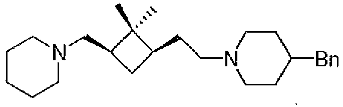

- **¹H-NMR (250 MHz, CDCl₃)**
   0.9 (s, 3H), 1.1 (s, 3H), 1.3 (m, 6H), 1.6 (m, 6H), 1.9 (m, 4H), 2.1 (m, 6H), 2.4 (m, 5H), 2.5 (d, J= 8 Hz, 2H), 2.9 (m, 2H), 7.2 (m, 3H), 7.3 (m, 2H).
- **MS (IFE-IT), m/z (%) :** 383.3 (M+1⁺,100)

### Example compound 41: 4-(((1R,3R)-2,2-Dimethyl-3-(2-(4-phenylpiperidin-1-yl)ethyl)cyclobutyl)methyl)morpholine

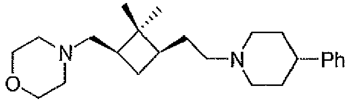

- **¹H-NMR (250 MHz, CDCl₃)**
   1.0 (s, 3H), 1.1 (s, 3H), 1.5 (m, 1H), 1.6 (m, 1 H), 1.8 (m, 6H), 2.1 (m, 7H), 2.4 (m, 7H), 3.1 (m, 1H), 3.7 (m, 4H), 7.3 (m, 5H).
- **MS (IFE-IT), m/z (%):** 371.3 (M+1⁺,100)

### Example compound 51: 1-(2-((1R,3R)-2,2-Dimethyl-3-((piperidin-1-yl)methyl)cyclobutyl)ethyl)-4-phenylpiperidine

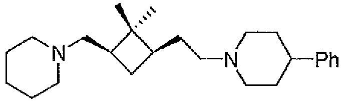

- **¹H-NMR (250 MHz, CDCl₃)**
   0.9 (s, 3H), 1.1 (s, 3H), 1.4 (m, 2H), 1.6 (m, 6H), 1.8 (m, 5H), 2.1 (m, 6H), 2.4 (m, 9H), 3.1 (m, 1H), 7.3 (m, 5H).
- **MS (IFE-IT), mlz (%):** 369.3 (M+1⁺,100)

### Example compound 40:4-(((1R,3R)-2,2-Dimethyl-(2-(3-phenylpiperidin-1-yl)ethyl)cyclobuty!)methyl)morpholine

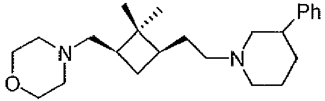

- **¹H-NMR (250 MHz, CDCl₃)**
   0.9 (s, 3H), 1.1 (s, 3H), 1.3 (m, 2H), 1.6 (m, 2H), 1.7 (m, 2H), 2.0 (m, 3H), 2.2 (m, 4H), 2.4 (m, 9H), 3.2 (m, 1H), 3.7 (m, 4H), 7.3 (m, 5H).
- **MS (IFE-IT), m/z (%):** 371.2 (M+1⁺,100)

### Example compound 43:4-(((1R,3R)-3-(2-(1H-Imidazol-1-yl)ethyl)-2,2-dimethylcyclohutyl)methyl)morpholine

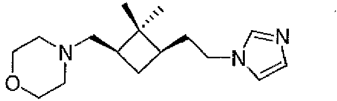

- **¹H-NMR (250 MHz, CDCl₃)**
   1.0 (s, 3H), 1.1 (s, 3H), 1.8 (m, 3H), 2.1 (m, 4H), 2.4 (m, 5H), 3.7 (m, 4H), 3.9 (m, 2H), 6.9 (s, 1H), 7.1 (s, 1 H), 7.5 (s, 1H).
- **MS (IFE-IT), m/z (%):** 278.1 (M+1⁺,100)

### Example compound 48: 1-(((1R,3R)-3-(2-(1H-midazol-1yl)ethyl)-2,2-dimethylcyclobutyl)methyl)piperidine

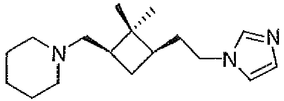

- **¹H-NMR (250 MHz, CDCl₃)**
   0.9 (s, 3H), 1.0 (s, 3H), 1.6 (m, 6H), 1.7 (m, 3H), 2.1 (m, 4H), 2.4 (m, 5H), 3.9 (m, 2H), 6.9 (s, 1H), 7.0 (s, 1H), 7.4 (s, 1H).
- **MS (IFE-IT), m/z (%):** 276.2 (M+1⁺,100)

### Example compound 44: 4-(((1R,3R)-3-(2-(1H-Pyrazol-1-yl)ethyl)-2,2-dimethylcyclobutyl)methyl)morpholine

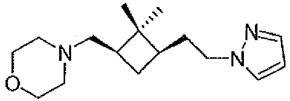

- **¹H-RMN (250 MHz, CDCl₃**)
   1.0 (s, 3H), 1.1 (s, 3H), 1.8 (m, 2H), 2.0 (m, 5H), 2.4 (m, 5H), 3.7 (m, 4H), 4.1 (m, 2H), 6.2 (t, *J*=2.5 Hz, 1H), 7.3 (d, *J*=2.5 Hz, 1H). 7.5 (d, *J*=2.5 Hz, 1H).
- **MS (IFE-IT), m/z (%):** 278.1 (M+1⁺,100)

### Example compound 49: -(((1R,3R)-3-(2-1H-Pyrazol-1-yl)ethyl)-2,2-dimethylcyclobutyl)methyl)piperidine

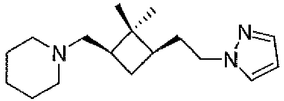

- **¹H-NMR (250 MHz, CDCl₃]**
   0.9 (s, 3H), 1.0 (s, 3H), 1.6 (m, 6H), 1.8 (m, 3H), 2.1 (m, 4H), 2.3 (m, 5H), 4.0 (m, 2H), 6.2 (t, *J*= 2 Hz, 1H), 7.3 (d, *J=* 2.8 Hz, 1H), 7.5 (d, *J=* 1.6 Hz, 1H).
- **MS (IFE-IT], m/z (%):** 276.2 (M+1⁺,100)

### Example compound 45:4-(((1R,3R)-3-(2-(1H-1,2,4-Triazol-1-yl)ethyl)-2,2-dimethylcyclobutylymethylymorpholine

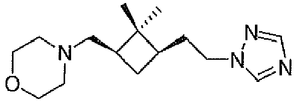

- **¹H-NMR (250 MHz, CDCl₃)**
   1.0 (s, 3H), 1.1 (s, 3H), 1.8 (m, 3H), 2.1 (m, 4H), 2.4 (m, 5H), 3.9 (m, 4H), 4.1 (m, 2H), 7.9 (s, 1 H), 8.0 (s. 1H).
- **MS (IFE-IT), m/z (%):** 279.1 (M+1⁺,100)

### Example compound 50: 1-(((1R,3R)-3-(2-(1H-1,2,4-Triazol-1-yl)ethyl)-2,2-dimethylcyclobutyl)methyl)piperidine

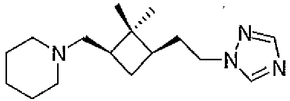

- **¹H-NMR (250 MHz, CDCl₃)**
   0.9 (s, 3H), 1.1 (s, 3H), 1.6 (m, 6H), 1.8 (m, 3H), 2.1 (m, 4H), 2.5 (m, 5H), 4.0 (m, 2H), 8.0 (s, 1H), 8.1 (s, 1H).
- **MS (IFE-IT), m/z (%):** 277.2 (M+1⁺,100)

### Example compound 47:1,2,3,4-Tetrahydro-2-(2-((1R,3R)-2,2-dimethyl-3-(morpholinomethyl)cyclobutyl)ethyl) isoquinoline

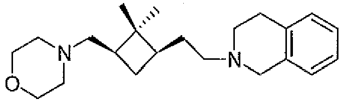

- **¹H-RMN (250 MHz, CDCl₃)**
   1.0 (s, 3H), 1.1 (s, 3H), 1.5 (m, 2H), 1.6 (m, 1H), 1.8 (m, 1 H), 2.2 (m, 3H), 2.5 (m, 7H), 2.8 (m, 2H), 3.0 (m, 2H), 3.7 (s, 2H), 3.7 (m, 4H), 7.0 (m, 1H), 7.1 (m, 3H).
- **MS (IFE-IT), m/z (%):** 343.3 (M+1⁺,100)

### Example compound 54: 1,2,3,4-Tetrahydro-2-(2-((1R,3R)-2,2-dimethyl-3-((piperidin-1-yl)methyl)cyclobutyl)ethyl)isoquinoline

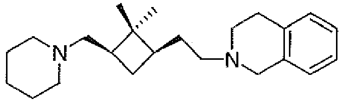

- **¹H-RMN (250 MHz, CDCl₃)**
   0.9 (s, 3H), 1.1 (s. 3H), 1.5 (m, 2H), 1.7 (m, 6H), 1.9 (m, 1H), 2.2 (m, 4H), 2.4 (m, 6H), 2.7 (m, 2H), 2.8 (s, 1H), 2.9 (m, 2H), 3.6 (m, 2H), 7.0 (m, 1H), 7.1 (m, 3H).
- **MS (IFE-IT), m/z (%):** 341.3 (M+1⁺,100)

### Preparation of example compound 15:

### 4-(((1R,3R)-2,2-Dimethyl-3-(2-phenoxyethyl)cyclobutyl)methyl)morpholine)

1.6 M n-BuLi in hexane (2.6 mL, 4.2 mmol) was added to an ice-cooled solution of phenol (350 mg, 3.7 mmol) in anhydrous THF (10 mL) under nitrogen atmosphere, and the mixture was stirred at 0 °C for 1 h. Then 2-((1R,3R)-2,2-dimethyl-3-(morpholinomethyl)cyclobutyl)ethyl methanesulfonate (500 mg, 1.6 mmol) in 20 mL THF was added and the mixture was subsequently heated to reflux for 6 days and evaporated to dryness. The residue was poured into EtOAc and the solution was washed with saturated aqueous NaHCO₃ and dried over MgSO₄. The solvent was removed under vacuo to give 879 mg of crude 4-(((1R,3R)-2,2-dimethyl-3-(2-phenoxyethyl)cyclobutyl)methyl)morpholine that was chromatographed twice on Baker^{®} silica gel (1:40 MeOH-CH₂Cl₂ and CH₂Cl₂ as the respective eluents) giving 178 mg of 4-(((1*R*,3*R*)-2,2-dimethyl-3-(2-phenoxyethyl)cyclobutyl)methyl)morpholine (36% yield).
- **¹H-NMR (250 MHz, CDCl₃)**
   1.0 (s, 3H), 1.1 (s, 3H), 1.7 (m, 2H), 2.1 (m, 5H), 2.4 (m, 5H), 3.7 (m, 4H), 3.9 (m, 2H), 6.9 (m, 3H), 7.3 (m, 2H).
- **¹³C-NMR (67.5 MHz, CDCl₃)**
   16.6 (1C), 29.9 (1C), 30.1 (1C), 30.5 (1C), 39.8 (1C), 40.2 (1C), 40.3 (1C), 54.3 (2C), 60.0 (1C), 66.2 (1C), 66.7 (2C), 114.5 (2C), 120.5 (1C), 129.5 (2C), 159.2 (1C).
- **MS (IFE-IT), m/z (%):** 304.2 (M+1⁺,100)
- **[α]=** +13 (*c*=0.9, CH₂Cl₂)

### Example compound 16 1-(((1R,3R)-2,2-Dimethyl-3-(2-phenoxyethyl)cyclobutyl)methyl)piperidine was prepared in a similar manner.

- **¹H-NMR (250 MHz, CDCl₃)**
   0.9 (s, 3H), 1.0 (s, 3H), 1.5 (m, 6H), 1.8 (m, 2H), 2.1 (m, 5H), 2.4 (m, 5H), 3.8 (m, 2H), 6.8 (m, 3H), 7.2 (m, 2H).
- **MS (IFE-IT), m/z (%)**: 302.2 (M+1⁺,100)

### Preparation of example compound 55:

### 2-((1R,3R)-2,2-Dimethyl-3-((piperidin-1-yl)methyl)cyclobutyl)ethyl pivalate

Triethylamine (0.8 mL, 5.7 mmol) and pivaloyl chloride (0.6 mL, 5.2 mmol) were added to an ice-cooled solution of 2-((1R,3R)-2,2-dimethyl-3-(piperidin-1-ylmethyl)cyclobutyl)ethanol (0.9 g, 4.0 mmol) in anhydrous dichloromethane (50 mL) under nitrogen atmosphere. After stirring at 0°C for 1 h, the reaction mixture was washed with saturated aqueous NaHCO₃ (4 x 25 mL) and dried over MgSO₄. The solvent was removed to afford 1.2 g of a residue that was cromatographed on Baker^{®} silica gel using 1:9 MeOH-CH₂Cl₂ as eluent to give 2-((1R,3R)-2,2-dimethyl-3-(piperidin-1-ylmethyl)cyclobutyl)ethyl pivalate (300 mg, 24% yield). The compound was dissolved in hot dichloromethane and allowed to stand at room temperature for 96 h under pentane atmosphere. The produced precipitate was filtered and dried to afford 2-((1R,3R)-2,2-dimethyl-3-(piperidin-1-ylmethyl)cyclobutyl)ethyl pivalate (250 mg, 20% yield).
- **¹H-NMR (250 MHz, CDCl₃)**
   0.9 (s, 3H), 1.1 (s, 3H), 1.2 (s, 9H), 1.5 (m, 4H), 1.8 (m, 3H), 2.0 (m, 1 H), 2.3 (m, 3H), 2.6 (m, 3H), 2.7 (m, 1H), 3.0 (m, 1 H), 3.4 (m, 2H), 4.0 (m, 2H).
- **¹³C-NMR (67.5 MHz, CDCl₃)**
   16.5 (1C), 21.9 (2C), 22.5 (1C), 27.1 (3C), 28.8 (1C), 29.2 (1C), 31.5 (1C), 36.7 (1C), 38.6 (1C), 40.2 (1C), 40.9 (1C), 52.7 (1C), 53.6 (1C), 58.2 (1C), 62.5 (1C), 178.5 (1C).
- **MS (IFE-IT), m/z (%):** 310.3 (M+1⁺,100)
- **[α]= -**16 (*c*=0.6, CH₂Cl₂)
- **mp=**198°C dec. (CH₂Cl₂/pentane)
   The example compound 56 2-((1*R*,3*R*)-2,2-Dimethyl-3-((piperidin-1-yl)methyl)cyclobutyl)ethyl acetate was prepared accordingly.
- **¹H-NMR (250 MHz, CDCl₃)**
   0.9 (s, 3H), 1.0 (s, 3H), 1.5 (m, 9H), 1.9 (m, 1H), 2.1 (s, 3H), 2.2 (m, 3H), 2.3 (m, 5H), 4.0 (m, 2H).
- **MS (IFE-IT), m/z (%):** 268.2 (M+1⁺,100)

### Preparation of compounds of general formula IX:

### tert-Butyl 2-((1R,3R)-3-((benzyloxy]methyl)-2,2-dimethylcyclobutyl)acetate

An ice-cooled mixture of tert-butyl 2-((1R,3R)-3-(hydroxymethyl)-2,2-dimethylcyclobutyl)acetate (2.5 g, 10.9 mmol) and 60 % by weight NaH in mineral oil (3.2 g, 78.8 mmol) in DMF (100 mL) was stirred for 1.5 h under nitrogen atmosphere. Then benzyl bromide (7.3 mL, 61.4 mmol) was added dropwise keeping the temperature at 0 °C. The light-protected mixture was allowed to warm and was stirred at room temperature for 72 h. The reaction mixture was diluted with wet EtOAc (100 mL) and water (40 mL) was carefully added. The resultant emulsion was washed with saturated aqueous NaHCO₃ (5x75 mL) and dried over MgSO₄. The solvents were removed under vacuo and the residue was lyophilized to afford tert-butyl 2-((1R,3R)-3-(benzyloxymethyl)-2,2-dimethylcyclobutyl)acetate (3.1 g, 85% yield) that was used in the next step without further purification.
- **¹H-NMR (250 MHz, CDCl₃)**
   0.9 (s, 3H), 1.1 (s, 3H), 1.5 (s, 9H), 2.2 (m, 6H), 3.5 (m, 2H), 4.5 (s, 2H), 7.4 (m, 5H).

### Preparation of compounds of general formula X:

### 2-((1R,3R)-3-((Benzyloxy)methyl)-2,2-dimethylcyclobutylyethanol

2M LiBH₄ (19 mL, 20.0 mmol) and dry MeOH (1.9 mL) were successively added to a solution of tert-butyl 2-((1R,3R)-3-(benzyloxymethyl)-2,2-dimethylcyclobutyl)acetate (700 mg, 2.5 mmol) in anydrous THF under nitrogen atmosphere. The mixture was stirred at room temperature for 15 minutes then heated to reflux for 24 h. Excess hydride was destroyed with MeOH and the mixture was poured into water and extracted with CH₂Cl₂ (5x80 mL). The combined organic extracts were dried over MgSO₄ and solvents were removed at reduced pressure to afford 2-((1R,3R)-3-(benzyloxymethyl)-2,2-dimethylcyclobutyl)ethanol (500 mg, 79% yield) that was used in the next step without further purification.
- ¹H-NMR (250 MHz, CDCl₃)
   1.0 (s, 3H), 1.1 (s, 3H), 1.6 (m, 2H), 2.0 (m, 4H), 3.4 (m, 2H), 3.6 (m, 2H), 4.5 (s, 2H), 7.4 (m, 5H).

### Preparation of compounds of general formula XI:

### 2-((1R,3R)-3-((Benzyloxy)methyl)-2,2-dimethylcyclobutyl)ethyl methanesulfonate

An ice-cooled solution of alcohol 2-((1R,3R)-3-(benzyloxymethyl)-2,2-dimethylcyclobutyl)ethanol (500 mg, 2.0 mmol), mesyl chloride (0.2 mL, 2.6 mmol), triethylamine ((0.4 mL, 2.8 mmol) in anhydrous CH₂Cl₂ (10 mL) was stirred for 1 h under nitrogen atmosphere. The reaction mixture was washed with saturated aqueous NaHCO₃ (4x10 mL) and the organic phase was dried over MgSO₄. The solvent was removed at reduced pressure to afford crude mesylate 2-((1*R*,3*R*)-3-((Benzyloxy)methyl)-2,2-dimethylcyclobutyl)ethyl methanesulfonate (510 mg, 78% yield) that was used in the next step without further purification.
- ¹H-NMR (250 MHz, CDCl₃)
   1.0 (s, 3H), 1.1 (s, 3H), 1.6 (m, 2H), 2.0 (m, 4H), 3.0 (s, 3H), 3.4 (m, 2H), 3.6 (m, 2H), 4.5 (s, 2H), 7.4 (m, 5H).
   The compound 2-((1*R*,3*R*)-3-(1-hydroxyethyl}-2,2-dimethylcyclobutyl)ethyl methanesulfonate was prepared accordingly. 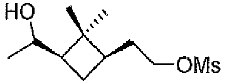
- ¹H-NMR (250 MHz, CDCl₃)
   1.0 (s, 3H), 1.1 (d, *J*= 5.5 Hz, 3H), 1.2 (s, 3H), 1.8 (m, 6H), 3.0 (s, 3H), 3.8 (m, 1H), 4.1 (m, 2H).

### Preparation of example compound 32:

### (2S,6R)-4(((1R,3R)-3-(2-(Benzyloxyethyl)-2,2-dimethylcyclobutyl)methyl)-2,6-dimethylmorpholine

A mixture of ((1R,3R)-3-(2-(benzyloxy)ethyl)-2,2-dimethylcyclobutyl)methyl methanesulfonate (500 mg, 1.5 mmol) and *cis*-(2*R*,6*R*)-dimethylmorpholine was heated to reflux for 24 h under nitrogen atmosphere. The reaction mixture was diluted with EtOAc (25 mL) and washed with saturated aqueous NaHCO₃. The organic layer was dried over MgSO₄ and the solvent was evaporated. The residue was chromatographed on Baker^{®} silica gel (1:9 MeOH-CH₂Cl₂) to provide (2S,6R)-4-(((1R,3R)-3-(2-(benzyloxy)ethyl)-2,2-dimethylcyclobutyl)methyl)-2,6-dimethylmorpholine (429 mg, 81% yield).
- ¹H-NMR (250 MHz, CDCl₃)
   0.9 (s, 3H), 1.1 (s, 3H), 1.2 (d, *J*= 6 Hz, 6H), 1.6 (m, 4H), 2.0 (m, 5H), 2.4 (dd, *J=* 10.5 Hz, 1H), 2.7 (m, 2H), 3.4 (m, 2H), 3.7 (m, 2H), 4.5 (s, 2H), 7.4 (m, 5H).
- ¹³C-NMR (67.5 MHz, CDCl₃)
   16.4 (1C), 19.1 (1C), 19.2 (C), 30.1 (1C), 30.3 (1C), 30.6 (1C), 39.9 (1C), 40.0 (1C), 40.1 (1C), 59.5 (2C), 60.2 (1C), 69.0 (2C), 71.6 (1C). 72.9 (1C), 127.4 (1C), 127.6 (2C), 128.3 (2C), 138.6 (1C).
- MS (IFE-IT), m/z (%): 346.2 (M+1⁺,100)
- [α]= + 8 (*c*=2.3, CH₂Cl₂)

### Preparation of example compound 29:

### 1-(((1R,3R)- 3-(2-(Banzyloxy)ethyl)-2,2-dimethylcyclobutyl)methyl)-4-phenylpiperidine

4-Phenylpiperidine (2.5 g, 15.5 mmol) was added to a solution of ((1R,3R)-3-(2-(benzyloxy)ethyl)-2,2-dimethylcyclobutyl)methyl methanesulfonate (250 mg, 0.8 mmol) in dry THF (15 mL) and the mixture was heated to reflux for 48 h under nitrogen atmosphere. The solvent was evaporated under vacuo and the residue was poured into EtOAc (25 mL) and successively washed with saturated aqueous NaHCO₃ and brine. The organic phase was dried over MgSO₄ and the solvent was removed. The residue (320 mg) was chromatographed on Baker^{®} silica gel (1:9 MeOH-CH₂Cl₂) to afford pure 1-(((1R,3R)-3-(2-(benzyloxy)ethyl)-2,2-dimethylcyclobutyl)methyl)-4-phenylpiperidine (144 mg, 50% yield).
- ¹H-NMR (250 MHz, CDCl₃)
   0.9 (s, 3H), 1.1 (s, 3H), 1.7 (m, 9H), 2.1 (m, 6H), 2.5 (m, 1H), 3.0 (m, 1H), 3.4 (m, 2H), 4.5 (s, 2H), 7.4 (m, 10H).
- ¹³C-NMR (67.5 MHz, CDCl₃)
   16.4 (1C), 30.1 (1C), 30.4 (1C), 31.2 (1C), 33.4 (1C), 33.5 (1C), 40.1 (1C), 40.2 (1C), 40.4 (1C), 42.6 (1C), 54.1 (1C), 55.8 (1C), 60.0 (1C), 69.1 (1C), 73.0 (1C), 126.0 (1C), 126.8 (2C), 127.5 (1C), 127.6 (2C), 128.3 (2C), 128.4 (2C), 138.6 (1 C), 146.4 (1C).
- MS (IFE-IT), m/z (%): 392.3 (M+1⁺,100)
- [α]= + 14 (*c*=1.9, CH₂Cl₂)

The following example compounds were prepared as described above:

### Example compound 3: 1-(((1S,3S)- 3-(2-(Benzyloxy)ethyl)-2,2-dimethylcyclobutyl)methyl)-4-phenylpiperidine

- [α]= -19 (*c*=0.3, CH₂Cl₂)

### Example compound 27: 1-(((1R,3R)-3-(2-(Benxy)oxy)ethy))-2,2-dimethylcyclobutyl)methyl)piperidine

- ¹H-NMR (250 MHz, CDCl₃)
   0.9 (s, 3H), 1.1 (s, 3H), 1.4 (m, 2H), 1.6 (m, 6H), 1.7 (m, 1H), 1.9 (m, 1H), 2.1 (m, 3H), 2.4 (m, 5H), 3.4 (m, 2H), 4.5 (s, 2H), 7.4 (m, 5H).
- MS (IFE-IT), m/z (%): 316.3 (M+1⁺,100)

### Example compound 2: 1-(((1S,3S)-3-(2-(Benzyloxy)ethyl)-2,2-dimethylcyciobutyl)methylpiperidine

- [α]= -13 (*c*=2.0, CH₂Cl₂)

### Example compound 31:4-(((1R,3R)-3-(2-(Benzyloxy)ethyl)-2,2-dimethylcyclobutyl)methyl)morpholine

- ¹H-NMR (250 MHz, CDCl₃)
   0.9 (s; 3H), 1.1 (s, 3H), 1.6 (m, 3H), 1.9 (m, 1 H), 2.0 (m, 2H), 2.1 (m, 1 H), 2.5 (m, 5H), 3.4 (m, 2H), 3.7 (m, 4H), 4.5 (s, 2H), 7.4 (m, 5H).
- MS (IFE-IT), m/z (%): 318.2 (M+1⁺,100)

### Example compound 1: 4-(((1S,3S)-3-(2-(Benzyloxy)ethyl)-2.2-dimethylcyclobutyl)methyl)morpholine

- **[α]=** -16 (*c*=0.7, CH₂Cl₂)

### Example compound 30: 4-(((1R,3R)-3-(2-(Benzyloxy)ethyl)-2,2-dimethylcyclobutyl)methyl)thiomorpholine

- ¹H-NMR (250 MHz, CDCl₃)
   0.9 (s, 3H), 1.1 (s, 3H), 1.5 (m, 1H), 1.7 (m, 1H), 1.9 (m, 1H), 2.0 (m, 2H), 2.2 (m, 1 H), 2.4 (m, 1 H), 2.7 (m, 9H), 3.4 (m, 2H), 4.5 (s, 2H), 7.4 (m, 5H).
- MS (IFE-IT), m/z (%): 334.2 (M+1⁺,100)

### Example compound 33: 1-(((1R,3R)-(2-(Benzyloxy)ethyl)-2,2-dimethylcyclobutyl)methyl)pyrrolidine

- ¹H-NMR (250 MHz, CDCl₃)
   0.9 (s, 3H), 1.1 (s, 3H), 1.6 (m, 2H), 1.8 (m, 4H), 2.1 (m, 5H), 2.4 (m, 4H), 2.6 (dd, *J*= 11.5 Hz, 1 H), 3.4 (m, 2H), 4.5 (s, 2H), 7.4 (m, 5H).
- ¹³C-NMR (67.5 MHz, CDCl₃)
   16.5 (1C), 23.3 (2C), 30.1 (1C), 30.3 (1C), 30.8 (1C), 40.0 (1C), 40.1 (1C), 42.1 (1C), 54.5 (2C), 57.2 (1C), 69.1 (1C), 73.0 (1C), 127.0 (1C), 127.5 (2C), 128.3 (2C), 138.5 (1C).
- MS (IFE-IT), m/z (%): 302.2 (M+1⁺,100)

### Example compound 28: 1-(((1R,3R)-3-(2-(Benzyloxy)ethyl)-2,2-dimethylcyclobutyl)methyl)-4-methylpiperidine

- ¹H-NMR (250 MHz, CDCl₃)
   0.9 (s, 3H), 0.9 (d, *J*= 4 Hz, 3H), 1.1 (s, 3H), 1.3 (m, 2H), 1.7 (m, 8H), 2.1 (m, 4H), 2.4 (dd, *J*= 10.5 Hz, 1H), 2.8 (m, 2H), 3.4 (m, 2H), 4.5 (s, 2H), 7.4 (m, 5H).
- ¹³C-NMR (67.5 MHz, CDCl₃)
   16.4 (2C), 22.0 (1C), 30.2 (1 C), 30.4 (1C), 30.7 (1C), 31.2 (1C), 34.2 (1C), 34.3 (1C), 40.2 (2C), 40.5 (1C), 53.8 (1C), 54.8 (1C), 60.1 (1C), 69.2 (1C), 73.0 (1C), 127.4 (2C), 127.5 (1C), 128.3 (2C), 138.6 (1C).
- MS (IFE-IT), m/z (%): 330.3 (M+1⁺,100)

### Example compound 34: 1-(((1R,3R)-3-(2-(Benzyloxy)ethyl)-2,2-dimethylcyclobutyl)methyl)-4-mothylpiperazine

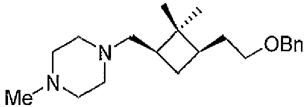

- ¹H-NMR (250 MHz, CDCl₃)
   0.9 (s, 3H), 1.1 (s, 3H), 1.5 (m, 1H), 1.7 (m, 1H), 1.9 (m, 2H), 2.1 (m, 4H), 2.3 (s, 3H), 2.5 (m, 8H), 3.4 (m, 2H), 4.5 (s, 2H), 7.3 (m, 5H).
- MS (IFE-IT), m/z (%): 331.3 (M+1⁺,100)

### Example compound 5: 1-(((1S,3S]-3-(2-(Benzyloxy]ethyl]-2,2-dimethy)cyctobuty)methy)-4-methy)piperazine

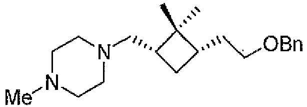

- [α]= -19 (*c*=0.4, CH₂Cl₂)

### Example compound 36: 1-(((1R,3R)- 3-(2-(Benzyloxy)ethyl)-2,2-dimethylcyclobutyl)methyl)-4-phenylpiperazine

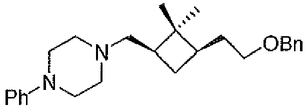

- ¹H-NMR (250 MHz, CDCl₃)
   0.9 (s, 3H), 1.1 (s, 3H), 1.6 (m, 3H), 2.1 (m, 4H), 2.5 (m, 5H), 3.2 (m, 4H), 3.4 (m, 2H), 4.5 (s, 2H), 6.9 (m, 3H), 7.4 (m, 7H).
- ¹³C-NMR (67.5 MHz, CDCl₃)
   16.4 (1C), 30.1 (1C), 30.4 (1C), 30.6 (1C), 40.0 (1C), 40.1 (2C), 49.1 (2C), 53.5 (2C), 59.6 (1C), 69.1 (1C), 72.9 (1C), 115.9 (2C), 119.5 (1C), 127.4 (1C), 127.6 (2C), 128.3 (2C), 129.0 (2C), 138.7 (1C), 151.4 (1C).
- MS (IFE-IT), m/z (%): 393.3 (M+1⁺,100)
- [α]= +11 (*c*=1.2, CH₂Cl₂)

### Example compound 4: 1-(((1S,3S)- 3-(2-(Benzyloxy)ethyl)-2,2-dimethylcyclobutyl)mothyl)-4-phonylpiperazine

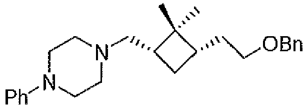

- [α]= -15 (*c*=0.3, CH₂Cl₂)

### Example compound 7:1-(((1R,3S)-3-((Benzyloxy)methyl)-2,2-dimethylcyclobutyl)methyl)piperidine

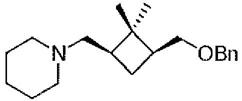

- **¹H-NMR (250 MHz, CDCl₃)**
   1.0 (s, 3H), 1.2 (s, 3H), 1.6 (m, 6H), 2.1 (m, 5H), 2.3 (m, 5H), 3.4 (m, 2H), 4.5 (d, *J*=2 Hz, 2H), 7.3 (m, 5H).
- **MS (IFE-IT), m/z (%):** 302.2 (M+1⁺,100)
- **[α]=** -8(*c=*0*.*5, CH₂Cl₂)

### Example compound 6: 4-(((1R,3S)-3-((Benzyloxy)methyl)-2,2-dimethylcyclobutyl)methyl)morpholine

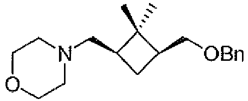

- **¹H-NMR (250 MHz, CDCl₃)**
   1.0 (s, 3H), 1.1 (s, 3H), 2.1 (m, 5H), 2.4 (m, 5H), 3.4 (m, 2H), 3.7 (m, 4H), 4.5 (d, *J*=2 Hz, 2H), 7.4 (m, 5H).
- **MS (IFE-IT), m/z (%):** 304.2 (M+1⁺,100)
- **[α]= -**3 (*c*=0.6, CH₂Cl₂

### Example compound 10:1-(((1R,3S)-3-((Benzyloxy)methyl)-2,2-dimethylcyclobutyl)methyl)-4-methylpiperazine

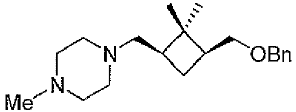

- **¹H-NMR (250 MHz, CDCl₃)**
   1.0 (s, 3H), 1.2 (s, 3H), 2.2 (m, 5H), 1.9 (m, 1H), 2.3 (s, 3H), 2.5 (m, 8H), 3.5 (m, 2H), 4.5 (d, *J=* 2 Hz, 2H), 7.4 (m, 5H).
- **MS (IFE-IT), m/z (%):** 317.2 (M+1⁺,100)
- **[α]=** -3(*c*=0.6, CF₂Cl₂)

### Example compound 9: 1-(((1R,3S)-3-((Benzyloxy)methyl)-2,2-dimethylcyclobutyl)methyl)-4-phenylpiperazine

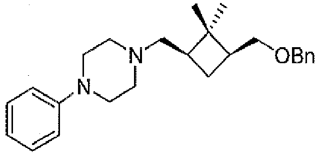

- **¹H-NMR (250 MHz, CDCl₃)**
   1.0 (s, 3H), 1.2 (s, 3H), 2.2 (m, 5H), 2.6 (m, 5H), 3.4 (m, 4H), 3.6 (m, 2H), 4.5 (d, J=2 Hz, 2H), 7.0 (m, 5H), 7.3 (m, 5H).
- **MS (IFE-IT), m/z (%):** 379.2 (M+1⁺,100)
- **[α]=** -9(*c*=0.2, CH₂Cl₂)

### Example compound 8: 1-(((1R,3S)-3-((Benzyloxy)methyl)-2,2-dimethylcyclobutyl)methyl)-4-phenylpiperidine

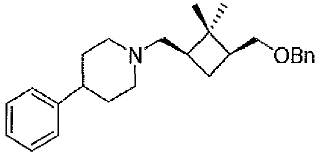

- **¹H-NMR (250 MHz, CDCl₃)**
   1.0 (s, 3H), 1.2 (s, 3H), 1.9 (m, 9H), 1.9 (m, 1 H), 2.2 (m, 4H), 3.1 (m, 1 H), 3.5 (m, 2H), 4.5 (d, *J*=2 Hz, 2H), 7.4 (m, 10H).
- **MS (IFE-IT), m/z (%)**: 378.3 (M+1⁺,100)
- **[α]=**-18(*c*=0.1, CH₂Cl₂)

### Preparation of compounds of general formula XII:

### 1-(2-((1R,3R)-3-((Benzyloxy)methyl)-2,2-dimethylcyclobutyl)ethyl)piperidine

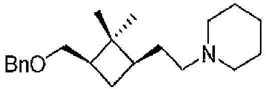

- **¹H-NMR (250 MHz, CDCl₃**)
   0.9 (s, 3H), 1.1 (s, 3H), 1.5 (m, 2H), 1.6 (m, 6H), 1.8 (m, 3H), 2.0 (m, 1H), 2.2 (m, 2H), 2.4 (m, 4H), 3.4 (m, 2H), 4.5 (s, 2H), 7.3 (m, 5H).
- **MS (IFE-IT), m/z (%):** 316.2 (M+1⁺,100)

### 4-(2-((1R,3R)-3-((Benzyloxy)methyl)-2,2-dimethylcyclobutyl)ethyl)morpholine

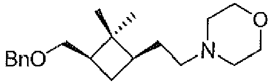

- **¹H-NMR (250 MHz, CDCl₃)**
   0.9 (s, 3H), 1.1 (s, 3H), 1.4 (m, 1H), 1.6 (m, 1H), 1.8 (m, 1H), 2.0 (m, 1 H), 2.2 (m, 4H), 2.4 (m, 4H), 3.4 (m, 2H), 3.7 (m, 4H), 4.5 (s, 2H), 7.3 (m, 5H).
- **MS (IFE-IT), m/z (%):** 318.2 (M+1⁺,100)

### Preparation of example compound 37: 1-(((1R,3R)-3-(2-(benzyloxy)ethyl)-2,2-dimethylcyclobutyl)methyl)4-phenylpiperazine oxalate

1-(((1R,3R)-3-(2-(benryloxy)ethyl)-2,2-dimethylcyclobutyl)methyl)-4-phenylpiperazine (83 mg, 0.2 mmol) was dissolved in hot EtOH and oxalic acid dihydrate (25.8 mg, 0.2 mmol) was added. The mixture was allowed to stand at room temperature for 72 h under pentane atmosphere. The produced precipitate was filtered and dried to afford the 1-(((1R,3R)-3-(2-(benzyloxy)ethyl)-2,2-dimethylcyclobutyl)methyl)-4-phenylpiperazine oxalate (39 mg, 40% yield).
- **¹H-NMR (250 MHz, MeOD-d₄)**
   1.0 (s, 3H), 1.1 (s, 3H), 1.6 (m, 3H), 2.1 (m, 4H), 2.5 (m, 5H), 3.2 (m, 4H), 3.4 (m, 2H), 4.5 (s, 2H), 7.0 (m, 3H), 7.3 (m, 7H).
- **MS (IFE-IT), m/z (%):** 393.3 (M+1⁺,100)
- **mp=** 162°C (EtOH/pentane)

The following product was prepared in a similar manner:

### Example compound 35: 1-(((1R,3R)-3-(2-(Benzyloxy)ethyl)-2,2-dimethylcyclobutyl)methyl)-4-methylpiperazine oxalate

- **¹H-NMR (250 MHz, MeOD-d₄)**
   0.9 (s, 3H), 1.1 (s, 3H), 1.5 (m, 3H), 1.7 (m, 1 H), 2.0 (m, 4H), 2.6 (s, 3H), 2.9 (m, 8H), 3.4 (m, 2H), 4.5 (s, 2H), 7.3 (m, 5H).
- **MS (IFE-IT), m/z (%):** 331.3 (M+1⁺,100)
- **mp=** 230°C dec. (EtOH/pentane)

A compound of general formula XIII, wherein R¹, R³ and R⁴ represent hydrogen and n is 1, can be prepared starting from (-)-α-pinene as depicted in scheme 2. below.

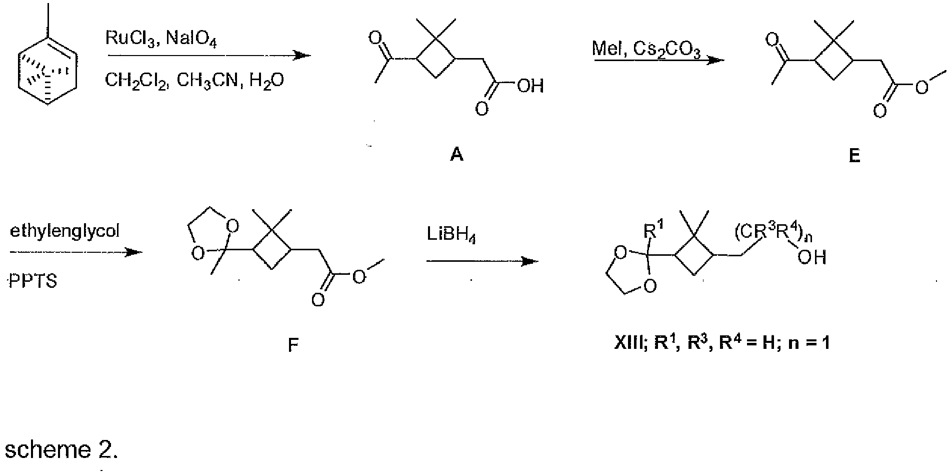

### Preparation of compounds of general formula XVI:

### 1-((1R,3R)-2,2-Dimethy)-3-(2-morphoHnoethy))cyclobutyl)ethanone

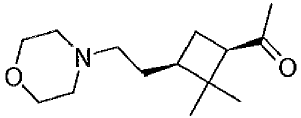

2M HCl (0.1 mL, 0.2 mmol) was added to a solution of 4-(2-((1R,3R)-2,2-dimethyl-3-(2-methyl-1,3-dioxolan-2-yl)cyclobutyl)ethyl)morpholine (200 mg, 0.7 mmol) in MeOH (10 mL) and the mixture was subsequently stirred at room temperature for 4.5 h and evaporated to dryness. The residue was poured into EtOAc and washed with saturated aqueous NaHCO₃. The organic phase was dried over MgSO₄ and the solvent was removed to afford 1-((1R,3R)-2,2-dimethyl-3-(2-morpholinoethyl)cyclobutyl)ethanone (120 mg, 71% yield) that was used in the next step without further purification.
- **¹H-NMR (250 MHz, CDCl₃)**
   0.9 (s, 3H), 1.3 (s, 3H), 1.5 (m, 2H), 1.9 (m, 3H), 2.0 (s, 3H), 2.2 (m, 2H), 2.4 (m, 4H), 2.8 (m, 1H), 3.7 (m, 4H).

The following compound of general formula XVI was prepared in a similar manner:

### 1-((1R,3R)-2,2-Dimethyl-3-(2-(piperidin-1-yl)ethyl)cyclobutyi)ethanone

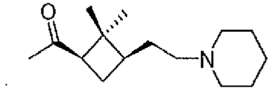

- **¹H-NMR (250 MHz, CDCl₃)**
   0.9 (s, 3H), 1.3 (s, 3H), 1.5 (m, 2H), 1.7 (m, 6H), 1.9 (m, 3H), 2.0 (s, 3H), 2.2 (m, 2H), 2.4 (m, 4H), 2.8 (m, 1H).

### Preparation of compounds of general formula XVII:

### Example compound 13: 1-((1R,3R)-2,2-Dimethyl-3-(2-morpholinoethyl)cyclobutyl)-1-phenylethanol

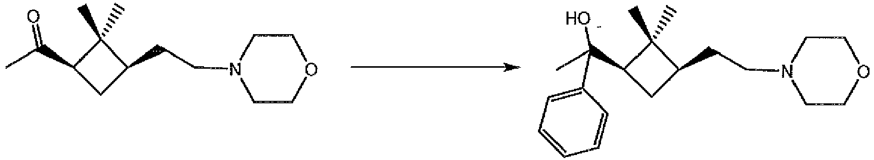

Phenyllithium [1.9 M in ether (0.8 mL, 1.5 mmol)] was added to an ice-cooled solution of 1-((1R,3R)-2,2-dimethyl-3-(2-morpholinoethyl)cyclobutyl)ethanone (170 mg, 0.7 mmol) in ahydrous THF (8 mL). The mixture was allowed to warm to room temperature and stirred overnight under nitrogen atmosphere. Excess of Phenyllithium was destroyed with wet ether and then water was added. The layers were separated and the solvent was evaporated from the organic phase. The residue was poured into EtOAc, washed with brine and dried over Mg S04. The solvent was removed and the residue (225 mg) was chroamtographed on Baker^{®} silica (1:3 EtOAc-hexane) to afford (S)-1-((1R,3R)-2,2-dimethyl-3-(2-morpholinoethyl)cyclobutyl)-1-phenylethanol (45 mg, 20% yield).
- **¹H-NMR (250 MHz, CDCl₃)**
   1.1 (s, 3H), 1.2 (s, 3H), 1.3 (m, 3H), 1.6 (s, 3H), 2.1 (m, 2H), 2.2 (m, 2H), 2.4 (m, 5H), 3.7 (m, 4H), 7.4 (m, 5H).
- **MS (IFE-IT), m/z (%):** 318.3 (M+1⁺,100)

The following product was prepared in a similar manner:

### Example compound 14: 1-((1R,3R)-2,2-Dimethyl-3-(2-(piperidin-1-yl)ethyl)cyclobutyl)-1-phenylethanol

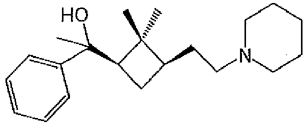

- **¹H-NMR (250 MHz, CDCl₃)**
   1.1 (s, 3H), 1.2 (s. 3H), 1.4 (m, 3H), 1.5 (m, 6H), 1.6 (s, 3H), 2.1 (m, 2H), 2.2 (m, 3H), 2.4 (m, 4H), 7.3 (m, 5H).
- **MS (IFE-IT), m/z (%):** 316.2 (M+1⁺,100)

### Example compound 12: 1-((1R,3R)-2,2-Dimethyl-3-(2-(piperidin-1-yl)ethyl)cyclobutyl)ethanol

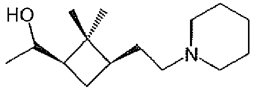

- **¹H-NMR (250 MHz, CDCl₃)**
   1.0 (s, 3H), 1.0 (d, *J=*6.25 Hz, 3H), 1.1 (s, 3H), 1.4 (m, 2H), 1.6 (m, 8H), 1.9 (m, 2H), 2.2 (m, 2H), 2.4 (m, 4H), 3.7 (m, 1H).
- **MS (IFE-IT), m/z (%):** 240.1 (M+1⁺,100)

### Example compound 11: 1-((1R,3R)-2,2-Dimethyl-3-(2-morpholinoethyl)-cyclobutyl)ethanol)

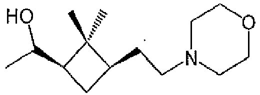

- **¹H-NMR (250 MHz, CDCl₃)**
   1.0 (s, 3H), 1.1 (d, *J*=6.75 Hz, 3H), 1.2 (s, 3H), 1.5 (m, 2H), 1.6 (m, 2H), 1.9 (m, 2H), 2.2 (m, 2H), 2.4 (m, 4H), 3.7 (m, 5H).
- **MS (IFE-IT), m/z (%)**: 242.1 (M+1⁺,100)

### Pharmacological data

The binding of the compounds of the present invention to sigma-1 receptors was tested as described above.

The substituted dimethylcyclobutyl compounds of general formula I show a high affinity to sigma1 receptors (table 1).

**Table 1.**

| **Compound according to example** | **Percent Inhibition Percent Concentration (µM) 0.1000 SIGMA-1 receptor** | **Percent Inhibition Percent Concentration (µM) 0.0100 SIGMA-1 receptor** | **Binding Sigma-1 Kᵢ (nM)** |
|---|---|---|---|
| 26 | - 50 | 23.9 | |
| 51 | 81,9 | 36.1 | |
| 48 | 72,4 | 34,3 | |
| 17 | 61,5 | 45,2 | 41,8 |
| 31 | 101,4 | 89,9 | 1,7 |
| 27 | 104,2 | 96,5 | 1,1 |
| 40 | 60,1 | 16,3 | |
| 54 | 87,1 | 42,2 | 11,7 |
| 47 | 92 | 36,8 | |
| 42 | 88 | 19,7 | |
| 25 | 98,3 | 83 | 1,2 |
| 49 | 92 | 45,7 | 4,9 |
| 53 | 73,6 | -6,6 | |
| 52 | 52,6 | -0,6 | |
| 50 | 20,9 | 2 | |
| 46 | 83 | 50,2 | 13,5 |
| 45 | 28,4 | 10,3 | |
| 44 | 82,8 | 41,7 | |
| 30 | 91,8 | 74 | 1 |
| 33 | 89,8 | 85,3 | 0.6±0.2 |
| 39 | 95,8 | 94,1 | 8,4 |
| 38 | 98,5 | 88,6 | 1.2 |
| 13 | 95,2 | 77,8 | 1,2 |
| 14 | 97,5 | 75,6 | 1,9 |
| 11 | 44,4 | -8,3 | |
| 12 | 44,1 | 5,5 | |
| 28 | 100,5 | 93,5 | 5 |
| 34 | 96,2 | 92,3 | 3,6 |
| 35 | 95,9 | 76,7 | 5,3 |
| 36 | 88,3 | 65,8 | 47 |
| 29 | 95,6 | 76,9 | 16,6 |
| 32 | 99,1 | 94,6 | 3,6 |
| 37 | 98,8 | 79,7 | 33,5 |
| 19 | 42,3 | 17,1 | |
| 16 | 96,4 | 97,5 | 2,2 |
| 15 | 95,4 | 93,6 | 4,2 |
| 18 | 44,8 | -21,4 | |
| 20 | 63,1 | 31.5 | |
| 23 | 70,6 | 27,6 | |
| 24 | 92 | 69,7 | 3,3 |
| 22 | 96,8 | 91,9 | 1,4 |
| 21 | 77,1 | 25,7 | |
| 7 | 101 | 94,5 | 0,79 |
| 6 | 98,4 | 91 | 1,3 |
| 10 | 99,5 | 86,7 | 0,95 |
| 9 | 94,7 | 60 | 14,6 |
| 8 | 101,2 | 48,3 | 10,9 |
| 2 | 95,9 | 66,5 | 1,5 |
| 1 | 79,7 | 62,8 | 6,1 |
| 5 | 92,4 | 42,5 | |
| 4 | 82,2 | 24,1 | |
| 3 | 97,7 | 30,2 | |

## Claims

1. A substituted dimethylcyclobutyl compound of general formula I, 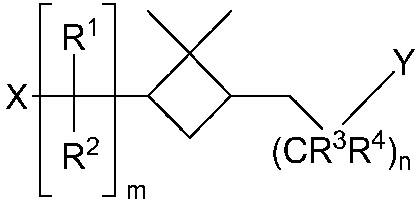 wherein
m is 1 or 2;
n is 0 or 1;
X and Y are different;
X represents a -OR⁵ moiety or a -NR⁶R⁷ moiety; or a (hetero)cycloaliphatic radical selected from the group consisting of pyrrolidinyl, piperidinyl, morpholinyl, thiomorpholinyl, piperazinyl, pyrazolidinyl, indolinyl, isoindolinyl, (1,2,3,4)-tetrahydroquinolinyl and (1,2,3,4)-tetrahydroisoquinolinyl, which may be unsubstituted or optionally substituted with 1, 2, 3, 4 or 5 substituent(s) independently selected from the group consisting of oxo (=O), thioxo (=S), methyl, ethyl, n-propyl, isopropyl, n-butyl, tert-butyl, sec-butyl, isobutyl, n-pentyl, -O-CH₃, -O-C₂H₅, F, Cl, Br, I, -CN, -CF₃, -OH, -SH and-NH₂;
Y represents a -OR⁸ moiety; a -NR⁹R¹⁰ moiety; a -C(=O)-OR¹¹ moiety; or a (hetero)cycloaliphatic radical selected from the group consisting of pyrrolidinyl, piperidinyl, morpholinyl, thiomorpholinyl, piperazinyl, pyrazolidinyl, indolinyl, isoindolinyl, (1,2,3,4)-tetrahydroquinolinyl, and (1,2,3,4)-tetrahydroisoquinolinyl, which may be unsubstituted or optionally substituted with 1, 2, 3, 4 or 5 substituent(s) independently selected from the group consisting of methyl, ethyl, n-propyl, isopropyl, n-butyl, tert-butyl, sec-butyl, isobutyl, n-pentyl, -O-CH₃, -O-C₂H₅, F, CI, Br, I, -CN, -CF₃, -OH, -SH and -NH₂;
R¹ represents a hydrogen atom or a radical selected from the group consisting of methyl, ethyl, n-propyl, isopropyl, n-butyl, sec-butyl, isobutyl, tert-butyl and n-pentyl;
R² represents a hydrogen atom; a radical selected from the group consisting of methyl, ethyl, n-propyl, isopropyl, n-butyl, sec-butyl, isobutyl, tert-butyl and n-pentyl; or an aryl radical selected from the group consisting of phenyl and naphthyl, which may be unsubstituted or optionally substituted with 1, 2, 3, 4 or 5 substituent(s) independently selected from the group consisting of methyl, ethyl, n-propyl, isopropyl, n-butyl, tert-butyl, sec-butyl, isobutyl, n-pentyl, -O-CH₃, -O-C₂H₅ -S-CH₃, -S-C₂H₅, -C(=O)-OH, -C(=O)-O-CH₃,-C(=O)-O-C₂H₅, -C(=O)-CH₃, -C(=O)-C₂H₅, F, Cl, Br, I, -CN, -CF₃, -OCF₃,-SCF₃, -OH, -SH, -NH₂, -NO₂, -CHO, -CF₂H and -CFH₂;
R³ and R⁴ both represent a hydrogen atom;
R⁵ and R⁸, independently of one another, each represent a hydrogen atom; a radical selected from the group consisting of methyl, ethyl, n-propyl, isopropyl, n-butyl, sec-butyl, isobutyl, tert-butyl and n-pentyl; an aryl radical selected from the group consisting of phenyl and naphthyl, which may be bonded via a -(CH₂)₁,_{2 or 3}-group and which may be unsubstituted or optionally substituted with 1, 2, 3, 4 or 5 substituent(s) independently selected from the group consisting of methyl, ethyl, n-propyl, isopropyl, n-butyl, tert-butyl, sec-butyl, isobutyl, n-pentyl, -O-CH₃, -O-C₂H₅, -S-CH₃, -S-C₂H₅, -C(=O)-OH, -C(=O)-O-CH₃, -C(=O)-O-C₂H₅, -C(=O)-CH₃, -C(=O)-C₂H₅, F, Cl, Br, I, -CN, -CF₃, -OCF₃, -SCF₃, -OH, -SH, -NH₂, -NO₂, -CHO, -CF₂H,-CFH₂ and -S(=O)₂-CH₃; or a -C(=O)-R¹² moiety;
with the proviso that
if R⁵ represents hydrogen and m is 1,
R¹ represents; a radical selected from the group consisting of methyl, ethyl, n-propyl, isopropyl, n-butyl, sec-butyl, isobutyl, tert-butyl and n-pentyl;
and
R² represents a radical selected from the group consisting of methyl, ethyl, n-propyl, isopropyl, n-butyl, sec-butyl, isobutyl, tert-butyl and n-pentyl; or an aryl radical selected from the group consisting of phenyl and naphthyl, which may be unsubstituted or optionally substituted with 1, 2, 3, 4 or 5 substituent(s) independently selected from the group consisting of methyl, ethyl, n-propyl, isopropyl, n-butyl, tert-butyl, sec-butyl, isobutyl, n-pentyl, -O-CH₃, -O-C₂H₅ -S-CH₃, -S-C₂H₅, -C(=O)-OH, -C(=O)-O-CH₃, -C(=O)-O-C₂H₅, - (=O)-CH₃, -C(=O)-C₂H₅, F, Cl, Br, I, -CN, -CF₃, -OCF₃, -SCF₃, -OH, -SH, - NH₂, -NO₂, -CHO, -CF₂H and -CFH₂
R⁶, R⁷, R⁹ and R¹⁰, independently of one another, each represent a radical selected from the group consisting of methyl, ethyl, n-propyl, isopropyl, n-butyl, sec-butyl, isobutyl, tert-butyl and n-pentyl; or an aryl radical selected from the group consisting of phenyl and naphthyl, which may be bonded via a -(CH₂)_{1, 2 or 3}-group and which may be unsubstituted or optionally substituted with 1, 2, 3, 4 or 5 substituent(s) independently selected from the group consisting of methyl, ethyl, n-propyl, isopropyl, n-butyl, tert-butyl, sec-butyl, isobutyl, -O-CH₃, -O-C₂H₅, -S-CH₃, -S-C₂H₅, -C(=O)-OH, -C(=O)-O-CH₃, -C(=O)-O-C₂H₅, -C(=O)-CH₃, -C(=O)-C₂H₅, F, Cl, Br, I, -CN, -CF₃, - OCF₃, -SCF₃, -OH, -SH, -NH₂-NO₂, -CHO, -CF₂H, -CFH₂, -C(=O)-NH₂ and - S(=O)₂-CH₃;
or R⁶ and R⁷ together with the bridging nitrogen atom form a moiety selected from the group consisting of 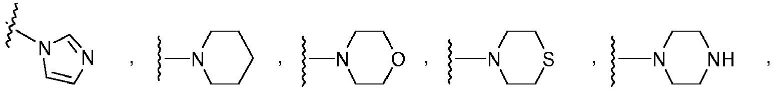 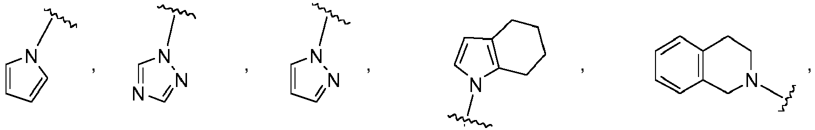 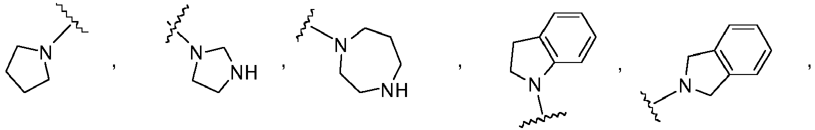 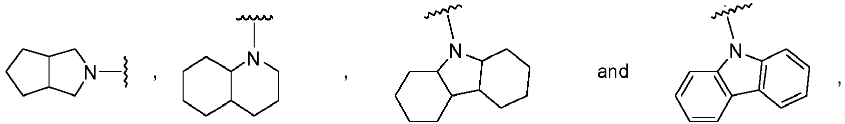 which may be unsubstituted or optionally substituted in any position including the -NH-groups with 1, 2, 3, 4 or 5 substituent(s) independently selected from the group consisting of oxo (=O), methyl, ethyl, n-propyl, isopropyl, n-butyl, tert-butyl, sec-butyl, -O-CH₃, -O-C₂H₅, -S-CH₃, -S-C₂H₅, -C(=O)-OH, -C(=O)-O-CH₃, -C(=O)-O-C₂H₅, -C (=O)-O-CH₂-CH₂-CH₃, -C(=O)-O-CH(CH₃)₂, - C(=O)-O-C(CH₃)₃, -C(=O)-CH₃, -C(=O)-C₂H₅, -C(=O)-CH₂-CH₂-CHₛ, -C(=O)-CH(CH₃)₂, -C(=O)-C(CH₃)₃, F, Cl, Br, I, -CN, -CF₃, -OCF₃, -SCF₃, -OH, -SH, - NH₂, -NO₂, -CHO, -CF₂H, -CFH₂, -C(=O)-NH₂, -C(=O)-NH-CH₃, -C(=O)-NH-C₂H₅, -C(=O)-N(CH₃)₂, -C(=O)-N(C₂H₅)₂, -S(=O)₂-CH₃, phenyl, phenethyl and benzyl, whereby said cyclic substituents may be unsubstituted or substituted by 1, 2 or 3 substituent(s) independently selected from the group consisting of methyl, ethyl, n-propyl, isopropyl, methoxy, ethoxy, F, Cl, Br, -CN, -CF₃, - OCF₃, -SCF₃, -OH, -SH, -NH₂ and -NO₂;
or R⁹ and R¹⁰ together with the bridging nitrogen atom form a moiety selected from the group consisting of; 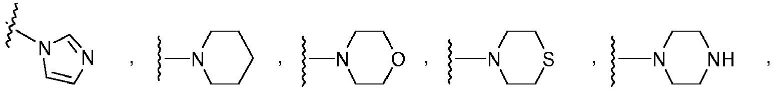 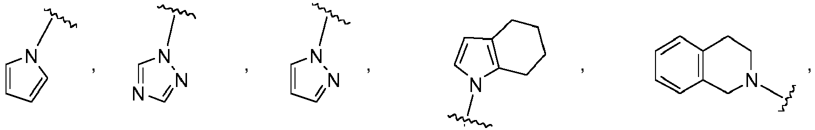 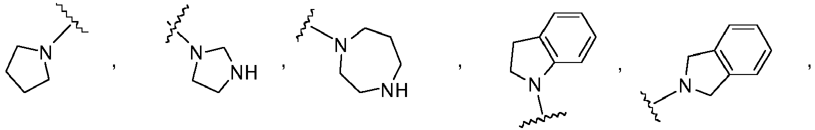 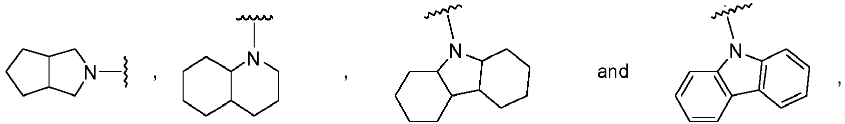 which may be unsubstituted or optionally substituted in any position including the -NH-groups with 1, 2, 3, 4 or 5 substituent(s) independently selected from the group consisting of oxo (=O), methyl, ethyl, n-propyl, isopropyl, n-butyl, tert-butyl, sec-butyl, -O-CH₃, -O-C₂H₅, -S-CH₃, -S-C₂H₅, - C(=O)-OH, -C(=O)-O-CH₃, -C(=O)-O-C₂H₅, -C (=O)-O-CH₂-CH₂-CH₃, -C(=O)-O-CH(CH₃)₂, -C(=O)-O-C(CH₃)₃, -C(=O)-CH₃, -C(=O)-C₂H₅, -C(=O)-CH₂-CH₂-CH₃, -C(=O)-CH(CH₃)₂, -C(=O)-C(CH₃)₃, F, Cl, Br, I, -CN, -CF₃, -OCF₃, - SCF₃, -OH, -SH, -NH₂, -NO₂, -CHO, -CF₂H, -CFH₂, -C(=O)-NH₂, -C(=O)-NH-CH₃, -C(=O)-NH-C₂H₅, -C(=O)-N(CH₃)₂, -C(=O)-N(C₂H₅)₂, -S(=O)₂-CH₃, phenyl, phenethyl and benzyl, whereby said cyclic substituents may be unsubstituted or substituted by 1, 2 or 3 substituent(s) independently selected from the group consisting of methyl, ethyl, n-propyl, isopropyl, methoxy, ethoxy, F, Cl, Br, -CN, -CF₃, -OCF₃, -SCF₃, -OH, -SH, -NH₂ and - NO₂;
R¹¹ represents a radical selected from the group consisting of methyl, ethyl, n-propyl, isopropyl, n-butyl, sec-butyl, isobutyl, tert-butyl and n-pentyl; and
R¹² represents a hydrogen atom; a radical selected from the group consisting of methyl, ethyl, n-propyl, isopropyl, n-butyl, sec-butyl, isobutyl, tert-butyl and n-pentyl;
optionally in form of one of its stereoisomers, preferably enantiomers or diasteromers, a racemate or in form of a mixture of at least two of its stereoisomers, preferably enantiomers and/or diastereomers, in any mixing ratio, or a salt thereof, or a corresponding solvate thereof.

2. A compound according to claim 1, **characterized in that** m is 1;
X represents a -OR⁵ moiety or a -NR⁶R⁷ moiety;
Y represents a -OR⁸ moiety; a -NR⁹R¹⁰ moiety or a -C(=O)-OR¹¹ moiety;
R⁵ and R⁸, independently of one another, each represent a hydrogen atom; a radical selected from the group consisting of methyl, ethyl, n-propyl, isopropyl, n-butyl, sec-butyl, isobutyl, tert-butyl and n-pentyl; an aryl radical selected from the group consisting of phenyl and naphthyl, which may be bonded via a -(CH₂)_{1,2} or ₃-group and which may be unsubstituted or
optionally substituted with 1, 2, 3, 4 or 5 substituent(s) independently selected from the group consisting of methyl, ethyl, n-propyl, isopropyl, n-butyl, tert-butyl, -O-CH₃, -O-C₂H₅, F, Cl, Br, I, -CN, -CF₃, -OCF₃, -SCF₃, -OH, -SH, -NH₂, -NO₂, -CHO, -CF₂H, -CFH₂ and -S(=O)₂-CH₃; or a -C(=O)-R¹² moiety;
R⁶, R⁷, R⁹ and R¹⁰, independently of one another, each represent a radical selected from the group consisting of methyl, ethyl, n-propyl, isopropyl, n-butyl, sec-butyl, isobutyl, tert-butyl and n-pentyl; or an aryl radical selected from the group consisting of phenyl and naphthyl, which may be bonded via a -(CH₂)_{1,2} or ₃-group and which may be unsubstituted or optionally substituted with 1, 2, 3, 4 or 5 substituent(s) independently selected from the group consisting of methyl, ethyl, n-propyl, isopropyl, n-butyl, tert-butyl, sec-butyl, isobutyl, -O-CH₃, -O-C₂H₅, -S-CH₃, -S-C₂H₅, F, Cl, Br, I, -CN, -CF₃, - OCF₃, -SCF₃, -OH, -SH, -NH₂-NO₂, -CHO, -CF₂H, -CFH₂, -C(=O)-NH₂ and - S(=O)₂-CH₃;
or R⁶ and R⁷ together with the bridging nitrogen atom form a moiety selected from the group consisting of 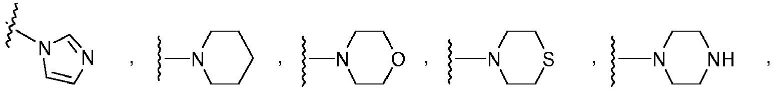 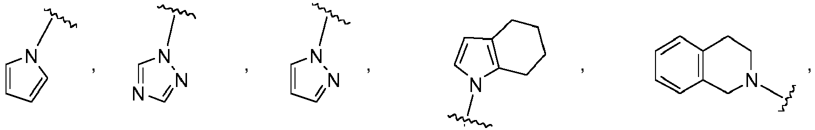 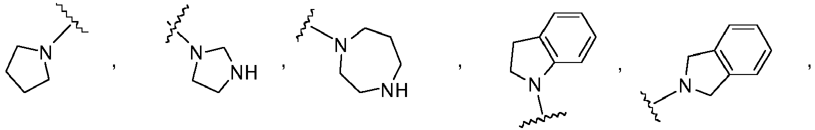 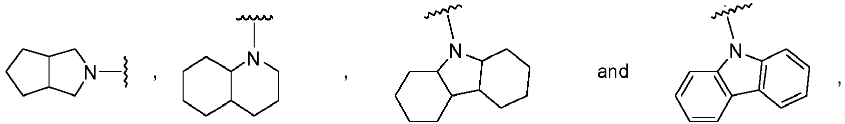 which may be unsubstituted or optionally substituted in any position including the -NH-groups with 1, 2, 3, 4 or 5 substituent(s) independently selected from the group consisting of oxo (=O), methyl, ethyl, n-propyl, isopropyl, n-butyl, tert-butyl, sec-butyl, -O-CH₃, -O-C₂H₅, -S-CH₃, -S-C₂H₅, -C(=O)-OH, -C(=O)-O-CH₃, -C(=O)-O-C₂H₅, -C (=O)-O-CH₂-CH₂-CH₃, -C(=O)-O-CH(CH₃)₂, - C(=O)-O-C(CH₃)₃, -C(=O)-CH₃, -C(=O)-C₂H₅, -C(=O)-CH₂-CH₂-CH₃, -C(=O)-CH(CH₃)₂, -C(=O)-C(CH₃)₃, F, Cl, Br, I, -CN, -CF₃, -OCF₃, -SCF₃, -OH, -SH, - NH₂, -NO₂, -CHO, -CF₂H, -CFH₂, -C(=O)-NH₂, -C(=O)-NH-CH₃, -C(=O)-NH-C₂H₅, -C(=O)-N(CH₃)₂, -C(=O)-N(C₂H₅)₂, -S(=O)₂-CH₃, phenyl, phenethyl and benzyl, whereby said cyclic substituents may be unsubstituted or substituted by 1, 2 or 3 substituent(s) independently selected from the group consisting of methyl, ethyl, n-propyl, isopropyl, methoxy, ethoxy, F, Cl, Br, -CN, -CF₃, - OCF₃, -SCF₃, -OH, -SH, -NH₂ and -NO₂;
or R⁹ and R¹⁰ together with the bridging nitrogen atom form a moiety selected from the group consisting of 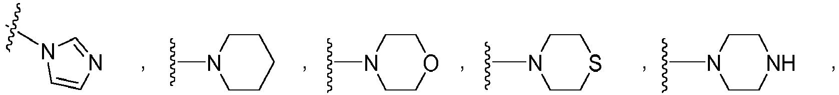 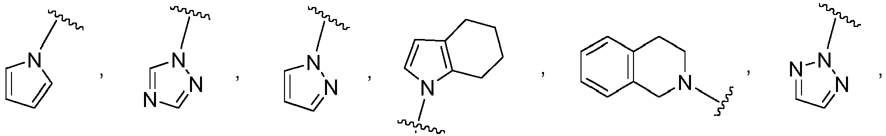 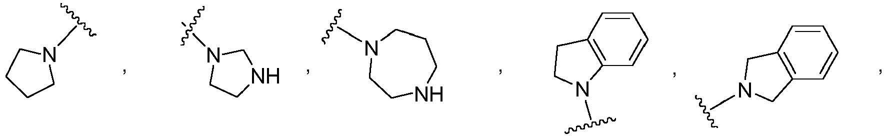 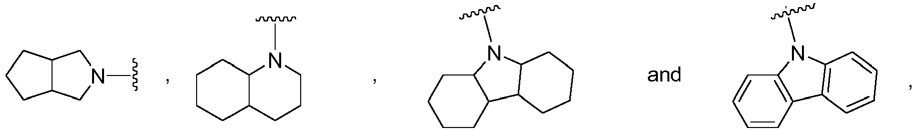 which may be unsubstituted or optionally substituted in any position including the -NH-groups with 1, 2, 3, 4 or 5 substituent(s) independently selected from the group consisting of oxo (=O), methyl, ethyl, n-propyl, isopropyl, n-butyl, tert-butyl, sec-butyl, -O-CH₃, -O-C₂H₅, -S-CH₃, -S-C₂H₅, -C(=O)-OH, -C(=O)-O-CH₃, -C(=O)-O-C₂H₅, -C(=O)-O-CH₂-CH₂-CH₃, -C(=O)-O-CH(CH₃)₂, - C(=O)-O-C(CH₃)₃, -C(=O)-CH₃, -C(=O)-C₂H₅, -C(=O)-CH₂-CH₂-CH₃, -C(=O)-CH(CH₃)₂, -C(=O)-C(CH₃)₃, F, Cl, Br, I, -CN, -CF₃, -OCF₃, -SCF₃, -OH, -SH, - NH₂, -NO₂, -CHO, -CF₂H, -CFH₂, -C(=O)-NH₂, -C(=O)-NH-CH₃, -C(=O)-NH-C₂H₅, -C(=O)-N(CH₃)₂, -C(=O)-N(C₂H₅)₂, -S(=O)₂-CH₃, phenyl, phenethyl and benzyl, whereby said cyclic substituents may be unsubstituted or substituted by 1, 2 or 3 substituent(s) independently selected from the group consisting of methyl, ethyl, n-propyl, isopropyl, methoxy, ethoxy, F, Cl, Br, -CN, -CF₃, - OCF₃, -SCF₃, -OH, -SH, -NH₂ and -NO₂.

3. A compound according to claim 1 or 2, **characterized in that** m is 1;
X represents a -OR⁵ moiety or a -NR⁶R⁷ moiety;
Y represents a -OR⁸ moiety; a -NR⁹R¹⁰ moiety or a -C(=O)-OR¹¹ moiety;
R² represents a hydrogen atom; a radical selected from the group consisting of methyl, ethyl, n-propyl, isopropyl, n-butyl, sec-butyl, isobutyl, tert-butyl and n-pentyl; or a phenyl radical which may be unsubstituted or substituted with 1, 2, 3, 4 or 5 substituent(s) independently selected from the group consisting of methyl, ethyl, n-propyl, isopropyl, n-butyl, tert-butyl, sec-butyl, isobutyl, n-pentyl, -O-CH₃, -O-C₂H₅, F, Cl, Br, I, -CN, -CF₃, -OCF₃ and -SCF₃;
R⁵ and R⁸, independently of one another, each represent a hydrogen atom; a phenyl radical, which may be bonded via a -(CH₂)_{1,2} or ₃-group and which may be unsubstituted or optionally substituted with 1, 2, 3, 4 or 5 substituent(s) independently selected from the group consisting of methyl, ethyl, n-propyl, isopropyl, n-butyl, tert-butyl, -O-CH₃, -O-C₂H₅, F, Cl, Br, I, -CN, -CF₃ and - OCF₃; or a -C(=O)-R¹² moiety;
R⁶, R⁷, R⁹ and R¹⁰, independently of one another, each represent a radical selected from the group consisting of methyl, ethyl, n-propyl, isopropyl, n-butyl, sec-butyl, isobutyl, tert-butyl and n-pentyl; or a phenyl radical, which may be bonded via a -(CH₂)_{1,2} or ₃-group and which may be unsubstituted or optionally substituted with 1, 2, 3, 4 or 5 substituent(s) independently selected from the group consisting of methyl, ethyl, n-propyl, isopropyl, n-butyl, tert-butyl, sec-butyl, isobutyl, -O-CH₃, -O-C₂H₅, F, Cl, Br, I, -CN, -CF₃, - OCF₃ and -SCF₃;
or R⁶ and R⁷ together with the bridging nitrogen atom form a moiety selected from the group consisting of 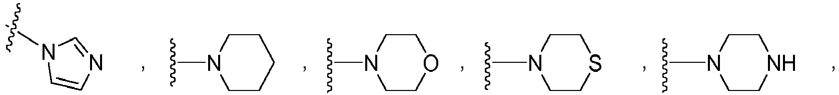 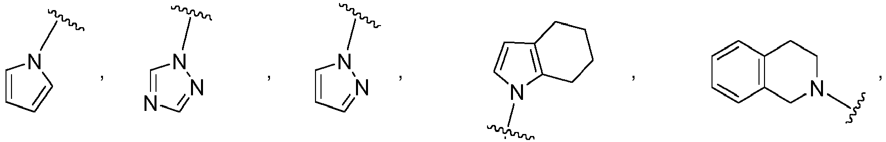 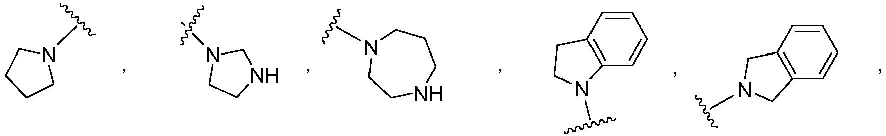 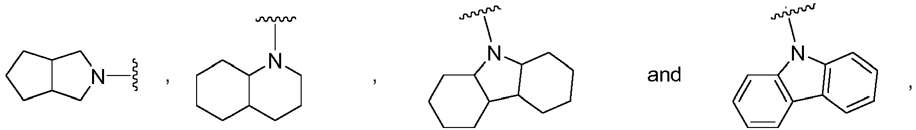 which may be unsubstituted or optionally substituted in any position including the -NH-groups with 1, 2, 3, 4 or 5 substituent(s) independently selected from the group consisting of oxo (=O), methyl, ethyl, n-propyl, isopropyl, n-butyl, tert-butyl, sec-butyl, phenyl, phenethyl and benzyl, whereby said cyclic substituents may be unsubstituted or substituted by 1, 2 or 3 substituent(s) independently selected from the group consisting of methyl, ethyl, n-propyl, isopropyl, methoxy, ethoxy, F, Cl, Br, -CN, -CF₃, -OCF₃, -SCF₃, -OH, -SH, - NH₂ and -NO₂;
or R⁹ and R¹⁰ together with the bridging nitrogen atom form a moiety selected from the group consisting of 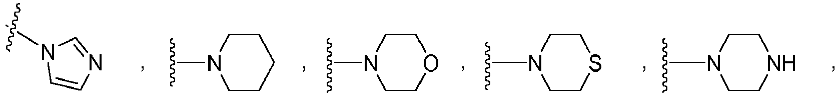 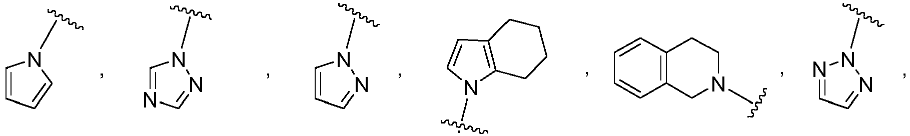 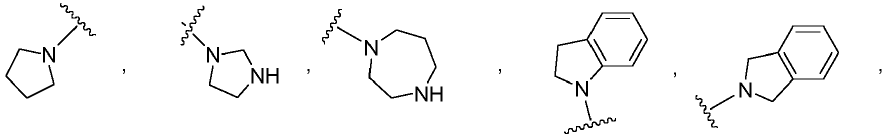 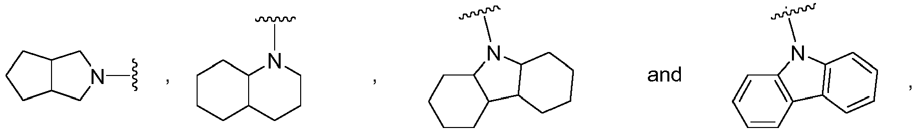 which may be unsubstituted or optionally substituted in any position including the -NH-groups with 1, 2, 3, 4 or 5 substituent(s) independently selected from the group consisting of oxo (=O), methyl, ethyl, n-propyl, isopropyl, n-butyl, tert-butyl, sec-butyl, phenyl, phenethyl and benzyl, whereby said cyclic substituents may be unsubstituted or substituted by 1, 2 or 3 substituent(s) independently selected from the group consisting of methyl, ethyl, n-propyl, isopropyl, methoxy, ethoxy, F, Cl, Br, -CN, -CF₃, -OCF₃, -SCF₃, -OH, -SH, - NH₂ and-NO₂.

4. A compound according to one or more of claims 1 to 3, **characterized in that** m is 1;
X represents a -OR⁵ moiety or a -NR⁶R⁷ moiety;
Y represents a -OR⁸ moiety; a -NR⁹R¹⁰ moiety or a -C(=O)-OR¹¹ moiety;
R² represents a hydrogen atom; a radical selected from the group consisting of methyl, ethyl, n-propyl, isopropyl, n-butyl, sec-butyl, isobutyl, tert-butyl and n-pentyl; or a phenyl radical which may be unsubstituted or substituted with 1, 2, 3, 4 or 5 substituent(s) independently selected from the group consisting of methyl, ethyl, n-propyl, isopropyl, n-butyl, tert-butyl, sec-butyl, isobutyl, n-pentyl, -O-CH₃, -O-C₂H₅, F, Cl, Br, I, -CN, -CF₃, -OCF₃ and -SCF₃;
R⁵ and R⁸, independently of one another, each represent a hydrogen atom; or a phenyl radical, which may be bonded via a -(CH₂)_{1, 2} or ₃-group and which may be unsubstituted or optionally substituted with 1, 2, 3, 4 or 5 substituent(s) independently selected from the group consisting of methyl, ethyl, n-propyl, isopropyl, n-butyl, tert-butyl, -O-CH₃, -O-C₂H₅, F, Cl, Br, I, - CN, -CF₃ and -OCF₃; or a -C(=O)-R¹² moiety;
R⁶, R⁷, R⁹ and R¹⁰, independently of one another, each represent a radical selected from the group consisting of methyl, ethyl, n-propyl, isopropyl, n-butyl, sec-butyl, isobutyl, tert-butyl and n-pentyl; or a phenyl radical, which is bonded via a -(CH₂)_{1,2 or 3}-group and which may be unsubstituted or optionally substituted with 1, 2, 3, 4 or 5 substituent(s) independently selected from the group consisting of methyl, ethyl, n-propyl, isopropyl, n-butyl, tert-butyl, sec-butyl, isobutyl, -O-CH₃, -O-C₂H₅, F, Cl, Br, I, -CN, -CF₃, - OCF₃ and -SCF₃;
or R⁶ and R⁷ together with the bridging nitrogen atom form a moiety selected from the group consisting of 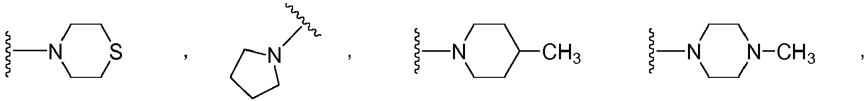 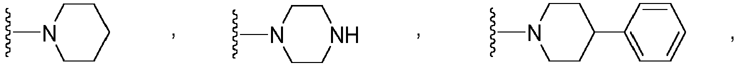 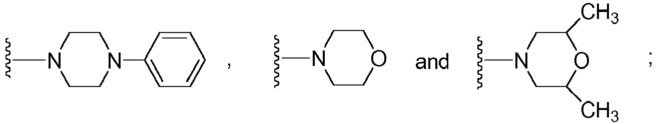 or R⁹ and R¹⁰ together with the bridging nitrogen atom form a moiety selected from the group consisting of

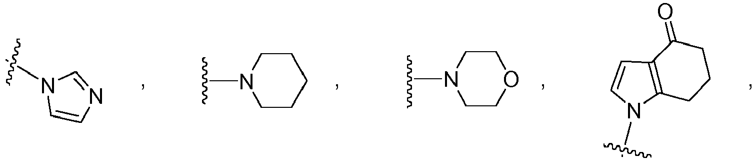

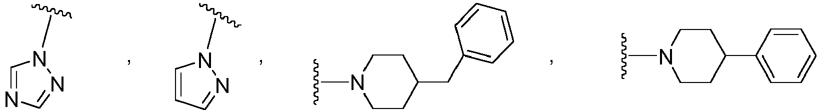

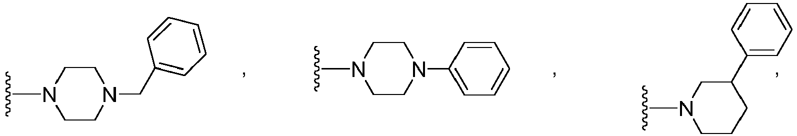

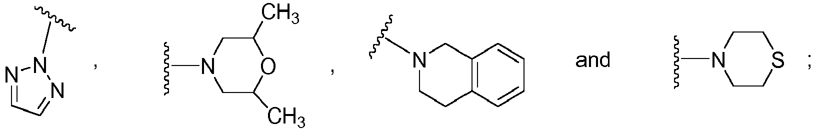

5. A compound of general formula le according to one or more of claims 1 or 2, 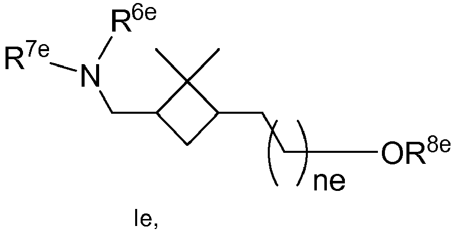 wherein ne is 0 or 1 ;
R^{6e} and R^{7e}, independently of one another, each represent a radical selected from the group consisting of methyl, ethyl, n-propyl, isopropyl, n-butyl, sec-butyl, isobutyl, tert-butyl and n-pentyl; or an aryl radical selected from the group consisting of phenyl and naphthyl, which may be bonded via a -(CH₂)₁, _{2 or 3}-group and which may be unsubstituted or optionally substituted with 1, 2, 3, 4 or 5 substituent(s) independently selected from the group consisting of methyl, ethyl, n-propyl, isopropyl, n-butyl, tert-butyl, sec-butyl, isobutyl, -O-CH₃, -O-C₂H₅, -S-CH₃, -S-C₂H₅, F, Cl, Br, I, -CN, -CF₃, -OCF₃, -SCF₃, -OH, - SH, -NH₂-NO₂, -CHO, -CF₂H, -CFH₂, -C(=O)-NH₂ and -S(=O)₂-CH₃;
or R^{6e} and R^{7e} together with the bridging nitrogen atom form a moiety selected from the group consisting of 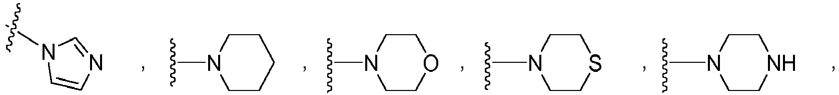 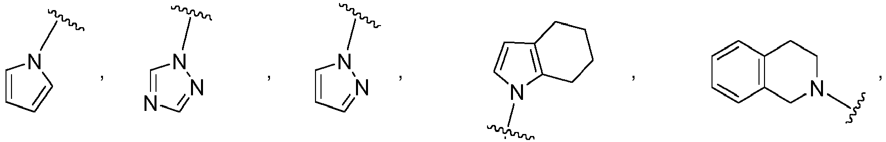 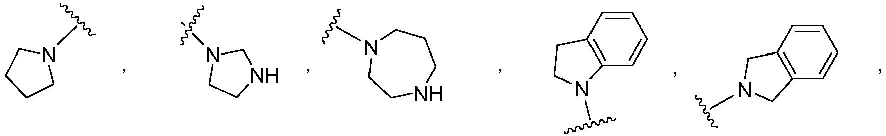 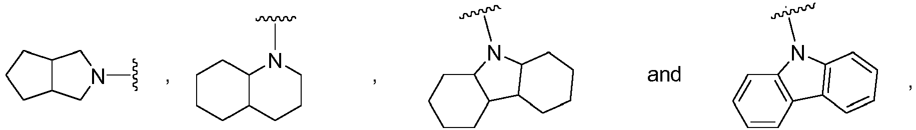 which may be unsubstituted or optionally substituted in any position including the -NH-groups with 1, 2, 3, 4 or 5 substituent(s) independently selected from the group consisting of oxo (=O), methyl, ethyl, n-propyl, isopropyl, n-butyl, tert-butyl, sec-butyl, -O-CH₃, -O-C₂H₅, -S-CH₃, -S-C₂H₅, -C(=O)-OH, -C(=O)-O-CH₃, -C(=O)-O-C₂H₅, -C(=O)-O-CH₂-CH₂-CH₃, -C(=O)-O-CH(CH₃)₂, - C(=O)-O-C(CH₃)₃, -C(=O)-CH₃, -C(=O)-C₂H₅, -C(=O)-CH₂-CH₂-CH₃, -C(=O)-CH(CH₃)₂, -C(=O)-C(CH₃)₃, F, Cl, Br, I, -CN, -CF₃, -OCF₃, -SCF₃, -OH, -SH, - NH₂, -NO₂, -CHO, -CF₂H, -CFH₂, -C(=O)-NH₂, -C(=O)-NH-CH₃, -C(=O)-NH-C₂H₅, -C(=O)-N(CH₃)₂, -C(=O)-N(C₂H₅)₂, -S(=O)₂-CH₃, phenyl, phenethyl and benzyl, whereby said cyclic substituents may be unsubstituted or substituted by 1, 2 or 3 substituent(s) independently selected from the group consisting of methyl, ethyl, n-propyl, isopropyl, methoxy, ethoxy, F, Cl, Br, -CN, -CF₃, - OCF₃, -SCF₃, -OH, -SH, -NH₂ and -NO₂;
R^{8e} represents a hydrogen atom; a radical selected from the group consisting of methyl, ethyl, n-propyl, isopropyl, n-butyl, sec-butyl, isobutyl, tert-butyl and n-pentyl; an aryl radical selected from the group consisting of phenyl and naphthyl, which may be bonded via a -(CH₂)_{1,2 or 3}-group and which may be unsubstituted or optionally substituted with 1, 2, 3, 4 or 5 substituent(s) independently selected from the group consisting of methyl, ethyl, n-propyl, isopropyl, n-butyl, tert-butyl, -O-CH₃, -O-C₂H₅-F, Cl, Br, I, -CN, -CF₃, -OCF₃, - SCF₃, -OH, -SH, -NH₂, -NO₂, -CHO, -CF₂H, -CFH₂ and -S(=O)₂-CH₃, or a - C(=O)-R^{12e} moiety; and R^{12e} represents a radical selected from the group consisting of methyl, ethyl, n-propyl, isopropyl, n-butyl, sec-butyl, isobutyl, tert-butyl and n-pentyl; optionally in form of one of its stereoisomers, preferably enantiomers or diasteromers, a racemate or in form of a mixture of at least two of its stereoisomers, preferably enantiomers and/or diastereomers, in any mixing ratio, or a salt thereof, or a corresponding solvate thereof.

6. A compound according to claim 5, **characterized in that** the stereoisomers have the general formulae If or Ig or Ih or Ij, 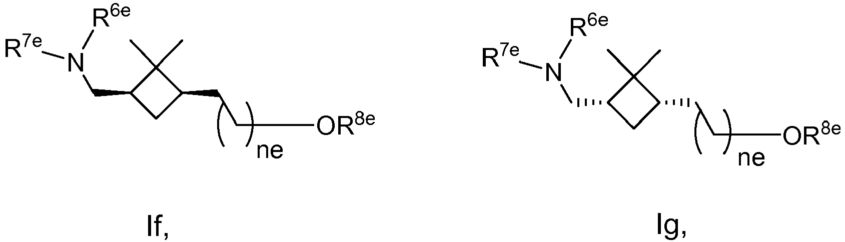 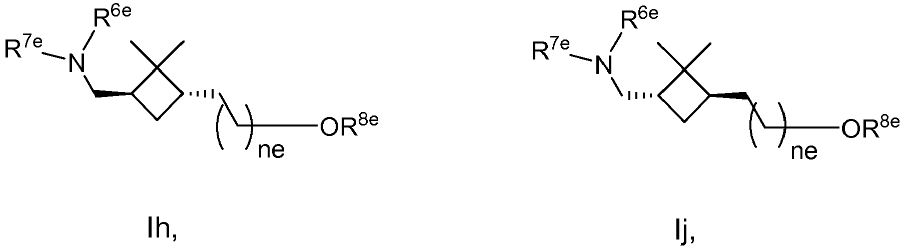 wherein ne, R^{6e}, R^{7e} and R^{8e} are defined as in claim 5.

7. A compound of general formula Ik according to one or more of claims 1 to 2, 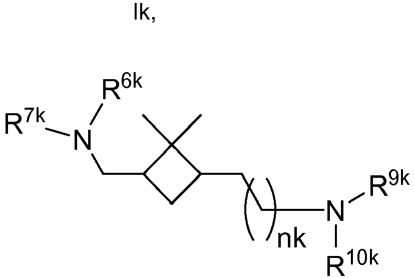 wherein
R^{6k}R^{7k}N- and R^{9k}R^{10k}N- are different;
R^{6k}, R^{7k}, R^{9k} and R^{10k}, independently of one another, each represent a radical selected from the group consisting of methyl, ethyl, n-propyl, isopropyl, n-butyl, sec-butyl, isobutyl, tert-butyl and n-pentyl; or an aryl radical selected from the group consisting of phenyl and naphthyl, which may be bonded via a -(CH₂)_{1,2 or 3}-group and which may be unsubstituted or optionally substituted with 1, 2, 3, 4 or 5 substituent(s) independently selected from the group consisting of methyl, ethyl, n-propyl, isopropyl, n-butyl, tert-butyl, sec-butyl, isobutyl, -O-CH₃, -O-C₂H₅, -S-CH₃, -S-C₂H₅, F, Cl, Br, I, -CN, -CF₃, - OCF₃, -SCF₃, -OH, -SH, -NH₂-NO₂, -CHO, -CF₂H, -CFH₂, -C(=O)-NH₂ and - S(=O)₂-CH₃;
or R^{6k} and R^{7k} together with the bridging nitrogen atom form a moiety selected from the group consisting of 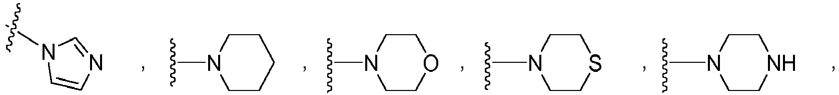 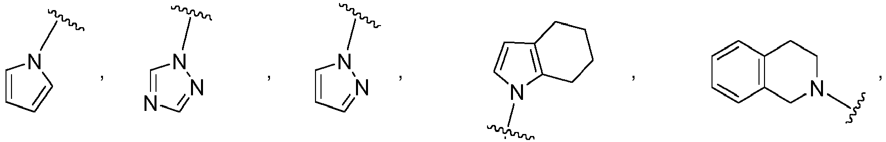 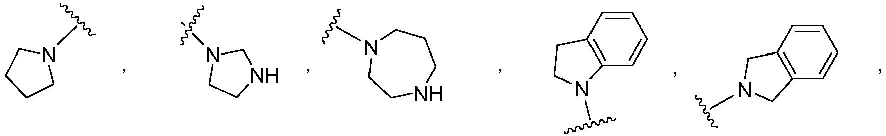 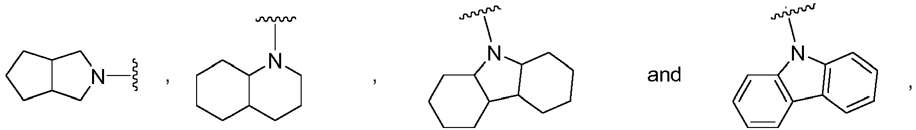 which may be unsubstituted or optionally substituted in any position including the -NH-groups with 1, 2, 3, 4 or 5 substituent(s) independently selected from the group consisting of oxo (=O), methyl, ethyl, n-propyl, isopropyl, n-butyl, tert-butyl, sec-butyl, -O-CH₃, -O-C₂H₅, -S-CH₃, -S-C₂H₅, -C(=O)-OH, -C(=O)-O-CH₃, -C(=O)-O-C₂H₅, -C(=O)-O-CH₂-CH₂-CH₃, -C(=O)-O-CH(CH₃)₂, - C(=O)-O-C(CH₃)₃, -C(=O)-CH₃, -C(=O)-C₂H₅, -C(=O)-CH₂-CH₂-CH₃, -C(=O)-CH(CH₃)₂, -C(=O)-C(CH₃)₃, F, Cl, Br, I, -CN, -CF₃, -OCF₃, -SCF₃, -OH, -SH, - NH₂, -NO₂, -CHO, -CF₂H, -CFH₂, -C(=O)-NH₂, -C(=O)-NH-CH₃, -C(=O)-NH-C₂H₅, -C(=O)-N(CH₃)₂, -C(=O)-N(C₂H₅)₂, -S(=O)₂-CH₃, phenyl, phenethyl and benzyl, whereby said cyclic substituents may be unsubstituted or substituted by 1, 2 or 3 substituent(s) independently selected from the group consisting of methyl, ethyl, n-propyl, isopropyl, methoxy, ethoxy, F, Cl, Br, -CN, -CF₃, - OCF₃, -SCF₃, -OH, -SH, -NH₂ and -NO₂;
or R^{9k} and R^{10k} together with the bridging nitrogen atom form a moiety selected from the group consisting of 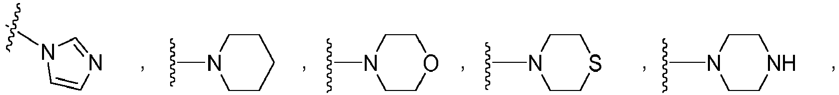 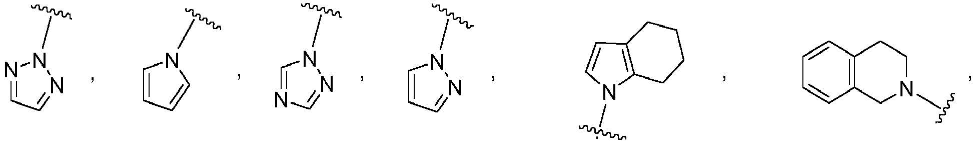 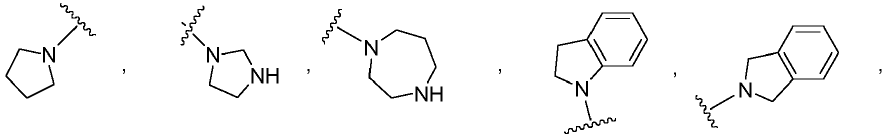 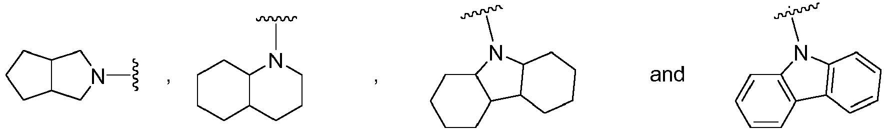 which may be unsubstituted or optionally substituted in any position including the -NH-groups with 1, 2, 3, 4 or 5 substituent(s) independently selected from the group consisting of oxo (=O), methyl, ethyl, n-propyl, isopropyl, n-butyl, tert-butyl, sec-butyl, -O-CH₃, -O-C₂H₅, -S-CH₃, -S-C₂H₅, -C(=O)-OH, -C(=O)-O-CH₃, -C(=O)-O-C₂H₅, -C(=O)-O-CH₂-CH₂-CH₃, -C(=O)-O-CH(CH₃)₂, - C(=O)-O-C(CH₃)₃, -C(=O)-CH₃, -C(=O)-C₂H₅, -C(=O)-CH₂-CH₂-CH₃, -C(=O)-CH(CH₃)₂, -C(=O)-C(CH₃)₃, F, Cl, Br, I, -CN, -CF₃, -OCF₃, -SCF₃, -OH, -SH, - NH₂, -NO₂, -CHO, -CF₂H, -CFH₂, -C(=O)-NH₂, -C(=O)-NH-CH₃, -C(=O)-NH-C₂H₅, -C(=O)-N(CH₃)₂, -C(=O)-N(C₂H₅)₂, -S(=O)₂-CH₃, phenyl, phenethyl and benzyl, whereby said cyclic substituents may be unsubstituted or substituted by 1, 2 or 3 substituent(s) independently selected from the group consisting of methyl, ethyl, n-propyl, isopropyl, methoxy, ethoxy, F, Cl, Br, -CN, -CF₃, - OCF₃, -SCF₃, -OH, -SH, -NH₂ and -NO₂;
optionally in form of one of its stereoisomers, preferably enantiomers or diasteromers, a racemate or in form of a mixture of at least two of its stereoisomers, preferably enantiomers and/or diastereomers, in any mixing ratio, or a salt thereof, or a corresponding solvate thereof.

8. A compound according to claim 7, **characterized in that** the stereoisomers have the general formulae Im or In or lo or Ip, 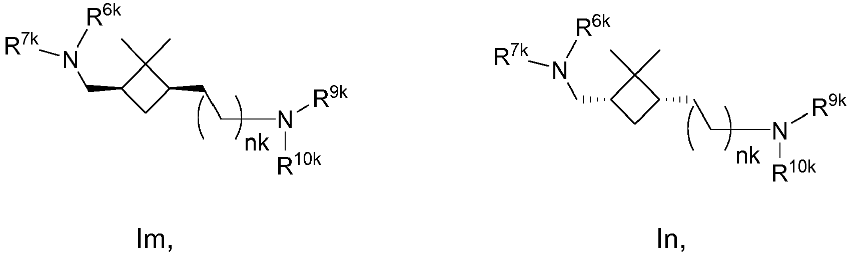 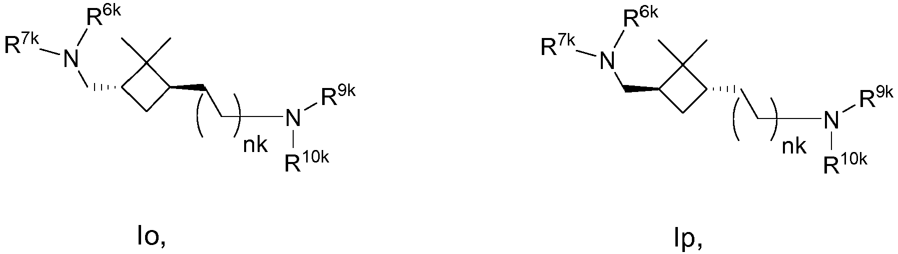 wherein nk, R^{6k}, R^{7k}, R^{9k} and R^{10k} are defined as in claim 7.

9. A compound according to one or more of claims 1 to 8 selected from the group consisting of
[1] 4-(((1S,3S)-3-(2-(benzyloxy)ethyl)-2,2-dimethylcyclobutyl)methyl)-morpholine
[2] 1-(((1S,3S)-3-(2-(benzyloxy)ethyl)-2,2-dimethylcyclobutyl)methyl)-piperidine
[3] 1-(((1S,3S)-3-(2-(benzyloxy)ethyl)-2,2-dimethylcyclobutyl)methyl)-4-phenylpiperidine
[4] 1-(((1S,3S)-3-(2-(benzyloxy)ethyl)-2,2-dimethylcyclobutyl)methyl)-4-phenylpiperazine
[5] 1-(((1S,3S)-3-(2-(benzyloxy)ethyl)-2,2-dimethylcyclobutyl)methyl)-4-methylpiperazine
[6] 4-(((1R,3S)-3-(benzyloxymethyl)-2,2-dimethylcyclobutyl)methyl)-morpholine
[7] 1-(((1R,3S)-3-(benzyloxymethyl)-2,2-dimethylcyclobutyl)methyl)-piperidine
[8] 1-(((1R,3S)-3-(benzyloxymethyl)-2,2-dimethylcyclobutyl)methyl)-4-phenylpiperidine
[9] 1-(((1R,3S)-3-(benzyloxymethyl)-2,2-dimethylcyclobutyl)methyl)-4-phenylpiperazine
[10] 1-(((1R,3S)-3-(benzyloxymethyl)-2,2-dimethylcyclobutyl)methyl)-4-methylpiperazine
[13] 1-((1R,3R)-2,2-dimethyl-3-(2-morpholinoethyl)cyclobutyl)-1-phenylethanol
[14] 1-((1R,3R)-2,2-dimethyl-3-(2-(piperidin-1-yl)ethyl)cyclobutyl)-1-phenylethanol
[15] 4-(((1R,3R)-2,2-dimethyl-3-(2-phenoxyethyl)cyclobutyl)methyl)-morpholine
[16] 1-(((1R,3R)-2,2-dimethyl-3-(2-phenoxyethyl)cyclobutyl)methyl)piperidine
[17] 2-((1R,3R)-2,2-dimethyl-3-(piperidin-1-ylmethyl)cyclobutyl)ethanol
[18] 2-((1S,3S)-2,2-dimethyl-3-(piperidin-1-ylmethyl)cyclobutyl)ethanol
[19] 2-((1R,3R)-2,2-dimethyl-3-(morpholinomethyl)cyclobutyl)ethanol
[20] 2-((1S,3S)-2,2-dimethyl-3-(morpholinomethyl)cyclobutyl)ethanol
[21] 2-((1R,3R)-2,2-dimethyl-3-((4-methylpiperidin-1-yl)methyl)cyclobutyl)-ethanol
[22] 2-((1R,3R)-2,2-dimethyl-3-((4-phenylpiperidin-1-yl)methyl)cyclobutyl)-ethanol
[23] 2-((1R,3R)-3-(((2S,6R)-2,6-dimethylmorpholino)methyl)-2,2-dimethylcyclobutyl)ethanol
[24] 2-((1R,3R)-3-(N-benzyl-N-methylamino)methyl)-2,2-dimethylcyclobutyl)-ethanol
[26] methyl 2-((1R,3R)-2,2-dimethyl-3-(piperidin-1-ylmethyl)cyclobutyl)-acetate
[27] 1-(((1R,3R)-3-(2-(benzyloxy)ethyl)-2,2-dimethylcyclobutyl)methyl)-piperidine
[28] 1-(((1R,3R)-3-(2-(benzyloxy)ethyl)-2,2-dimethylcyclobutyl)methyl)-4-methylpiperidine
[29] 1-(((1R,3R)-3-(2-(benzyloxy)ethyl)-2,2-dimethylcyclobutyl)methyl)-4-phenylpiperidine
[30] 4-(((1R,3R)-3-(2-(benzyloxy)ethyl)-2,2-dimethylcyclobutyl)methyl)-thiomorpholine
[31] 4-(((1R,3R)-3-(2-(benzyloxy)ethyl)-2,2-dimethylcyclobutyl)methyl)-morpholine
[32] (2S,6R)-4-(((1R,3R)-3-(2-(benzyloxy)ethyl)-2,2-dimethylcyclobutyl)-methyl)-2,6-dimethylmorpholine
[33] 1-(((1R,3R)-3-(2-(benzyloxy)ethyl)-2,2-dimethylcyclobutyl)methyl)-pyrrolidine
[34] 1-(((1R,3R)-3-(2-(benzyloxy)ethyl)-2,2-dimethylcyclobutyl)methyl)-4-methylpiperazine
[35] 1-(((1R,3R)-3-(2-(benzyloxy)ethyl)-2,2-dimethylcyclobutyl)methyl)-4-methylpiperazine oxalate
[36] 1-(((1R,3R)-3-(2-(benzyloxy)ethyl)-2,2-dimethylcyclobutyl)methyl)-4-phenylpiperazine
[37] 1-(((1R,3R)-3-(2-(benzyloxy)ethyl)-2,2-dimethylcyclobutyl)methyl)-4-phenylpiperazine oxalate
[38] 1-(2-((1R,3R)-3-(benzyloxymethyl)-2,2-dimethylcyclobutyl)ethyl)-piperidine
[39] 4-(2-((1R,3R)-3-(benzyloxymethyl)-2,2-dimethylcyclobutyl)ethyl)-morpholine
[40] 4-(((1R,3R)-2,2-dimethyl-3-(2-(3-phenylpiperidin-1-yl)ethyl)cyclobutyl)-methyl)-morpholine
[41] 4-(((1R,3R)-2,2-dimethyl-3-(2-(4-phenylpiperidin-1-yl)ethyl)cyclobutyl)-methyl)morpholine
[42] 4-(((1R,3R)-3-(2-(4-benzylpiperidin-1-yl)ethyl)-2,2-dimethylcyclobutyl)-methyl)morpholine
[43] 4-(((1 R,3R)-3-(2-(1 H-imidazol-1-yl)ethyl)-2,2-dimethylcyclobutyl)-methyl)morpholine
[44] 4-(((1 R,3R)-3-(2-(1 H-pyrazol-1-yl)ethyl)-2,2-dimethylcyclobutyl)-methyl)morpholine
[45] 4-(((1R,3R)-3-(2-(1H-1,2,4-triazol-1-yl)ethyl)-2,2-dimethylcyclobutyl)-methyl)morpholine
[46] 1-(2-((1 R,3R)-2,2-dimethyl-3-(morpholinomethyl)cyclobutyl)ethyl)-6,7-dihydro-1H-indol-4(5H)-one
[47] 2-(2-((1R,3R)-2,2-dimethyl-3-(morpholinomethyl)cyclobutyl)ethyl)-1,2,3,4-tetrahydroisoquinoline
[48] 1-(((1 R,3R)-3-(2-(1 H-imidazol-1-yl)ethyl)-2,2-dimethylcyclobutyl)-methyl)piperidine
[49] 1-(((1 R,3R)-3-(2-(1 H-pyrazol-1-yl)ethyl)-2,2-dimethylcyclobutyl)-methyl)piperidine
[50] 1-(((1R,3R)-3-(2-(1H-1,2,4-triazol-1-yl)ethyl)-2,2-dimethylcyclobutyl)-methyl)piperidine
[51] 1-(2-((1R,3R)-2,2-dimethyl-3-(piperidin-1-ylmethyl)cyclobutyl)ethyl)-4-phenylpiperidine
[52] 4-benzyl-1-(2-((1R,3R)-2,2-dimethyl-3-(piperidin-1-ylmethyl)cyclobutyl)-ethyl)piperidine
[53] 1-(2-((1R,3R)-2,2-dimethyl-3-(piperidin-1-ylmethyl)cyclobutyl)ethyl)-6,7-dihydro-1H-indol-4(5H)-one
[54] 2-(2-((1R,3R)-2,2-dimethyl-3-(piperidin-1-ylmethyl)cyclobutyl)ethyl)-1,2,3,4-tetrahydroisoquinoline
[55] 2-((1*R*,3*R*)-2,2-Dimethyl-3-((piperidin-1-yl)methyl)cyclobutyl)ethyl pivalate
[56] 2-((1*R*,3*R*)-2,2-Dimethyl-3-((piperidin-1-yl)methyl)cyclobutyl)ethyl acetate
optionally in form of one of its stereoisomers, preferably enantiomers or diasteromers, a racemate or in form of a mixture of at least two of its stereoisomers, preferably enantiomers and/or diastereomers, in any mixing ratio, or a salt thereof, or a corresponding solvate thereof.

10. Process for the preparation of a compound of general formula I according to one or more of claims 1 to 9, **characterized in that** at least one compound of general formula II, 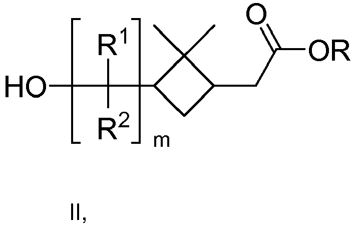 wherein m, R¹ and R² have the meaning according to one or more of claims 1 to 9 and R represents a linear or branched C₁₋₅-alkyl radical or a benzyl radical,
is reacted with methane sulfonylchloride, *p*-toluene sulfonylchloride, trifluormethane sulfonylchloride, thionyl chloride or tetrabromethane, preferably in a reaction medium, preferably in the presence of at least one base, to yield at least one compound of general formula III, 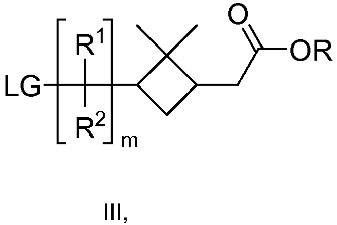 wherein m, R¹ and R² have the meaning given above, R represents a linear or branched C₁₋₅-alkyl radical and LG represents -O-S(=O)₂-CH₃, -O-S(=O)₂-p-toluyl, -O-S(=O)₂-CF₃, Cl or Br, which is optionally purified and/or isolated, and at least one compound of general formula III is reacted with at least one compound of general formula HNR⁶R⁷, wherein R⁶ and R⁷ have the meaning according to one or more of claims 1 to 9, preferably in a reaction medium, preferably in the presence of at least one base, to yield at least one compound of general formula IV, 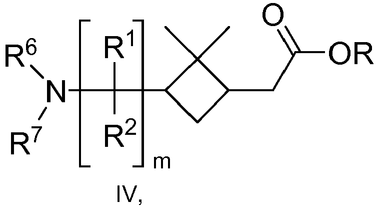 wherein m, R¹, R², R⁶ and R⁷ have the meaning given above and R represents a linear or branched C₁₋₅-alkyl radical, which is optionally purified and/or isolated,
and at least one compound of general formula IV is reacted with at least one reducing agent selected from the group consisting of lithium borohydride, sodium borohydride, lithium aluminium hydride and diborane, preferably with lithium borohydride, preferably in a reaction medium, to yield at least one compound of general formula V, 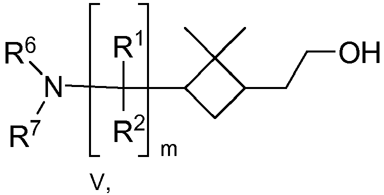 wherein m, R¹, R², R⁶ and R⁷ have the meaning given above, which is optionally purified and/or isolated,
and at least one compound of general formula V is reacted with methane sulfonylchloride, p-toluene sulfonylchloride, trifluormethane sulfonylchloride, thionyl chloride or tetrabromethane,
preferably in a reaction medium, preferably in the presence of at least one base, to yield at least one compound of general formula VI, 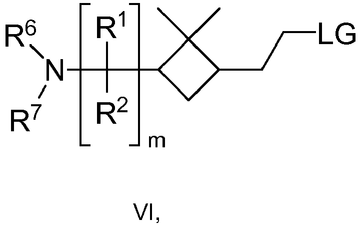 wherein m, R¹, R², R⁶ and R⁷ have the meaning given above and LG represents -O-S(=O)₂-CH₃, -O-S(=O)₂-p-toluyl, -O-S(=O)₂-CF₃, Cl or Br, which is optionally purified and/or isolated,
and at least one compound of general formula VI is reacted with at least one compound of general formula HNR⁹R¹⁰, wherein R⁹ and R¹⁰ have the meaning according to one or more of claims 1 to 9, preferably in a reaction medium, preferably in the presence of at least one base, to yield at least one compound of general formula VII, 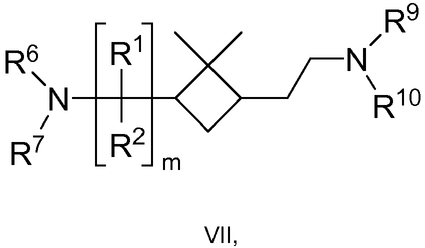 wherein m, R¹, R², R⁶, R⁷, R⁹ and R¹⁰ have the meaning given above, which is optionally purified and/or isolated,
or at least one compound of general formula VI is reacted with at least one compound of general formula M-OR⁸, wherein R⁸ has the meaning according to one or more of claims 1 to 9 and M represents a monovalent kation selected from the group consisting of sodium, magnesium, potassium and lithium, preferably M represents a lithium kation, preferably in a reaction medium, preferably in the presence of at least one base, to yield at least one compound of general formula VIII, 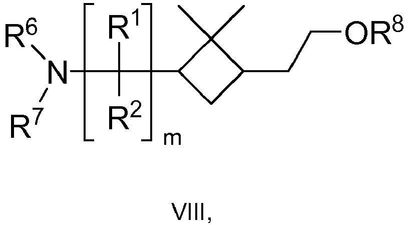 wherein m, R¹, R², R⁶, R⁷ and R⁸ have the meaning given above, which is optionally purified and/or isolated;
and optionally at least one compound of general formula VIII, wherein R⁸ represents hydrogen, is reacted with at least one compound of general formula LG-C(=O)-R¹², wherein R¹² has the meaning according to one or more of claims 1 to 9 and LG represents a leaving group, preferably a leaving group selected from the group consisting of chlorine and bromine, preferably in a reaction medium, preferably in the presence of at least one base,
or with at least one compound of general formula HO-C(=O)-R¹², wherein R¹² has the meaning according to one or more of claims 1 to 9, preferably in a reaction medium, preferably in the presence of at least one base, preferably in the presence of at least one coupling agent, to yield at least one compound of general formula VIIIa, 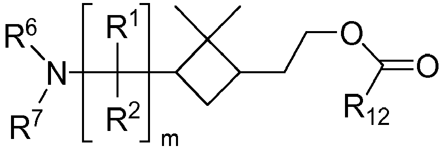 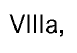 wherein m, R¹, R², R⁶, R⁷ and R¹² have the meaning given above, which is optionally purified and/or isolated.

11. Process for the preparation of a compound of general formula I according to one or more of claims 1 to 9, **characterized in that** at least one compound of general formula II, 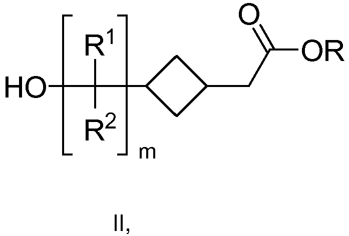 wherein m, R¹ and R² have the meaning according to one or more of claims 1 to 9 and R represents a linear or branched C₁₋₅-alkyl radical, is reacted with at least one compound of general formula R⁵-LG, wherein R⁵ has the meaning according to one or more of claims 1 to 9 and LG represents a leaving group, preferably LG represents a leaving group selected from the group consisting of chlorine, bromine, -O-S(=O)₂-CH₃, -O-S(=O)₂-CF₃ and - O-S(=O)₂-*p*-toluyl, more preferably LG represents bromine, preferably in a reaction medium, preferably in the presence of at least one base, more preferably in the presence of at least one base selected from the group consisting of butyllithium, sodium ethoxide, potassium tert-butoxide, sodium hydride and potassium hydride, to yield at least one compound of general formula IX, 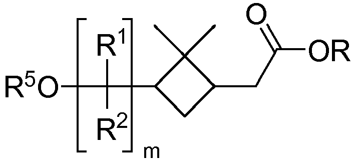 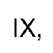 wherein m, R¹, R² and R⁵ have the meaning given above and R represents a linear or branched C₁₋₅-alkyl radical, which is optionally purified and/or isolated,
and at least one compound of general formula IX is reacted with at least one reducing agent selected from the group consisting of lithium borohydride, sodium borohydride, lithium aluminium hydride and diborane, preferably with lithium borohydride, preferably in a reaction medium, to yield at least one compound of general formula X, 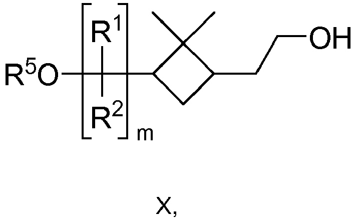 wherein m, R¹, R² and R⁵ have the meaning given above, which is optionally purified and/or isolated,
and optionally at least one compound of general formula X, is reacted with at least one compound of general formula LG-C(=O)-R¹², wherein R¹² has the meaning according to one or more of claims 1 to 9 and LG represents a leaving group, preferably a leaving group selected from the group consisting of chlorine and bromine, preferably in a reaction medium, preferably in the presence of at least one base,
or with at least one compound of general formula HO-C(=O)-R¹², wherein R¹² has the meaning according to one or more of claims 1 to 9, preferably in a reaction medium, preferably in the presence of at least one base, preferably in the presence of at least one coupling agent,
to yield at least one compound of general formula Xa, 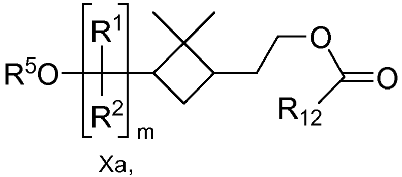 wherein m, R¹, R² and R⁵ have the meaning given above, which is optionally purified and/or isolated,
and at least one compound of general formula X is reacted with methane sulfonylchloride, p-toluene sulfonylchloride, trifluormethane sulfonylchloride, thionyl chloride or tetrabromethane, preferably in a reaction medium, preferably in the presence of at least one base, to yield at least one compound of general formula XI, 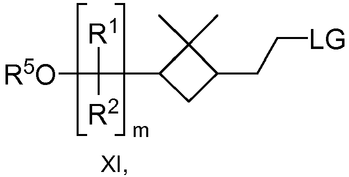 wherein m, R¹, R² and R⁵ have the meaning given above and LG represents -O-S(=O)₂-CH₃, -O-S(=O)₂-p-toluyl, -O-S(=O)₂-CF₃, Cl or Br, which is optionally purified and/or isolated,
and at least one compound of general formula XI is reacted with at least one compound of general formula HNR⁹R¹⁰, wherein R⁹ and R¹⁰ have the meaning according to one or more of claims 1 to 9, preferably in a reaction medium, preferably in the presence of at least one base, to yield at least one compound of general formula XII, 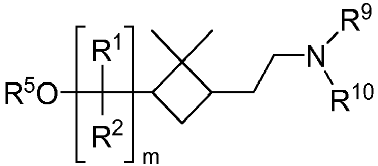 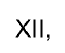 wherein m, R¹, R², R⁵, R⁹ and R¹⁰ have the meaning given above, which is optionally purified and/or isolated.

12. Process for the preparation of a compound of general formula I according to one or more of claims 1 to 9, **characterized in that** at least one compound of general formula XIII, 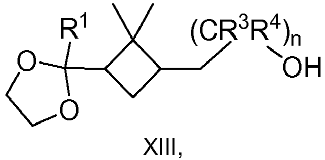 wherein R¹, R3 and R⁴ have the meaning according to one or more of claims 1 to 9, is reacted with methane sulfonylchloride, p-toluene sulfonylchloride, trifluormethane sulfonylchloride, thionyl chloride or tetrabromethane, preferably in a reaction medium, preferably in the presence of at least one base, to yield at least one compound of general formula XIV, 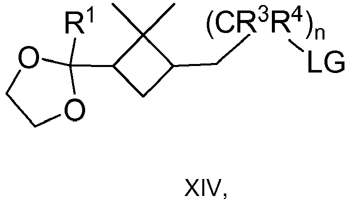 wherein R¹, R³ and R⁴ have have the meaning given above and LG represents -O-S(=O)₂-CH₃, -O-S(=O)₂-p-toluyl, -O-S(=O)₂-CF₃, Cl or Br, which is optionally purified and/or isolated,
and at least one compound of general formula XIV is reacted with at least one compound of general formula HNR⁹R¹⁰, wherein R⁹ and R¹⁰ have the meaning according to one or more of claims 1 to 9, preferably in a reaction medium, preferably in the presence of at least one base, to yield at least one compound of general formula XV, 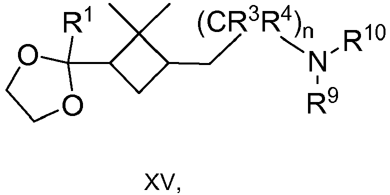 wherein R¹, R³, R⁴, R⁹ and R¹⁰ have the meaning given above, which is optionally purified and/or isolated,
and at least one compound of general formula XV is reacted with at least one reagent selected from the group consisting of hydrochloric acid, pyridinium *p-*toluenesulfonate, sulfonic acid and trifluoroacetic acid , preferably with pyridinium *p*-toluenesulfonate, preferably in a reaction medium, to yield at least one compound of general formula XVI, 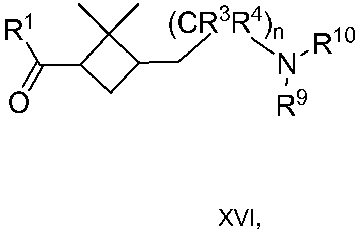 wherein R¹, R³, R⁴, R⁹ and R¹⁰ have the meaning given above, which is optionally purified and/or isolated,
and at least one compound of general formula XVI is reacted with at least one compound of general formula R²-Li, wherein R² has the meaning according to one or more of claims 1 to 9, or R²-Mg-Z, wherein R² has the meaning according to one or more of claims 1 to 9 and Z represents an anion selected from the group consisting of bromide and chloride, preferably in a reaction medium, to yield at least one compound of general formula XVII, 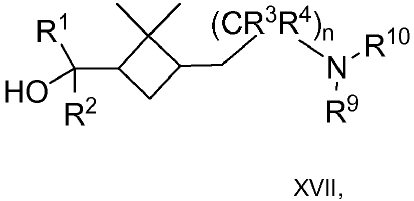 wherein R¹, R², R³, R⁴, R⁹ and R¹⁰ have the meaning given above, which is optionally purified and/or isolated,
and optionally at least one compound of general formula XVII is reacted with at least one compound of general formula R⁵-LG, wherein R⁵ has the meaning according to one or more of claims 1 to 9 and LG represents a leaving group, preferably LG represents a leaving group selected from the group consisting of chlorine, bromine, -O-S(=O)₂-CH₃, -O-S(=O)₂-CF₃ and - O-S(=O)₂-*p*-toluyl more preferably LG represents bromine, preferably in a reaction medium, preferably in the presence of at least one base, more preferably in the presence of at least one base selected from the group consisting of butyllithium, sodium ethoxide, potassium tert-butoxide, sodium hydride and potassium hydride, to yield at least one compound of general formula XVIII, 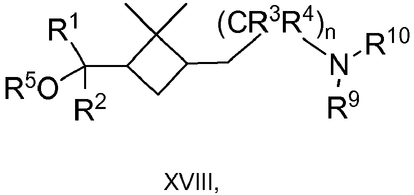 wherein R¹, R², R³, R⁴, R⁵, R⁹ and R¹⁰ have the meaning given above, which is optionally purified and/or isolated.

13. Process for the preparation of a compound of genera formula I according to one or more of claims 1 to 9, **characterized in that** at least one compound of general formula IXX, 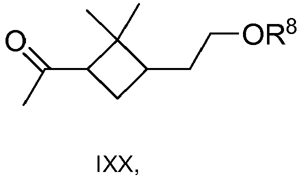 wherein R⁸ has the meaning according to one or more of claims 1 to 9, is reacted with NaOBr, preferably in a reaction medium, more preferably in a reaction medium selected from the group consisting of dioxane, THF and water or a mixture thereof, to yield at least one compound of general formula XX, 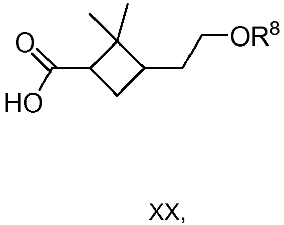 wherein R⁸ has the meaning according to one or more of claims 1 to 9, which is optionally purified and/or isolated,
and at least one compound of general formula XX is reacted with methyl iodide, preferably in a reaction medium, preferably in the presence of at least one base, more preferably in the presence of caesium carbonate, to yield at least one compound of general formula XXI, 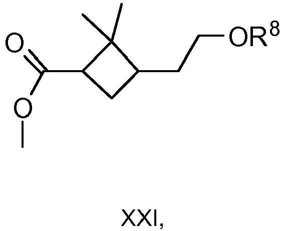 wherein R⁸ has the meaning according to one or more of claims 1 to 9, which is optionally purified and/or isolated,
and at least one compound of general formula XXI is reacted with at least one reducing agent selected from the group consisting of lithium borohydride, sodium borohydride, lithium aluminium hydride and diborane, preferably with lithium borohydride, preferably in a reaction medium, to yield at least one compound of general formula XXII, 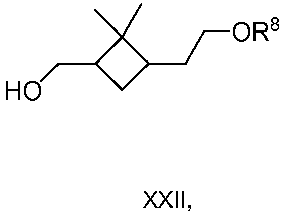 wherein R⁸ has the meaning according to one or more of claims 1 to 9, which is optionally purified and/or isolated.

14. A medicament comprising at least one compound of general formula I according to one or more of claims 1 to 9 and optionally at least one physiologically acceptable auxiliary agent.

15. A medicament according to claim 14, for use in the prophylaxis and/or treatment of pain, preferably neuropathic pain, allodynia, analgesia, causalgia, central pain, dysesthesia, hyperesthesia, hyperalgesia, hypoalgesia, hypoesthesia, or neuralgia, more preferably neuropathic pain, hyperalgesia or allodynia.

16. A substituted dimethylcyclobutyl compound of general formula I, 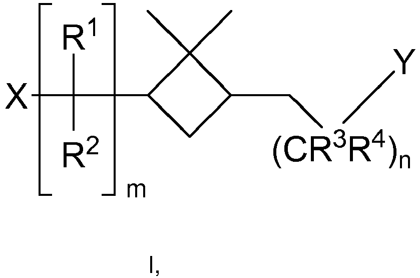 Wherein m, n, X, Y, R¹, R², R³ and R⁴ are as defined in claim 1,
optionally in form of one of its stereoisomers, preferably enantiomers or diasteromers, a racemate or in form of a mixture of at least two of its stereoisomers, preferably enantiomers and/or diastereomers, in any mixing ratio, or a salt thereof, or a corresponding solvate thereof,
for use in the treatment and/or prophylaxis of pain, preferably neuropathic pain, allodynia, analgesia, causalgia, central pain, dysesthesia, hyperesthesia, hyperalgesia, hypoalgesia, hypoesthesia, or neuralgia, more preferably neuropathic pain, hyperalgesia or allodynia.

## Patentansprüche

1. Substituierte Dimethylcyclobutylverbindung der allgemeinen Formel I: 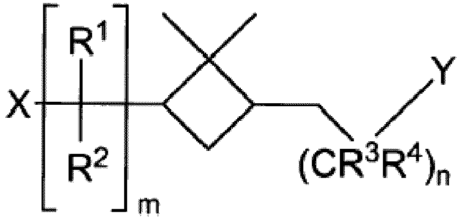 wobei
m 1 oder 2 ist;
n 0 oder 1 ist;
X und Y voneinander verschieden sind;
X für eine OR⁵-Struktureinheit oder eine -NR⁶R⁷-Struktureinheit steht, oder für einen (hetero)cycloaliphatischen Rest, der aus der Gruppe, die aus Pyrrolidinyl, Piperidinyl, Morpholinyl, Thiomorpholinyl, Piperazinyl, Pyrazolidinyl, Indolinyl, Isoindolinyl, (1,2,3,4)-Tetrahydrochinolinyl und (1,2,3,4)-Tetrahydroisochinolinyl besteht, ausgewählt ist und unsubstituiert oder gegebenenfalls mit 1, 2, 3, 4 oder 5 Substituenten substituiert sein kann, die unabhängig aus der Gruppe ausgewählt sind, die aus Oxo (=O), Thioxo (=S), Methyl, Ethyl, n-Propyl, Isopropyl, n-Butyl, tert-Butyl, sek-Butyl, Isobutyl, n-Pentyl, -O-CH₃, -O-C₂H₅, F, Cl, Br, I, -CN, -CF₃, -OH, -SH und -NH₂ besteht;
Y für eine -OR⁸-Struktureinheit, eine -NR⁹R¹⁰-Struktureinheit, eine -C(=O)-OR¹¹-Struktureinheit oder einen (hetero)cycloaliphatischen Rest steht, der aus der Gruppe, die aus Pyrrolidinyl, Piperidinyl, Morpholinyl, Thiomorpholinyl, Piperazinyl, Pyrazolidinyl, Indolinyl, Isoindolinyl, (1,2,3,4)-Tetrahydrochinolinyl und (1,2,3,4)-Tetrahydroisochinolinyl besteht, ausgewählt ist und unsubstituiert oder gegebenenfalls mit 1, 2, 3, 4 oder 5 Substituenten substituiert sein kann, die unabhängig aus der Gruppe ausgewählt sind, die aus Methyl, Ethyl, n-Propyl, Isopropyl, n-Butyl, tert-Butyl, sek-Butyl, Isobutyl, n-Pentyl, -O-CH₃, -O-C₂H₅, F, Cl, Br, I, -CN, -CF₃, -OH, -SH und -NH₂ besteht;
R¹ für ein Wasserstoffatom oder einen Rest steht, der aus der Gruppe ausgewählt ist, die aus Methyl, Ethyl, n-Propyl, Isopropyl, n-Butyl, sek-Butyl, Isobutyl, tert-Butyl und n-Pentyl besteht;
R² steht für ein Wasserstoffatom; für einen Rest, der aus der Gruppe ausgewählt ist, die aus Methyl, Ethyl, n-Propyl, Isopropyl, n-Butyl, sek-Butyl, Isobutyl, tert-Butyl und n-Pentyl besteht; oder für einen Arylrest, der aus der Gruppe, die aus Phenyl und Naphthyl besteht, ausgewählt ist und die unsubstituiert oder gegebenenfalls mit 1, 2, 3, 4 oder 5 Substituenten substituiert sein können, die unabhängig aus der Gruppe ausgewählt sind, die aus Methyl, Ethyl, n-Propyl, Isopropyl, n-Butyl, tert-Butyl, sek-Butyl, Isobutyl, n-Pentyl, -O-CH₃, -O-C₂H₅, -S-CH₃, -S-C₂H₅,-C(=O)-OH, -C(=O)-O-CH₃, -C(=O)-O-C₂H₅, -C(=O)-CH₃, -C(=O)-C₂H₅, F, Cl, Br, I, -CN, -CF₃, -OCF₃, -SCF₃, -OH, -SH, -NH₂, -NO₂, -CHO, -CF₂H und -CFH₂ besteht;
R³ und R⁴ beide für ein Wasserstoffatom stehen;
R⁵ und R⁸ stehen unabhängig voneinander jeweils für ein Wasserstoffatom; für einen Rest, der aus der Gruppe ausgewählt ist, die aus Methyl, Ethyl, n-Propyl, Isopropyl, n-Butyl, sek-Butyl, Isobutyl, tert-Butyl und n-Pentyl besteht; für einen Arylrest, der aus der Gruppe, die aus Phenyl und Naphthyl besteht, ausgewählt ist, und über eine -(CH₂)_{1,2 oder 3}-Gruppe gebunden sein können und die unsubstituiert oder gegebenenfalls mit 1, 2, 3, 4 oder 5 Substituenten substituiert sein können, die unabhängig aus der Gruppe ausgewählt sind, die aus Methyl, Ethyl, n-Propyl, Isopropyl, n-Butyl, tert-Butyl, sek-Butyl, Isobutyl, n-Pentyl, -O-CH₃, -O-C₂H₅, -S-CH₃, -S-C₂H₅, -C(=O)-OH, -C(=O)-O-CH₃,-C(=O)-O-C₂H₅, -C(=O)-CH₃, -C(=O)-C₂H₅, F, Cl, Br, I, -CN, -CF₃, -OCF₃,-SCF₃, -OH, -SH, -NH₂, -NO₂, -CHO, -CF₂H, -CFH₂ und -S(=O)₂-CH₃ besteht; oder für eine -C(=O)-R¹²-Struktureinheit;
mit der Maßgabe, dass
wenn R⁵ für Wasserstoff steht und m = 1 ist,
dann R¹ für einen Rest steht, der aus der Gruppe ausgewählt ist, die aus Methyl, Ethyl, n-Propyl, Isopropyl, n-Butyl, sek-Butyl, Isobutyl, tert-Butyl und n-Pentyl besteht, und
R² steht für einen Rest, der aus der Gruppe ausgewählt ist, die aus Methyl, Ethyl, n-Propyl, Isopropyl, n-Butyl, sek-Butyl, Isobutyl, tert-Butyl und n-Pentyl besteht; oder für einen Arylrest, der aus der Gruppe, die aus Phenyl und Naphthyl besteht, ausgewählt ist und die unsubstituiert oder gegebenenfalls mit 1, 2, 3, 4 oder 5 Substituenten substituiert sein können, die unabhängig aus der Gruppe ausgewählt sind, die aus Methyl, Ethyl, n-Propyl, Isopropyl, n-Butyl, tert-Butyl, sek-Butyl, Isobutyl, n-Pentyl, -O-CH₃, -O-C₂H₅, -S-CH₃, -S-C₂H₅, -C(=O)-OH, -C(=O)-O-CH₃, -C(=O)-O-C₂H₅,-C(=O)-CH₃, -C(=O)-C₂H₅, F, Cl, Br, I, -CN, -CF₃, -OCF₃, -SCF₃, -OH, -SH, -NH₂, -NO₂, -CHO, -CF₂H und -CFH₂ besteht;
R⁶, R⁷, R⁹ und R¹⁰ stehen unabhängig voneinander jeweils für einen Rest, der aus der Gruppe ausgewählt ist, die aus Methyl, Ethyl, n-Propyl, Isopropyl, n-Butyl, sek-Butyl, Isobutyl, tert-Butyl und n-Pentyl besteht;
oder für einen Arylrest, der aus der Gruppe, die aus Phenyl und Naphthyl besteht, ausgewählt ist und die über eine -(CH₂)_{1,2 oder 3}-Gruppe gebunden sein können und die unsubstituiert oder gegebenenfalls mit 1, 2, 3, 4 oder 5 Substituenten substituiert sein können, die unabhängig aus der Gruppe ausgewählt sind, die aus Methyl, Ethyl, n-Propyl, Isopropyl, n-Butyl, tert-Butyl, sek-Butyl, Isobutyl, -O-CH₃, -O-C₂H₅, -S-CH₃, -S-C₂H₅, -C(=O)-OH, -C(=O)-O-CH₃, -C(=O)-O-C₂H₅, -C(=O)-CH₃, -C(=O)-C₂H₅, F, Cl, Br, I, -CN, -CF₃, -OCF₃, -SCF₃, -OH, -SH, -NH₂, -NO₂, -CHO, -CF₂H, -CFH₂, -C(=O)-NH₂ und -S(=O)₂-CH₃ besteht;
oder R⁶ und R⁷ zusammen mit dem verbrückenden Stickstoffatom eine Struktureinheit bilden, die aus der Gruppe, die aus 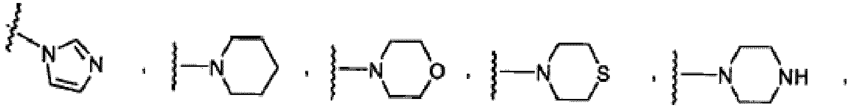 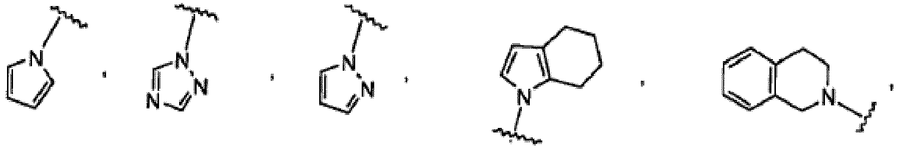 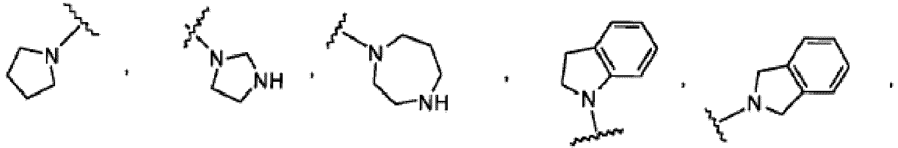 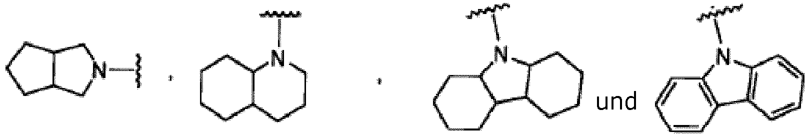 besteht, ausgewählt ist und die unsubstituiert oder gegebenenfalls auf beliebiger Position einschließlich der -NH-Gruppen mit 1, 2, 3, 4 oder 5 Substituenten substituiert sein können, die unabhängig aus der Gruppe ausgewählt sind, die aus Oxo (=O), Methyl, Ethyl, n-Propyl, Isopropyl, n-Butyl, tert-Butyl, sek-Butyl, -O-CH₃, -O-C₂H₅, -S-CH₃, -S-C₂H₅, -C(=O)-OH, -C(=O)-O-CH₃, -C(=O)-O-C₂H₅, -C(=O)-O-CH₂-CH₂-CH₃, -C(=O)-O-CH(CH₃)₂, -C(=O)-O-C(CH₃)₃, -C(=O)-CH₃, -C(=O)-C₂H₅, -C(=O)-CH₂-CH₂CH₃, -C(=O)-CH(CH₃)₂, -C(=O)-C(CH₃)₃, F, Cl, Br, I, -CN, -CF₃, -OCF₃, -SCF₃, -OH, -SH, -NH₂, -NO₂, -CHO, -CF₂H, -CFH₂, -C(=O)-NH₂, -C(=O)-NH-CH₃, -C(=O)-NH-, C₂H₅, -C(=O)-N(CH₃)₂, -C(=O)-N(C₂H₅)₂, -S(=O)₂-CH₃, Phenyl, Phenethyl und Benzyl besteht, wobei die cyclischen Substituenten unsubstituiert oder mit 1, 2 oder 3 Substituenten substituiert sein können, die unabhängig aus der Gruppe ausgewählt sind, die aus Methyl, Ethyl, n-Propyl, Isopropyl, Methoxy, Ethoxy, F, Cl, Br,-CN, -CF₃, -OCF₃, -SCF₃, -OH, -SH, -NH₂ und -NO₂ besteht;
oder R⁹ und R¹⁰ zusammen mit dem verbrückenden Stickstoffatom eine Struktureinheit bilden, die aus der Gruppe, die aus 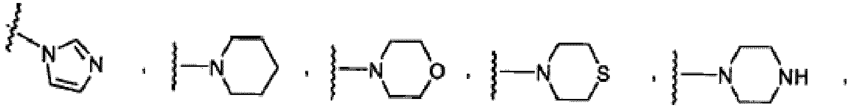 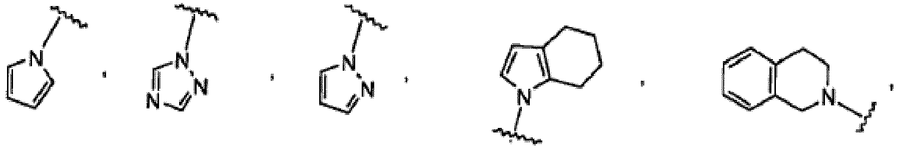 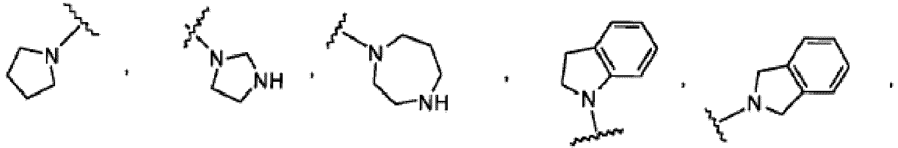 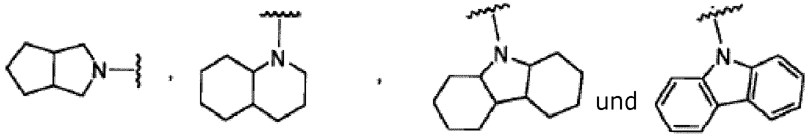 besteht, ausgewählt ist und die unsubstituiert oder gegebenenfalls auf beliebiger Position einschließlich der -NH-Gruppen mit 1, 2, 3, 4 oder 5 Substituenten substituiert sein können, die unabhängig aus der Gruppe ausgewählt sind, die aus Oxo (=O), Methyl, Ethyl, n-Propyl, Isopropyl, n-Butyl, tert-Butyl, sek-Butyl, -O-CH₃, -O-C₂H₅, -S-CH₃, -S-C₂H₅, -C(=O)-OH, -C(=O)-O-CH₃, -C(=O)-O-C₂H₅, -C(=O)-O-CH₂-CH₂-CH₃, -C(=O)-O-CH(CH₃)₂, -C(=O)-O-C(CH₃)₃, -C(=O)-CH₃, -C(=O)-C₂H₅, -C(=O)-CH₂-CH₂CH₃, -C(=O)-CH(CH₃)₂, -C(=O)-C(CH₃)₃, F, Cl, Br, I, -CN, -CF₃, -OCF₃, -SCF₃, -OH, -SH, -NH₂, -NO₂, -CHO, -CF₂H, -CFH₂, -C(=O)-NH₂, -C(=O)-NH-CH₃, -C(=O)-NH-, C₂H₅, -C(=O)-N(CH₃)₂, -C(=O)-N(C₂H₅)₂, -S(=O)₂-CH₃, Phenyl, Phenethyl und Benzyl besteht, wobei die cyclischen Substituenten unsubstituiert oder mit 1, 2 oder 3 Substituenten substituiert sein können, die unabhängig aus der Gruppe ausgewählt sind, die aus Methyl, Ethyl, n-Propyl, Isopropyl, Methoxy, Ethoxy, F, Cl, Br,-CN, -CF₃, -OCF₃, -SCF₃, -OH, -SH, -NH₂ und -NO₂ besteht;
R¹¹ für einen Rest steht, der aus der Gruppe ausgewählt ist, die aus Methyl, Ethyl, n-Propyl, Isopropyl, n-Butyl, sek-Butyl, Isobutyl, tert-Butyl und n-Pentyl besteht; und
R¹² steht für ein Wasserstoffatom oder einen Rest, der aus der Gruppe ausgewählt ist, die aus Methyl, Ethyl, n-Propyl, Isopropyl, n-Butyl, sek-Butyl, Isobutyl, tert-Butyl und n-Pentyl besteht;
gegebenenfalls in Form von einem ihrer Stereoisomere, vorzugsweise Enantiomere oder Diastereomere, eines Racemats oder in Form eines Gemischs von wenigstens zwei ihrer Stereoisomere, vorzugsweise Enantiomere und/oder Diastereomere, in beliebigem Mischungsverhältnis oder ein Salz davon oder ein entsprechendes Solvat davon.

2. Verbindung gemäß Anspruch 1, **dadurch gekennzeichnet, dass**
m 1 ist;
X für eine OR⁵-Struktureinheit oder eine -NR⁶R⁷-Struktureinheit steht;
Y für eine -OR⁸-Struktureinheit, eine -NR⁹R¹⁰-Struktureinheit oder eine -C(=O)-OR¹¹-Struktureinheit steht;
R⁵ und R⁸ stehen unabhängig voneinander jeweils für ein Wasserstoffatom; für einen Rest, der aus der Gruppe ausgewählt ist, die aus Methyl, Ethyl, n-Propyl, Isopropyl, n-Butyl, sek-Butyl, Isobutyl, tert-Butyl und n-Pentyl besteht; für einen Arylrest, der aus der Gruppe, die aus Phenyl und Naphthyl besteht, ausgewählt ist und die über eine -(CH₂)_{1, 2 oder 3}-Gruppe gebunden sein können und die unsubstituiert oder gegebenenfalls mit 1, 2, 3, 4 oder 5 Substituenten substituiert sein können, die unabhängig aus der Gruppe ausgewählt sind, die aus Methyl, Ethyl, n-Propyl, Isopropyl, n-Butyl, tert-Butyl, -O-CH₃, -O-C₂H₅, F, Cl, Br, I, -CN, -CF₃, -OCF₃, -SCF₃, -OH, -SH, -NH₂, -NO₂, -CHO, -CF₂H, -CFH₂ und -S(=O)₂-CH₃ besteht; oder für eine -C(=O)-R¹²-Struktureinheit;
R⁶, R⁷, R⁹ und R¹⁰ stehen unabhängig voneinander jeweils für einen Rest, der aus der Gruppe ausgewählt ist, die aus Methyl, Ethyl, n-Propyl, Isopropyl, n-Butyl, sek-Butyl, Isobutyl, tert-Butyl und n-Pentyl besteht; oder für einen Arylrest, der aus der Gruppe, die aus Phenyl und Naphthyl besteht, ausgewählt ist und die über eine -(CH₂)_{1, 2 oder 3}-Gruppe gebunden sein können und die unsubstituiert oder gegebenenfalls mit 1, 2, 3, 4 oder 5 Substituenten substituiert sein können, die unabhängig aus der Gruppe ausgewählt sind, die aus Methyl, Ethyl, n-Propyl, Isopropyl, n-Butyl, tert-Butyl, sek-Butyl, Isobutyl, -O-CH₃, -O-C₂H₅, -S-CH₃, -S-C₂H₅, F, Cl, Br, I, -CN, -CF₃, -OCF₃, -SCF₃, -OH, -SH, -NH₂, -NO₂, -CHO, -CF₂H, -CFH₂, -C(=O)-NH₂ und -S(=O)₂-CH₃ besteht;
oder R⁶ und R⁷ zusammen mit dem verbrückenden Stickstoffatom eine Struktureinheit bilden, die aus der Gruppe, die aus 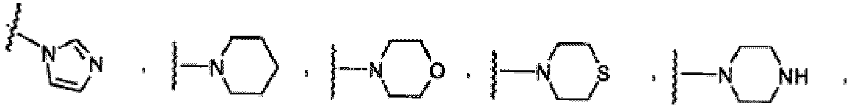 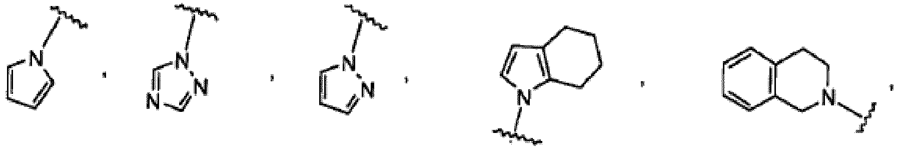 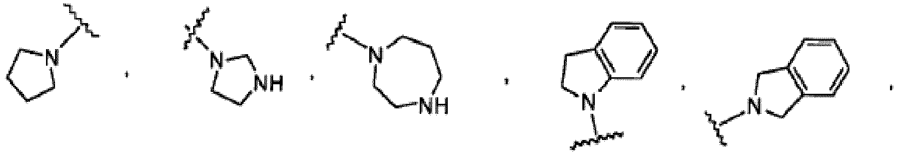 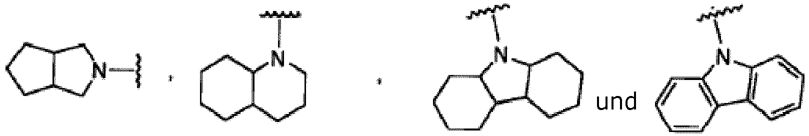 besteht, ausgewählt ist und die unsubstituiert oder gegebenenfalls auf beliebiger Position einschließlich der -NH-Gruppen mit 1, 2, 3, 4 oder 5 Substituenten substituiert sein können, die unabhängig aus der Gruppe ausgewählt sind, die aus Oxo (=O), Methyl, Ethyl, n-Propyl, Isopropyl, n-Butyl, tert-Butyl, sek-Butyl, -O-CH₃, -O-C₂H₅, -S-CH₃, -S-C₂H₅, -C(=O)-OH, -C(=O)-O-CH₃, -C(=O)-O-C₂H₅, -C(=O)-O-CH₂-CH₂-CH₃, -C(=O)-O-CH(CH₃)₂, -C(=O)-O-C(CH₃)₃, -C(=O)-CH₃, -C(=O)-C₂H₅, -C(=O)-CH₂-CH₂CH₃, -C(=O)-CH(CH₃)₂, -C(=O)-C(CH₃)₃, F, Cl, Br, I, -CN, -CF₃, -OCF₃, -SCF₃, -OH, -SH, -NH₂, -NO₂, -CHO, -CF₂H, -CFH₂, -C(=O)-NH₂, -C(=O)-NH-CH₃, -C(=O)-NH-, C₂H₅, -C(=O)-N(CH₃)₂, -C(=O)-N(C₂H₅)₂, -S(=O)₂-CH₃, Phenyl, Phenethyl und Benzyl besteht, wobei die cyclischen Substituenten unsubstituiert oder mit 1, 2 oder 3 Substituenten substituiert sein können, die unabhängig aus der Gruppe ausgewählt sind, die aus Methyl, Ethyl, n-Propyl, Isopropyl, Methoxy, Ethoxy, F, Cl, Br,-CN, -CF₃, -OCF₃, -SCF₃, -OH, -SH, -NH₂ und -NO₂ besteht;
oder R⁹ und R¹⁰ zusammen mit dem verbrückenden Stickstoffatom eine Struktureinheit bilden, die aus der Gruppe, die aus 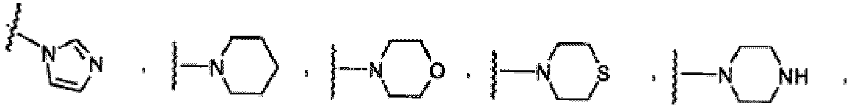 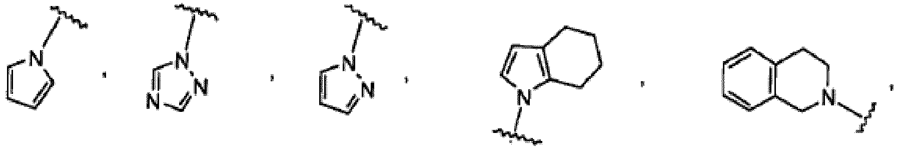 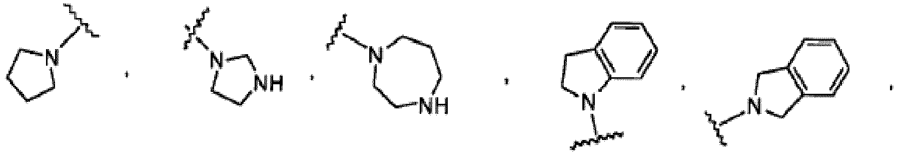 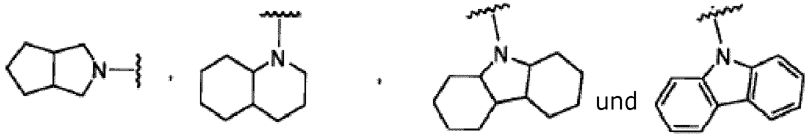 besteht, ausgewählt ist und die unsubstituiert oder gegebenenfalls auf beliebiger Position einschließlich der -NH-Gruppen mit 1, 2, 3, 4 oder 5 Substituenten substituiert sein können, die unabhängig aus der Gruppe ausgewählt sind, die aus Oxo (=O), Methyl, Ethyl, n-Propyl, Isopropyl, n-Butyl, tert-Butyl, sek-Butyl, -O-CH₃, -O-C₂H₅, -S-CH₃, -S-C₂H₅, -C(=O)-OH, -C(=O)-O-CH₃, -C(=O)-O-C₂H₅, -C(=O)-O-CH₂-CH₂-CH₃, -C(=O)-O-CH(CH₃)₂, -C(=O)-O-C(CH₃)₃, -C(=O)-CH₃, -C(=O)-C₂H₅, -C(=O)-CH₂-CH₂CH₃, -C(=O)-CH(CH₃)₂, -C(=O)-C(CH₃)₃, F, Cl, Br, I, -CN, -CF₃, -OCF₃, -SCF₃, -OH, -SH, -NH₂, -NO₂, -CHO, -CF₂H, -CFH₂, -C(=O)-NH₂, -C(=O)-NH-CH₃, -C(=O)-NH-, C₂H₅, -C(=O)-N(CH₃)₂, -C(=O)-N(C₂H₅)₂, -S(=O)₂-CH₃, Phenyl, Phenethyl und Benzyl besteht, wobei die cyclischen Substituenten unsubstituiert oder mit 1, 2 oder 3 Substituenten substituiert sein können, die unabhängig aus der Gruppe ausgewählt sind, die aus Methyl, Ethyl, n-Propyl, Isopropyl, Methoxy, Ethoxy, F, Cl, Br,-CN, -CF₃, -OCF₃, -SCF₃, -OH, -SH, -NH₂ und -NO₂ besteht.

3. Verbindung gemäß Anspruch 1 oder 2, **dadurch gekennzeichnet, dass**
m 1 ist;
X für eine OR⁵-Struktureinheit oder eine -NR⁶R⁷-Struktureinheit steht;
Y für eine -OR⁸-Struktureinheit, eine -NR⁹R¹⁰-Struktureinheit oder eine -C(=O)-OR¹¹-Struktureinheit steht;
R² steht für ein Wasserstoffatom; für einen Rest, der aus der Gruppe ausgewählt ist, die aus Methyl, Ethyl, n-Propyl, Isopropyl, n-Butyl, sek-Butyl, Isobutyl, tert-Butyl und n-Pentyl besteht; oder für einen Phenylrest, und die unsubstituiert oder mit 1, 2, 3, 4 oder 5 Substituenten substituiert sein können, die unabhängig aus der Gruppe ausgewählt sind, die aus Methyl, Ethyl, n-Propyl, Isopropyl, n-Butyl, tert-Butyl, sek-Butyl, Isobutyl, n-Pentyl, -O-CH₃, -O-C₂H₅, F, Cl, Br, I, -CN, -CF₃, -OCF₃ und -SCF₃ besteht;
R⁵ und R⁸ stehen unabhängig voneinander jeweils für ein Wasserstoffatom; oder für einen Phenylrest, der über eine -(CH₂)_{1, 2} oder ₃-Gruppe gebunden sein kann und der unsubstituiert oder gegebenenfalls mit 1, 2, 3, 4 oder 5 Substituenten substituiert sein kann, die unabhängig aus der Gruppe ausgewählt sind, die aus Methyl, Ethyl, n-Propyl, Isopropyl, n-Butyl, tert-Butyl, -O-CH₃, -O-C₂H₅, F, Cl, Br, I, -CN, -CF₃ und -OCF₃ besteht; oder für eine -C(=O)-R¹²-Struktureinheit;
R⁶, R⁷, R⁹ und R¹⁰ stehen unabhängig voneinander jeweils für einen Rest, der aus der Gruppe ausgewählt ist, die aus Methyl, Ethyl, n-Propyl, Isopropyl, n-Butyl, sek-Butyl, Isobutyl, tert-Butyl und n-Pentyl besteht; oder für einen Phenylrest, und die über eine -(CH₂)_{1, 2 oder 3}-Gruppe gebunden sein können und die unsubstituiert oder gegebenenfalls mit 1, 2, 3, 4 oder 5 Substituenten substituiert sein kann, die unabhängig aus der Gruppe ausgewählt sind, die aus Methyl, Ethyl, n-Propyl, Isopropyl, n-Butyl, tert-Butyl, sek-Butyl, Isobutyl, -O-CH₃, -O-C₂H₅, F, Cl, Br, I, -CN,-CF₃, -OCF₃ und -SCF₃ besteht;
oder R⁶ und R⁷ zusammen mit dem verbrückenden Stickstoffatom eine Struktureinheit bilden, die aus der Gruppe, die aus 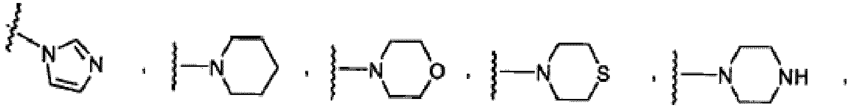 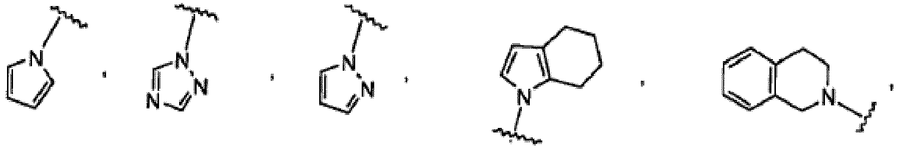 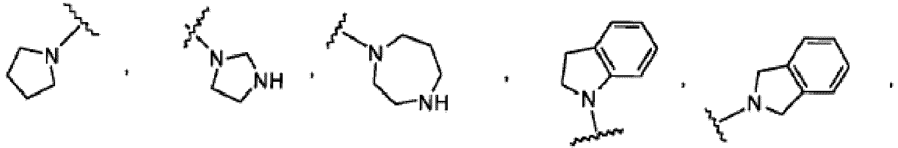 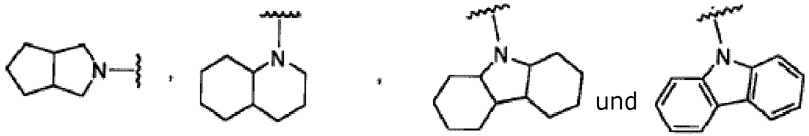 besteht, ausgewählt ist und die unsubstituiert oder gegebenenfalls auf beliebiger Position einschließlich der -NH-Gruppen mit 1, 2, 3, 4 oder 5 Substituenten substituiert sein können, die unabhängig aus der Gruppe ausgewählt sind, die aus Oxo (=O), Methyl, Ethyl, n-Propyl, Isopropyl, n-Butyl, tert-Butyl, sek-Butyl, Phenyl, Phenethyl und Benzyl besteht, wobei die cyclischen Substituenten unsubstituiert oder mit 1, 2 oder 3 Substituenten substituiert sein können, die unabhängig aus der Gruppe ausgewählt sind, die aus Methyl, Ethyl, n-Propyl, Isopropyl, Methoxy, Ethoxy, F, Cl, Br, -CN, -CF₃, -OCF₃, -SCF₃, -OH, -SH, -NH₂ und -NO₂ besteht;
oder R⁹ und R¹⁰ zusammen mit dem verbrückenden Stickstoffatom eine Struktureinheit bilden, die aus der Gruppe, die aus 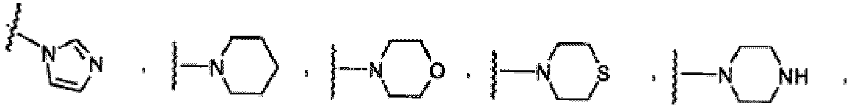 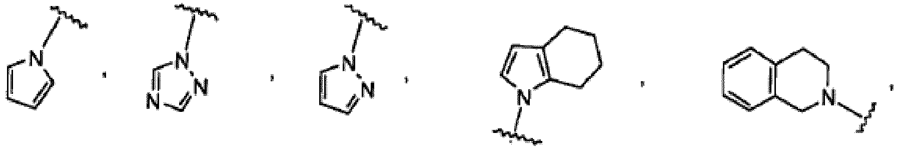 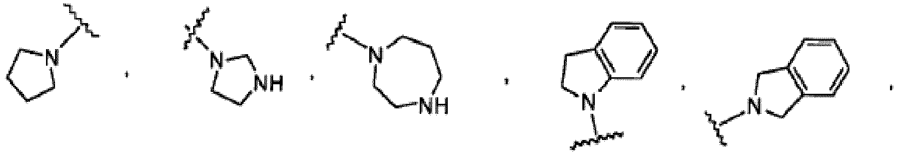 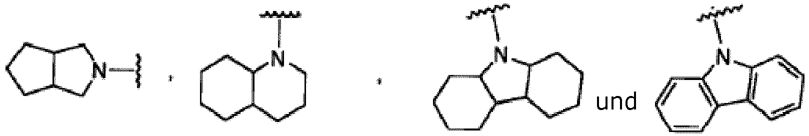 besteht, ausgewählt ist und die unsubstituiert oder gegebenenfalls auf beliebiger Position einschließlich der -NH-Gruppen mit 1, 2, 3, 4 oder 5 Substituenten substituiert sein kann, die unabhängig aus der Gruppe ausgewählt sind, die aus Oxo (=O), Methyl, Ethyl, n-Propyl, Isopropyl, n-Butyl, tert-Butyl, sek-Butyl, Phenyl, Phenethyl und Benzyl besteht, wobei die cyclischen Substituenten unsubstituiert oder mit 1, 2 oder 3 Substituenten substituiert sein können, die unabhängig aus der Gruppe ausgewählt sind, die aus Methyl, Ethyl, n-Propyl, Isopropyl, Methoxy, Ethoxy, F, Cl, Br, -CN, -CF₃, -OCF₃, -SCF₃, -OH, -SH, -NH₂ und -NO₂ besteht.

4. Verbindung gemäß einem oder mehreren der Ansprüche 1 bis 3, **dadurch gekennzeichnet, dass**
m = 1 ist;
X für eine OR⁵-Struktureinheit oder eine -NR⁶R⁷-Struktureinheit steht;
Y für eine -OR⁸-Struktureinheit, eine -NR⁹R¹⁰-Struktureinheit oder eine -C(=O)-OR¹¹-Struktureinheit steht;
R² steht für ein Wasserstoffatom; für einen Rest, der aus der Gruppe ausgewählt ist, die aus Methyl, Ethyl, n-Propyl, Isopropyl, n-Butyl, sek-Butyl, Isobutyl, tert-Butyl und n-Pentyl besteht; oder für einen Phenylrest, und die unsubstituiert oder mit 1, 2, 3, 4 oder 5 Substituenten substituiert sein können, die unabhängig aus der Gruppe ausgewählt sind, die aus Methyl, Ethyl, n-Propyl, Isopropyl, n-Butyl, tert-Butyl, sek-Butyl, Isobutyl, n-Pentyl, -O-CH₃, -O-C₂H₅, F, Cl, Br, I, -CN, -CF₃, -OCF₃ und -SCF₃ besteht;
R⁵ und R⁸ stehen unabhängig voneinander jeweils für ein Wasserstoffatom; oder für einen Phenylrest, der über eine -(CH₂)_{1, 2 oder 3}-Gruppe gebunden sein kann und der unsubstituiert oder gegebenenfalls mit 1, 2, 3, 4 oder 5 Substituenten substituiert sein kann, die unabhängig aus der Gruppe ausgewählt sind, die aus Methyl, Ethyl, n-Propyl, Isopropyl, n-Butyl, tert-Butyl, -O-CH₃, -O-C₂H₅, F, Cl, Br, I, -CN, -CF₃ und -OCF₃ besteht; oder für eine -C(=O)-R¹²-Struktureinheit;
R⁶, R⁷, R⁹ und R¹⁰ stehen unabhängig voneinander jeweils für einen Rest, der aus der Gruppe ausgewählt ist, die aus Methyl, Ethyl, n-Propyl, Isopropyl, n-Butyl, sek-Butyl, Isobutyl, tert-Butyl und n-Pentyl besteht; oder für einen Phenylrest, der über eine -(CH₂)_{1, 2 oder 3}-Gruppe gebunden sein kann und die unsubstituiert oder gegebenenfalls mit 1, 2, 3, 4 oder 5 Substituenten substituiert sein können, die unabhängig aus der Gruppe ausgewählt sind, die aus Methyl, Ethyl, n-Propyl, Isopropyl, n-Butyl, tert-Butyl, sek-Butyl, Isobutyl, -O-CH₃, -O-C₂H₅, F, Cl, Br, I, -CN, -CF₃, -OCF₃ und -SCF₃ besteht;
oder R⁶ und R⁷ zusammen mit dem verbrückenden Stickstoffatom eine Struktureinheit bilden, die aus der Gruppe ausgewählt ist, die aus 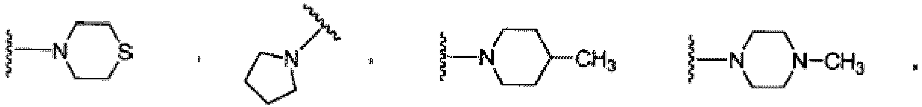 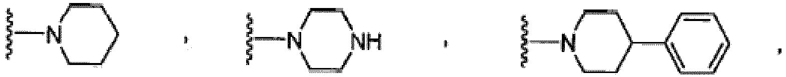 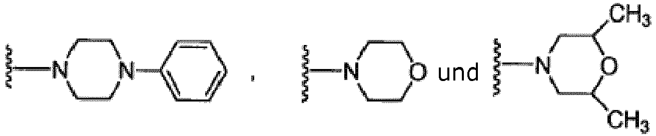 besteht;
oder R⁹ und R¹⁰ zusammen mit dem verbrückenden Stickstoffatom eine Struktureinheit bilden, die aus der Gruppe ausgewählt ist, die aus 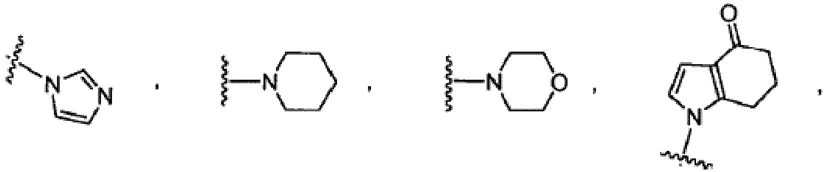 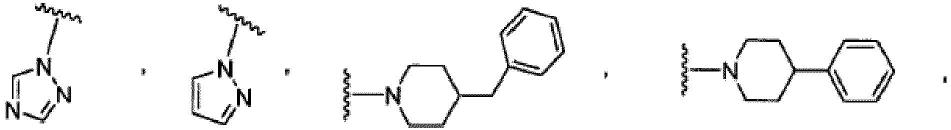 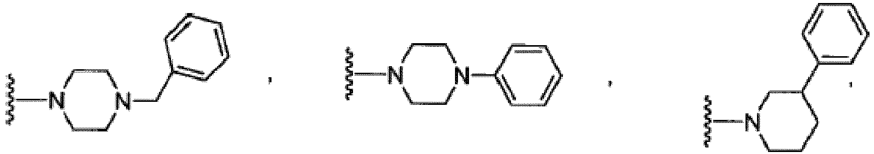 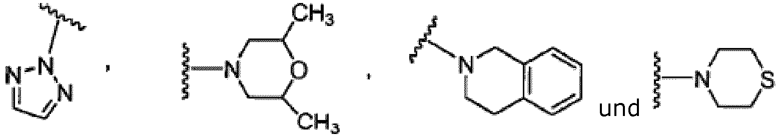 besteht.

5. Verbindung der allgemeinen Formel Ie gemäß einem oder mehreren der Ansprüche 1 oder 2: 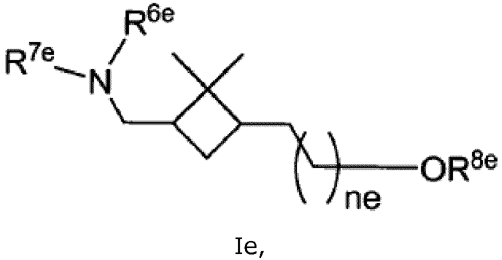 wobei ne = 0 oder 1 ist;
R^{6e} und R^{7e} stehen unabhängig voneinander jeweils für einen Rest, der aus der Gruppe ausgewählt ist, die aus Methyl, Ethyl, n-Propyl, Isopropyl, n-Butyl, sek-Butyl, Isobutyl, tert-Butyl und n-Pentyl besteht; oder für einen Arylrest, der aus der Gruppe, die aus Phenyl und Naphthyl besteht, ausgewählt ist und die über eine -(CH₂)_{1, 2 oder 3}-Gruppe gebunden sein können und die unsubstituiert oder gegebenenfalls mit 1, 2, 3, 4 oder 5 Substituenten substituiert sein können, die unabhängig aus der Gruppe ausgewählt sind, die aus Methyl, Ethyl, n-Propyl, Isopropyl, n-Butyl, tert-Butyl, sek-Butyl, Isobutyl, -O-CH₃, -O-C₂H₅, -S-CH₃, -S-C₂H₅, F, Cl, Br, I, -CN, -CF₃, -OCF₃, -SCF₃, -OH, -SH, -NH₂, -NO₂, -CHO, -CF₂H, -CFH₂, -C(=O)-NH₂ und -S(=O)₂-CH₃ besteht;
oder R^{6e} und R^{7e} zusammen mit dem verbrückenden Stickstoffatom eine Struktureinheit bilden, die aus der Gruppe, die aus 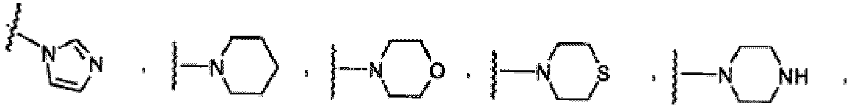 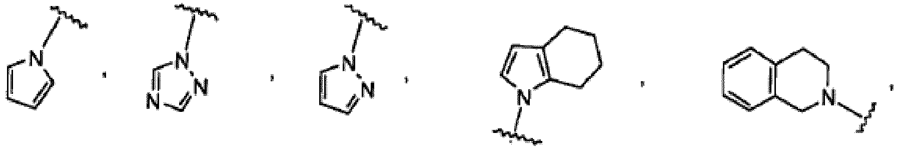 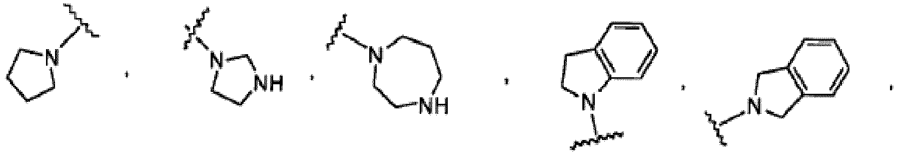 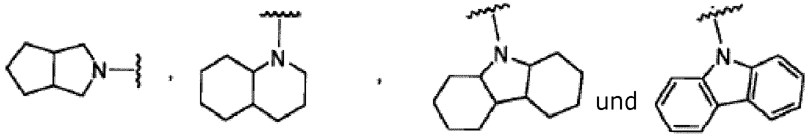 besteht, ausgewählt ist und die unsubstituiert oder gegebenenfalls auf beliebiger Position einschließlich der -NH-Gruppen mit 1, 2, 3, 4 oder 5 Substituenten substituiert sein können, die unabhängig aus der Gruppe ausgewählt sind, die aus Oxo (=O), Methyl, Ethyl, n-Propyl, Isopropyl, n-Butyl, tert-Butyl, sek-Butyl, -O-CH₃, -O-C₂H₅, -S-CH₃, -S-C₂H₅, -C(=O)-OH, -C(=O)-O-CH₃, -C(=O)-O-C₂H₅, -C(=O)-O-CH₂-CH₂-CH₃, -C(=O)-O-CH(CH₃)₂, -C(=O)-O-C(CH₃)₃, -C(=O)-CH₃, -C(=O)-C₂H₅, -C(=O)-CH₂-CH₂CH₃, -C(=O)-CH(CH₃)₂, -C(=O)-C(CH₃)₃, F, Cl, Br, I, -CN, -CF₃, -OCF₃, -SCF₃, -OH, -SH, -NH₂, -NO₂, -CHO, -CF₂H, -CFH₂, -C(=O)-NH₂, -C(=O)-NH-CH₃, -C(=O)-NH-, C₂H₅, -C(=O)-N(CH₃)₂, -C(=O)-N(C₂H₅)₂, -S(=O)₂-CH₃, Phenyl, Phenethyl und Benzyl besteht, wobei die cyclischen Substituenten unsubstituiert oder mit 1, 2 oder 3 Substituenten substituiert sein können, die unabhängig aus der Gruppe ausgewählt sind, die aus Methyl, Ethyl, n-Propyl, Isopropyl, Methoxy, Ethoxy, F, Cl, Br,-CN, -CF₃, -OCF₃, -SCF₃, -OH, -SH, -NH₂ und -NO₂ besteht;
R^{8e} steht für ein Wasserstoffatom steht; für einen Rest, der aus der Gruppe ausgewählt ist, die aus Methyl, Ethyl, n-Propyl, Isopropyl, n-Butyl, sek-Butyl, Isobutyl, tert-Butyl und n-Pentyl besteht; für einen Arylrest, der aus der Gruppe, die aus Phenyl und Naphthyl besteht, ausgewählt ist und die über eine -(CH₂)_{1, 2 oder 3}-Gruppe gebunden sein können und die unsubstituiert oder gegebenenfalls mit 1, 2, 3, 4 oder 5 Substituenten substituiert sein können, die unabhängig aus der Gruppe ausgewählt sind, die aus Methyl, Ethyl, n-Propyl, Isopropyl, n-Butyl, tert-Butyl, -O-CH₃, -O-C₂H₅, F, Cl, Br, I, -CN, -CF₃, -OCF₃, -SCF₃, -OH, -SH, -NH₂, -NO₂, -CHO,-CF₂H, -CFH₂ und -S(=O)₂-CH₃ besteht; oder für eine -C(=O)-R^{12e}-Struktureinheit;
und R^{12e} für einen Rest steht, der aus der Gruppe ausgewählt ist, die aus Methyl, Ethyl, n-Propyl, Isopropyl, n-Butyl, sek-Butyl, Isobutyl, tert-Butyl und n-Pentyl besteht;
gegebenenfalls in Form von einem ihrer Stereoisomere, vorzugsweise Enantiomere oder Diastereomere, eines Racemats oder in Form eines Gemischs von wenigstens zwei ihrer Stereoisomere, vorzugsweise Enantiomere und/oder Diastereomere, in beliebigem Mischungsverhältnis oder ein Salz davon oder ein entsprechendes Solvat davon.

6. Verbindung gemäß Anspruch 5, **dadurch gekennzeichnet, dass** die Stereoisomere die allgemeinen Formeln If oder Ig oder Ih oder Ij aufweisen: 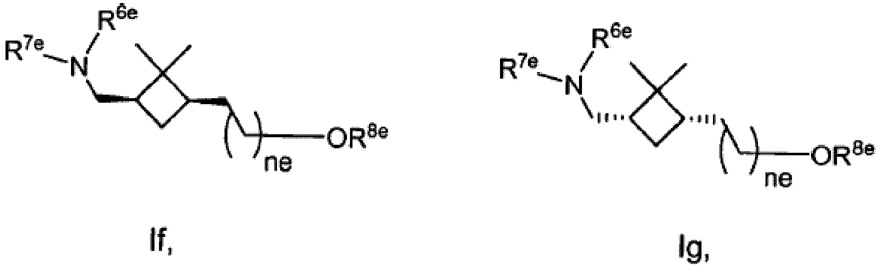 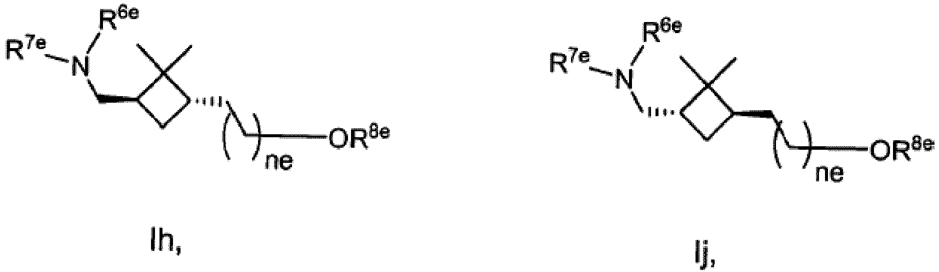 wobei ne, R^{6e}, R^{7e} und R^{8e} wie in Anspruch 5 definiert sind.

7. Verbindung der allgemeinen Formel Ik gemäß einem oder mehreren der Ansprüche 1 oder 2: 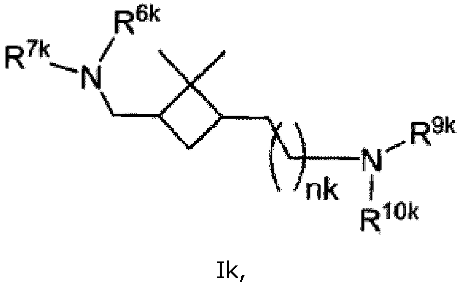 wobei
R^{6k}R^{7k}N- und R^{9k}R^{10k}N- voneinander verschieden sind;
R^{6k}, R^{7k}, R^{9k} und R^{10k} stehen unabhängig voneinander jeweils für einen Rest stehen, der aus der Gruppe ausgewählt ist, die aus Methyl, Ethyl, n-Propyl, Isopropyl, n-Butyl, sek-Butyl, Isobutyl, tert-Butyl und n-Pentyl besteht; oder für einen Arylrest, der aus der Gruppe, die aus Phenyl und Naphthyl besteht, ausgewählt ist und die über eine -(CH₂)_{1, 2 oder 3}-Gruppe gebunden sein können und die unsubstituiert oder gegebenenfalls mit 1, 2, 3, 4 oder 5 Substituenten substituiert sein können, die unabhängig aus der Gruppe ausgewählt sind, die aus Methyl, Ethyl, n-Propyl, Isopropyl, n-Butyl, tert-Butyl, sek-Butyl, Isobutyl, -O-CH₃, -O-C₂H₅, -S-CH₃, -S-C₂H₅, F, Cl, Br, I, -CN, -CF₃, -OCF₃, -SCF₃, -OH, -SH, -NH₂, -NO₂, -CHO, -CF₂H,-CFH₂, -C(=O)-NH₂ und -S(=O)₂-CH₃ besteht;
oder R^{6k} und R^{7k} zusammen mit dem verbrückenden Stickstoffatom eine Struktureinheit bilden, die aus der Gruppe, die aus 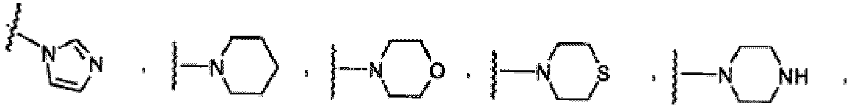 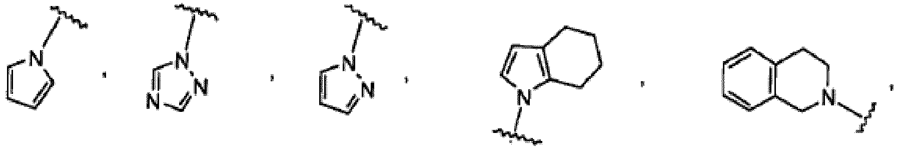 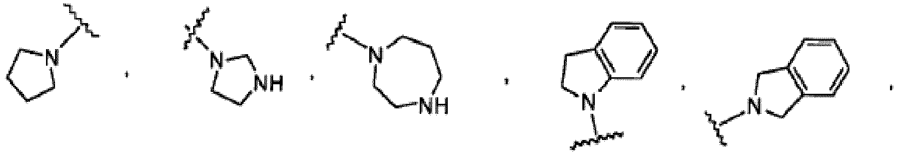 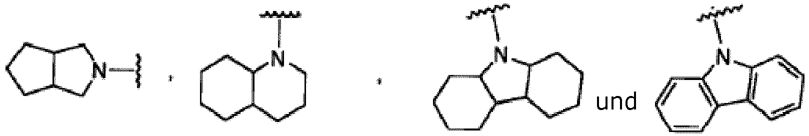 besteht, ausgewählt ist und die unsubstituiert oder gegebenenfalls auf beliebiger Position einschließlich der -NH-Gruppen mit 1, 2, 3, 4 oder 5 Substituenten substituiert sein können, die unabhängig aus der Gruppe ausgewählt sind, die aus Oxo (=O), Methyl, Ethyl, n-Propyl, Isopropyl, n-Butyl, tert-Butyl, sek-Butyl, -O-CH₃, -O-C₂H₅, -S-CH₃, -S-C₂H₅, -C(=O)-OH, -C(=O)-O-CH₃, -C(=O)-O-C₂H₅, -C(=O)-O-CH₂-CH₂-CH₃, -C(=O)-O-CH(CH₃)₂, -C(=O)-O-C(CH₃)₃, -C(=O)-CH₃, -C(=O)-C₂H₅, -C(=O)-CH₂-CH₂CH₃, -C(=O)-CH(CH₃)₂, -C(=O)-C(CH₃)₃, F, Cl, Br, I, -CN, -CF₃, -OCF₃, -SCF₃, -OH, -SH, -NH₂, -NO₂, -CHO, -CF₂H, -CFH₂, -C(=O)-NH₂, -C(=O)-NH-CH₃, -C(=O)-NH-, C₂H₅, -C(=O)-N(CH₃)₂, -C(=O)-N(C₂H₅)₂, -S(=O)₂-CH₃, Phenyl, Phenethyl und Benzyl besteht, wobei die cyclischen Substituenten unsubstituiert oder mit 1, 2 oder 3 Substituenten substituiert sein können, die unabhängig aus der Gruppe ausgewählt sind, die aus Methyl, Ethyl, n-Propyl, Isopropyl, Methoxy, Ethoxy, F, Cl, Br,-CN, -CF₃, -OCF₃, -SCF₃, -OH, -SH, -NH₂ und -NO₂ besteht; oder R⁹ und R¹⁰ zusammen mit dem verbrückenden Stickstoffatom eine Struktureinheit bilden, die aus der Gruppe, die aus 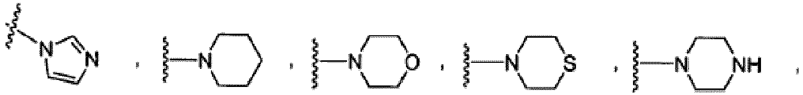 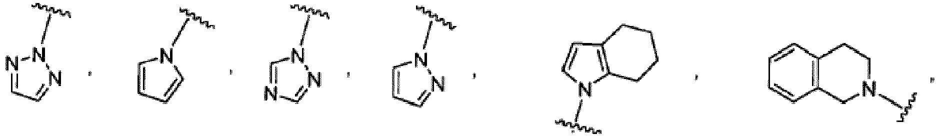 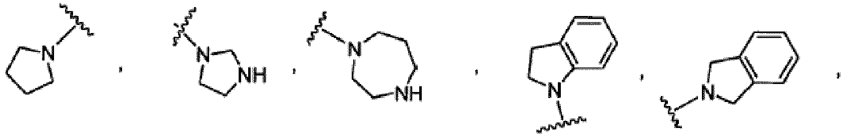 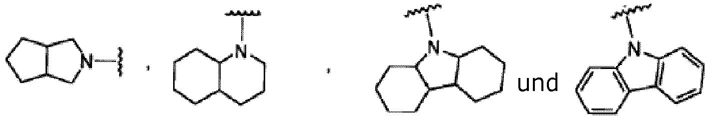 besteht, ausgewählt ist und die unsubstituiert oder gegebenenfalls auf beliebiger Position einschließlich der -NH-Gruppen mit 1, 2, 3, 4 oder 5 Substituenten substituiert sein können, die unabhängig aus der Gruppe ausgewählt sind, die aus Oxo (=O), Methyl, Ethyl, n-Propyl, Isopropyl, n-Butyl, tert-Butyl, sek-Butyl, -O-CH₃, -O-C₂H₅, -S-CH₃, -S-C₂H₅, -C(=O)-OH, -C(=O)-O-CH₃, -C(=O)-O-C₂H₅, -C(=O)-O-CH₂-CH₂-CH₃, -C(=O)-O-CH(CH₃)₂, -C(=O)-O-C(CH₃)₃, -C(=O)-CH₃, -C(=O)-C₂H₅, -C(=O)-CH₂-CH₂CH₃, -C(=O)-CH(CH₃)₂, -C(=O)-C(CH₃)₃, F, Cl, Br, I, -CN, -CF₃, -OCF₃, -SCF₃, -OH, -SH, -NH₂, -NO₂, -CHO, -CF₂H, -CFH₂, -C(=O)-NH₂, -C(=O)-NH-CH₃, -C(=O)-NH-, C₂H₅, -C(=O)-N(CH₃)₂, -C(=O)-N(C₂H₅)₂, -S(=O)₂-CH₃, Phenyl, Phenethyl und Benzyl besteht, wobei die cyclischen Substituenten unsubstituiert oder mit 1, 2 oder 3 Substituenten substituiert sein können, die unabhängig aus der Gruppe ausgewählt sind, die aus Methyl, Ethyl, n-Propyl, Isopropyl, Methoxy, Ethoxy, F, Cl, Br, - CN, -CF₃, -OCF₃, -SCF₃, -OH, -SH, -NH₂ und -NO₂ besteht; gegebenenfalls in Form von einem ihrer Stereoisomere, vorzugsweise Enantiomere oder Diastereomere, eines Racemats oder in Form eines Gemischs von wenigstens zwei ihrer Stereoisomere, vorzugsweise Enantiomere und/oder Diastereomere, in beliebigem Mischungsverhältnis oder ein Salz davon oder ein entsprechendes Solvat davon.

8. Verbindung gemäß Anspruch 7, **dadurch gekennzeichnet, dass** die Stereoisomere die allgemeinen Formeln Im oder In oder Io oder Ip aufweisen: 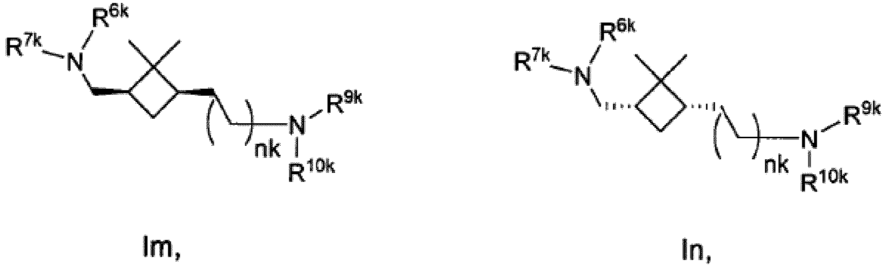 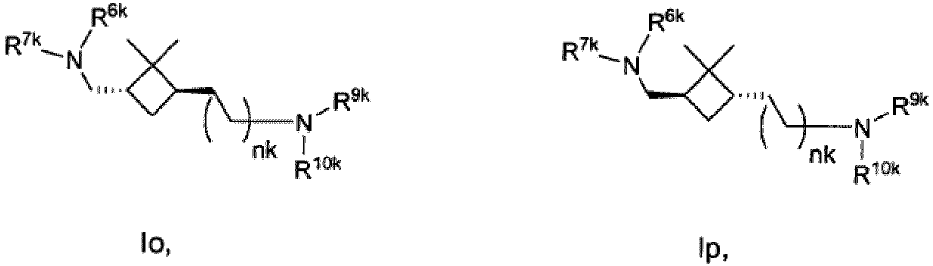 wobei nk, R^{6k}, R^{7k}, R^{9k} und R^{10k} wie in Anspruch 7 definiert sind.

9. Verbindung gemäß einem oder mehreren der Ansprüche 1 bis 8, ausgewählt aus der Gruppe, bestehend aus:
[1] 4-(((1S,3S)-3-(2-(Benzyloxy)ethyl)-2,2-dimethylcyclobutyl)methyl)morpholin
[2] 1-(((1S,3S)-3-(2-(Benzyloxy)ethyl)-2,2-dimethylcyclobutyl)methyl)piperidin
[3] 1-(((1S,3S)-3-(2-(Benzyloxy)ethyl)-2,2-dimethylcyclobutyl)methyl)-4-phenylpiperidin
[4] 1-(((1S,3S)-3-(2-(Benzyloxy)ethyl)-2,2-dimethylcyclobutyl)methyl)-4-phenylpiperazin
[5] 1-(((1S,3S)-3-(2-(Benzyloxy)ethyl)-2,2-dimethylcyclobutyl)methyl)-4-methylpiperazin
[6] 4-(((1R,3S)-3-(Benzyloxymethyl)-2,2-dimethylcyclobutyl)methyl)-morpholin
[7] 1-(((1R,3S)-3-(Benzyloxymethyl)-2,2-dimethylcyclobutyl)methyl)-piperidin
[8] 1-(((1R,3S)-3-(Benzyloxymethyl)-2,2-dimethylcyclobutyl)methyl)-4-phenylpiperidin
[9] 1-(((1R,3S)-3-(Benzyloxymethyl)-2,2-dimethylcyclobutyl)methyl)-4-phenylpiperazin
[10] 1-(((1R,3S)-3-(Benzyloxymethyl)-2,2-dimethylcyclobutyl)methyl)-4-methylpiperazin
[13] 1-((1R,3R)-2,2-Dimethyl-3-(2-morpholinoethyl)cyclobutyl)-1-phenylethanol
[14] 1-((1R,3R)-2,2-Dimethyl-3-(2-(piperidin-1-yl)ethyl)cyclobutyl)-1-phenylethanol
[15] 4-(((1R,3R)-2,2-Dimethyl-3-(2-phenoxyethyl)cyclobutyl)methyl)-morpholin
[16] 1-(((1R,3R)-2,2-Dimethyl-3-(2-phenoxyethyl)cyclobutyl)methyl)-piperidin
[17] 2-((1R,3R)-2,2-Dimethyl-3-(piperidin-1-ylmethyl)cyclobutyl)ethanol
[18] 2-((1S,3S)-2,2-Dimethyl-3-(piperidin-1-ylmethyl)cyclobutyl)ethanol
[19] 2-((1R,3R)-2,2-Dimethyl-3-(morpholinomethyl)cyclobutyl)ethanol
[20] 2-((1S,3S)-2,2-Dimethyl-3-(morpholinomethyl)cyclobutyl)ethanol
[21] 2-((1R,3R)-2,2-Dimethyl-3-((4-methylpiperidin-1-yl)methyl)cyclobutyl)ethanol
[22] 2-((1R,3R)-2,2-Dimethyl-3-((4-phenylpiperidin-1-yl)methyl)cyclobutyl)ethanol
[23] 2-((1R,3R)-3-(((2S,6R)-2,6-Dimethylmorpholino)methyl)-2,2-di-methylcyclobutyl)ethanol
[24] 2-((1R,3R)-3-(N-Benzyl-N-methylamino)methyl)-2,2-dimethylcyclobutyl)ethanol
[26] Methyl-2-((1R,3R)-2,2-dimethyl-3-(piperidin-1-ylmethyl)cyclobutyl)acetat
[27] 1-(((1R,3R)-3-(2-(Benzyloxy)ethyl)-2,2-dimethylcyclobutyl)methyl)piperidin
[28] 1-(((1R,3R)-3-(2-(Benzyloxy)ethyl)-2,2-dimethylcyclobutyl)methyl)-4-methylpiperidin
[29] 1-(((1R,3R)-3-(2-(Benzyloxy)ethyl)-2,2-dimethylcyclobutyl)methyl)-4-phenylpiperidin
[30] 4-(((1R,3R)-3-(2-(Benzyloxy)ethyl)-2,2-dimethylcyclobutyl)methyl)thiomorpholin
[31] 4-(((1R,3R)-3-(2-(Benzyloxy)ethyl)-2,2-dimethylcyclobutyl)methyl)morpholin
[32] (2S,6R)-4-(((1R,3R)-3-(2-(Benzyloxy)ethyl)-2,2-dimethylcyclobutyl)methyl)-2,6-dimethylmorpholin
[33] 1-(((1R,3R)-3-(2-(Benzyloxy)ethyl)-2,2-dimethylcyclobutyl)methyl)pyrrolidin
[34] 1-(((1R,3R)-3-(2-(Benzyloxy)ethyl)-2,2-dimethylcyclobutyl)methyl)-4-methylpiperazin
[35] 1-(((1R,3R)-3-(2-(Benzyloxy)ethyl)-2,2-dimethylcyclobutyl)methyl)-4-methylpiperazinoxalat
[36] 1-(((1R,3R)-3-(2-(Benzyloxy)ethyl)-2,2-dimethylcyclobutyl)methyl)-4-phenylpiperazin
[37] 1-(((1R,3R)-3-(2-(Benzyloxy)ethyl)-2,2-dimethylcyclobutyl)methyl)-4-phenylpiperazinoxalat
[38] 1-(2-((1R,3R)-3-(Benzyloxymethyl)-2,2-dimethylcyclobutyl)ethyl)-piperidin
[39] 4-(2-((1R,3R)-3-(Benzyloxymethyl)-2,2-dimethylcyclobutyl)ethyl)-morpholin
[40] 4-(((1R,3R)-2,2-Dimethyl-3-(2-(3-phenylpiperidin-1-yl)ethyl)cyclo-butyl)methyl)morpholin
[41] 4-(((1R,3R)-2,2-Dimethyl-3-(2-(4-phenylpiperidin-1-yl)ethyl)cyclo-butyl)methyl)morpholin
[42] 4-(((1R,3R)-3-(2-(4-Benzylpiperidin-1-yl)ethyl)-2,2-dimethylcyclo-butyl)methyl)morpholin
[43] 4-(((1R,3R)-3-(2-(1H-Imidazol-1-yl)ethyl)-2,2-dimethylcyclobutyl)-methyl)morpholin
[44] 4-(((1R,3R)-3-(2-(1H-Pyrazol-1-yl)ethyl)-2,2-dimethylcyclobutyl)-methyl)morpholin
[45] 4-(((1R,3R)-3-(2-(1H-1,2,4-Triazol-1-yl)ethyl)-2,2-dimethylcyclo-butyl)methyl)morpholin
[46] 1-(2-((1R,3R)-2,2-Dimethyl-3-(morpholinomethyl)cyclobutyl)ethyl)-6,7-dihydro-1H-indol-4(5H)-on
[47] 2-(2-((1R,3R)-2,2-Dimethyl-3-(morpholinomethyl)cyclobutyl)ethyl)-1,2,3,4-tetrahydroisochinolin
[48] 1-(((1R,3R)-3-(2-(1H-Imidazol-1-yl)ethyl)-2,2-dimethylcyclobutyl)-methyl)piperidin
[49] 1-(((1R,3R)-3-(2-(1H-Pyrazol-1-yl)ethyl)-2,2-dimethylcyclobutyl)-methyl)piperidindimethylcyclobutyl)methyl)piperidin [51] 1-(2-((1R,3R)-2,2-Dimethyl-3-(piperidin-1-ylmethyl)cyclobutyl)eth-yl)-4-phenylpiperidin
[52] 4-Benzyl-1-(2-((1R,3R)-2,2-dimethyl-3-(piperidin-1-ylmethyl)-cyclobutyl)ethyl)piperidin
[53] 1-(2-((1R,3R)-2,2-Dimethyl-3-(piperidin-1-ylmethyl)cyclobutyl)ethyl)-6,7-dihydro-1H-indol-4(5H)-on
[54] 2-(2-((1R,3R)-2,2-Dimethyl-3-(piperidin-1-ylmethyl)cyclobutyl)ethyl)-1,2,3,4-tetrahydroisochinolin
[55] 2-((1R,3R)-2,2-Dimethyl-3-((piperidin-1-yl)methyl)cyclobutyl)ethylpivalat
[56] 2-((1R,3R)-2,2-Dimethyl-3-((piperidin-1-yl)methyl)cyclobutyl)ethylacetat
gegebenenfalls in Form von einem ihrer Stereoisomere, vorzugsweise Enantiomere oder Diastereomere, eines Racemats oder in Form eines Gemischs von wenigstens zwei ihrer Stereoisomere, vorzugsweise Enantiomere und/oder Diastereomere, in beliebigem Mischungsverhältnis oder ein Salz davon oder ein entsprechendes Solvat davon.

10. Verfahren zur Herstellung einer Verbindung der allgemeinen Formel I gemäß einem oder mehreren der Ansprüche 1 bis 9, **dadurch gekennzeichnet, dass** wenigstens eine Verbindung der allgemeinen Formel II 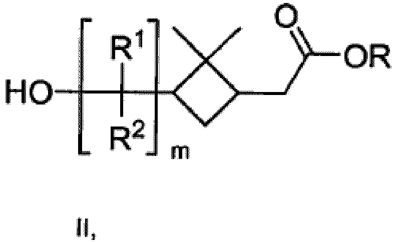 wobei m, R¹ und R² die Bedeutung gemäß einem oder mehreren der Ansprüche 1 bis 9 haben und R für einen linearen oder verzweigten C₁₋₅-Alkylrest oder einen Benzylrest steht,
mit Methansulfonylchlorid, p-Toluolsulfonylchlorid, Trifluormethansulfonylchlorid, Thionylchlorid oder Tetrabromethan umgesetzt wird, vorzugsweise in einem Reaktionsmedium, vorzugsweise in Gegenwart wenigstens einer Base, was wenigstens eine Verbindung der allgemeinen Formel III ergibt 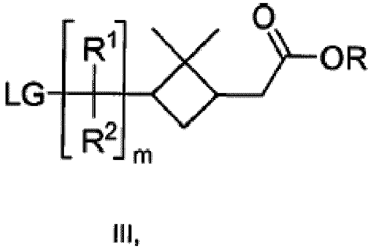 wobei m, R¹ und R² die oben angegebene Bedeutung haben, R für einen linearen oder verzweigten C₁₋₅-Alkylrest steht und LG für -O-S(=O)₂-CH₃, -O-S(=O)₂-p-Tolyl, -O-S(=O)₂-CF₃, Cl oder Br steht, welche gegebenenfalls gereinigt und/oder isoliert wird, und wenigstens eine Verbindung der allgemeinen Formel III mit wenigstens einer Verbindung der allgemeinen Formel HNR⁶R⁷ umgesetzt wird, wobei R⁶ und R⁷ die Bedeutung gemäß einem oder mehreren der Ansprüche 1 bis 9 haben, vorzugsweise in einem Reaktionsmedium, vorzugsweise in Gegenwart wenigstens einer Base, was wenigstens eine Verbindung der allgemeinen Formel IV ergibt 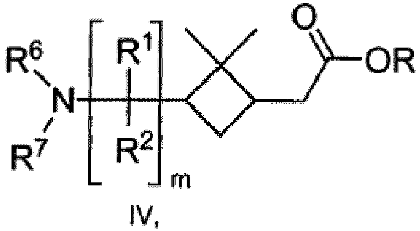 wobei m, R¹, R², R⁶ und R⁷ die oben angegebene Bedeutung haben und R für einen linearen oder verzweigten C₁₋₅-Alkylrest steht, welche gegebenenfalls gereinigt und/oder isoliert wird,
und wenigstens eine Verbindung der allgemeinen Formel IV mit wenigstens einem Reduktionsmittel, das aus der Gruppe ausgewählt ist, die aus Lithiumborhydrid, Natriumborhydrid, Lithiumaluminiumhydrid und Diboran besteht, vorzugsweise mit Lithiumborhydrid, umgesetzt wird, vorzugsweise in einem Reaktionsmedium, was wenigstens eine Verbindung der allgemeinen Formel V ergibt 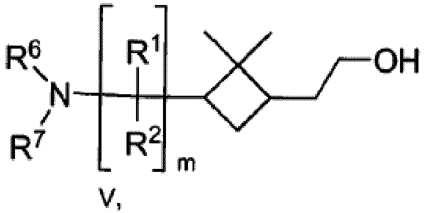 wobei m, R¹, R², R⁶ und R⁷ die oben angegebene Bedeutung haben, welche gegebenenfalls gereinigt und/oder isoliert wird,
und wenigstens eine Verbindung der allgemeinen Formel V mit Methansulfonylchlorid, p-Toluolsulfonylchlorid, Trifluormethansulfonylchlorid, Thionylchlorid oder Tetrabromethan umgesetzt wird, vorzugsweise in einem Reaktionsmedium, vorzugsweise in Gegenwart wenigstens einer Base, was wenigstens eine Verbindung der allgemeinen Formel VI ergibt 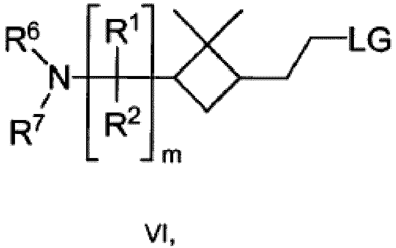 wobei m, R¹, R², R⁶ und R⁷ die oben angegebene Bedeutung haben und LG für -OS(=O)₂-CH₃, -O-S(=O)₂-p-Tolyl, -OS(=O)₂-CF₃, Cl oder Br steht, welche gegebenenfalls gereinigt und/oder isoliert wird,
und wenigstens eine Verbindung der allgemeinen Formel VI mit wenigstens einer Verbindung der allgemeinen Formel HNR⁹R¹⁰ umgesetzt wird, wobei R⁹ und R¹⁰ die Bedeutung gemäß einem oder mehreren der Ansprüche 1 bis 9 haben, vorzugsweise in einem Reaktionsmedium, vorzugsweise in Gegenwart wenigstens einer Base, was wenigstens eine Verbindung der allgemeinen Formel VII ergibt 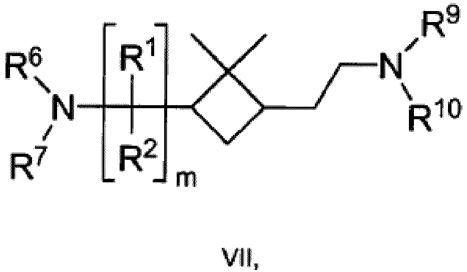 wobei m, R¹, R², R⁶, R⁷, R⁹ und R¹⁰ die oben angegebene Bedeutung haben, welche gegebenenfalls gereinigt und/oder isoliert wird,
oder wenigstens eine Verbindung der allgemeinen Formel VI mit wenigstens einer Verbindung der allgemeinen Formel M-OR⁸ umgesetzt wird, wobei R⁸ die Bedeutung gemäß einem oder mehreren der Ansprüche 1 bis 9 hat und M für ein einwertiges Kation steht, das aus der Gruppe ausgewählt ist, die aus Natrium, Magnesium, Kalium und Lithium besteht, wobei M vorzugsweise für ein Lithiumkation steht, vorzugsweise in einem Reaktionsmedium, vorzugsweise in Gegenwart wenigstens einer Base, was wenigstens eine Verbindung der allgemeinen Formel VIII ergibt 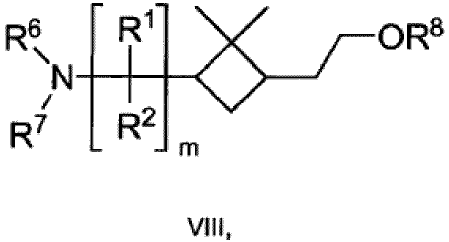 wobei m, R¹, R², R⁶, R⁷ und R⁸ die oben angegebene Bedeutung haben, welche gegebenenfalls gereinigt und/oder isoliert wird, und gegebenenfalls wenigstens eine Verbindung der allgemeinen Formel VIII, wobei R⁸ für Wasserstoff steht, mit wenigstens einer Verbindung der allgemeinen Formel LG-C(=O)-R¹² umgesetzt wird, wobei R¹² die Bedeutung gemäß einem oder mehreren der Ansprüche 1 bis 9 hat und LG für eine Abgangsgruppe steht, vorzugsweise eine Abgangsgruppe, die aus der Gruppe ausgewählt ist, die aus Chlor und Brom besteht, vorzugsweise in einem Reaktionsmedium, vorzugsweise in Gegenwart wenigstens einer Base,
oder mit wenigstens einer Verbindung der allgemeinen Formel HO-C(=O)-R¹² umgesetzt wird, wobei R¹² die Bedeutung gemäß einem oder mehreren der Ansprüche 1 bis 9 hat, vorzugsweise in einem Reaktionsmedium, vorzugsweise in Gegenwart wenigstens einer Base, vorzugsweise in Gegenwart wenigstens eines Kopplungsmittels,
was wenigstens eine Verbindung der allgemeinen Formel VIIIa ergibt 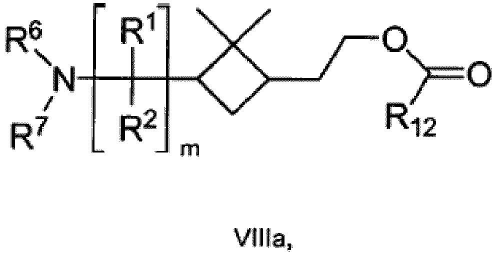 wobei m, R¹, R², R⁶, R⁷ und R¹² die oben angegebene Bedeutung haben, welche gegebenenfalls gereinigt und/oder isoliert wird.

11. Verfahren zur Herstellung einer Verbindung der allgemeinen Formel I gemäß einem oder mehreren der Ansprüche 1 bis 9, **dadurch gekennzeichnet, dass** wenigstens eine Verbindung der allgemeinen Formel II 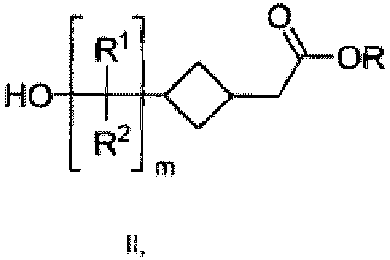 wobei m, R¹ und R² die Bedeutung gemäß einem oder mehreren der Ansprüche 1 bis 9 haben und R für einen linearen oder verzweigten C₁₋₅-Alkylrest steht, mit wenigstens einer Verbindung der allgemeinen Formel R⁵-LG umgesetzt wird, wobei R⁵ die Bedeutung gemäß einem oder mehreren der Ansprüche 1 bis 9 hat und LG für eine Abgangsgruppe steht, wobei LG vorzugsweise für eine Abgangsgruppe steht, die aus der Gruppe ausgewählt ist, die aus Chlor, Brom, -O-S(=O)₂-CH₃, -O-S(=O)₂-CF₃ und -O-S(=O)₂-p-Tolyl besteht, wobei LG besonders bevorzugt für Brom steht, vorzugsweise in einem Reaktionsmedium, vorzugsweise in Gegenwart wenigstens einer Base, besonders bevorzugt in Gegenwart wenigstens einer Base, die aus der Gruppe ausgewählt ist, die aus Butyllithium, Natriumethoxid, Kalium-tert-butoxid, Natriumhydrid und Kaliumhydrid besteht, was wenigstens eine Verbindung der allgemeinen Formel IX ergibt, 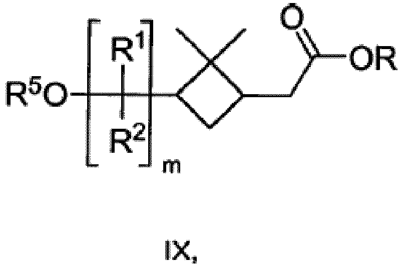 wobei m, R¹, R² und R⁵ die oben angegebene Bedeutung haben und R für einen linearen oder verzweigten C₁₋₅-Alkylrest steht, welche gegebenenfalls gereinigt und/oder isoliert wird,
und wenigstens eine Verbindung der allgemeinen Formel IX mit wenigstens einem Reduktionsmittel, das aus der Gruppe ausgewählt ist, die aus Lithiumborhydrid, Natriumborhydrid, Lithiumaluminiumhydrid und Diboran besteht, vorzugsweise mit Lithiumborhydrid, umgesetzt wird, vorzugsweise in einem Reaktionsmedium, was wenigstens eine Verbindung der allgemeinen Formel X ergibt 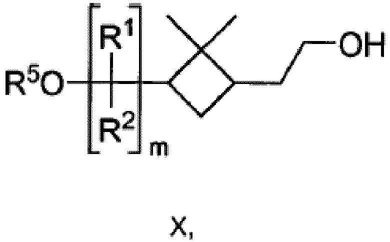 wobei m, R¹, R² und R⁵ die oben angegebene Bedeutung haben, welche gegebenenfalls gereinigt und/oder isoliert wird,
und gegebenenfalls wenigstens eine Verbindung der allgemeinen Formel X mit wenigstens einer Verbindung der allgemeinen Formel LG-C(=O)-R¹² umgesetzt wird, wobei R¹² die Bedeutung gemäß einem oder mehreren der Ansprüche 1 bis 9 hat und LG für eine Abgangsgruppe steht, vorzugsweise eine Abgangsgruppe, die aus der Gruppe ausgewählt ist, die aus Chlor und Brom besteht, vorzugsweise in einem Reaktionsmedium, vorzugsweise in Gegenwart wenigstens einer Base,
oder mit wenigstens einer Verbindung der allgemeinen Formel HO-C(=O)-R¹² umgesetzt wird, wobei R¹² die Bedeutung gemäß einem oder mehreren der Ansprüche 1 bis 9 hat, vorzugsweise in einem Reaktionsmedium, vorzugsweise in Gegenwart wenigstens einer Base, vorzugsweise in Gegenwart wenigstens eines Kopplungsmittels,
was wenigstens eine Verbindung der allgemeinen Formel Xa ergibt 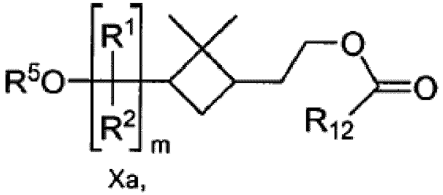 wobei m, R¹, R² und R⁵ die oben angegebene Bedeutung haben, welche gegebenenfalls gereinigt und/oder isoliert wird,
und wenigstens eine Verbindung der allgemeinen Formel X mit Methansulfonylchlorid, p-Toluolsulfonylchlorid, Trifluormethansulfonylchlorid, Thionylchlorid oder Tetrabromethan umgesetzt wird, vorzugsweise in einem Reaktionsmedium, vorzugsweise in Gegenwart wenigstens einer Base, was wenigstens eine Verbindung der allgemeinen Formel XI ergibt 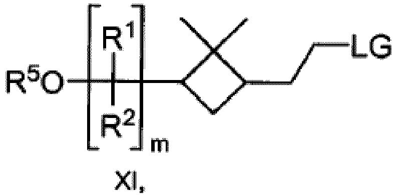 wobei m, R¹, R² und R⁵ die oben angegebene Bedeutung haben und LG für -O-S(=O)₂-CH₃, -O-S(=O)₂-p-Tolyl, -O-S(=O)₂-CF₃, Cl oder Br steht, welche gegebenenfalls gereinigt und/oder isoliert wird,
und wenigstens eine Verbindung der allgemeinen Formel X mit wenigstens einer Verbindung der allgemeinen Formel HNR⁹R¹⁰ umgesetzt wird, wobei R⁹ und R¹⁰ die Bedeutung gemäß einem oder mehreren der Ansprüche 1 bis 9 haben, vorzugsweise in einem Reaktionsmedium, vorzugsweise in Gegenwart wenigstens einer Base, was wenigstens eine Verbindung der allgemeinen Formel XII ergibt 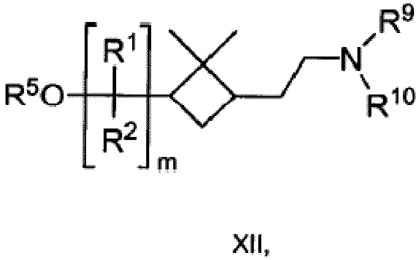 wobei m, R¹, R², R⁵, R⁹ und R¹⁰ die oben angegebene Bedeutung haben, welche gegebenenfalls gereinigt und/oder isoliert wird.

12. Verfahren zur Herstellung einer Verbindung der allgemeinen Formel I gemäß einem oder mehreren der Ansprüche 1 bis 9, **dadurch gekennzeichnet, dass** wenigstens eine Verbindung der allgemeinen Formel XIII 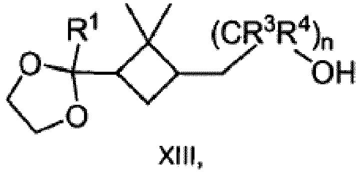 wobei R¹, R³ und R⁴ die Bedeutung gemäß einem oder mehreren der Ansprüche 1 bis 9 haben, mit Methansulfonylchlorid, p-Toluolsulfonylchlorid, Trifluormethansulfonylchlorid, Thionylchlorid oder Tetrabromethan umgesetzt wird, vorzugsweise in einem Reaktionsmedium, vorzugsweise in Gegenwart wenigstens einer Base, was wenigstens eine Verbindung der allgemeinen Formel XIV ergibt 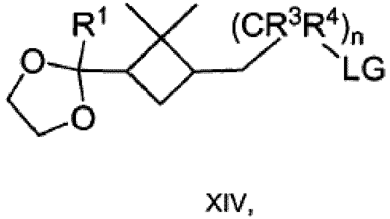 wobei R¹, R³ und R⁴ die oben angegebene Bedeutung haben und LG für - O-S(=O)₂-CH₃, -O-S(=O)₂-p-Tolyl, -O-S(=O)₂-CF₃, Cl oder Br steht, welche gegebenenfalls gereinigt und/oder isoliert wird,
und wenigstens eine Verbindung der allgemeinen Formel XIV mit wenigstens einer Verbindung der allgemeinen Formel HNR⁹R¹⁰ umgesetzt wird, wobei R⁹ und R¹⁰ die Bedeutung gemäß einem oder mehreren der Ansprüche 1 bis 9 haben, vorzugsweise in einem Reaktionsmedium, vorzugsweise in Gegenwart wenigstens einer Base, was wenigstens eine Verbindung der allgemeinen Formel XV ergibt 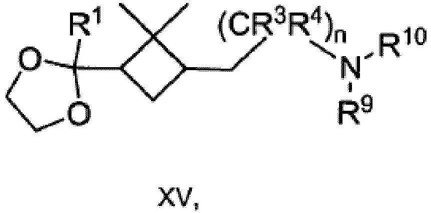 wobei R¹, R³, R⁴, R⁹ und R¹⁰ die oben angegebene Bedeutung haben, welche gegebenenfalls gereinigt und/oder isoliert wird,
und wenigstens eine Verbindung der allgemeinen Formel XV mit wenigstens einem Reagens, das aus der Gruppe ausgewählt ist, die aus Chlorwasserstoffsäure, Pyridinium-p-toluolsulfonat, Sulfonsäure und Trifluoressigsäure, vorzugsweise mit Pyridinium-p-toluolsulfonat, umgesetzt wird, vorzugsweise in einem Reaktionsmedium, was wenigstens eine Verbindung der allgemeinen Formel XVI ergibt 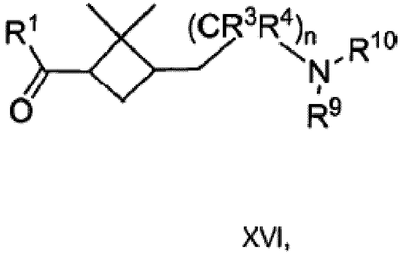 wobei R¹, R³, R⁴, R⁹ und R¹⁰ die oben angegebene Bedeutung haben, welche gegebenenfalls gereinigt und/oder isoliert wird,
und wenigstens eine Verbindung der allgemeinen Formel XVI mit wenigstens einer Verbindung der allgemeinen Formel R²-Li, wobei R² die Bedeutung gemäß einem oder mehreren der Ansprüche 1 bis 9 hat, oder R²-Mg-Z, wobei R² die Bedeutung gemäß einem oder mehreren der Ansprüche 1 bis 9 hat und Z für ein Anion steht, das aus der Gruppe ausgewählt ist, die aus Bromid und Chlorid besteht, umgesetzt wird, vorzugsweise in einem Reaktionsmedium, was wenigstens eine Verbindung der allgemeinen Formel XVII ergibt 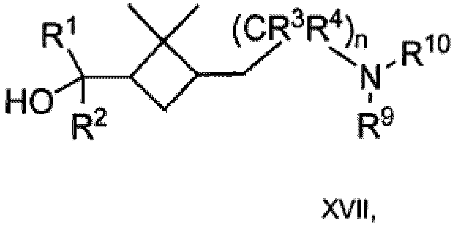 wobei R¹, R², R³, R⁴, R⁹ und R¹⁰ die oben angegebene Bedeutung haben, welche gegebenenfalls gereinigt und/oder isoliert wird,
und gegebenenfalls wenigstens eine Verbindung der allgemeinen Formel XVII mit wenigstens einer Verbindung der allgemeinen Formel R⁵-LG umgesetzt wird, wobei R⁵ die Bedeutung gemäß einem oder mehreren der Ansprüche 1 bis 9 hat und LG für eine Abgangsgruppe steht, wobei LG vorzugsweise für eine Abgangsgruppe steht, die aus der Gruppe ausgewählt ist, die aus Chlor, Brom, -O-S(=O)₂-CH₃, -O-S(=O)₂-CF₃ und -O-S(=O)₂-p-Tolyl besteht, wobei LG besonders bevorzugt für Brom steht, vorzugsweise in einem Reaktionsmedium, vorzugsweise in Gegenwart wenigstens einer Base, besonders bevorzugt in Gegenwart wenigstens einer Base, die aus der Gruppe ausgewählt ist, die aus Butyllithium, Natriumethoxid, Kalium-tert-butoxid, Natriumhydrid und Kaliumhydrid besteht, was wenigstens eine Verbindung der allgemeinen Formel XVIII ergibt, 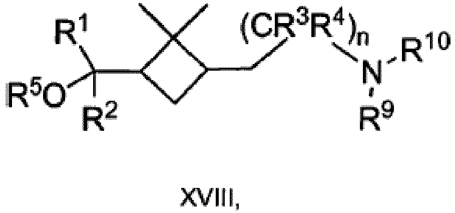 wobei R¹, R², R³, R⁴, R⁵, R⁹ und R¹⁰ die oben angegebene Bedeutung haben, welche gegebenenfalls gereinigt und/oder isoliert wird.

13. Verfahren zur Herstellung einer Verbindung der allgemeinen Formel I gemäß einem oder mehreren der Ansprüche 1 bis 9, **dadurch gekennzeichnet, dass** wenigstens eine Verbindung der allgemeinen Formel IXX 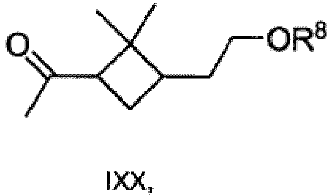 wobei R⁸ die Bedeutung gemäß einem oder mehreren der Ansprüche 1 bis 9 hat, mit NaOBr umgesetzt wird, vorzugsweise in einem Reaktionsmedium, besonders bevorzugt in einem Reaktionsmedium, das aus der Gruppe, die aus Dioxan, THF und Wasser besteht, oder einem Gemisch davon ausgewählt ist, was wenigstens eine Verbindung der allgemeinen Formel XX ergibt, 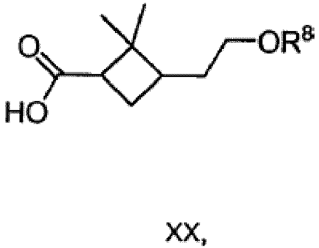 wobei R⁸ die Bedeutung gemäß einem oder mehreren der Ansprüche 1 bis 9 hat, welche gegebenenfalls gereinigt und/oder isoliert wird,
und wenigstens eine Verbindung der allgemeinen Formel XX mit Methyliodid umgesetzt wird, vorzugsweise in einem Reaktionsmedium, vorzugsweise in Gegenwart wenigstens einer Base, besonders bevorzugt in Gegenwart von Cäsiumcarbonat, was wenigstens eine Verbindung der allgemeinen Formel XXI ergibt, 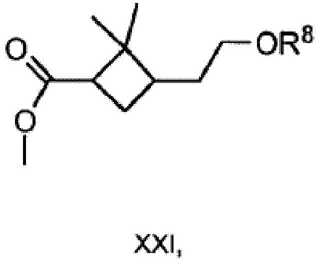 wobei R⁸ die Bedeutung gemäß einem oder mehreren der Ansprüche 1 bis 9 hat, welche gegebenenfalls gereinigt und/oder isoliert wird,
und wenigstens eine Verbindung der allgemeinen Formel XXI mit wenigstens einem Reduktionsmittel, das aus der Gruppe ausgewählt ist, die aus Lithiumborhydrid, Natriumborhydrid, Lithiumaluminiumhydrid und Diboran besteht, vorzugsweise mit Lithiumborhydrid, umgesetzt wird, vorzugsweise in einem Reaktionsmedium, was wenigstens eine Verbindung der allgemeinen Formel XXII ergibt 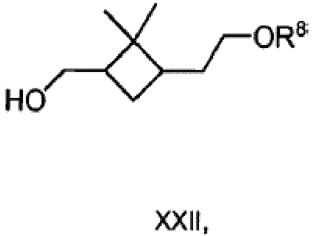 wobei R⁸ die Bedeutung gemäß einem oder mehreren der Ansprüche 1 bis 9 hat, welche gegebenenfalls gereinigt und/oder isoliert wird.

14. Medikament, umfassend wenigstens eine Verbindung der allgemeinen Formel I gemäß einem oder mehreren der Ansprüche 1 bis 9 und gegebenenfalls wenigstens ein physiologisch annehmbares Hilfsmittel.

15. Medikament gemäß Anspruch 14 zur Verwendung bei der Prophylaxe und/oder Behandlung von Schmerzen, vorzugsweise neuropathischen Schmerzen, Allodynie, Analgesie, Kausalgie, zentralen Schmerzen, Dysästhesie, Hyperästhesie, Hyperalgesie, Hypoalgesie, Hypoästhesie oder Neuralgie, besonders bevorzugt neuropathischen Schmerzen, Hyperalgesie oder Allodynie.

16. Substituierte Dimethylcyclobutylverbindung der allgemeinen Formel I: 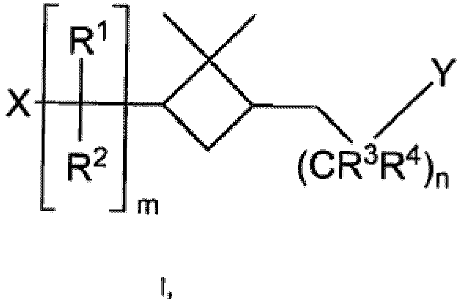 wobei m, n, X, Y, R¹, R², R³ und R⁴ wie in Anspruch 1 definiert sind,
gegebenenfalls in Form von einem ihrer Stereoisomere, vorzugsweise Enantiomere oder Diastereomere, eines Racemats oder in Form eines Gemischs von wenigstens zwei ihrer Stereoisomere, vorzugsweise Enantiomere und/oder Diastereomere, in beliebigem Mischungsverhältnis oder ein Salz davon oder ein entsprechendes Solvat davon
zur Verwendung bei der Behandlung und/oder Prophylaxe von Schmerzen, vorzugsweise neuropathischen Schmerzen, Allodynie, Analgesie, Kausalgie, zentralen Schmerzen, Dysästhesie, Hyperästhesie, Hyperalgesie, Hypoalgesie, Hypoästhesie oder Neuralgie, besonders bevorzugt neuropathischen Schmerzen, Hyperalgesie oder Allodynie.

## Revendications

1. Composé diméthylcyclobutyle substitué de formule générale I, 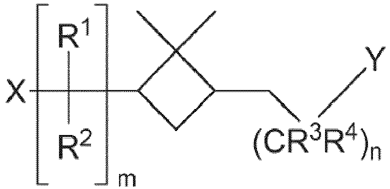 dans laquelle
m est 1 ou 2 ;
n est 0 ou 1 ;
X et Y sont différents ;
X représente un groupement -OR⁵ ou un groupement -NR⁶R⁷ ; ou un radical (hétéro)cycloaliphatique choisi dans le groupe constitué de pyrrolidinyle, pipéridinyle, morpholinyle, thiomorpholinyle, pipérazinyle, pyrazolidinyle, indolinyle, isoindolinyle, (1,2,3,4)-tétrahydroquinolinyle et (1,2,3,4)-tétrahydroisoquinolinyle, qui peut être non substitué ou éventuellement substitué par 1, 2, 3, 4 ou 5 substituants choisis indépendamment dans le groupe constitué de oxo (=0), thioxo (=S), méthyle, éthyle, n-propyle, isopropyle, n-butyle, tert-butyle, sec-butyle, isobutyle, n-pentyle, -O-CH₃, -O-C₂H₅, F, Cl, Br, I, -CN, -CF₃, -OH, -SH et -NH₂ ;
Y représente un groupement -OR⁸ ; un groupement -NR⁹R¹⁰ ; un groupement -C(=O)-OR¹¹ ; ou un radical (hétéro)cycloaliphatique choisi dans le groupe constitué de pyrrolidinyle, pipéridinyle, morpholinyle, thiomorpholinyle, pipérazinyle, pyrazolidinyle, indolinyle, isoindolinyle, (1,2,3,4)-tétrahydroquinolinyle et (1,2,3,4)-tétrahydroisoquinolinyle, qui peut être non substitué ou éventuellement substitué par 1, 2, 3, 4 ou 5 substituants choisis indépendamment dans le groupe constitué de méthyle, éthyle, n-propyle, isopropyle, n-butyle, tert-butyle, sec-butyle, isobutyle, n-pentyle, -O-CH₃, -O-C₂H₅, F, Cl, Br, I, -CN, -CF₃, -OH, -SH et -NH₂ ;
R¹ représente un atome d'hydrogène ou un radical choisi dans le groupe constitué de méthyle, éthyle, n-propyle, isopropyle, n-butyle, sec-butyle, isobutyle, tert-butyle et n-pentyle ;
R² représente un atome d'hydrogène ; un radical choisi dans le groupe constitué de méthyle, éthyle, n-propyle, isopropyle, n-butyle, sec-butyle, isobutyle, tert-butyle et n-pentyle ; ou un radical aryle choisi dans le groupe constitué de phényle et napthyle, qui peut être non substitué ou éventuellement substitué par 1, 2, 3, 4 ou 5 substituants choisis indépendamment dans le groupe constitué de méthyle, éthyle, n-propyle, isopropyle, n-butyle, tert-butyle, sec-butyle, isobutyle, n-pentyle, -O-CH₃, -O-C₂H₅, -S-CH₃, -S-C₂H₅, -C(=O)-OH, -C(=O)-O-CH₃, -C(=O)-O-C₂H₅, -C(=O)-CH₃, -C(=O)-C₂H₅, F, Cl, Br, I, -CN, -CF₃, -OCF₃, -SCF₃, -OH, -SH, -NH₂, -NO₂, -CHO, -CF₂H et -CFH₂ ;
R³ et R⁴ représentent tous deux un atome d'hydrogène ;
R⁵ et R⁸, indépendamment l'un de l'autre, représentent chacun un atome d'hydrogène ; un radical choisi dans le groupe constitué de méthyle, éthyle, n-propyle, isopropyle, n-butyle, sec-butyle, isobutyle, tert-butyle et n-pentyle ; un radical aryle choisi dans le groupe constitué de phényle et napthyle, qui peut être lié par un groupe -(CH₂)_{1, 2 ou 3} et qui peut être non substitué ou éventuellement substitué par 1, 2, 3, 4 ou 5 substituants choisis indépendamment dans le groupe constitué de méthyle, éthyle, n-propyle, isopropyle, n-butyle, tert-butyle, sec-butyle, isobutyle, n-pentyle, -O-CH₃, -O-C₂H₅, -S-CH₃, -S-C₂H₅, -C(=O)-OH, -C(=O)-O-CH₃, -C(=O)-O-C₂H₅, -C(=O)-CH₃, -C(=O)-C₂H₅, F, Cl, Br, I, -CN, -CF₃, -OCF₃, -SCF₃, -OH, -SH, -NH₂, -NO₂, -CHO, -CF₂H, -CFH₂ et -S(=O)₂-CH₃ ; ou un groupement -C(=O)-R¹;
à condition que
si R⁵ représente un hydrogène et m est 1,
R¹ représente un radical choisi dans le groupe constitué de méthyle, éthyle, n-propyle, isopropyle, n-butyle, sec-butyle, isobutyle, tert-butyle et n-pentyle ; et
R² représente un radical choisi dans le groupe constitué de méthyle, éthyle, n-propyle, isopropyle, n-butyle, sec-butyle, isobutyle, tert-butyle et n-pentyle ; ou un radical aryle choisi dans le groupe constitué de phényle et napthyle, qui peut être non substitué ou éventuellement substitué par 1, 2, 3, 4 ou 5 substituants choisis indépendamment dans le groupe constitué de méthyle, éthyle, n-propyle, isopropyle, n-butyle, tert-butyle, sec-butyle, isobutyle, n-pentyle, -O-CH₃, -O-C₂H₅, -S-CH₃, -S-C₂H₅, -C(=O)-OH, -C(=O)-O-CH₃, -C(=O)-O-C₂H₅, -C(=O)-CH₃, -C(=O)-C₂H₅, F, Cl, Br, I, -CN, -CF₃, -OCF₃, -SCF₃, -OH, -SH, -NH₂, -NO₂, -CHO, -CF₂H et -CFH₂ ;
R⁶, R⁷, R⁹ et R¹⁰, indépendamment l'un de l'autre, représentent chacun un radical choisi dans le groupe constitué de méthyle, éthyle, n-propyle, isopropyle, n-butyle, sec-butyle, isobutyle, tert-butyle et n-pentyle ; ou un radical aryle choisi dans le groupe constitué de phényle et napthyle, qui peut être lié par un groupe -(CH₂)_{1, 2 ou 3} et qui peut être non substitué ou éventuellement substitué par 1, 2, 3, 4 ou 5 substituants choisis indépendamment dans le groupe constitué de méthyle, éthyle, n-propyle, isopropyle, n-butyle, tert-butyle, sec-butyle, isobutyle, -O-CH₃, -O-C₂H₅, -S-CH₃, -S-C₂H₅, -C(=O)-OH, -C(=O)-O-CH₃, -C(=O)-O-C₂H₅, -C(=O)-CH₃, -C(=O)-C₂H₅, F, Cl, Br, I, -CN, -CF₃, -OCF₃, -SCF₃, -OH, -SH, -NH₂-NO₂, -CHO, -CF₂H, -CFH₂, -C(=O)-NH₂ et -S(=O)₂-CH₃ ;
ou R⁶ et R⁷ conjointement à l'atome d'azote pontant forment un groupement choisi dans le groupe constitué de 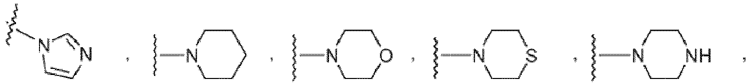 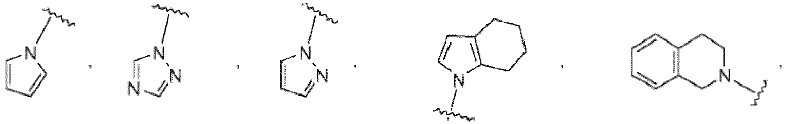 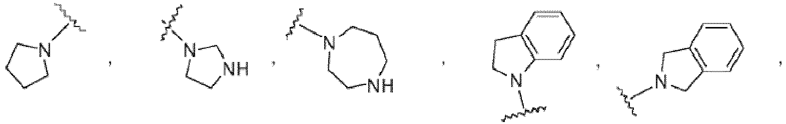 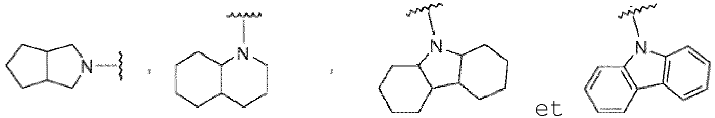 qui peut être non substitué ou éventuellement substitué en une quelconque position y compris les groupes -NH par 1, 2, 3, 4 ou 5 substituants choisis indépendamment dans le groupe constitué de oxo (=0), méthyle, éthyle, n-propyle, isopropyle, n-butyle, tert-butyle, sec-butyle, -O-CH₃, -O-C₂H₅, -S-CH₃, -S-C₂H₅, -C(=O)-OH, -C(=O)-O-CH₃, -C(=O)-O-C₂H₅, -C(=O)-O-CH₂-CH₂-CH₃, -C(=O)-O-CH(CH₃)₂-, -C(=O)-O-C(CH₃)₃-, -C(=O)-CH₃, -C(=O)-C₂H₅, -C(=O)-CH₂-CH₂-CH₃, -C(=O)-CH(CH₃)₂, -C(=O)-C(CH₃)₃, F, Cl, Br, I, -CN, -CF₃, -OCF₃, -SCF₃, -OH, -SH, -NH₂, -NO₂, -CHO, -CF₂H, -CFH₂, -C(=O)-NH₂, -C(=O)-NH-CH₃, -C(=O)-NH-C₂H₅, -C(=O)-N(CH₃)₂, -C(=O)-N(C₂H₅)₂, -S(=O)₂-CH₃, phényle, phénéthyle et benzyle, dans lequel lesdits substituants cycliques peuvent être non substitués ou substitués par 1, 2 ou 3 substituants choisis indépendamment dans le groupe constitué de méthyle, éthyle, n-propyle, isopropyle, méthoxy, éthoxy, F, Cl, Br, -CN, -CF₃, -OCF₃, -SCF₃, -OH, -SH, -NH₂ et -NO₂ ;
ou R⁹ et R¹⁰ conjointement à l'atome d'azote pontant forment un groupement choisi dans le groupe constitué de 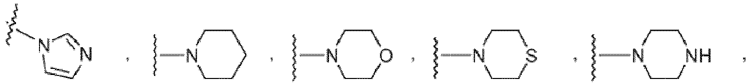 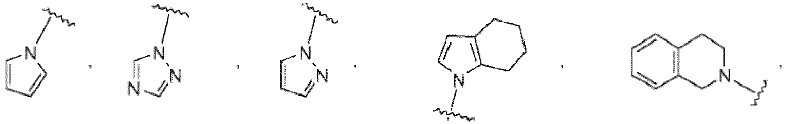 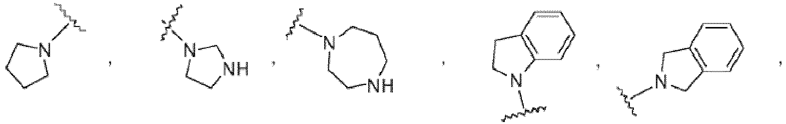 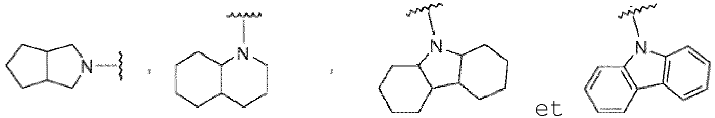 qui peut être non substitué ou éventuellement substitué en une quelconque position y compris les groupes -NH par 1, 2, 3, 4 ou 5 substituants choisis indépendamment dans le groupe constitué de oxo (=0), méthyle, éthyle, n-propyle, isopropyle, n-butyle, tert-butyle, sec-butyle, -O-CH₃, -O-C₂H₅, -S-CH₃, -S-C₂H₅, -C(=O)-OH, -C(=O)-O-CH₃, -C(=O)-O-C₂H₅, -C(=O)-O-CH₂-CH₂-CH₃, -C(=O)-O-CH(CH₃)₂, -C(=O)-O-C(CH₃)₃, -C(=O)-CH₃, -C(=O)-C₂H₅, -C(=O)-CH₂-CH₂-CH₃, -C(=O)-CH(CH₃)₂, -C(=O)-C(CH₃)₃, F, Cl, Br, I, -CN, -CF₃, -OCF₃, -SCF₃, -OH, -SH, -NH₂, -NO₂, -CHO, -CF₂H, -CFH₂, -C(=O)-NH₂, -C(=O)-NH-CH₃, -C(=O)-NH-C₂H₅, -C(=O)-N(CH₃)₂, -C(=O)-N(C₂H₅)₂, -S(=O)₂-CH₃, phényle, phénéthyle et benzyle, dans lequel lesdits substituants cycliques peuvent être non substitués ou substitués par 1, 2 ou 3 substituants choisis indépendamment dans le groupe constitué de méthyle, éthyle, n-propyle, isopropyle, méthoxy, éthoxy, F, Cl, Br, -CN, -CF₃, -OCF₃, -SCF₃, -OH, -SH, -NH₂ et -NO₂ ;
R¹¹ représente un radical choisi dans le groupe constitué de méthyle, éthyle, n-propyle, isopropyle, n-butyle, sec-butyle, isobutyle, tert-butyle et n-pentyle ; et
R¹² représente un atome d'hydrogène ; un radical choisi dans le groupe constitué de méthyle, éthyle, n-propyle, isopropyle, n-butyle, sec-butyle, isobutyle, tert-butyle et n-pentyle ;
éventuellement sous la forme d'un de ses stéréoisomères, de préférence des énantiomères ou des diastéréoisomères, un racémique ou sous la forme d'un mélange d'au moins deux de ses stéréoisomères, de préférence des énantiomères et/ou des diastéréoisomères, dans un quelconque rapport de mélange, ou un sel de celui-ci, ou un solvate correspondant de celui-ci.

2. Composé selon la revendication 1, **caractérisé en ce que** m est 1 ;
X représente un groupement -OR⁵ ou un groupement -NR⁶R⁷ ; Y représente un groupement -OR⁸ ; un groupement -NR⁹R¹⁰ ou un groupement -C(=O)-OR¹¹ ;
R⁵ et R⁸, indépendamment l'un de l'autre, représentent chacun un atome d'hydrogène ; un radical choisi dans le groupe constitué de méthyle, éthyle, n-propyle, isopropyle, n-butyle, sec-butyle, isobutyle, tert-butyle et n-pentyle ; un radical aryle choisi dans le groupe constitué de phényle et napthyle, qui peut être lié par un groupe -(CH₂)_{1, 2 ou 3} et qui peut être non substitué ou éventuellement substitué par 1, 2, 3, 4 ou 5 substituants choisis indépendamment dans le groupe constitué de méthyle, éthyle, n-propyle, isopropyle, n-butyle, tert-butyle, -O-CH₃, -O-C₂H₅, F, Cl, Br, I, -CN, -CF₃, -OCF₃, -SCF₃, -OH, -SH, -NH₂, -NO₂, -CHO, -CF₂H, -CFH₂ et -S(=O)₂-CH₃ ; ou un groupement -C(=O)-R¹²;
R⁶, R⁷, R⁹ et R¹⁰, indépendamment l'un de l'autre, représentent chacun un radical choisi dans le groupe constitué de méthyle, éthyle, n-propyle, isopropyle, n-butyle, sec-butyle, isobutyle, tert-butyle et n-pentyle ; ou un radical aryle choisi dans le groupe constitué de phényle et napthyle, qui peut être lié par un groupe -(CH₂)_{1, 2} ou ₃ et qui peut être non substitué ou éventuellement substitué par 1, 2, 3, 4 ou 5 substituants choisis indépendamment dans le groupe constitué de méthyle, éthyle, n-propyle, isopropyle, n-butyle, tert-butyle, sec-butyle, isobutyle, -O-CH₃, -O-C₂H₅, -S-CH₃, -S-C₂H₅, F, Cl, Br, I, -CN, -CF₃, -OCF₃, -SCF₃, -OH, -SH, -NH₂-NO₂, -CHO, -CF₂H, -CFH₂, -C(=O)-NH₂ et -S(=O)₂-CH₃ ;
ou R⁶ et R⁷ conjointement à l'atome d'azote pontant forment un groupement choisi dans le groupe constitué de 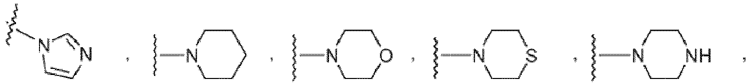 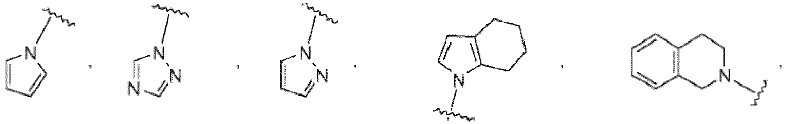 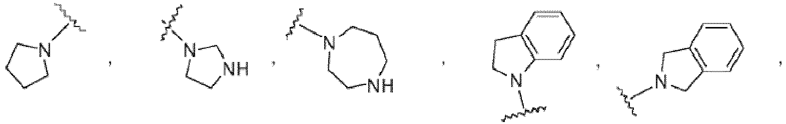 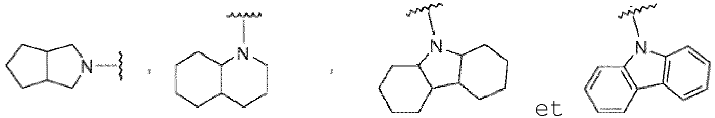 qui peut être non substitué ou éventuellement substitué en une quelconque position y compris les groupes -NH par 1, 2, 3, 4 ou 5 substituants choisis indépendamment dans le groupe constitué de oxo (=0), méthyle, éthyle, n-propyle, isopropyle, n-butyle, tert-butyle, sec-butyle, -O-CH₃, -O-C₂H₅, -S-CH₃, -S-C₂H₅, -C(=O)-OH, -C(=O)-O-CH₃, -C(=O)-O-C₂H₅, -C(=O)-O-CH₂-CH₂-CH₃, -C(=O)-O-CH(CH₃)₂, -C(=O)-O-C(CH₃)₃, -C(=O)-CH₃, -C(=O)-C₂H₅, -C(=O)-CH₂-CH₂-CH₃, -C(=O)-CH(CH₃)₂, -C(=O)-C(CH₃)₃, F, Cl, Br, I, -CN, -CF₃, -OCF₃, -SCF₃, -OH, -SH, -NH₂, -NO₂, -CHO, -CF₂H, -CFH₂, -C(=O)-NH₂, -C(=O)-NH-CH₃, -C(=O)-NH-C₂H₅, -C(=O)-N(CH₃)₂, -C(=O)-N(C₂H₅)₂, -S(=O)₂-CH₃, phényle, phénéthyle et benzyle, dans lequel lesdits substituants cycliques peuvent être non substitués ou substitués par 1, 2 ou 3 substituants choisis indépendamment dans le groupe constitué de méthyle, éthyle, n-propyle, isopropyle, méthoxy, éthoxy, F, Cl, Br, -CN, -CF₃, -OCF₃, -SCF₃, -OH, -SH, -NH₂ et -NO₂ ;
ou R⁹ et R¹⁰ conjointement à l'atome d'azote pontant forment un groupement choisi dans le groupe constitué de 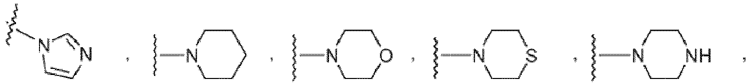 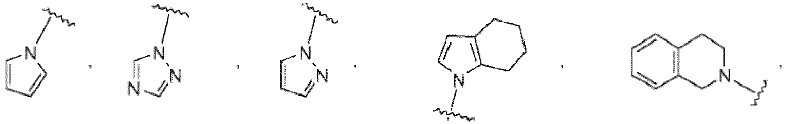 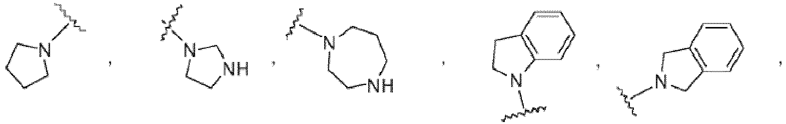 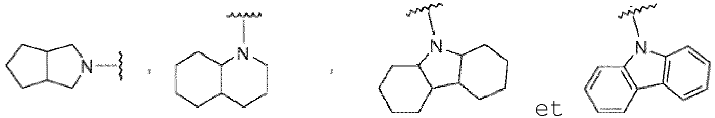 qui peut être non substitué ou éventuellement substitué en une quelconque position y compris les groupes -NH par 1, 2, 3, 4 ou 5 substituants choisis indépendamment dans le groupe constitué de oxo (=0), méthyle, éthyle, n-propyle, isopropyle, n-butyle, tert-butyle, sec-butyle, -O-CH₃, -O-C₂H₅, -S-CH₃, -S-C₂H₅, -C(=O)-OH, -C(=O)-O-CH₃, -C(=O)-O-C₂H₅, -C(=O)-O-CH₂-CH₂-CH₃, -C(=O)-O-CH(CH₃)₂, -C(=O)-O-C(CH₃)₃, -C(=O)-CH₃, -C(=O)-C₂H₅, -C(=O)-CH₂-CH₂-CH₃, -C(=O)-CH(CH₃)₂, -C(=O)-C(CH₃)₃, F, Cl, Br, I, -CN, -CF₃, -OCF₃, -SCF₃, -OH, -SH, -NH₂, -NO₂, -CHO, -CF₂H, -CFH₂, -C(=O)-NH₂, -C(=O)-NH-CH₃, -C(=O)-NH-C₂H₅, -C(=O)-N(CH₃)₂, -C(=O)-N(C₂H₅)₂, -S(=O)₂-CH₃, phényle, phénéthyle et benzyle, dans lequel lesdits substituants cycliques peuvent être non substitués ou substitués par 1, 2 ou 3 substituants choisis indépendamment dans le groupe constitué de méthyle, éthyle, n-propyle, isopropyle, méthoxy, éthoxy, F, Cl, Br, -CN, -CF₃, -OCF₃, -SCF₃, -OH, -SH, -NH₂ et -NO₂.

3. Composé selon la revendication 1 ou 2, **caractérisé en ce que**
m est 1 ;
X représente un groupement -OR⁵ ou un groupement -NR⁶R⁷ ; Y représente un groupement -OR⁸ ; un groupement -NR⁹R¹⁰ ou un groupement -C(=O)-OR¹¹ ;
R² représente un atome d'hydrogène ; un radical choisi dans le groupe constitué de méthyle, éthyle, n-propyle, isopropyle, n-butyle, sec-butyle, isobutyle, tert-butyle et n-pentyle ; ou un radical phényle qui peut être non substitué ou substitué par 1, 2, 3, 4 ou 5 substituants choisis indépendamment dans le groupe constitué de méthyle, éthyle, n-propyle, isopropyle, n-butyle, tert-butyle, sec-butyle, isobutyle, n-pentyle, -O-CH₃, -O-C₂H₅, F, Cl, Br, I, -CN, -CF₃, -OCF₃ et -SCF₃ ;
R⁵ et R⁸, indépendamment l'un de l'autre, représentent chacun un atome d'hydrogène ; un radical phényle, qui peut être lié par un groupe - (CH₂)₁, ou ₃ et qui peut être non substitué ou éventuellement substitué par 1, 2, 3, 4 ou 5 substituants choisis indépendamment dans le groupe constitué de méthyle, éthyle, n-propyle, isopropyle, n-butyle, tert-butyle, -O-CH₃, -O-C₂H₅, F, Cl, Br, I, -CN, -CF₃ et -OCF₃ ; ou un groupement -C(=O)-R¹² ;
R⁶, R⁷, R⁹ et R¹⁰, indépendamment l'un de l'autre, représentent chacun un radical choisi dans le groupe constitué de méthyle, éthyle, n-propyle, isopropyle, n-butyle, sec-butyle, isobutyle, tert-butyle et n-pentyle ; ou un radical phényle, qui peut être lié par un groupe -(CH₂)_{1, 2} ou ₃ et qui peut être non substitué ou éventuellement substitué par 1, 2, 3, 4 ou 5 substituants choisis indépendamment dans le groupe constitué de méthyle, éthyle, n-propyle, isopropyle, n-butyle, tert-butyle, sec-butyle, isobutyle, -O-CH₃, -O-C₂H₅, F, Cl, Br, I, -CN, -CF₃, -OCF₃ et -SCF₃ ;
ou R⁶ et R⁷ conjointement à l'atome d'azote pontant forment un groupement choisi dans le groupe constitué de 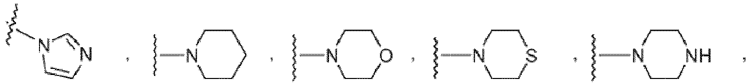 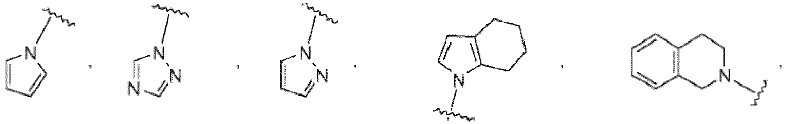 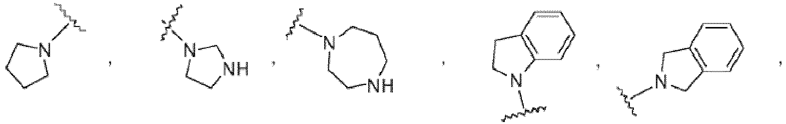 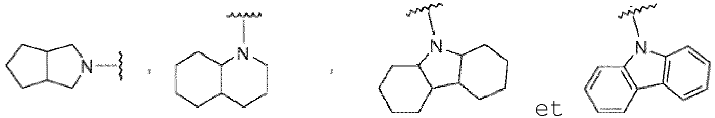 qui peut être non substitué ou éventuellement substitué en une quelconque position y compris les groupes -NH par 1, 2, 3, 4 ou 5 substituants choisis indépendamment dans le groupe constitué de oxo (=0), méthyle, éthyle, n-propyle, isopropyle, n-butyle, tert-butyle, sec-butyle, phényle, phénéthyle et benzyle, dans lequel lesdits substituants cycliques peuvent être non substitués ou substitués par 1, 2 ou 3 substituants choisis indépendamment dans le groupe constitué de méthyle, éthyle, n-propyle, isopropyle, méthoxy, éthoxy, F, Cl, Br, -CN, -CF₃, -OCF₃, -SCF₃, -OH, -SH, -NH₂ et -NO₂ ;
ou R⁹ et R¹⁰ conjointement à l'atome d'azote pontant forment un groupement choisi dans le groupe constitué de 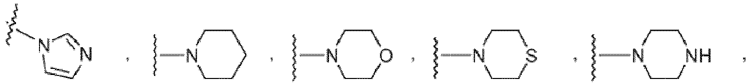 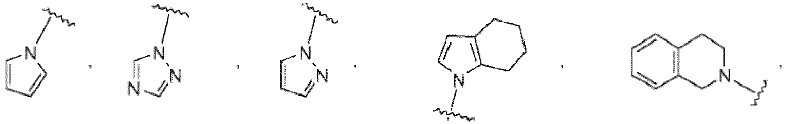 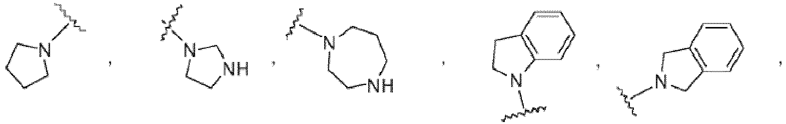 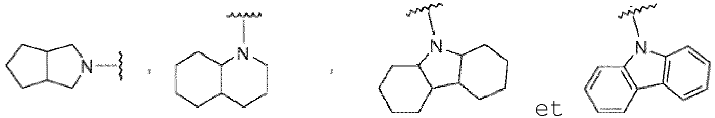 qui peut être non substitué ou éventuellement substitué en une quelconque position y compris les groupes -NH par 1, 2, 3, 4 ou 5 substituants choisis indépendamment dans le groupe constitué de oxo (=0), méthyle, éthyle, n-propyle, isopropyle, n-butyle, tert-butyle, sec-butyle, phényle, phénéthyle et benzyle, dans lequel lesdits substituants cycliques peuvent être non substitués ou substitués par 1, 2 ou 3 substituants choisis indépendamment dans le groupe constitué de méthyle, éthyle, n-propyle, isopropyle, méthoxy, éthoxy, F, Cl, Br, -CN, -CF₃, -OCF₃, -SCF₃, -OH, -SH, -NH₂ et -NO₂.

4. Composé selon une ou plusieurs des revendications 1 à 3, **caractérisé en ce que**
m est 1 ;
X représente un groupement -OR⁵ ou un groupement -NR⁶R⁷ ; Y représente un groupement -OR⁸ ; un groupement -NR⁹R¹⁰ ou un groupement -C(=O)-OR¹¹ ;
R² représente un atome d'hydrogène ; un radical choisi dans le groupe constitué de méthyle, éthyle, n-propyle, isopropyle, n-butyle, sec-butyle, isobutyle, tert-butyle et n-pentyle ; ou un radical phényle qui peut être non substitué ou substitué par 1, 2, 3, 4 ou 5 substituants choisis indépendamment dans le groupe constitué de méthyle, éthyle, n-propyle, isopropyle, n-butyle, tert-butyle, sec-butyle, isobutyle, n-pentyle, -O-CH₃, -O-C₂H₅, F, Cl, Br, I, -CN, -CF₃, -OCF₃ et -SCF₃ ;
R⁵ et R⁸, indépendamment l'un de l'autre, représentent chacun un atome d'hydrogène ; un radical phényle, qui peut être lié par un groupe -(CH₂)_{1, ou 3} et qui peut être non substitué ou éventuellement substitué par 1, 2, 3, 4 ou 5 substituants choisis indépendamment dans le groupe constitué de méthyle, éthyle, n-propyle, isopropyle, n-butyle, tert-butyle, -O-CH₃, -O-C₂H₅, F, Cl, Br, I, -CN, -CF₃ et -OCF₃ ; ou un groupement -C(=O)-R¹² ;
R⁶, R⁷, R⁹ et R¹⁰, indépendamment l'un de l'autre, représentent chacun un radical choisi dans le groupe constitué de méthyle, éthyle, n-propyle, isopropyle, n-butyle, sec-butyle, isobutyle, tert-butyle et n-pentyle ; ou un radical phényle, qui peut être lié par un groupe -(CH₂)_{1, 2 ou 3} et qui peut être non substitué ou éventuellement substitué par 1, 2, 3, 4 ou 5 substituants choisis indépendamment dans le groupe constitué de méthyle, éthyle, n-propyle, isopropyle, n-butyle, tert-butyle, sec-butyle, isobutyle, -O-CH₃, -O-C₂H₅, F, Cl, Br, I, -CN, -CF₃, -OCF₃ et -SCF₃ ;
ou R⁶ et R⁷ conjointement à l'atome d'azote pontant forment un groupement choisi dans le groupe constitué de 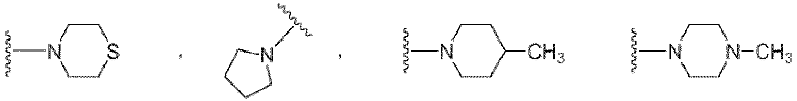 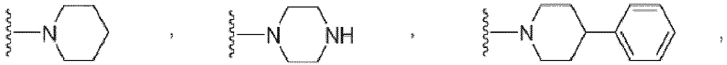 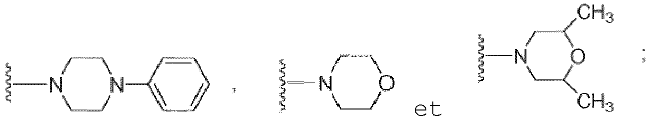 ou R⁹ et R¹⁰ conjointement à l'atome d'azote pontant forment un groupement choisi dans le groupe constitué de

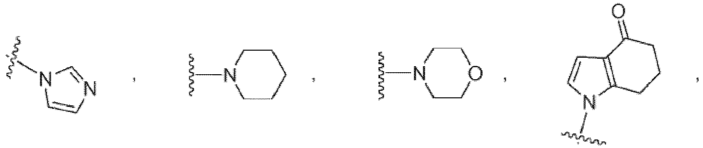

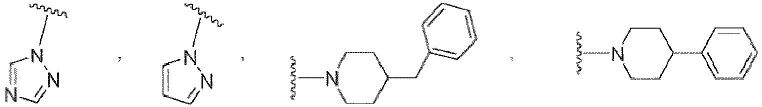

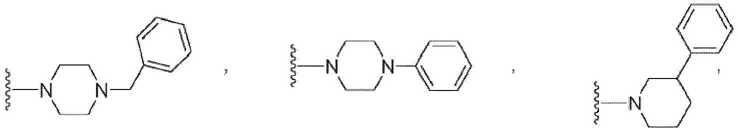

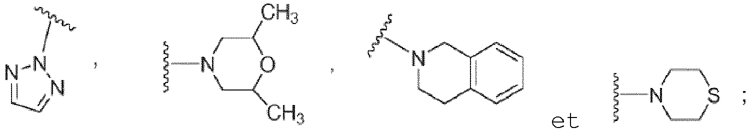

5. Composé de formule générale Ie selon une ou plusieurs des revendications 1 ou 2, 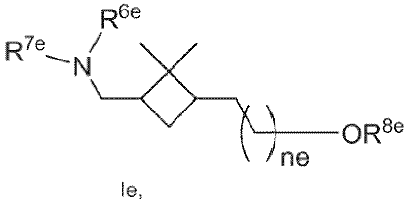 dans laquelle
ne est 0 ou 1 ;
R^{6e} et R^{7e}, indépendamment l'un de l'autre, représentent chacun un radical choisi dans le groupe constitué de méthyle, éthyle, n-propyle, isopropyle, n-butyle, sec-butyle, isobutyle, tert-butyle et n-pentyle ; ou un radical aryle choisi dans le groupe constitué de phényle et napthyle, qui peut être lié par un groupe - (CH₂)_{1, 2 ou 3} et qui peut être non substitué ou éventuellement substitué par 1, 2, 3, 4 ou 5 substituants choisis indépendamment dans le groupe constitué de méthyle, éthyle, n-propyle, isopropyle, n-butyle, tert-butyle, sec-butyle, isobutyle, -O-CH₃, -O-C₂H₅, -S-CH₃, -S-C₂H₅, F, Cl, Br, I, -CN, -CF₃, -OCF₃, -SCF₃, -OH, -SH, -NH₂-NO₂, -CHO, -CF₂H, -CFH₂, -C(=O)-NH₂ et -S(=O)₂-CH₃ ;
ou R^{6e} et R^{7e} conjointement à l'atome d'azote pontant forment un groupement choisi dans le groupe constitué de 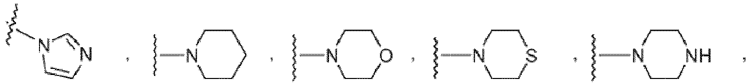 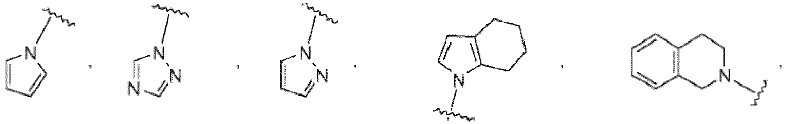 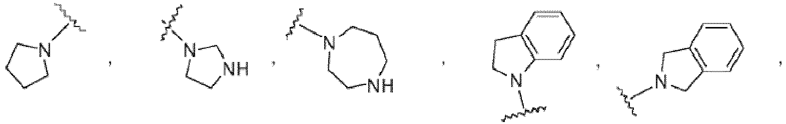 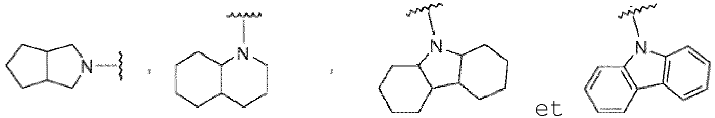 qui peut être non substitué ou éventuellement substitué en une quelconque position y compris les groupes -NH par 1, 2, 3, 4 ou 5 substituants choisis indépendamment dans le groupe constitué de oxo (=0), méthyle, éthyle, n-propyle, isopropyle, n-butyle, tert-butyle, sec-butyle, -O-CH₃, -O-C₂H₅, -S-CH₃, -S-C₂H₅, -C(=O)-OH, -C(=O)-O-CH₃, -C(=O)-O-C₂H₅, -C(=O)-O-CH₂-CH₂-CH₃, -C(=O)-O-CH(CH₃)₂, -C(=O)-O-C(CH₃)₃, -C(=O)-CH₃, -C(=O)-C₂H₅, -C(=O)-CH₂-CH₂-CH₃, -C(=O)-CH(CH₃)₂, -C(=O)-C(CH₃)₃, F, Cl, Br, I, -CN, -CF₃, -OCF₃, -SCF₃, -OH, -SH, -NH₂, -NO₂, -CHO, -CF₂H, -CFH₂, -C(=O)-NH₂, -C(=O)-NH-CH₃, -C(=O)-NH-C₂H₅, -C(=O)-N(CH₃)₂, -C(=O)-N(C₂H₅)₂, -S(=O)₂-CH₃, phényle, phénéthyle et benzyle, dans lequel lesdits substituants cycliques peuvent être non substitués ou substitués par 1, 2 ou 3 substituants choisis indépendamment dans le groupe constitué de méthyle, éthyle, n-propyle, isopropyle, méthoxy, éthoxy, F, Cl, Br, -CN, -CF₃, -OCF₃, -SCF₃, -OH, -SH, -NH₂ et -NO₂ ;
R^{8e} représente un atome d'hydrogène ; un radical choisi dans le groupe constitué de méthyle, éthyle, n-propyle, isopropyle, n-butyle, sec-butyle, isobutyle, tert-butyle et n-pentyle ; un radical aryle choisi dans le groupe constitué de phényle et napthyle, qui peut être lié par un groupe -(CH₂)_{1, 2 ou 3} et qui peut être non substitué ou éventuellement substitué par 1, 2, 3, 4 ou 5 substituants choisis indépendamment dans le groupe constitué de méthyle, éthyle, n-propyle, isopropyle, n-butyle, tert-butyle, -O-CH₃, -O-C₂H₅, F, Cl, Br, I, -CN, -CF₃, -OCF₃, -SCF₃, -OH, -SH, -NH₂, -NO₂, -CHO, -CF₂H, -CFH₂ et -S(=O)₂-CH₃ ; ou un groupement -C(=O)-R^{12e} ;
et R^{12e} représente un radical choisi dans le groupe constitué de méthyle, éthyle, n-propyle, isopropyle, n-butyle, sec-butyle, isobutyle, tert-butyle et n-pentyle ;
éventuellement sous la forme d'un de ses stéréoisomères, de préférence des énantiomères ou des diastéréoisomères, un racémique ou sous la forme d'un mélange d'au moins deux de ses stéréoisomères, de préférence des énantiomères et/ou des diastéréoisomères, dans un quelconque rapport de mélange, ou un sel de celui-ci, ou un solvate correspondant de celui-ci.

6. Composé selon la revendication 5, **caractérisé en ce que** les stéréoisomères ont les formules générales If ou Ig ou Ih ou Ij, 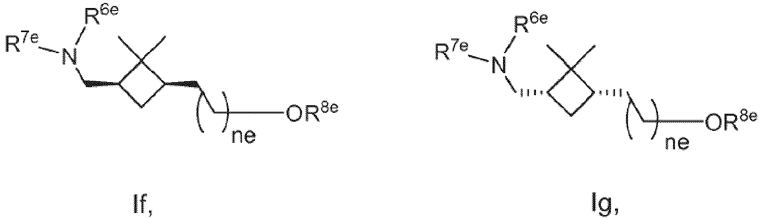 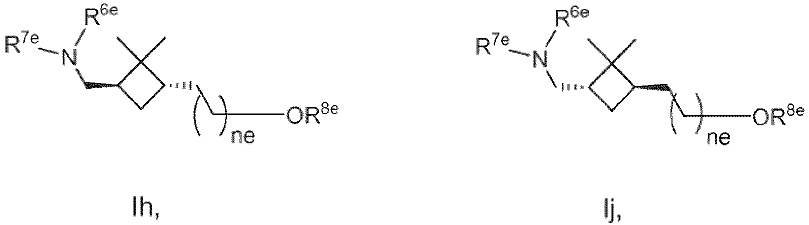 dans lesquelles ne, R^{6e}, R^{7e} et R^{8e} sont tels que définis dans la revendication 5.

7. Composé de formule générale Ik selon une ou plusieurs des revendications 1 à 2, 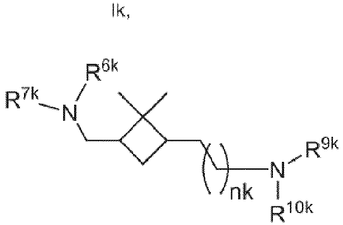 dans laquelle
R^{6k}R^{7k}N- et R^{9k}R^{10k}N- sont différents ;
R^{6k} R^{7k}, R^{9k} et R^{10k}, indépendamment l'un de l'autre, représentent chacun un radical choisi dans le groupe constitué de méthyle, éthyle, n-propyle, isopropyle, n-butyle, sec-butyle, isobutyle, tert-butyle et n-pentyle ; ou un radical aryle choisi dans le groupe constitué de phényle et napthyle, qui peut être lié par un groupe -(CH₂)_{1, 2 ou 3} et qui peut être non substitué ou éventuellement substitué par 1, 2, 3, 4 ou 5 substituants choisis indépendamment dans le groupe constitué de méthyle, éthyle, n-propyle, isopropyle, n-butyle, tert-butyle, sec-butyle, isobutyle, -O-CH₃, -O-C₂H₅, -S-CH₃, -S-C₂H₅, F, Cl, Br, I, -CN, -CF₃, -OCF₃, -SCF₃, -OH, -SH, -NH₂-NO₂, -CHO, -CF₂H, -CFH₂, -C(=O)-NH₂ et -S(=O)₂-CH₃ ;
ou R^{6k} et R^{7k} conjointement à l'atome d'azote pontant forment un groupement choisi dans le groupe constitué de 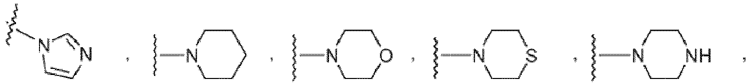 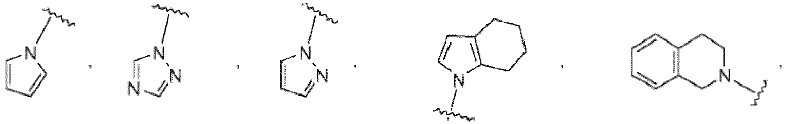 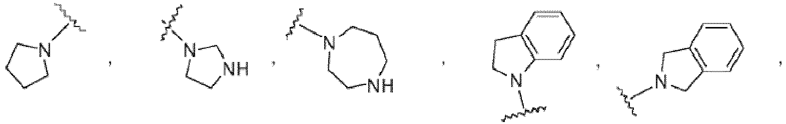 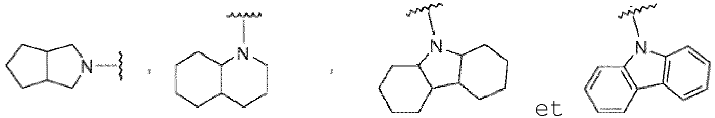 qui peut être non substitué ou éventuellement substitué en une quelconque position y compris les groupes -NH par 1, 2, 3, 4 ou 5 substituants choisis indépendamment dans le groupe constitué de oxo (=0), méthyle, éthyle, n-propyle, isopropyle, n-butyle, tert-butyle, sec-butyle, -O-CH₃, -O-C₂H₅, -S-CH₃, -S-C₂H₅, -C(=O)-OH, -C(=O)-O-CH₃, -C(=O)-O-C₂H₅, -C(=O)-O-CH₂-CH₂-CH₃, -C(=O)-O-CH(CH₃)₂, -C(=O)-O-C(CH₃)₃, -C(=O)-CH₃, -C(=O)-C₂H₅, -C(=O)-CH₂-CH₂-CH₃, -C(=O)-CH(CH₃)₂, -C(=O)-C(CH₃)₃, F, Cl, Br, I, -CN, -CF₃, -OCF₃, -SCF₃, -OH, -SH, -NH₂, -NO₂, -CHO, -CF₂H, -CFH₂, -C(=O)-NH₂, -C(=O)-NH-CH₃, -C(=O)-NH-C₂H₅, -C(=O)-N(CH₃)₂, -C(=O)-N(C₂H₅)₂, -S(=O)₂-CH₃, phényle, phénéthyle et benzyle, dans lequel lesdits substituants cycliques peuvent être non substitués ou substitués par 1, 2 ou 3 substituants choisis indépendamment dans le groupe constitué de méthyle, éthyle, n-propyle, isopropyle, méthoxy, éthoxy, F, Cl, Br, -CN, -CF₃, -OCF₃, -SCF₃, -OH, -SH, -NH₂ et -NO₂ ;
ou R^{9k} et R^{10k} conjointement à l'atome d'azote pontant forment un groupement choisi dans le groupe constitué de 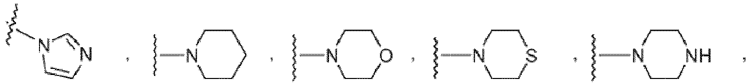 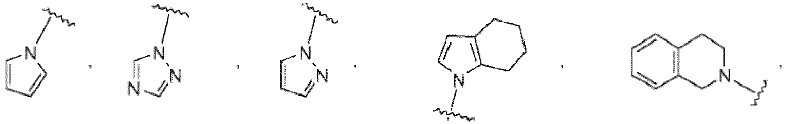 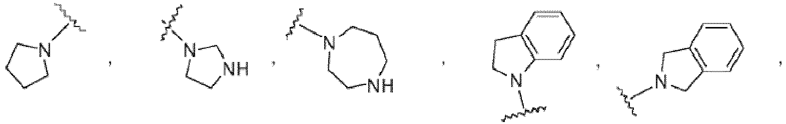 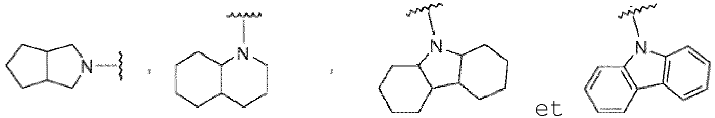 qui peut être non substitué ou éventuellement substitué en une quelconque position y compris les groupes -NH par 1, 2, 3, 4 ou 5 substituants choisis indépendamment dans le groupe constitué de oxo (=0), méthyle, éthyle, n-propyle, isopropyle, n-butyle, tert-butyle, sec-butyle, -O-CH₃, -O-C₂H₅, -S-CH₃, -S-C₂H₅, -C(=O)-OH, -C(=O)-O-CH₃, -C(=O)-O-C₂H₅, -C(=O)-O-CH₂-CH₂-CH₃, -C(=O)-O-CH(CH₃)₂, -C(=O)-O-C(CH₃)₃, -C(=O)-CH₃, -C(=O)-C₂H₅, -C(=O)-CH₂-CH₂-CH₃, -C(=O)-CH(CH₃)₂, -C(=O)-C(CH₃)₃, F, Cl, Br, I, -CN, -CF₃, -OCF₃, -SCF₃, -OH, -SH, -NH₂, -NO₂, -CHO, -CF₂H, -CFH₂, -C(=O)-NH₂, -C(=O)-NH-CH₃, -C(=O)-NH-C₂H₅, -C(=O)-N(CH₃)₂, -C(=O)-N(C₂H₅)₂, -S(=O)₂-CH₃, phényle, phénéthyle et benzyle, dans lequel lesdits substituants cycliques peuvent être non substitués ou substitués par 1, 2 ou 3 substituants choisis indépendamment dans le groupe constitué de méthyle, éthyle, n-propyle, isopropyle, méthoxy, éthoxy, F, Cl, Br, -CN, -CF₃, -OCF₃, -SCF₃, -OH, -SH, -NH₂ et -NO₂ ;
éventuellement sous la forme d'un de ses stéréoisomères, de préférence des énantiomères ou des diastéréoisomères, un racémique ou sous la forme d'un mélange d'au moins deux de ses stéréoisomères, de préférence des énantiomères et/ou des diastéréoisomères, dans un quelconque rapport de mélange, ou un sel de celui-ci, ou un solvate correspondant de celui-ci.

8. Composé selon la revendication 7, **caractérisé en ce que** les stéréoisomères ont les formules générales Im ou In ou Io ou Ip, 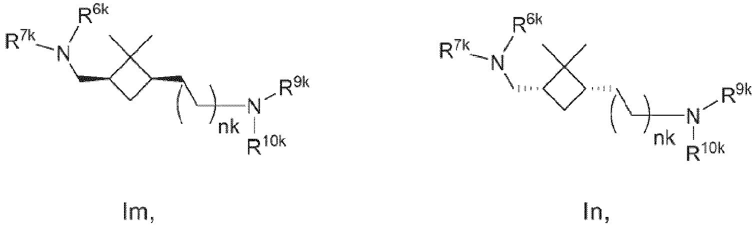 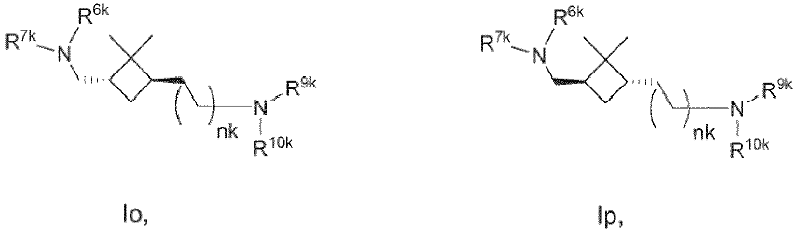 dans lesquelles nk, R^{6k}, R^{7k}, R^{9k} et R^{10k} sont tels que définis dans la revendication 7.

9. Composé selon une ou plusieurs des revendications 1 à 8 choisi dans le groupe constitué de
[1] la 4-(((1S,3S)-3-(2-(benzyloxy)éthyl)-2,2-diméthylcyclobutyl)méthyl)-morpholine
[2] la 1-(((1S,3S)-3-(2-(benzyloxy)éthyl)-2,2-diméthylcyclobutyl)méthyl)-pipéridine
[3] la 1-(((1S,3S)-3-(2-(benzyloxy)éthyl)-2,2-diméthylcyclobutyl)méthyl)-4-phénylpipéridine
[4] la 1-(((1S,3S)-3-(2-(benzyloxy)éthyl)-2,2-diméthylcyclobutyl)méthyl)-4-phénylpipérazine
[5] la 1-(((1S,3S)-3-(2-(benzyloxy)éthyl)-2,2-diméthylcyclobutyl)méthyl)-4-méthylpipérazine
[6] la 4-(((1R,3S)-3-(benzyloxyméthyl)-2,2-diméthylcyclobutyl)méthyl)-morpholine
[7] la 1-(((1R,3S)-3-(benzyloxyméthyl)-2,2-diméthylcyclobutyl)méthyl)-pipéridine
[8] la 1-(((1R,3S)-3-(benzyloxyméthyl)-2,2-diméthylcyclobutyl)méthyl)-4-phénylpipéridine
[9] la 1-(((1R,3S)-3-(benzyloxyméthyl)-2,2-diméthylcyclobutyl)méthyl)-4-phénylpipérazine
[10] la 1-(((1R,3S)-3-(benzyloxyméthyl)-2,2-diméthylcyclobutyl)méthyl)-4-méthylpipérazine
[13] le 1-((1R,3R)-2,2-diméthyl-3-(2-morpholinoéthyl)cyclobutyl)-1-phényléthanol
[14] le 1-((1R,3R)-2,2-diméthyl-3-(2-(pipéridin-1-yl)éthyl)cyclobutyl)-1-phényléthanol
[15] la 4-(((1R,3R)-2,2-diméthyl-3-(2-phénoxyéthyl)cyclobutyl)méthyl)-morpholine
[16] la 1-(((1R,3R)-2,2-diméthyl-3-(2-phénoxyéthyl)cyclobutyl)méthyl)-pipéridine
[17] le 2-((1R,3R)-2,2-diméthyl-3-(pipéridin-1-ylméthyl)cyclobutyl)éthanol
[18] le 2-((1S,3S)-2,2-diméthyl-3-(pipéridin-1-ylméthyl)cyclobutyl)éthanol
[19] le 2-((1R,3R)-2,2-diméthyl-3-(morpholinométhyl)cyclobutyl)éthanol
[20] le 2-((1S,3S)-2,2-diméthyl-3-(morpholinométhyl)cyclobutyl)éthanol
[21] le 2-((1R,3R)-2,2-diméthyl-3-((4-méthylpipéridin-1-yl)méthyl)cyclobutyl)éthanol
[22] le 2-((1R,3R)-2,2-diméthyl-3-((4-phénylpipéridin-1-yl)méthyl)cyclobutyl)éthanol
[23] le 2-((1R,3R)-3-(((2S,6R)-2,6-diméthyl-morpholino)méthyl)-2,2-diméthylcyclobutyl)éthanol
[24] le 2-((1R,3R)-3-(N-benzyl-N-méthylamino)méthyl)-2,2-diméthylcyclobutyl)éthanol
[26] le 2-((1R,3R)-2,2-diméthyl-3-(pipéridin-1-ylméthyl)cyclobutyl)acétate de méthyle
[27] la 1-(((1R,3R)-3-(2-(benzyloxy)éthyl)-2,2-diméthylcyclobutyl)méthyl)-pipéridine
[28] la 1-(((1R,3R)-3-(2-(benzyloxy)éthyl)-2,2-diméthylcyclobutyl)méthyl)-4-méthylpipéridine
[29] la 1-(((1R,3R)-3-(2-(benzyloxy)éthyl)-2,2-diméthylcyclobutyl)méthyl)-4-phénylpipéridine
[30] la 4-(((1R,3R)-3-(2-(benzyloxy)éthyl)-2,2-diméthylcyclobutyl)méthyl)-thiomorpholine
[31] la 4-(((1R,3R)-3-(2-(benzyloxy)éthyl)-2,2-diméthylcyclobutyl)méthyl)-morpholine
[32] la (2S,6R)-4-(((1R,3R)-3-(2-(benzyloxy)éthyl)-2,2-diméthylcyclobutyl)méthyl)-2,6-diméthylmorpholine
[33] la 1-(((1R,3R)-3-(2-(benzyloxy)éthyl)-2,2-diméthylcyclobutyl)méthyl)-pyrrolidine
[34] la 1-(((1R,3R)-3-(2-(benzyloxy)éthyl)-2,2-diméthylcyclobutyl)méthyl)-4-méthylpipérazine
[35] l'oxalate de 1-(((1R,3R)-3-(2-(benzyloxy)éthyl)-2,2-diméthylcyclobutyl)méthyl)-4-méthylpipériazine
[36] la 1-(((1R,3R)-3-(2-(benzyloxy)éthyl)-2,2-diméthylcyclobutyl)méthyl)-4-phénylpipérazine
[37] l'oxalate de 1-(((1R,3R)-3-(2-(benzyloxy)éthyl)-2,2-diméthylcyclobutyl)méthyl)-4-phénylpipérazine
[38] la 1-(2-((1R,3R)-3-(benzyloxyméthyl)-2,2-diméthylcyclobutyl)éthyl)-pipéridine
[39] la 4-(2-((1R,3R)-3-(benzyloxyméthyl)-2,2-diméthylcyclobutyl)éthyl)-morpholine
[40] la 4-(((1R,3R)-2,2-diméthyl-3-(2-(3-phénylpipéridin-1-yl)éthyl)cyclobutyl)méthyl)-morpholine
[41] la 4-(((1R,3R)-2,2-diméthyl-3-(2-(4-phénylpipéridin-1-yl)éthyl)cyclobutyl)méthyl)morpholine
[42] la 4-(((1R,3R)-3-(2-(4-benzylpipéridin-1-yl)éthyl)-2,2-diméthylcyclobutyl)méthyl)morpholine
[43] la 4-(((1R,3R)-3-(2-(1H-imidazol-1-yl)éthyl)-2,2-diméthylcyclobutyl)méthyl)morpholine
[44] la 4-(((1R,3R)-3-(2-(1H-pyrazol-1-yl)éthyl)-2,2-diméthylcyclobutyl)méthyl)morpholine
[45] la 4-(((1R,3R)-3-(2-(1H-1,2,4-triazol-1-yl)éthyl)-2,2-diméthylcyclobutyl)méthyl)morpholine
[46] la 1-(2-((1R,3R)-2,2-diméthyl-3-(morpholinométhyl)cyclobutyl)éthyl)-6,7-dihydro-1H-indol-4(5H)-one
[47] la 2-(2-((1R,3R)-2,2-diméthyl-3-(morpholinométhyl)cyclobutyl)éthyl)-1,2,3,4-tétrahydroisoquinoline
[48] la 1-(((1R,3R)-3-(2-(1H-imidazol-1-yl)éthyl)-2,2-diméthylcyclobutyl)méthyl)pipéridine
[49] la 1-(((1R,3R)-3-(2-(1H-pyrazol-1-yl)éthyl)-2,2-diméthylcyclobutyl)méthyl)pipéridine
[50] la 1-(((1R,3R)-3-(2-(1H-1,2,4-triazol-1-yl)éthyl)-2,2-diméthylcyclobutyl)méthyl)pipéridine
[51] la 1-(2-((1R,3R)-2,2-diméthyl-3-(pipéridin-1-ylméthyl)cyclobutyl)éthyl)-4-phénylpipéridine
[52] la 4-benzyl-1-(2-((1R,3R)-2,2-diméthyl-3-(pipéridin-1-ylméthyl)cyclobutyl)éthyl)pipéridine
[53] la 1-(2-((1R,3R)-2,2-diméthyl-3-(pipéridin-1-ylméthyl)cyclobutyl)éthyl)-6,7-dihydro-1H-indol-4(5H)-one
[54] la 2-(2-((1R,3R)-2,2-diméthyl-3-(pipéridin-1-ylméthyl)cyclobutyl)éthyl)-1,2,3,4-tétrahydroisoquinoline
[55] le pivalate de 2-((1R,3R)-2,2-diméthyl-3-((pipéridin-1-yl)méthyl)cyclobutyl)éthyle
[56] l'acétate de 2-((1R,3R)-2,2-diméthyl-3-((pipéridin-1-yl)méthyl)cyclobutyl)éthyle
éventuellement sous la forme d'un de ses stéréoisomères, de préférence des énantiomères ou des diastéréoisomères, un racémique ou sous la forme d'un mélange d'au moins deux de ses stéréoisomères, de préférence des énantiomères et/ou des diastéréoisomères, dans un quelconque rapport de mélange, ou un sel de celui-ci, ou un solvate correspondant de celui-ci.

10. Procédé de préparation d'un composé de formule générale I selon une ou plusieurs des revendications 1 à 9, **caractérisé en ce qu'**au moins un composé de formule générale II, 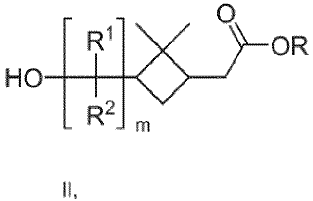 dans laquelle m, R¹ et R² ont la signification selon une ou plusieurs des revendications 1 à 9 et R représente un radical alkyle en C₁-C₅ linéaire ou ramifié ou un radical benzyle,
est mis à réagir avec du chlorure de méthane-sulfonyle, du chlorure de *p*-toluène-sulfonyle, du chlorure de trifluorométhane-sulfonyle, du chlorure de thionyle ou du tétrabromoéthane, de préférence dans un milieu réactionnel, de préférence en présence d'au moins une base, pour donner au moins un composé de formule générale III, 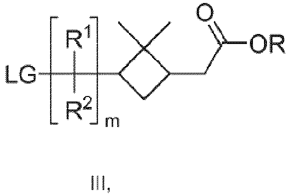 dans laquelle m, R¹ et R² ont la signification donnée ci-dessus, R représente un radical alkyle en C₁-C₅ linéaire ou ramifié et LG représente -O-S(=O)₂-CH₃, -O-S(=O)₂-p-toluyle, -O-S(=O)₂-CF₃, Cl ou Br, qui est éventuellement purifié et/ou isolé,
et au moins un composé de formule générale III est mis à réagir avec au moins un composé de formule générale HNR⁶R⁷, dans laquelle R⁶ et R⁷ ont la signification selon une ou plusieurs des revendications 1 à 9, de préférence dans un milieu réactionnel, de préférence en présence d'au moins une base, pour donner au moins un composé de formule générale IV, 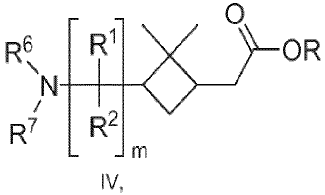 dans laquelle m, R¹, R², R⁶ et R⁷ ont la signification donnée ci-dessus et R représente un radical alkyle en C₁-C₅ linéaire ou ramifié, qui est éventuellement purifié et/ou isolé,
et au moins un composé de formule générale IV est mis à réagir avec au moins un agent réducteur choisi dans le groupe constitué du borohydrure de lithium, du borohydrure de sodium, de l'hydrure de lithium-aluminium et du diborane, de préférence avec du borohydrure de lithium, de préférence dans un milieu réactionnel, pour donner au moins un composé de formule générale V, 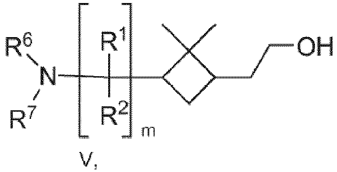 dans laquelle m, R¹, R², R⁶ et R⁷ ont la signification donnée ci-dessus, qui est éventuellement purifié et/ou isolé,
et au moins un composé de formule générale V est mis à réagir avec du chlorure de méthane-sulfonyle, du chlorure de *p*-toluène-sulfonyle, du chlorure de trifluorométhane-sulfonyle, du chlorure de thionyle ou du tétrabromoéthane, de préférence dans un milieu réactionnel, de préférence en présence d'au moins une base, pour donner au moins un composé de formule générale VI, 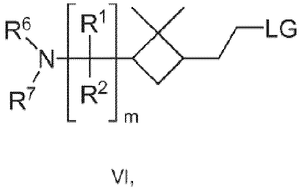 dans laquelle m, R¹, R², R⁶ et R⁷ ont la signification donnée ci-dessus et LG représente -O-S(=O)₂-CH₃, -O-S(=O)₂-p-toluyle, -O-S(=O)₂-CF₃, Cl ou Br, qui est éventuellement purifié et/ou isolé,
et au moins un composé de formule générale VI est mis à réagir avec au moins un composé de formule générale HNR⁹R¹⁰, dans laquelle R⁹ et R¹⁰ ont la signification selon une ou plusieurs des revendications 1 à 9, de préférence dans un milieu réactionnel, de préférence en présence d'au moins une base, pour donner au moins un composé de formule générale VII, 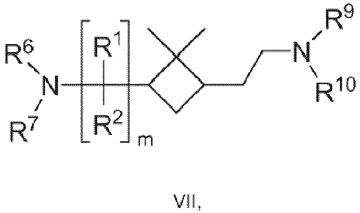 dans laquelle m, R¹, R², R⁶, R⁷, R⁹ et R¹⁰ ont la signification donnée ci-dessus, qui est éventuellement purifié et/ou isolé,
ou au moins un composé de formule générale VI est mis à réagir avec au moins un composé de formule générale M-OR⁸, dans laquelle R⁸ a la signification selon une ou plusieurs des revendications 1 à 9 et M représente un cation monovalent choisi dans le groupe constitué du sodium, du magnésium, du potassium et du lithium, de préférence M représente un cation de lithium, de préférence dans un milieu réactionnel, de préférence en présence d'au moins une base, pour donner au moins un composé de formule générale VIII, 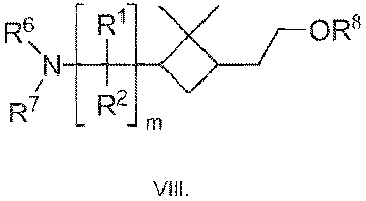 dans laquelle m, R¹, R², R⁶, R⁷ et R⁸ ont la signification donnée ci-dessus, qui est éventuellement purifié et/ou isolé,
et éventuellement au moins un composé de formule générale VIII, dans laquelle R⁸ représente un hydrogène, est mis à réagir avec au moins un composé de formule générale LG-C(=O)-R¹², dans laquelle R¹² a la signification selon une ou plusieurs des revendications 1 à 9 et LG représente un groupe partant, de préférence un groupe partant choisi dans le groupe constitué du chlore et du brome, de préférence dans un milieu réactionnel, de préférence en présence d'au moins une base, ou avec au moins un composé de formule générale HO-C(=O)-R¹², dans laquelle R¹² a la signification selon une ou plusieurs des revendications 1 à 9, de préférence dans un milieu réactionnel, de préférence en présence d'au moins une base, de préférence en présence d'au moins un agent de couplage,
pour donner au moins un composé de formule générale VIIIa, 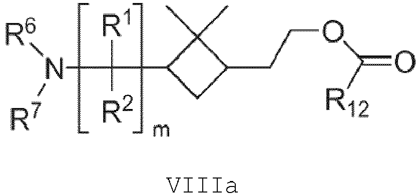 dans laquelle m, R¹, R², R⁶, R⁷ et R¹² ont la signification donnée ci-dessus, qui est éventuellement purifié et/ou isolé.

11. Procédé de préparation d'un composé de formule générale I selon une ou plusieurs des revendications 1 à 9, **caractérisé en ce qu'**au moins un composé de formule générale II, 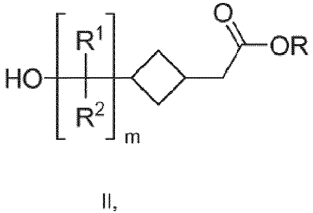 dans laquelle m, R¹ et R² ont la signification selon une ou plusieurs des revendications 1 à 9 et R représente un radical alkyle en C₁-C₅ linéaire ou ramifié, est mis à réagir avec au moins un composé de formule générale R⁵-LG, dans laquelle R⁵ a la signification selon une ou plusieurs des revendications 1 à 9 et LG représente un groupe partant, de préférence LG représente un groupe partant choisi dans le groupe constitué du chlore, du brome, -O-S(=O)₂-CH₃, -O-S(=O)₂-CF₃ et -O-S(=O)₂-_{P}-toluyle, de manière davantage préférée LG représente le brome, de préférence dans un milieu réactionnel, de préférence en présence d'au moins une base, de manière davantage préférée en présence d'au moins une base choisie dans le groupe constitué du butyl-lithium, de l'éthoxyde de sodium, du tert-butoxyde de potassium, de l'hydrure de sodium et de l'hydrure de potassium, pour donner au moins un composé de formule générale IX, 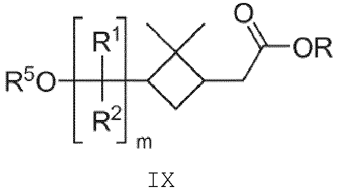 dans laquelle m, R¹, R² et R⁵ ont la signification donnée ci-dessus et R représente un radical alkyle en C₁-C₅ linéaire ou ramifié, qui est éventuellement purifié et/ou isolé,
et au moins un composé de formule générale IX est mis à réagir avec au moins un agent réducteur choisi dans le groupe constitué du borohydrure de lithium, du borohydrure de sodium, de l'hydrure de lithium-aluminium et du diborane, de préférence avec du borohydrure de lithium, de préférence dans un milieu réactionnel, pour donner au moins un composé de formule générale X, 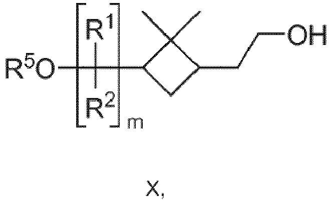 dans laquelle m, R¹, R² et R⁵ ont la signification donnée ci-dessus, qui est éventuellement purifié et/ou isolé, et éventuellement au moins un composé de formule générale X est mis à réagir avec au moins un composé de formule générale LG-C(=O)-R¹², dans laquelle R¹² a la signification selon une ou plusieurs des revendications 1 à 9 et LG représente un groupe partant, de préférence un groupe partant choisi dans le groupe constitué du chlore et du brome, de préférence dans un milieu réactionnel, de préférence en présence d'au moins une base,
ou avec au moins un composé de formule générale HO-C(=O)-R¹², dans laquelle R¹² a la signification selon une ou plusieurs des revendications 1 à 9, de préférence dans un milieu réactionnel, de préférence en présence d'au moins une base, de préférence en présence d'au moins un agent de couplage,
pour donner au moins un composé de formule générale Xa, 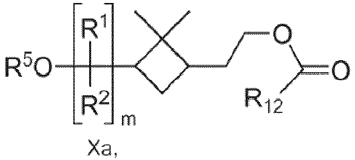 dans laquelle m, R¹, R² et R⁵ ont la signification donnée ci-dessus, qui est éventuellement purifié et/ou isolé, et au moins un composé de formule générale X est mis à réagir avec du chlorure de méthane-sulfonyle, du chlorure de *p*-toluène-sulfonyle, du chlorure de trifluorométhane-sulfonyle, du chlorure de thionyle ou du tétrabromoéthane, de préférence dans un milieu réactionnel, de préférence en présence d'au moins une base, pour donner au moins un composé de formule générale XI, 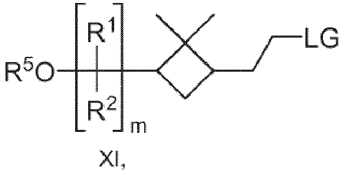 dans laquelle m, R¹, R² et R⁵ ont la signification donnée ci-dessus et LG représente -O-S(=O)₂-CH₃, -O-S(=O)₂-p-toluyle, -O-S(=O)₂-CF₃, Cl ou Br, qui est éventuellement purifié et/ou isolé,
et au moins un composé de formule générale XI est mis à réagir avec au moins un composé de formule générale HNR⁹R¹⁰, dans laquelle R⁹ et R¹⁰ ont la signification selon une ou plusieurs des revendications 1 à 9, de préférence dans un milieu réactionnel, de préférence en présence d'au moins une base, pour donner au moins un composé de formule générale XII, 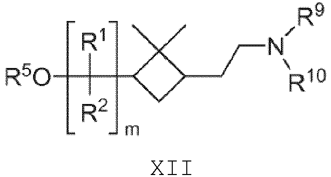 dans laquelle m, R¹, R², R⁵, R⁹ et R¹⁰ ont la signification donnée ci-dessus, qui est éventuellement purifié et/ou isolé.

12. Procédé de préparation d'un composé de formule générale I selon une ou plusieurs des revendications 1 à 9, **caractérisé en ce qu'**au moins un composé de formule générale XIII, 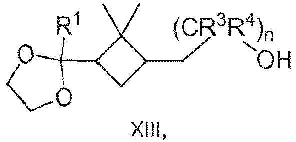 dans laquelle R¹, R³ et R⁴ ont la signification selon une ou plusieurs des revendications 1 à 9, est mis à réagir avec du chlorure de méthane-sulfonyle, du chlorure de *p-*toluène-sulfonyle, du chlorure de trifluorométhane-sulfonyle, du chlorure de thionyle ou du tétrabromoéthane, de préférence dans un milieu réactionnel, de préférence en présence d'au moins une base, pour donner au moins un composé de formule générale XIV, 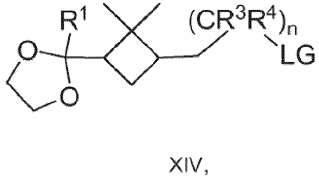 dans laquelle R¹, R³ et R⁴ ont la signification donnée ci-dessus et LG représente -O-S(=O)₂-CH₃, -O-S(=O)₂-p-toluyle, -O-S(=O)₂-CF₃, Cl ou Br, qui est éventuellement purifié et/ou isolé,
et au moins un composé de formule générale XIV est mis à réagir avec au moins un composé de formule générale HNR⁹R¹⁰ dans laquelle R⁹ et R¹⁰ ont la signification selon une ou plusieurs des revendications 1 à 9, de préférence dans un milieu réactionnel, de préférence en présence d'au moins une base, pour donner au moins un composé de formule générale XV, 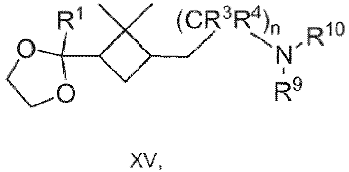 dans laquelle R¹, R³, R⁴, R⁹ et R¹⁰ ont la signification donnée ci-dessus, qui est éventuellement purifié et/ou isolé,
et au moins un composé de formule générale XV est mis à réagir avec au moins un réactif choisi dans le groupe constitué de l'acide chlorhydrique, du p-toluènesulfonate de pyridinium, de l'acide sulfonique et de l'acide trifluoroacétique, de préférence avec du p-toluènesulfonate de pyridinium, de préférence dans un milieu réactionnel, pour donner au moins un composé de formule générale XVI, 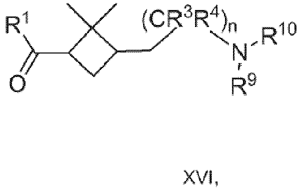 dans laquelle R¹, R³, R⁴, R⁹ et R¹⁰ ont la signification donnée ci-dessus, qui est éventuellement purifié et/ou isolé,
et au moins un composé de formule générale XVI est mis à réagir avec au moins un composé de formule générale R²-Li, dans laquelle R² a la signification selon une ou plusieurs des revendications 1 à 9, ou R²-Mg-Z, dans laquelle R² a la signification selon une ou plusieurs des revendications 1 à 9 et Z représente un anion choisi dans le groupe constitué du brome et du chlore, de préférence dans un milieu réactionnel, pour donner au moins un composé de formule générale XVII, 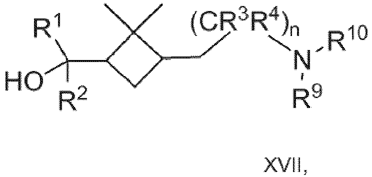 dans laquelle R¹, R², R³, R⁴, R⁹ et R¹⁰ ont la signification donnée ci-dessus, qui est éventuellement purifié et/ou isolé,
et éventuellement au moins un composé de formule générale XVII est mis à réagir avec au moins un composé de formule générale R⁵-LG, dans laquelle R⁵ a la signification selon une ou plusieurs des revendications 1 à 9 et LG représente un groupe partant, de préférence LG représente un groupe partant choisi dans le groupe constitué du chlore et du brome, -O-S(=O)₂-CH₃, -O-S(=O)₂-CF₃ et -O-S(=O)₂-p-toluyle, de manière davantage préférée LG représente le brome, de préférence dans un milieu réactionnel, de préférence en présence d'au moins une base, de manière davantage préférée en présence d'au moins une base choisie dans le groupe constitué du butyl-lithium, de l'éthoxyde de sodium, du tert-butoxyde de potassium, de l'hydrure de sodium et de l'hydrure de potassium, pour donner au moins un composé de formule générale XVIII, 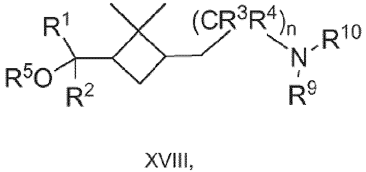 dans laquelle R¹, R², R³, R⁴, R⁵, R⁹ et R¹⁰ ont la signification donnée ci-dessus, qui est éventuellement purifié et/ou isolé.

13. Procédé de préparation d'un composé de formule générale I selon une ou plusieurs des revendications 1 à 9, **caractérisé en ce qu'**au moins un composé de formule générale IXX, 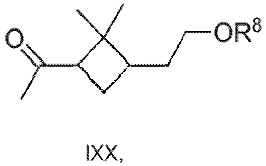 dans laquelle R⁸ a la signification selon une ou plusieurs des revendications 1 à 9, est mis à réagir avec NaOBr, de préférence dans un milieu réactionnel, de manière davantage préférée dans un milieu réactionnel choisi dans le groupe constitué du dioxane, du THF et de l'eau ou d'un mélange de ceux-ci, pour donner au moins un composé de formule générale XX, 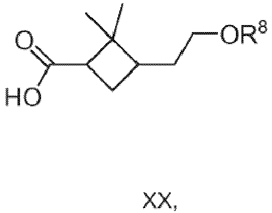 dans laquelle R⁸ a la signification selon une ou plusieurs des revendications 1 à 9, qui est éventuellement purifié et/ou isolé,
et au moins un composé de formule générale XX est mis à réagir avec de l'iodure de méthyle, de préférence dans un milieu réactionnel, de préférence en présence d'au moins une base, de manière davantage préférée en présence de carbonate de caesium, pour donner au moins un composé de formule générale XXI, 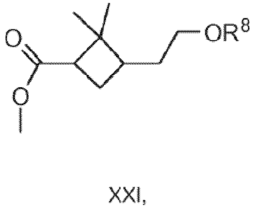 dans laquelle R⁸ a la signification selon une ou plusieurs des revendications 1 à 9, qui est éventuellement purifié et/ou isolé,
et au moins un composé de formule générale XXI est mis à réagir avec au moins un agent réducteur choisi dans le groupe constitué du borohydrure de lithium, du borohydrure de sodium, de l'hydrure de lithium-aluminium et du diborane, de préférence avec du borohydrure de lithium, de préférence dans un milieu réactionnel, pour donner au moins un composé de formule générale XXII, 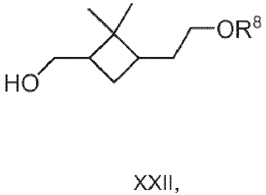 dans laquelle R⁸ a la signification selon une ou plusieurs des revendications 1 à 9, qui est éventuellement purifié et/ou isolé.

14. Médicament comprenant au moins un composé de formule générale I selon une ou plusieurs des revendications 1 à 9 et éventuellement au moins un agent auxiliaire acceptable sur le plan physiologique.

15. Médicament selon la revendication 14, pour une utilisation dans la prophylaxie et/ou le traitement de la douleur, de préférence d'une douleur neuropathique, de l'allodynie, de l'analgésie, de la causalgie, d'une douleur centrale, de la dysesthésie, de l'hyperesthésie, de l'hyperalgésie, de l'hypoalgésie, de l'hypoesthésie ou de la névralgie, de manière davantage préférée d'une douleur neurophatique, de l'hyperalgésie ou de l'allodynie.

16. Composé diméthylcyclobutyle substitué de formule générale I 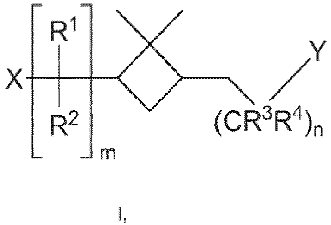 dans laquelle m, n, X, Y, R¹, R², R³ et R⁴ sont tels que définis dans la revendication 1,
éventuellement sous la forme d'un de ses stéréoisomères, de préférence des énantiomères ou des diastéréoisomères, un racémique ou sous la forme d'un mélange d'au moins deux de ses stéréoisomères, de préférence des énantiomères et/ou des diastéréoisomères, dans un quelconque rapport de mélange, ou un sel de celui-ci, ou un solvate correspondant de celui-ci,
pour une utilisation dans la prophylaxie et/ou le traitement de la douleur, de préférence d'une douleur neuropathique, de l'allodynie, de l'analgésie, de la causalgie, d'une douleur centrale, de la dysesthésie, de l'hyperesthésie, de l'hyperalgésie, de l'hypoalgésie, de l'hypoesthésie ou de la névralgie, de manière davantage préférée d'une douleur neurophatique, de l'hyperalgésie ou de l'allodynie.
